# EUROPEAN PATENT SPECIFICATION

(11) **EP 1 737 463 B1**
(45) Date of publication and mention of the grant of the patent: **03.12.2008**
(21) Application number: 04806139.4
(22) Date of filing: 20.12.2004
(51) Int. Cl.: A61K 31/505, C07D 239/42, A61P 35/00, C07D 405/12, C07D 417/12, C07D 413/12, C07D 401/12, C07D 413/14, C07D 401/06, C07D 417/06, C07D 403/12

(54) **PYRIMIDINES WITH TIE2 (TEK) ACTIVITY**
PYRIMIDINE MIT TIE2 (TEK) AKTIVITÄT
PYRIMIDINES A ACTIVITE TIE2 (TEK)

(30) Priority: 24.12.2003 GB 0330000; 29.07.2004 GB 0416849
(43) Date of publication of application: 03.01.2007
(73) Proprietor: AstraZeneca AB, 151 85 Södertälje (SE)
(72) Inventor: JONES, Clifford David, AstraZeneca R & D Alderley, Macclesfield, Cheshire SK10 4TG (GB); LUKE, Richard William Arthur, AstraZeneca R & D, Macclesfield, Cheshire SK10 4TG (GB); MCCOULL, William, AstraZeneca R & D Alderley, Macclesfield, Cheshire SK10 4TG (GB)
(86) International application number: PCT/GB2004/005337
(87) International publication number: WO 2005/060970

(56) References cited:
- WO-A-02/08205
- WO-A-03/029209
- WO-A-03/080625

## Description

This invention relates to compounds, or pharmaceutically acceptable salts thereof, which possess anti-angiogenic activity and are accordingly useful in methods of treatment of disease states associated with angiogenesis in the animal or human body. The invention also concerns processes for the preparation of the compounds, pharmaceutical compositions containing the compounds as active ingredient, and methods for the use of the compounds in the manufacture of medicaments for use in the production of anti-angiogenic effects in warm-blooded animals such as humans.

The Tie2 receptor tyrosine kinase (also known as TEK) is expressed predominantly in endothelial and haematopoietic cells and is essential for vessel formation and maintenance (Jones, N. et al. Nature Reviews Molecular Cell Biology. 2001: 2, 257-67).

Angiogenesis is a fundamental process defined as the generation of new blood vessels from existing vasculature. It is a vital yet complex biological process required for the formation and physiological functions of virtually all the organs. Normally it is transient in nature and is controlled by the local balance of angiogenic and angiostatic factors in a multistep process involving vessel sprouting, branching and tubule formation by endothelial cells (involving processes such as activation of endothelial cells (ECs), vessel destabilisation, synthesis and release of degradative enzymes, EC migration, EC proliferation, EC organisation and differentiation and vessel maturation).

Normal angiogenesis plays an important role in a variety of processes and is under stringent control. In the adult, physiological angiogenesis is largely confined to wound healing and several components of female reproductive function and embryonic development. In undesirable or pathological angiogenesis, the local balance between angiogenic and angiostatic factors is dysregulated leading to inappropriate and/or structurally abnormal blood vessel formation. Pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacology. Science. 16: 57-66; Folkman, 1995, Nature Medicine 1: 27-31). In cancer, growth of primary and secondary tumours beyond 1-2 mm³ requires angiogenesis (Folkman, J. New England Journal of Medicine 1995; 33, 1757-1763).

In diseases such as cancer in which progression is dependant on aberrant angiogenesis, blocking the process can lead to prevention of disease advancement (Folkman, J. 1995, Nature Medicine. 1: 27-31). Many factors are described in the scientific literature that are believed to play important critical roles in the regulation of angiogenesis. Two major classes of angiogenic factors are the vascular endothelial growth factor (VEGF) and the angiopoietins. These polypeptide moieties interact with their respective receptors (transmembrane tyrosine kinases which are predominantly endothelial cell specific) and induce cellular responses via ligand mediated signal transduction. It has been speculated that VEGF and the angiopoietins co-operate to regulate various aspects of the angiogenic process during both normal and pathological angiogenesis via signalling through their respective receptors.

Receptor tyrosine kinases (RTKs) are important in the transmission of biochemical signals across the plasma membrane of cells. These transmembrane molecules characteristically consist of an extracellular ligand-binding domain connected through a segment in the plasma membrane to an intracellular tyrosine kinase domain. Binding of ligand to the receptor results in stimulation of the receptor-associated tyrosine kinase activity that leads to phosphorylation of tyrosine residues on both the receptor and other intracellular molecules. These changes in tyrosine phosphorylation initiate a signalling cascade leading to a variety of cellular responses. To date, at least nineteen distinct RTK subfamilies, defined by amino acid sequence homology, have been identified. One of these subfamilies is presently comprised by the fms-like tyrosine kinase receptor, Flt or Flt1, the kinase insert domain-containing receptor, KDR (also referred to as Flk-1), and another fms-like tyrosine kinase receptor, Flt4. Two of these related RTKs, Flt and KDR, have been shown to bind VEGF with high affinity (De Vries et al, 1992, Science 255: 989-991; Terman et al, 1992, Biochem. Biophys. Res. Comm. 1992, 187: 1579-1586). Binding of VEGF to these receptors expressed in heterologous cells has been associated with changes in the tyrosine phosphorylation status of cellular proteins and calcium fluxes.

Recently a second family of predominantly endothelial cell specific receptors that regulate vessel destabilisation and maturation have been identified. The Tie receptors and their ligands, the angiopoietins, co-operate closely with VEGF during both normal and pathological angiogenesis. The transmembrane receptors Tie 1 and Tie2, constitute a family of endothelial cell specific tyrosine kinase receptors involved in maintenance of blood vessel integrity and which are involved in angiogenic outgrowth and vessel remodelling. Structurally Tie1 and Tie2 share a number of features (e.g. the intracellular domains of both these receptors each contain a tyrosine kinase domain interrupted by a kinase insert region) and thus constitute a distinct RTK subfamily. Overall sequence identity between Tie1 and Tie2 receptors at the amino acid level is 44% while their intracellular domains exhibit 76% homology. Targeted disruption of the Tie1 gene results in a lethal phenotype characterised by extensive haemorrhage and poor microvessel integrity (Puri, M. et al. 1995 EMBO Journal: 14:5884-5891). Transgenic mice deficient in Tie2 display defects in vessel sprouting and remodelling and display a lethal phenotype in mid gestation (E9.5-10.5) caused by severe defects in embryonic vasculature (Sato, T. et al. 1995 Nature 370: 70-74).

To date no ligands have been identified for Tie1 and little is known regarding its signalling abilities. However, Tie1 is believed to influence Tie2 signalling via heterodimerisation with the Tie2 receptor, hence potentially modulating the ability of Tie2 to autophosphorylate (Marron, M. et al. 2000 Journal of Biological Chemistry: 275, 39741-39746) and recent chimaeric Tie1 receptor studies have indicated that Tie-1 may inhibit apoptosis via the PI 3 kinase/Akt signal transduction pathway (Kontos, C.D., et al., 2002 Molecular and Cellular Biology: 22, 1704-1713). In contrast, a number of ligands, designated the angiopoietins have been identified for Tie2 of which Angiopoietin 1 (Ang1) is the best characterised. Binding of Ang1 induces tyrosine phosphorylation of the Tie2 receptor via autophosphorylation and subsequently activation of its signalling pathways via signal transduction. Ang2 has been reported to antagonise these effects in endothelial cells (Maisonpierre, P. et al. 1997 Science: 277, 55-60). The knock-out and transgenic manipulation of Tie2 and its ligands suggest that stringent spatial and temporal control of Tie2 signalling is imperative for the correct development of new vasculature. There are also reports of at least another two ligands (Ang3 and Ang4) as well as the possibility of heterodimerisation between the angiopoietin ligands that has the potential to modify their activity (agonistic/antagonistic) on association with the receptor. Activation of the Tie2 receptor by Ang1 inhibits apoptosis (Papapetropoulos, A., et al., 2000 Journal of Biological Chemistry: 275 9102-9105), promotes sprouting in vascular endothelial cells (Witzenbicher, B., et al., 1998 Journal of Biological Chemistry: 273,18514-18521) and *in vivo* promotes blood vessel maturation during angiogenesis and reduces the permeability and consequent leakage from adult microvessels (Thurston, G. et al., 2000 Nature Medicine: 6, 460-463). Thus activated Tie2 receptor is reported to be involved in the branching, sprouting and outgrowth of new vessels and recruitment and interaction of periendothelial support cells important in maintaining vessel integrity and overall appears to be consistent with promoting microvessel stability. Absence of Tie2 activation or inhibition of Tie2 auto phosphorylation may lead to a loss of vascular structure and matrix/cell contacts (Thurston, G., Cell Tissue Res (2003), 314: 61-69) and in turn may trigger endothelial cell death, especially in the absence of survival or growth stimuli. On the basis of the above reported effects due to Tie2 kinase activity, inhibiting Tie2 kinase may provide an anti-angiogenic effect and thus have application in the therapy of disease states associated with pathological angiogenesis. Tie2 expression has been shown to be up-regulated in the neovasculature of a variety of tumours (e.g. Peters, K.G. et al, (British Journal of Cancer, 1998; 77,51-56) suggesting that inhibiting Tie2 kinase activity will result in anti-angiogenic activity. In support of this hypothesis, studies with soluble Tie2 receptor (extracellular domain) (Pengnian, L. et al., 1997, Journal of Clinical Investigation 1997: 100, 2072-2078 and Pengnian, L. et al., 1998, Proceedings of the National Academy of Sciences 1998: 95, 8829-8834) have shown anti-tumour activity in *in vivo* tumour models. In addition these experiments also indicate that disruption of the Tie2 signalling pathways in a normal healthy individual may be well tolerated as no adverse toxicities were observed in these studies.

Examination of human primary breast cancer samples and human and murine breast cancer cell lines (Stratmann, A., et al., 2001, International Journal of Cancer: 91, 273-282) indicate that Tie2 dependant pathways of tumour angiogenesis may exist alongside KDR dependant pathways and, in fact, may operate both independently (Siemeister G., et al., 1999 Cancer Research: 59, 3185-3191) as well as in concert with each other (e.g. VEGF A and Ang1 reported to collaborate to induce angiogenesis and produce non-leaky mature vessels Thurston, G, et al., 1999 Science: 286, 2511-2514). It is quite possible that a mix of such angiogenic processes even exist within a single tumour.

Tie2 has also been shown to play a role in the vascular abnormality called venous malformation (VM) (Mulliken, J.B. & Young, A.E. 1998, Vascular Birthmarks: W. B. Sanders, Philadelphia). Such defects can either be inherited or can arise sporadically. VM's are commonly found in the skin or mucosal membranes but can affect any organ. Typically lesions appear as spongy, blue to purple vascular masses composed of numerous dilated vascular channels lined by endothelial cells. Among the inherited forms of this disease the most common defect appears to be a Tie2 kinase mutation C2545T in the Tie2 coding sequence (Calvert, J.T., et al., 1999 Human Molecular genetics: 8, 1279-1289), which produces a R849W amino acid substitution in the kinase domain. Analysis of this Tie2 mutant indicates that it is constitutively activated even in the absence of ligand (Vikkula, M., et al., 1996 Cell: 87, 1181-1190).

Upregulation of Tie2 expression has also been found within the vascular synovial pannus of arthritic joints in humans, which is consistent with the role of inappropriate neovascularisation.

Such examples provide further indications that inhibition of Tie2 phosphorylation and subsequent signal transduction will be useful in treating disorders and other occurrences of inappropriate neovascularisation. To date only a few inhibitors of Tie2 are known in the art For example, Internation Application No: WO 04/013141 describes a group of condensed pyridines and pyrimidines and International Application No: WO 04/058776 describes a group of pryidine and pyrimidine compounds. There is thus a need to identify additional Tie2 inhibitors that could exploit the full therapeutic potential of inhibiting/ modulating the Tie2 signalling pathways.

WO 03/029209 and WO 03/080625 describe groups of compounds having Tie2 inhibitory activity with a five-membered ring fused to a pyrimidinyl ring.

We have found that certain compounds possess inhibitory activity for the Tie2 receptor tyrosine kinase and accordingly have value in the treatment of disease states associated with pathological angiogenesis such as cancer, rheumatoid arthritis, and other diseases where active angiogenesis is undesirable.

According to a first aspect of the present invention there is provided a compound of the Formula I: 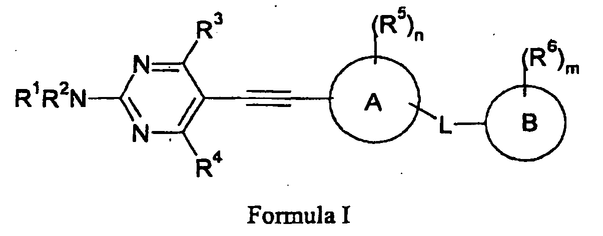 wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0,1, 2, 3, 4, 5 or 6, or a 5 or 6 membered heteroaryl ring, or **R¹ and R²** together with the nitrogen atom to which they are attached represent a saturated or partially saturated 3 to 7 membered heterocyclic ring optionally containing another hetero atom selected from N or O; wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino or di(1-6C)alkylamino, carbamoyl, mono(1-6C)alkylcarbamoyl, di-[(1-6C)alkyl]carbamoyl, -N(R^{d})C(O)(1-6C)alkyl in which R^{d} is hydrogen or (1-6C)alkyl, a saturated or partially saturated 3 to 7 membered heterocyclic ring or a 5 or 6 membered heteroaryl ring;
   wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or-N(R^{d})C(O)(1-6C)alkyl groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di(1-6C)alkylamino, wherein the (1-6C)alkyl and the (1-6C)alkoxy groups are optionally substituted by one or more groups independently selected from hydroxy, amino, mono(1-6C)alkylamino or di-(1-6C)alkylamino;
   and wherein any heterocyclic and heteroaryl rings within R¹ and/or R² are optionally independently substituted by one or more of the following: (1-4C)alkyl, (1-4C)alkoxy, (1-4C)alkoxy(1-4C)alkyl, hydroxy, amino, mono(1-6C)alkylamino, di-[(1-6C)alkyl]amino, a saturated or partially saturated 3 to 7 membered heterocyclic ring or -C(O)(CH_{2)z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
**R³ and R⁴** are independently selected from hydrogen, (1-6C)alkyl or (1-6C)alkoxy wherein the (1-6C)alkyl and the (1-6C)alkoxy groups are optionally substituted by one or more groups independently selected from: fluoro, hydroxy, (1-6C)alkyl, (1-6C)alkoxy, amino, mono(1-6C)alkylamino or di(1-6C)alkylamino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring wherein said heterocyclic and heteroaryl rings are optionally independently substituted by one or more of the following: (1-4C)alkyl, (1-4C)alkoxy, hydroxy, amino, mono(1-6C)alkylamino or di(1-6C)alkylamino or a saturated or partially saturated 3 to 7 membered heterocyclic ring;
   or one of **R³** and **R⁴** is as defined above and the other represents a group **-NR¹R²** as defined above.
**A** represents an aryl or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyclopropyl, cyano, halo, (1-6C)alkoxy or (1-6C)alkyl wherein the (1-6C)allcyl and the (1-6C)alkoxy groups are optionally substituted by cyano or by one or more fluoro;
n is 0,1, 2 or 3;
**L** is attached meta or para on ring A with respect to the point of attachment of the ethynyl group and represents -C(R^{a}R^{b})C(O)N(R⁹)-, -N(R⁸)C(O)C(R^{a}R^{b})-, -N(R⁸)C(O)N(R⁹)-, N(R⁸)C(O)-O-, or -OC(O)-N(R⁹)- wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl ;
**B** represents a (3-7C)cycloalkyl ring, a saturated or partially saturated 3 to 7 membered heterocyclic ring, an aryl or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl; or a 8, 9 or 10 membered bicyclic group which optionally contains 1, 2, 3 or 4 heteroatoms independently selected from N, O and S and which is saturated, partially saturated or aromatic;
**R⁶** is selected from halo, cyano, oxo, a (3-7C)cycloalkyl ring, a saturated or partially saturated 3 to 7 membered heterocyclic ring and -N(R^{c})C(O)(1-6C)alkyl in which R^{c} is hydrogen or (1-6C)alkyl;
   or **R⁶** is selected from (1-6C)alkyl, -S(O)ₚ-(1-6C)alkyl wherein p is 0, 1 or 2, or (1-6C)alkoxy,
   wherein the (1-6C)alkyl, -S(O)ₚ-(1-6C)alkyl and the (1-6C)alkoxy groups are optionally substituted by one or more groups independently selected from: cyano, fluoro, hydroxy, (1-6C)alkoxy, amino, mono(1-6C)alkylamino, di(1-6C)alkylamino, a (3-7C)cycloalkyl ring or a saturated or partially saturated 3 to 7 membered heterocyclic ring; and
   wherein the (3-7C)cycloalkyl ring and saturated or partially saturated 3 to 7 membered heterocyclic ring are optionally independently substituted by one or more groups selected from (1-6C)alkyl; and
m **is 0, 1, 2 or 3;**
and when B is a (3-7C)cycloalkyl ring, a saturated or partially saturated 3 to 7 membered heterocyclic ring or a saturated or partially saturated 8, 9 or 10 membered bicyclic group, the rings and the bicyclic group optionally bear 1 or 2 oxo or thioxo substituents;
   and salts thereof, particularily pharmaceutically acceptable salts thereof.

According to another aspect of the present invention there is provided a compound of the Formula I wherein:
**R¹ and R²** are independently selected from hydrogen, (1-6C)alkylsulphonyl, phenyl (CH₂)ᵤ- whereine u is 0, 1, 2, 3, 4 , 5 or 6, (1-6C)alkanoyl, (1-6C)alkyl, (1-6C)alkoxycarbonyl, or (3-6C)cycloalkyl(CH₂)ₓ- in which x is 0, 1, 2, 3, 4 , 5 or 6 or a 5 or 6 membered heteroaryl ring, or **R¹ and R²** together with the nitrogen atom to which they are attached represent a saturated or partially saturated 3 to 7 membered heterocyclic ring optionally containing another hetero atom selected from N or O;
   wherein the (1-6C)alkyl, the (1-6C)alkanoyl and the (3-6C)cycloalkyl groups are optionally substituted by one or more groups independently selected from: fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino or di(1-6C)alkylamino, carbamoyl, mono(1-6C)alkylcarbamoyl, di-[(1-6C)alkyl]carbamoyl or -N(R^{d})C(O)(1-6C)alkyl in which R^{d} is hydrogen or (1-6C)alkyl, a saturated or partially saturated 3 to 7 membered heterocyclic ring or a 5 or 6 membered heteroaryl ring
   wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or -N(R^{d})C(O)(1-6C)alkyl groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and the (1-6C)alkoxy groups are optionally substituted by one or more groups independently selected from hydroxy, amino, mono(1-6C)alkylamino or di-[(1-6C)alkyl]amino;
   wherein any heterocyclic and heteroaryl rings within R¹ and/or R² are optionally independently substituted by one or more of the following: (1-4C)alkyl, (1-4C)alkoxy, (1-4C)alkoxy(1-4C)alkyl, hydroxy, amino, mono(1-6C)alkylamino, di-[(1-6C)alkyl]amino, a saturated or partially saturated 3 to 7 membered heterocyclic ring or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
**R³ and R⁴** are independently selected from hydrogen, (1-6C)alkyl or (1-6C)alkoxy,
   wherein the (1-6C)alkyl and the (1-6C)alkoxy groups are optionally substituted by one or more groups independently selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
A represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyclopropyl, cyano, halo, (1-6C)alkoxy or (1-6C)alkyl, wherein the (1-6C)alkyl and the (1-6C)alkoxy groups are optionally substituted by cyano or by one or more fluoro;
**n** is 0, 1, 2 or 3;
**L** is attached meta or para on ring A with respect to the point of attachment of the ethynyl group and represents -C(R^{a}R^{b})C(O)N(R⁹)-, -N(R⁸)C(O)C(R^{a}R^{b})-, -N(R⁸)C(O)N(R⁹)-, -N(R⁸)C(O) O-, or -OC(O)-N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
**R⁶** is selected from halo, cyano, oxo, a (3-7C)cycloalkyl ring, a saturated or partially, saturated 3 to 7 membered heterocyclic ring or -N(R^{a})C(O)(1-6C)alkyl in which R^{a} is hydrogen or (1-6C)alkyl; or
   **R⁶** is selected from (1-6C)alkyl, -S(O)ₚ-(1-6C)alkyl wherein p is 0, 1 or 2, or (1-6C)alkoxy, wherein the (1-6C)alkyl and the (1-6C)alkoxy groups are optionally substituted by one or more groups independently selected from: cyano, fluoro, hydroxy, (1-6C)alkoxy, amino, mono(1-6C)alkylamino, di(1-6C)alkylamino, a (3-7C)cycloalkyl ring or a saturated or partially saturated 3 to 7 membered heterocyclic ring;
   wherein the (3-7C)cycloalkyl ring and saturated or partially saturated 3 to 7 membered heterocyclic ring are optionally independently substituted by one or more groups selected from (1-6C)alkyl; and
**m** is 0, 1, 2 or 3;
and when B is a (3-7C)cycloalkyl ring, a saturated or partially saturated 3 to 7 membered heterocyclic ring or a saturated or partially saturated 8, 9 or 10 membered bicyclic group, the rings and the bicyclic group optionally bear 1 or 2 oxo or thioxo substituents;
   and salts thereof, particularily pharmaceutically acceptable salts thereof.

According to a second aspect of the present invention there is provided a compound of the Formula I wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, or (3-6C)cycloalkyl(CH₂)ₓ- in which x is 0, 1, 2, 3, 4, 5 or 6, or **R¹ and R²** together with the nitrogen atom to which they are attached represent a saturated or partially saturated 3 to 7 membered heterocyclic ring optionally containing another hetero atom selected from N or O;
   wherein the alkyl and the cycloalkyl groups are optionally substituted by one or more groups selected from fluoro, hydroxy, (1-6C)alkyl, (1-6C)allcoxy, amino, mono(1-6C)alkylamino or di-[(1-6C)alkyl]amino, a saturated or partially saturated 3 to 7 membered heterocyclic ring or a 5 or 6 membered heteroaryl ring wherein said heterocyclic and heteroaryl rings are optionally independently substituted by one or more of the following: (1-4C)alkyl, hydroxy, amino, mono(1-6C)alkylamino or di-[(1-6C)alkyl]amino or a saturated or partially saturated 3 to 7 membered heterocyclic ring;
   and wherein the phenyl is optionally substituted by one or more groups selected from halo, (1-6C)alkyl, (1-6C)alkoxy, amino, mono(1-6C)alkylamino or di-[(1-6C)alkyl]amino, wherein the (1-6C)alkyl or (1-6C)alkoxy are optionally substituted by hydroxy, amino, mono(1-6C)alkylamino or di-[(1-6C)alkyl]amino;
**R³ and R⁴** are independently selected from hydrogen, (1-6C)alkyl or (1-6C)alkoxy,
   wherein the alkyl and the alkoxy groups are optionally substituted by one or more groups selected from fluoro, hydroxy, (1-6C)alkyl, (1-6C)alkoxy, amino, mono(1-6C)alkylamino or di-[(1-6C)alkyl]amino, a saturated or partially saturated 3 to 7 membered heterocyclic ring or a 5 or 6 membered heteroaryl ring wherein said heterocyclic and heteroaryl rings are optionally independently substituted by one or more of the following: (1-4C)alkyl, hydroxy, amino, mono(1-6C)alkylamino or di-[(1-6C)alkyl]amino or a saturated or partially saturated 3 to 7 membered heterocyclic ring; or one of **R³ and R⁴** is as defined above and the other represents a group **-NR¹R²** as defined above
A represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyano, halo, (1-6C)alkoxy or (1-6C)alkyl optionally substituted by cyano or by one or more fluoro;
**n** is 0, 1, 2 or 3;
L is attached meta or para on ring A with respect to the point of attachment of the ethynyl group and represents -C(R^{a}R^{b})C(O)N(R⁹), -N(R⁸)C(O)C(R^{a}R^{b})- , -N(R⁸)C(O)N(R⁹)-, -N(R⁸)C(O)O-, or -OC(O) N(R⁹)- wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl ;
**B** represents a (3-7C)cycloalkyl ring, an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁶** is selected from halo, cyano, a saturated or partially saturated 3 to 7 membered heterocyclic ring or an alkanoylamino group -N(R^{c})C(O)(1-6C)alkyl in which R^{c} is hydrogen or (1-6C)alkyl; or
   **R⁶** is selected from (1-6C)alkyl or (1-6C)alkoxy, wherein the alkyl and the alkoxy groups are optionally substituted by one or more groups selected from cyano, fluoro, hydroxy, (1-6C)alkoxy, amino, mono(1-6C)alkylamino, di-[(1-6C)alkyl]amino, or a saturated or partially saturated 3 to 7 membered heterocyclic ring; and
**m** is 0, 1, 2 or 3; and when m is at least 2 then two substituents on adjacent carbon atoms in ring B may together represent a methylenedioxy group;
   and salts thereof, particularily pharmaceutically acceptable salts thereof;

According to a further feature of the second aspect of the present invention there is provided a compound of the Formula I wherein:
**R¹ and R²** are independently selected from hydrogen, (1-6C)alkylsulphonyl, phenyl (CH₂)ᵤ- whereine u is 0, 1, 2, 3, 4 , 5 or 6, (1-6C)alkanoyl, (1-6C)allcyl, (1-6C)alkoxycarbonyl, or (3-6C)cycloalkyl(CH₂)ₓ- in which x is 0, 1, 2, 3, 4 , 5 or 6 or **R¹ and R²** together with the nitrogen atom to which they are attached represent a saturated or partially saturated 3 to 7 membered heterocyclic ring optionally containing another hetero atom selected from N or O; wherein the alkyl and the cycloalkyl groups are optionally substituted by one or more groups selected from: fluoro, hydroxy, (1-6C)alkyl, (1-6C)alkoxy, amino, mono(1-6C)alkylamino or di(1-6C)alkylamino, a saturated or partially saturated 3 to 7 membered heterocyclic ring or a 5 or 6 membered heteroaryl ring wherein said heterocyclic and heteroaryl rings are optionally independently substituted by one or more of the following: (1-4C)alkyl, hydroxy, amino, mono(1-6C)alkylamino or di(1-6C)alkylamino or a saturated or partially saturated 3 to 7 membered heterocyclic ring; and the phenyl is optionally substituted by one or more groups selected from: halo, (1-6C)alkyl, (1-6C)alkoxy, amino, mono(1-6C)alkylamino or di(1-6C)alkylamino, wherein the (1-6C)alkyl or (1-6C)alkoxy are optionally substituted by hydroxy, amino, mono(1-6C)alkylamino or di(1-6C)alkylamino;
**R³ and R⁴** are independently selected from hydrogen, (1-6C)alkyl or (1-6C)alkoxy wherein the alkyl and the alkoxy groups are optionally substituted by one or more groups selected from: fluoro, hydroxy, (1-6C)alkyl, (1-6C)alkoxy, amino, mono(1-6C)alkylamino or di(1-6C)alkylamino, a saturated or partially saturated 3 to 7 membered heterocyclic ring or a 5 or 6 membered heteroaryl ring wherein said heterocyclic and heteroaryl rings are optionally independently substituted by one or more of the following: (1-4C)alkyl, hydroxy, amino, mono(1-6C)alkylamino or di(1-6C)alkylamino or a saturated or partially saturated 3 to 7 membered heterocyclic ring;
**A** represents an aryl or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from (1-6C)alkoxy, cyano, halo or (1-6C)alkyl optionally substituted by cyano or by one or more fluoro;
**n** is 0, 1, 2 or 3;
**L** is attached meta or para on ring A with respect to the point of attachment of the ethynyl group and represents -C(R^{a}R^{b}C(O)N(R⁹)-, N(R⁸)C(O)C(R^{a}R^{b})-, -N(R⁸)C(O)N(R⁹)-, -N(R⁸)C(O)O-, or -OC(O)-N(R⁹)- wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl ;
**B** represents a (3-7C)cycloalkyl ring, an aryl or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁶** is selected from halo, cyano, a saturated or partially saturated 3 to 7 membered heterocyclic ring or an alkanoylamino group -N(R^{a})C(O)(1-6C)alkyl in which R^{a} is hydrogen or (1-6C)alkyl; or **R⁶** is selected from (1-6C)alkyl or (1-6C)alkoxy, wherein the alkyl and the alkoxy groups are optionally substituted by one or more groups selected from: cyano, fluoro, hydroxy, (1-6C)alkoxy, amino, mono(1-6C)alkylamino, di(1-6C)alkylamino, or a saturated or partially saturated 3 to 7 membered heterocyclic ring; and
**m** is 0, 1, 2 or 3; and when m is at least 2 then two substituents on adjacent carbon atoms in ring B may together represent a methylenedioxy group;
   and salts thereof, particularily pharmaceutically acceptable salts thereof.

According to a third aspect of the present invention there is provided a compound of the Formula I:
wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6, or a 5 or 6 membered heteroaryl ring, or **R¹ and R²** together with the nitrogen atom to which they are attached represent a saturated or partially saturated 3 to 7 membered heterocyclic ring optionally containing another hetero atom selected from N or O;
   wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or an alkanoylamino group N(R^{d})C(O)(1-6C)alkyl in which R^{d} is hydrogen or (1-6C)alkyl, or a saturated or partially saturated 3 to 7 membered heterocyclic ring, or a 5 or 6 membered heteroaryl ring, wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or alkanoylamino groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and the (1-6C)alkoxy groups are optionally substituted by one or more groups independently selected from hydroxy, amino, mono(1-6C)alkylamino or di-[(1-6C)alkyl]amino;
   and wherein any heterocyclic and heteroaryl rings within R¹ and/or R² are optionally independently substituted by one or more of the following: (1-4C)alkyl, (1-4C)alkoxy, (1-4C)alkoxy(1-4C)alkyl, hydroxy, amino, mono(1-6C)alkylamino, di-[(1-6C)alkyl]amino, a saturated or partially saturated 3 to 7 membered heterocyclic ring or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
**R³ and R⁴** are independently selected from hydrogen, (1-6C)alkyl or (1-6C)alkoxy,
   wherein the (1-6C)alkyl and the (1-6C)alkoxy groups are optionally substituted by one or more groups independently selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
   or one of **R³** and **R⁴** is as defined above and the other represents a group **-NR¹R²** as defined above;
**A** represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyclopropyl, cyano, halo, (1-6C)alkoxy or (1-6C)alkyl, wherein the (1-6C)alkyl and the (1-6C)alkoxy groups are optionally substituted by cyano or by one or more fluoro;
**n** is 0,1,2 or 3;
**L** is attached meta or para on ring A with respect to the point of attachment of the ethynyl group and represents -C(R^{a}R^{b})C(O)N(R⁹), -N(R⁸)C(O)C(R^{a}R^{b})-, -N(R⁸)C(O)N(R⁹)-, N(R⁸)C(O)O-, or -OC(O)-N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
**R⁶** is selected from halo, cyano, a (3-7C)cycloalkyl ring, a saturated or partially saturated 3 to 7 membered heterocyclic ring or an alkanoylamino group -N(R^{c})C(O)(1-6C)alkyl in which R^{c} is hydrogen or (1-6C)alkyl; or
   **R⁶** is selected from (1-6C)alkyl or (1-6C)alkoxy, wherein the (1-6C)alkyl and the (1-6C)alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring; and
**m** is 0, 1, 2 or 3;
and when B is a (3-7C)cycloalkyl ring, a saturated or partially saturated 3 to 7 membered heterocyclic ring or a saturated or partially saturated 8, 9 or 10 membered bicyclic group, the rings and the bicyclic group optionally bear 1 or 2 oxo or thioxo substituents;
   and salts thereof, particularily pharmaceutically acceptable salts thereof.

According to another feature of the third aspect of the invention of the present invention there is provided a compound of the Formula I wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6, or a 5 or 6 membered heteroaryl ring, or **R¹ and R²** together with the nitrogen atom to which they are attached represent a saturated or partially saturated 3 to 7 membered heterocyclic ring optionally containing another hetero atom selected from N or O;
   wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or an alkanoylamino group -N(R^{d})C(O)(1-6C)alkyl in which R^{d} is hydrogen or (1-6C)alkyl, or a saturated or partially saturated 3 to 7 membered heterocyclic ring, or a 5 or 6 membered heteroaryl ring, wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or alkanoylamino groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and the (1-6C)alkoxy groups are optionally substituted by one or more groups independently selected from hydroxy, amino, mono(1-6C)alkylamino or di-[(1-6C)alkyl]amino;
   and wherein any heterocyclic and heteroaryl rings within R¹ and/or R² are optionally independently substituted by one or more of the following: (1-4C)alkyl, (1-4C)alkoxy, (1-4C)alkoxy(1-4C)alkyl, hydroxy, amino, mono(1-6C)alkylamino, di-[(1-6C)alkyl]amino, a saturated or partially saturated 3 to 7 membered heterocyclic ring or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7-membered heterocyclic ring;
   and provided that when R¹ and/or R² is a (1C)alkanoyl group, then the (1C)alkanoyl is not substituted by fluoro or hydroxy;
**R³ and R⁴** are independently selected from hydrogen, (1-6C)alkyl or (1-6C)alkoxy,
   wherein the (1-6C)alkyl and the (1-6C)alkoxy groups are optionally substituted by one or more groups independently selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
**A** represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyclopropyl, cyano, halo, (1-6C)alkoxy or (1-6C)alkyl, wherein the (1-6C)alkyl and the (1-6C)alkoxy groups are optionally substituted by cyano or by one or more fluoro;
**n** is 0, 1, 2 or 3;
**L** is attached meta or para on ring A with respect to the point of attachment of the ethynyl group and represents -C(R^{a}R^{b}C(O)N(R⁹)-, -N(R⁸)C(O)C(R^{a}R^{b})-, -N(R⁸)C(O)N(R⁹)-, -N(R⁸)C(O)O-, or -OC(O)-NR⁹, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
**R⁶** is selected from halo, cyano, a (3-7C)cycloalkyl ring, a saturated or partially saturated 3 to 7 membered heterocyclic ring or an alkanoylamino group -N(R^{c})C(O)(1-6C)alkyl in which R^{c} is hydrogen or (1-6C)alkyl; or
   **R⁶** is selected from (1-6C)alkyl or (1-6C)alkoxy, wherein the (1-6C)alkyl and the (1-6C)alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring; and
**m** is 0, 1,2 or 3;
and when **B** is a (3-7C)cycloalkyl ring, a saturated or partially saturated 3 to 7 membered heterocyclic ring or a saturated or partially saturated 8, 9 or 10 membered bicyclic group, the rings and the bicyclic group optionally bear 1 or 2 oxo or thioxo substituents;
   and salts thereof, particularily pharmaceutically acceptable salts thereof. In this specification the generic term "alkyl" includes both straight-chain and branched-chain alkyl groups such as propyl, isopropyl and tert-butyl. However references to individual alkyl groups such as "propyl" are specific for the straight-chain version only, references to individual branched-chain alkyl groups such as "isopropyl" are specific for the branched-chain version only. An analogous convention applies to other generic terms, for example (1-6C)alkoxy includes methoxy, ethoxy and isopropoxy, (1-6C)alkylamino includes methylamino, isopropyl amino and ethylamino, and di-[(1-6Calkyl]amino includes dimethylamino, diethylamino and N-methyl-N-isoproylamino. The generic term aryl refers to phenyl or naphthyl, particularily phenyl.

It is to be understood that, insofar as certain of the compounds of Formula I defined above may exist in optically active or racemic forms by virtue of one or more asymmetric carbon atoms, the invention includes in its definition any such optically active or racemic form which possesses the above-mentioned activity. The synthesis of optically active forms may be carried out by standard techniques of organic chemistry well known in the art, for example by synthesis from optically active starting materials or by resolution of a racemic form. Similarly, the above-mentioned activity may be evaluated using the standard laboratory techniques referred to hereinafter.
Suitable values for the generic radicals referred to above include those set out below.

Suitable 5 or 6 membered heteroaryl rings include, for example furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, 1,4,5-triazinyl or pyrazinyl. Particular 5 or 6 membered heteroaryl rings include imidazolyl, pyridyl, thiazolyl, thiadiazolyl, pyrimidinyl, isoxazolyl, isothiazolyl and pyrazolyl.
Suitable saturated or partially saturated 3 to 7 membered heterocyclic rings include, for example oxiranyl, oxetanyl, tetrahydrofuranyl, tetrahydropyranyl, 2,3-dihydro-1,3-thiazolyl, 1,3-thiazolidinyl, 1,3-oxazolidinyl, oxepanyl, pyrrolinyl, pyrrolidinyl, morpholinyl, thiamorpholinyl (perhydro-1,4-thiazinyl), (8-oxa-3-azabicyclo[3.2.1]octyl), (7-oxa-3-azabicyclo[3.1.1]heptyl), perhydroazepinyl, perhydrooxazepinyl, tetrahydro-1,4-thiazinyl, 1-oxotetrahydrothienyl, 1,1-dioxotetrahydro-1,4-thiazinyl, piperidinyl, homopiperidinyl, piperazinyl, homopiperazinyl, dihydropyridinyl, tetrahydropyridinyl, dihydropyrimidinyl or tetrahydropyrimidinyl, preferably tetrahydrofuranyl, tetrahydropyranyl, pyrrolidinyl, morpholinyl, 1,1-dioxotetrahydro-4H-1,4-thiazinyl, piperidinyl or piperazinyl, more preferably tetrahydrofuran-3-yl, tetrahydropyran-4-yl, pyrrolidin-3-yl, morpholino, 1,1-dioxotetrahydro-4H-1,4-thiazin-4-yl, piperidino, piperidin-4-yl or piperazin-1-yl. A suitable value for such a group which bears 1 or 2 oxo or thioxo substituents is, for example, 2-oxopyrrolidinyl, 2-thioxopyrrolidinyl, 2-oxoimidazolidinyl, 2-thioxoimidazolidinyl, 2-oxopiperidinyl, 2,5-dioxopyrrolidinyl, 2,5-dioxoimidazolidinyl or 2,6-dioxopiperidinyl. The saturated or partially saturated 3 to 7 membered heterocyclic rings are optionally substituted by one or more (C1-6) alkyl groups and/or by one or more hydroxy.

Suitable 8, 9 or 10 membered bicyclic groups include thieno[2,3-b]furanyl, imidazolo[2,1-b]thiazolyl, dihydrocyclopentathiazolyl, tetrahydrocyclopenta[c]pyrazolyl, furo[3,2-b]furanyl, pyrrolopyrrole, thienopyrazolyl, thieno[2,3-b]thiophenyl, indolizinyl, indolyl, isoindolyl, 3H-indolyl, indolin-yl, benzo[b]furanyl, benzo[b]thiophenyl, IH-indazolyl, benzimidazolyl, benzthiazolyl, purinyl, 4H-quinolizinyl, quinolinyl, isoquinolinyl, cinnolinyl, phthalazinyl, quinazolinyl, quinoxalinyl, 1,8-naphthyridinyl, pteridinyl, chromanyl, isochromanyl, indenyl, naphthalenyl, 2,3-dihydro-1,4-benzodioxinyl, 1,3-benzodioxol-5-yl, decalin and norbornane. Particular 8, 9 or 10 membered bicyclic groups include thieno[2,3-b]furanyl, indolizinyl, indolyl, isoindolyl, 3H-indolyl, indolin-yl, benzo[b]furanyl, benzo[b]thiophenyl, IH-indazolyl, benzimidazolyl, benzthiazolyl, purinyl, 4H-quinolizinyl, quinolinyl, isoquinolinyl, cinnolinyl, phthalazinyl, quinazolinyl, quinoxalinyl, 1,8-naphthyridinyl, pteridinyl, chromanyl, isochromanyl, indenyl, naphthalenyl, 2,3-dihydro-1,4-benzodioxinyl and 1,3-benzodioxol-5-yl.

The bicyclic groups are optionally substituted by one or more groups R⁶ as hereinbefore defined.

The group A may particularly be attached to the ethynyl group via a carbon atom in the aryl group or in the 5 or 6 membered heteroaryl ring. The group B may particularly be attached to the group L via a carbon atom.

Suitable values for any of the the substituents herein, for example the 'R' groups (R¹ to R⁶) or for various groups within a A, B or L group include
- for halo: fluoro, chloro, bromo and iodo;
- for (1-6C)alkyl:: methyl, ethyl, propyl, isopropyl and tert-butyl;
- for (1-6C)alkoxy:: methoxy, ethoxy, propoxy, isopropoxy and butoxy;
- for (1-6C)alkylsulfonyl:: methylsulfonyl and ethylsulfonyl;
- for (1-6C)alkylamino:: methylamino, ethylamino, propylamino, isopropylamino and butylamino;
- for di-[(1-6C)alkyl]amino:: dimethylamino, diethylamino, N-ethyl-N-methylamino and diisopropylamino;
- for (1-6C)alkoxycarbonyl:: methoxycarbonyl, ethoxycarbonyl, propoxycarbonyl and tert-butoxycarbonyl;
- for (2-6C)alkanoyl:: acetyl and propionyl;
- for (1-6C)alkanoylamino:: acetamido and propionamido;
- for amino-(1-6C)allcyl:: aminomethyl, 2-aminoethyl, 1-aminoethyl and 3-aminopropyl;
- for (1-6C)alkylamino-(1-6C)alkyl:: methylaminomethyl, ethylaminomethyl, 1-methylaminoethyl, 2-methylaminoethyl, 2-ethylaminoethyl and 3-ethylaminopropyl;
- for di-[(1-6C)alkyl]amino-(1-6C)alkyl:: dimethylaminomethyl, diethylaminomethyl, 1-dimethylaminoethyl, 2-dimethylaminoethyl and 3-dimethylaminopropyl;
- for halogeno-(1=6C)alkyl:: chloromethyl, 2-chloroethyl, 1-chloroethyl and 3-chloropropyl;
- for hydroxy-(1-6C)alkyl:: hydroxymethyl, 2-hydroxyethyl, 1-hydroxyethyl and 3-hydroxypropyl;
- for (1-6C)alkoxy-(1-6C)alkyl:: methoxymethyl, ethoxymethyl, 1-methoxyethyl, 2-methoxyethyl, 2-ethoxyethyl and 3-methoxypropyl;
- for cyano-(1-6C)alkyl:: cyanomethyl, 2-cyanoethyl, 1-cyanoethyl and 3-cyanopropyl;
- for (3-7C)cycloalkyl:: cyclopropyl, cyclobutyl, cyclopentyl, cyclohexyl and cycloheptyl;
- for (1-6C)alkoxy(1-6C)alkoxy:: methoxymethoxy, methoxyethoxy, methoxypropoxy, methoxybutoxy, methoxyhexoxy, ethoxyethoxy, ethoxypropoxy, ethoxybutoxy, propoxypropoxy and propoxybutoxy;
- for (1-6C)alkoxy(1-6C)alkoxy(1-6C)alkoxy:: methoxymethoxymethoxy, methoxyethoxyethoxy, methoxypropoxymethoxy, methoxybutoxyethoxy, methoxyhexoxymethoxy, ethoxyethoxyethoxy, ethoxypropoxyethoxy, ethoxybutoxymethoxy, propoxypropoxymethoxy and propoxybutoxymethoxy;
- for mono(1-6C)alkylcarbamoyl:: N-methylcarbamoyl, N-ethylcarbamoyl and N-propylcarbamoyl; and
- for di-[(1-6C)alkyl]carbamoyl:: N,N-dimethylcarbamoyl, N-ethyl-N-methylcarbamoyl and N,N-diethylcarbamoyl.

When in this specification reference is made to a (1-4C)alkyl group it is to be understood that such groups refer to alkyl groups containing up to 4 carbon atoms. A skilled person will realise that representative examples of such groups are those listed above under (1-4C)alkyl that contain up to 4 carbon atoms, such as methyl, ethyl, propyl, isopropyl, butyl and tert-butyl. Similarly, reference to a (1-3C)alkyl group refers to alkyl groups containing up to 3 carbon atoms such as methyl, ethyl, propyl and isopropyl. A similar convention is adopted for the other groups listed above such as (1-4C)alkoxy, (2-4C)alkenyl, (2-4C)alkynyl and (2-4C)alkanoyl.

It is to be understood that certain compounds of the formula I may exist in solvated as well as unsolvated forms such as, for example, hydrated forms. It is to be understood that the invention encompasses all such solvated forms which exhibit an inhibitory effect on a Tie2 receptor tyrosine kinase.

It is also to be understood that certain compounds of the formula I may exhibit polymorphism, and that the invention encompasses all such forms which exhibit an inhibitory effect on a Tie2 receptor tyrosine kinase.

It is also to be understood that the invention relates to all tautomeric forms of the compounds of the formula I forms which exhibit an inhibitory effect on a Tie2 receptor tyrosine kinase.

Whilst pharmaceutically-acceptable salts of compounds of the invention are preferred, other non-pharmaceutically-acceptable salts of compounds of the invention may also be useful, for example, in the preparation of pharmaceutically-acceptable salts of compounds of the invention.

A suitable pharmaceutically acceptable salt of a compound of the formula I is, for example, an acid-addition salt of a compound of the formula I, for example an acid-addition salt with an inorganic or organic acid such as hydrochloric, hydrobromic, sulfuric, trifluoroacetic, citric or maleic acid; or, for example, a salt of a compound of the formula I which is sufficiently acidic, for example an alkali or alkaline earth metal salt such as a calcium or magnesium salt, or an ammonium salt, or a salt with an organic base such as methylamine, dimethylamine, trimethylamine, piperidine, morpholine or tris-(2-hydroxyethyl)amine.

Also provided as a further aspect of the invention are pro-drugs of compounds of the invention as herein before or herein after defined. Compounds of the invention may be administered in the form of a pro-drug which is broken down in the human or animal body to give a compound of the Formula (I). Examples of pro-drugs include in-vivo hydrolysable esters of a compound of the Formula (I).

Various forms of pro-drugs are known in the art. For examples of such pro-drug derivatives, see:
a) Design of Prodrugs, edited by H. Bundgaard, (Elsevier, 1985) and Methods in Enzymology, Vol. 42, p. 309-396, edited by K. Widder, et al. (Academic Press, 1985);
b) A Textbook of Drug Design and Development, edited by Krogsgaard-Larsen and H. Bundgaard, Chapter 5 "Design and Application of Prodrugs", by H. Bundgaard p. 113-191 (1991);
c) H. Bundgaard, Advanced Drug Delivery Reviews, 8, 1-38 (1992);
d) H. Bundgaard, et al., Journal of Pharmaceutical Sciences, 77, 285 (1988); and
e) N. Kakeya, et al., Chem Pharm Bull, 32, 692 (1984).

An in-vivo hydrolysable ester of a compound of the Formula (I) containing a hydroxy group is, for example, a pharmaceutically-acceptable ester which is hydrolysed in the human or animal body to produce the parent acid or alcohol. Suitable pharmaceutically-acceptable esters for carboxy include C₁₋₆ alkoxymethyl esters for example methoxymethyl, C₁₋₆alkanoyloxymethyl esters for example pivaloyloxymethyl, phthalidyl esters, C₃₋₈cycloalkoxycarbonyloxyC₁₋₆alkyl esters for example 1-cyclohexylcarbonyloxyethyl; 1,3-dioxolen-2-onylmethyl esters, for example 5-methyl-1,3-dioxolen-2-onylmethyl; and C₁₋₆alkoxycarbonyloxyethyl esters.

An in-vivo hydrolysable ester of a compound of the Formula (I) containing a hydroxy group includes inorganic esters such as phosphate esters (including phosphoramidic cyclic esters) and α-acyloxyalkyl ethers and related compounds which as a result of the in-vivo hydrolysis of the ester breakdown to give the parent hydroxy group/s. Examples of α-acyloxyalkyl ethers include acetoxymethoxy and 2,2-dimethylpropionyloxy-methoxy. A selection of in-vivo hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N=alkylcarbamoyl (to give carbamates), dialkylaminoacetyl and carboxyacetyl.

Particular novel compounds of the invention include, for example, compounds of the formula I, or salts, particularily pharmaceutically acceptable salts thereof, wherein, unless otherwise stated, each of R¹, R², R³, R⁴, R⁵, R⁶, A, B, L, m and n has any of the meanings defined hereinbefore or in paragraphs (a) to (eeee) hereinafter:-
(a) when n is 2 or 3, R⁵ may be the same or different;
(a') when m is 2 or 3, R⁶ may be the same or different;
(a") L is -CH₂C(O)N(R⁹)-, wherein R⁹ is hydrogen or (1-6C)alkyl (particularly R⁹ is hydrogen);-
(b) L is -N(R⁸)C(O)CH₂-, wherein R⁸ is hydrogen or (1-6C)alkyl (particularly R⁸ is hydrogen);
(c) L is -N(R⁸)C(O)N(R⁹)-, wherein R⁸ and R⁹ are independently selected from hydrogen and (1-6C)alkyl (particularly R⁸ and R⁹ are both hydrogen or in another embodiment one of R⁸ and R⁹ is hydrogen and the other is methyl)
(d) L is N(R⁸)C(O) O-, wherein R⁸ is hydrogen or (1-6C)allcyl (particularly R⁸ is hydrogen or in another embodiment R⁸ is methyl);
(e) L is -OC(O) N(R⁹)-, wherein R⁹ is hydrogen or (1-6C)alkyl (particularly R⁹ is hydrogen or in another embodiment R⁹ is methyl);
(e') L is selected from -N(R⁸)C(O)N(R⁹), -N(R⁸)C(O)O- or -N(R⁸)C(O)CH₂- wherein R⁸ and R⁹ independently represent hydrogen or (1-6C)alkyl.
(f) Ring B-R⁶, where m is 1 or 2, is selected from 2-methoxyphenyl, 2-fluoro-5-(trifluoromethyl)phenyl, 5-tert-butylisoxazol-3-yl, 3-(trifluoromethyl)phenyl, 3-morpholin-4-ylphenyl,3-methylisoxazol-5-yl, 5-tert-butyl-1,3,4-thiadiazol-2-yl and 3-acetylaminophenyl;
(f') Ring B-R⁶, where m is 1 or 2, is selected from 2-oxo-piperidin-3-yl, 2,2-dimethyltetrahydropyran-4-yl, 2-fluoro-phenyl, 3-fluoro-phenyl, 4-fluoro-phenyl, 2,5-difluoro-phenyl, 3,4-difluoro-phenyl, 4,5-difluoro-phenyl, 3-chloro-phenyl, 2-methoxyphenyl, 3-methoxyphenyl, 4-methoxyphenyl, 3-cyano-phenyl, 2-(trifluoromethyl)phenyl, 3-(trifluoromethyl)phenyl, 4-(trifluoromethyl)phenyl, 2-fluoro-5-(trifluoromethyl)phenyl, 3-acetylaminophenyl, 5-methyl-1,3,4-thiadiazol-2-yl, 5-t-butyl-1,3,4-thiadiazol-2-yl, 5-triflluoromethyl-1,3,4-thiadiazol-2-yl, 5-cyclopropyl-1,3,4-thiadiazol-2-yl, 5-ethyl-1,3,4-thiadiazol-2-yl, 5-isopropyl-1,3,4-thiadiazol-2-yl, 5-ethylthio-1,3,4-thiadiazol-2-yl, 3-methylisoxazol-5-yl, 5-methylisoxazol-3-yl, 5-t-butyl-isoxazol-3-yl, 4-t-butyl-thiazol-2-yl, 3-methyl-isothiazol-5-yl, 1-methyl-3-t-butyl-pyrazol-5-yl, 1-methyl-3-cyclopropyl-pyrazol-5-yl, 1-methyl-3-isopropyl-pyrazol-5-yl, 1-t-butyl-pyrazol-4-yl, 1-t-butyl-3-cyclopropyl-pyrazol-5-yl, 1-ethyl-pyrazol-3-yl, 1-isopropyl-pyrazol-3-yl, 5-isopropyl-1,3,4-oxadiazol-2-yl, 4-trifluoro-pyrid-2-yl and 3-fluoro-5-(4-methylpiperazin-1-yl)phenyl;
(f') Ring B-R⁶, where m is 1 or 2, is selected from 2-oxo-piperidin-3-yl, 1-methylpiperidin-4-yl, 1-propylpiperidin-4-yl, 2,2-dimethyltetrahydropyran-4-yl, 2-fluoro-phenyl, 3-fluoro-phenyl, 4-fluoro-phenyl, 3,4=dichloro-phenyl, 2,5-difluoro-phenyl, 3,4-difluoro-phenyl, 4,5-difluoro-phenyl, 3-chloro-phenyl, 2-methoxyphenyl, 3-methoxyphenyl, 4-methoxyphenyl, 3-cyano-phenyl, 2-(trifluoromethyl)phenyl, 3-(trifluoromethyl)phenyl, 4-(trifluoromethyl)phenyl, 2-fluoro-5-(trifluoromethyl)phenyl, 3-acetylaminophenyl, 2-morpholin-4-ylphenyl, 5-methyl-1,3,4-thiadiazol-2-yl, 5-t-butyl-1,3,4-thiadiazol-2-yl, 5-triflluoromethyl-1,3,4-thiadiazol-2-yl, 5-cyclopropyl-1,3,4-thiadiazol-2-yl, 5-ethyl-1,3,4-thiadiazol-2-yl, 5-isopropyl-1,3,4-thiadiazol-2-yl, 5-ethylthio-1,3,4-thiadiazol-2-yl, 3-methylisoxazol-5-yl, 5-methylisoxazol-3-yl, 5-t-butyl-isoxazol-3-yl, 4-t-butyl-thiazol-2-yl, 3-methyl-isothiazol-5-yl, 1-methyl-3-t-butyl-pyrazol-5-yl, 1-methyl-3-cyclopropyl-pyrazol-5-yl, 1-methyl-3-isopropyl-pyrazol-5-yl, 1-t-butyl-pyrazol-4-yl, 1-t-butyl-3-cyclopropyl-pyrazol-5-yl, 1-ethyl-pyrazol-3-yl, 1-isopropyl-pyrazol-3-yl, 5-isopropyl-1,3,4-oxadiazol-2-yl, 4-trifluoro-pyrid-2-yl, 3-fluoro-5-(4-methylpiperazin-1-yl)phenyl, 4-(trifluoromethyl)pyrid-3-yl and 4-(trifluoromethyl)pyrid-2-yl;
(g) R¹ and R² are both hydrogen, R³ and R⁴ are both hydrogen, n is 0, L is -NHC(O)NH-, and ring B-R⁶, where m is 1 or 2, is selected from 2-methoxyphenyl, 2-fluoro-5-(trifluoromethyl)phenyl, 5-tert-butylisoxazol-3-yl, 3-(trifluoromethyl)phenyl, 3-morpholin-4-ylphenyl, 3-methylisoxazol-5-yl, 5-tert-butyl-1,3,4-thiadiazol-2-yl and 3-acetylaminophenyl;
(g') R¹ and R² are both hydrogen, R³ and R⁴ are both hydrogen, n is 0, L is NHC(O)NH-, and ring B-R⁶, where m is 1 or 2, is selected from 2-methoxyphenyl, 2-fluoro-5-(trifluoromethyl)phenyl, 5-tert-butylisoxazol-3-yl, 3-(trifluoromethyl)phenyl, 3-morpholin-4-ylphenyl, 3-methylisoxazol-5-yl, 5-tert-butyl-1,3,4-thiadiazol-2-yl, 3-acetylaminophenyl, 1-t-methyl-3-t-butyl-pyrazol-5-yl; 1-methyl-3-cyclopropyl-pyrazol-5-yl, 1-methyl-3-isopropyl-pyrazol-5-yl, 1-t-butyl-pyrazol-4-yl, 1-t-butyl-3-cyclopropyl-pyrazol-5-yl and 1-ethyl-pyrazol-3-yl, 1-isopropyl-pyrazol-3-yl;
(h) R¹ and R² are independently selected from hydrogen, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6, or a 5 or 6 membered heteroaryl ring;
   wherein the (1-6C)alkyl, the (1-6C)alkanoyl and the (3-6C)cycloalkyl groups are optionally substituted by one or more groups (for example 1 or 2), which may be the same or different, selected from fluoro, hydroxy, (1-6C)alkyl,(1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or N(R^{d})C(O)(1-6C)alkyl in which R^{d} is hydrogen or (1-6C)alkyl, a saturated or partially saturated 3 to 7 membered heterocyclic ring or a 5 or 6 membered heteroaryl ring, wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di=[(1-6C)alkyl]carbamoyl and/or -N(R^{d})C(O)(1-6C)alkyl groups are optionally substituted by one or more (for example 1 or 2) hydroxy groups;
   wherein the phenyl is optionally substituted by one or more groups (for example 1 or 2), which may be the same or different, selected from halo, (1-6C)alkyl, (1-6C)alkoxy; amino, mono(1-6C)alkylamino or di-[(1-6C)alkyl]amino, wherein the (1-6C)alkyl and (1-6C)alkoxy groups are optionally substituted by one or more groups (for example 1 or 2), which may be the same or different, selected from hydroxy, amino, mono(1-6C)alkylamino or di-[(1-6C)alkyl]amino;
   and wherein any heterocyclic and heteroaryl rings within R¹ and/or R² are optionally substituted by one or more groups (for example 1 or 2), which may be the same or different, selected from (1-4C)alkyl, (1-4C)alkoxy, (1-4C)alkoxy(1-4C)alkyl, hydroxy, amino, mono(1-6C)alkylamino or di-[(1-6C)alkyl]amino, a saturated or partially saturated 3 to 7 membered heterocyclic ring or -C(O)(CH₂)_{z}Y wherein z is 0, 1, 2 or 3 and Y is selected from hydrogen, hydroxy, (1-4C)alkoxy, amino, mono(1-6C)allcylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
(i) R¹ and R² are independently selected from hydrogen, (1-6C)alkanoyl, (1-6C)alkyl, (1-6C)alkoxycarbonyl or (3-6C)cycloalkyl(CH₂)ₓ- in which x is 0, 1, 2, 3, 4, 5 or 6;
   wherein the (1-6C)alkyl, the (1-6C)alkanoyl and the (3-6C)cycloalkyl groups are optionally substituted by one or more groups (for example 1 or 2), which may be the same or different, as hereinbefore defined in (h);
   and wherein any heterocyclic and heteroaryl rings within R¹ and/or R² are optionally independently substituted by one or more groups (for example 1 or 2), which may be the same or different, as hereinbefore defined in (h);
(j) R¹ and R² are independently selected from hydrogen, (1-6C)alkanoyl and (1-6C)alkyl;
   wherein the (1-6C)alkyl and the (1-6C)alkanoyl groups are optionally substituted by one or more groups (for example 1 or 2), which may be the same or different, as hereinbefore defined in (h);
   and wherein any heterocyclic and heteroaryl rings within R¹ and/or R² are optionally independently substituted by one or more groups (for example 1 or 2), which may be the same or different, as hereinbefore defined in (h);
(k) R¹ is hydrogen and R² is selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)x- in which x is 0, 1, 2, 3, 4, 5 or 6, or a 5 or 6 membered heteroaryl ring;
   wherein the (1-6C)alkyl, the (1-6C)alkanoyl and the (3-6C)cycloalkyl groups are optionally substituted by one or more groups (for example 1 or 2), which may be the same or different, as hereinbefore defined in (h);
   wherein the phenyl is optionally substituted by one or more groups (for example 1 or 2), which may be the same or different, as hereinbefore defined in (h);
   and wherein any heterocyclic and heteroaryl rings within R² are optionally independently substituted by one or more groups (for example 1 or 2), which may be the same or different, as hereinbefore defined in (h);
(l) R¹ is hydrogen and R² is selected from hydrogen, (1-6C)alkanoyl, (1-6C)alkyl, (1-6C)alkoxycarbonyl or (3-6C)cycloalkyl(CH₂)ₓ- in which x is 0, 1, 2, 3, 4, 5 or 6;
   wherein the (1-6C)alkyl, the (1-6C)alkanoyl and the (3-6C)cycloalkyl groups are optionally substituted by one or more groups (for example 1 or 2), which may be the same or different, as hereinbefore defined in (h);
   and wherein any heterocyclic and heteroaryl rings within R² are optionally independently substituted by one or more groups (for example 1 or 2), which may be the same or different, as hereinbefore defined in (h);
(m) R¹ is hydrogen and R² is selected from hydrogen, (1-6C)alkanoyl and (1-6C)alkyl;
   wherein the (1-6C)alkyl and the (1-6C)alkanoyl groups are optionally substituted by one or more groups (for example 1 or 2), which may be the same or different, as hereinbefore defined in (h);
   and wherein any heterocyclic and heteroaryl rings within R² are optionally independently substituted by one or more groups (for example 1 or 2), which may be the same or different, as hereinbefore defined in (h);
(n) R¹ and R² are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6, or a 5 or 6 membered heteroaryl ring;
   wherein the (1-6C)alkyl, the (1-6C)alkanoyl and the (3-6C)cycloalkyl groups are optionally substituted by one or more groups (for example 1 or 2), which may be the same or different, selected from hydroxy, (1-6C)alkoxy, (1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or-N(R^{d})C(O)(1-6C)alkyl in which R^{d} is hydrogen or (1-6C)alkyl, a saturated or partially saturated 3 to 7 membered heterocyclic ring or a 5 or 6 membered heteroaryl ring, wherein the (1-6C)alkoxy and (1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or -N(R^{d})C(O)(1-6C)alkyl groups are optionally substituted by one or more (for example 1 or 2) hydroxy groups;
   and wherein the phenyl is optionally substituted by one or more groups (for example 1 or 2), which may be the same or different, selected from halo, (1-6C)alkyl, (1-6C)alkoxy, amino, mono(1-6C)alkylamino or di-[(1-6C)alkyl]amino, wherein the (1-6C)alkyl and (1-6C)alkoxy groups are optionally substituted by one or more groups (for example 1 or 2), which may be the same or different, selected from hydroxy, amino, mono(1-6C)alkylamino or di-[(1-6C)alkyl]amino;
   and wherein any heterocyclic and heteroaryl rings within R¹ and/or R² are optionally independently substituted by one or more groups (for example 1 or 2), which may be the same or different, selected from (1-4C)alkyl, (1-4C)alkoxy, (1-4C)alkoxy(1-4C)alkyl, hydroxy, amino, mono(1-6C)alkylamino or di-[(1-6C)alkyl]amino, or a saturated or partially saturated 3 to 7 membered heterocyclic ring or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)allcanoyl is not substituted by hydroxy;
(O) R¹ and R² are independently selected from hydrogen, (1-6C)alkanoyl, (1-6C)alkyl, (1-6C)alkoxycarbonyl or (3-6C)cycloalkyl(CH₂)ₓ- in which x is 0, 1, 2, 3, 4, 5 or 6;
   wherein the (1-6C)alkyl, the (1-6C)alkanoyl and the (3-6C)cycloalkyl groups are optionally substituted by one or more groups (for example 1 or 2), which may be the same or different, as hereinbefore defined in (n);
   and wherein any heterocyclic and heteroaryl rings within R¹ and/or R² are optionally independently substituted by one or more groups (for example 1 or 2), which may be the same or different, as hereinbefore defined in (n);
(p) R¹ and R² are independently selected from hydrogen, (1-6C)alkanoyl and (1-6C)alkyl;
   wherein the (1-6C)alkyl and the (1-6C)alkanoyl groups are optionally substituted by one or more groups (for example 1 or 2), which may be the same or different, as hereinbefore defined in (n);
   and wherein any heterocyclic and heteroaryl rings within R¹ and/or R² are optionally independently substituted by one or more groups (for example 1 or 2), which may be the same or different, as hereinbefore defined in (n);
(q) R¹ is hydrogen and R² is selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6, or a 5 or 6 membered heteroaryl ring;
   wherein the (1-6C)alkyl, the (1-6C)alkanoyl and the (3-6C)cycloalkyl groups are optionally substituted by one or more groups (for example 1 or 2), which may be the same or different, as hereinbefore defined in (n);
   and wherein the phenyl is optionally substituted by one or more groups (for example 1 or 2), which may be the same or different, as hereinbefore defined in (n);
   and wherein any heterocyclic and heteroaryl rings within R² are optionally independently substituted by one or more groups (for example 1 or 2), which may be the same or different, as hereinbefore defined in (n);
(r) R¹ is hydrogen and R² is selected from hydrogen, (1-6C)alkanoyl, (1-6C)alkyl, (1-6C)alkoxycarbonyl or (3-6C)cycloalkyl(CH₂)ₓ- in which x is 0, 1, 2, 3, 4, 5 or 6;
   wherein the (1-6C)alkyl, the (1-6C)alkanoyl and the (3-6C)cycloalkyl groups are optionally substituted by one or more groups (for example 1 or 2), which may be the same or different, as hereinbefore defined in (n);
   and wherein any heterocyclic and heteroaryl rings within R² are optionally independently substituted by one or more groups (for example 1 or 2), which may be the same or different, as hereinbefore defined in (n);
(s) R¹ is hydrogen and R² is selected from hydrogen, (1-6C)alkanoyl and (1-6C)alkyl;
   wherein the (1-6C)alkyl and the (1-6C)alkanoyl groups are optionally substituted by one or more groups (for example 1 or 2), which may be the same or different, as hereinbefore defined in (n);
   and wherein any heterocyclic and heteroaryl rings within R² are optionally independently substituted by one or more groups (for example 1 or 2), which may be the same or different, as hereinbefore defined in (n);
(t) R¹ is hydrogen and R² is selected from hydrogen, (1-6C)alkanoyl and (1-6C)alkyl;
   wherein the (1-6C)alkyl and the (1-6C)alkanoyl groups are optionally substituted by one or more groups (for example 1 or 2), which may be the same or different, selected from hydroxy, (1-4C)alkoxy, (1-4C)alkoxy(1-4C)alkoxy, amino, mono(1-3C)alkylamino, di(1-3C)alkylamino, carbamoyl or-N(R^{d})C(O)(1-3C)alkyl in which R^{d} is hydrogen or (1-3C)alkyl, a saturated 5 or 6 membered heterocyclic ring or a 5 or 6 membered heteroaryl ring, wherein the (1-4C)alkoxy and (1-4C)alkoxy(1-4C)alkoxy groups and the (1-3C)alkyl groups of the mono(1-3C)alkylamino, di-[(1-3C)alkyl]amino and/or -N(R^{d})C(O)(1-3C)alkyl groups are optionally substituted by one or more (for example 1 or 2) hydroxy groups;
   and wherein any heterocyclic and heteroaryl rings within R² are optionally independently substituted by one or more groups (for example 1 or 2), which may be the same or different, selected from (1-4C)alkyl, (1-4C)alkoxy, (1-4C)alkoxy(1-4C)alkyl, hydroxy, amino, mono(1-3C)alkylamino or di-[(1-3C)alkyl]amino, a saturated or partially saturated 3 to 7 membered heterocyclic ring or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R² is a (1C)alkanoyl group, then the (1C)alkanoyl is not substituted by hydroxy;
(u) R¹ is hydrogen and R² is selected from hydrogen, (1-3C)alkanoyl and (1-3C)alkyl;
   wherein the (1-3C)alkyl and the (1-3C)alkanoyl groups are optionally substituted by one or more groups (for example 1 or 2), which may be the same or different, as hereinbefore defined in (t);
   and wherein any heterocyclic and heteroaryl rings within R² are optionally independently substituted by one or more groups (for example 1 or 2), which may be the same or different, as hereinbefore defined in (t);
(v) R¹ is hydrogen and R² is selected from hydrogen and (1-6C)alkyl (particularly (1-3C)alkyl);
   wherein the (1-6C)alkyl (particularly (1-3C)alkyl) group is optionally substituted by one or more groups (for example 1 or 2), which may be the same or different, as hereinbefore defined in (t);
   and wherein any heterocyclic and heteroaryl rings within R² are optionally independently substituted by one or more groups (for example 1 or 2), which may be the same or different, as hereinbefore defined in (t);
(w) R¹ is hydrogen and R² is (1-6C)alkyl (particularly (1-3C)alkyl);
   wherein the (1-6C)alkyl (particularly (1-3C)alkyl) group is optionally substituted by one or more groups (for example 1 or 2), which may be the same or different, as hereinbefore defined in (t);
   and wherein any heterocyclic and heteroaryl rings within R² are optionally independently substituted by one or more groups (for example 1 or 2), which may be the same or different, as hereinbefore defined in (t);
(w') R¹ and R² are both hydrogen or R¹ is hydrogen or (1-6C)alkyl and R² is (1-6C)alkyl
   wherein (1-6Calkyl) is optionally substituted by hydroxy, amino, mono(1-6C)alkylamino or di(1-6C)alkylamino, carbamoyl, (1-6C)alkoxy, (1-6C)alkoxy(1-6C)alkoxy, -N(R^{d})C(O)(1-6C)alkyl in which R^{d} is hydrogen or (1-6C)alkyl, a saturated or partially saturated 3 to 7 membered heterocyclic ring or a 5 or 6 membered heteroaryl ring;
   wherein the (1-6C)alkoxy, mono(1-6C)alkylamino and -N(R^{d})C(O)(1-6C)alkyl groups are optionally substituted by hydroxy;
   wherein the a saturated or partially saturated 3 to 7 membered heterocyclic ring is optionally substituted by (1-4C)alkyl or -C(O)CH₂Y wherein Y is selected from hydroxy or di(1-6C)alkylamino.
(w") R¹ and R² are both hydrogen or R¹ is hydrogen or (1-6C)alkyl and R² is (1-6C)alkyl
   wherein (1-6Calkyl) is optionally substituted by hydroxy, amino, mono(1-6C)alkylamino or di(1-6C)alkylamino, aryl, a saturated or partially saturated 3 to 7 membered heterocyclic ring or a 5 or 6 membered heteroaryl ring;
   wherein an aryl ring or a saturated or partially saturated 3 to 7 membered heterocyclic ring or a 5 or 6 membered heteroaryl ring is optionally substituted by one or two groups independently selected from (1-4C)alkyl and (1-4C)alkoxy.
(w"') R¹ and R² are both hydrogen or R¹ is hydrogen or (1-6C)allcyl and R² is (1-6C)alkyl
   wherein (1-6Calkyl) is optionally substituted by hydroxy, amino, mono(1-6C)alkylamino or di(1-6C)alkylamino, carbamoyl, (1-6C)alkoxy, (1-6C)alkoxy(1-6C)alkoxy, -N(R^{d})C(O)(1-6C)alkyl in which R^{d} is hydrogen or (1-6C)alkyl, aryl, a saturated or partially saturated 3 to 7 membered heterocyclic ring or a 5 or 6 membered heteroaryl ring;
   wherein the (1-6C)alkoxy, mono(1-6C)alkylamino and -N(R^{d})C(O)(1-6C)alkyl groups are optionally substituted by hydroxy;
   wherein an aryl ring, a saturated or partially saturated 3 to 7 membered heterocyclic ring or a 5 or 6 membered heteroaryl ring is optionally substituted by (1-4C)alkyl, (1-4C)alkoxy or -C(O)CH₂Y wherein Y is selected from hydroxy or di(1-6C)alkylamino.
(x) R¹ and R² are independently selected from hydrogen, methyl, ethyl, propyl, 2-hydroxyethyl, 3-hydroxypropyl, 2-methoxyethyl, 3-methoxypropyl, 2-(2-hydroxyethoxy)ethyl, 2-aminoethyl, 3-aminopropyl, 4-aminobutyl, 2-(isopropylamino)ethyl, 3-(isopropylamino)propyl, 2-(dimethylamino)ethyl, 3-(dimethylamino)propyl, 4-(dimethylamino)butyl, 2-(dimethylamino)-1-methylethyl, carbamoylmethyl, 2-carbamoylethyl, 3-carbamoylpropyl, 2-(2-methoxyethoxy)acetyl, N-ethyl-2-hydroxyacetamide, 2-morpholin-4-ylethyl, 3-morpholin-4-ylpropyl, 2-pyrrolidin-1-ylethyl, 3-pyrrolidin-1-ylpropyl, 3-(4-methylpiperazin-1-yl)propyl, 3-piperidin-1-ylpropyl, 2-piperidin-1-ylethyl, 2-(1H-imidazol-4-yl)ethyl, 2-pyridin-2-ylethyl, 3-(1H-imidazol-1-yl)propyl and 2-pyridin-4-ylethyl;
(y) R¹ is hydrogen and R² is selected from hydrogen, methyl, ethyl, propyl, 2-hydroxyethyl, 3-hydroxypropyl, 2-methoxyethyl, 3-methoxypropyl, 2-(2-hydroxyethoxy)ethyl, 2-aminoethyl, 3-aminopropyl, 4-aminobutyl, 2-(isopropylamino)ethyl, 3-(isopropylamino)propyl, 2-(dimethylamino)ethyl, 3-(dimethylamino)propyl, 4-(dimethylamino)butyl, 2-(dimethylamino)-1-methylethyl, carbamoylmethyl, 2-carbamoylethyl, 3-carbamoylpropyl, 2-(2-methoxyethoxy)acetyl, N-ethyl-2-hydroxyacetamide, 2-morpholin-4-ylethyl, 3-morpholin-4-ylpropyl, 2-pyrrolidin-1-ylethyl, 3-pyrrolidin-1-ylpropyl, 3-(4-methylpiperazin-1-yl)propyl, 3-piperidin-1-ylpropyl, 2-piperidin-1-ylethyl, 2-(1H-imidazol-4-yl)ethyl, 2-pyridin-2-ylethyl, 3-(1H-imidazol-1-yl)propyl and 2-pyridin-4-ylethyl;
(y') R¹ is hydrogen and R² is selected from hydrogen, methyl, ethyl, propyl, 2-hydroxyethyl, 3-hydroxypropyl, 2-methoxyethyl, 3-methoxypropyl, 2-(2-hydroxyethoxy)ethyl, 2-aminoethyl, 3-aminopropyl, 4-aminobutyl, 2-(isopropylamino)ethyl, 3-(isopropylamino)propyl, 2-(dimethylamino)ethyl, 3-(dimethylamino)propyl, 4-(dimethylamino)butyl, 2-(dimethylamino)-1-methylethyl, carbamoylmethyl, 2-carbamoylethyl, 3-carbamoylpropyl, 2-(2-methoxyethoxy)acetyl, N-ethyl-2-hydroxyacetamide, 2-(2-hydroxyacetamido)ethyl, 3-[N-(2-hydroxyethyl)amino]propyl, 2-morpholin-4-ylethyl, 3-morpholin-4-ylpropyl, 2-[(1-methyl-2-morpholin-4-ylethyl), 2-pyrrolidin-1-ylethyl, 3-pyrrolidin-1-ylpropyl, 1-glycoloylpyrrolidin-2-yl)methyl, 1-(N,N-dimethylglycyl)pyrrolidin-2-yl, 2-piperazin-1-ylethyl, 3-piperazin-1-ylpropyl, 3-(4-methylpiperazin-1-yl)propyl, 3-piperidin-1-ylpropyl, 2-piperidin-1-ylethyl, 2-(1H-imidazol-4-yl)ethyl, 2-pyridin-2-ylethyl, 3-(1H-imidazol-1-yl)propyl and 2-pyridin-4-ylethyl;
(y") R¹ is hydrogen or methyl and R² is selected from hydrogen, methyl, 2-hydroxyethyl, 2-methoxyethyl, 3-methoxypropyl, 2-(2-hydroxyethoxy)ethyl, 2-aminoethyl, 3-aminopropyl, 4-aminobutyl, 2-(isopropylamino)ethyl, 3-(isopropylamino)propyl, 2-(dimethylamino)ethyl, 3-(dimethylamino)propyl, 4-(dimethylamino)butyl, 2-(dimethylamino)-1-methylethyl, carbamoylmethyl, 2-carbamoylethyl, 2-(2-methoxyethoxy)acetyl, 2-(2-hydroxyacetamido)ethyl, 3-[N-(2-hydroxyethyl)amino]propyl, 2-morpholin-4-ylethyl, 3-morpholin-4-ylpropyl, 2-[(1-methyl-2-morpholin-4-ylethyl), 2-pyrrolidin-1-ylethyl, 3-pyrrolidin-1-ylpropyl, 1-glycoloylpyrrolidin-2-yl)methyl, 1-(N,N-dimethylglycyl)pyrrolidin-2-yl, 2-piperazin-1-ylethyl; 3-piperazin-1-yipropyl, 3-(4-methylpiperazin-l-yl)propyl, 3-piperidin-1-ylpropyl, 2-(1H-imidazol-4-yl)ethyl, 2-pyridin-2-ylethyl, 3-(1H-imidazol-1-yl)propyl and 2-pyridin-4-ylethyl;
(y"') R¹ is hydrogen or methyl and R² is selected from hydrogen, methyl, 2-hydroxyethyl; 2-methoxyethyl, 3-methoxypropyl, 2-(2-hydroxyethoxy)ethyl, 2-aminoethyl, 3-aminopropyl, 4-aminobutyl, 2-(isopropylamino)ethyl, 3-(isopropylamino)propyl, 2-(dimethylamino)ethyl, 3-(dimethylamino)propyl, 4-(dimethylamino)butyl, 2-(dimethylamino)-1-methylethyl, carbamoylmethyl, 2-carbamoylethyl, 2-(2-methoxyethoxy)acetyl, 2-(2-hydroxyacetamido)ethyl, 3-[N-(2-hydroxyethyl)amino]propyl, 2-morpholin-4-ylethyl, 3-morpholin-4-ylpropyl, 2-[(1-methyl-2-morpholin-4-ylethyl), 2-pyrrolidin-1-ylethyl, 3-pyrrolidin-1-yipropyl, 1-glycoloylpyrrolidin-2-yl)methyl, 1-(N,N-dimethylglycyl)pyrrolidin-2-yl, 2-piperazin-1-ylethyl, 3-piperazin-1-ylpropyl, 3-(4-methylpiperazin-1-yl)propyl, 3-piperidin-1-ylpropyl, 2-(1H-imidazol-4-yl)ethyl, 2-pyridin-2-ylethyl, 3-(1H-imidazol-1-yl)propyl, 5-t-butyl-isoxazol-3-yl, 2-pyridin-4-ylethyl and and 2,4-dimethoxybenzyl;
(z) R¹ is hydrogen and R² is selected from 2-morpholin-4-ylethyl, 3-morpholin-4-ylpropyl, 3-piperidin-1-ylpropyl, 2-piperidin-1-ylethyl, 2-pyrrolidin-1-ylethyl and 3-pyrrolidin-1-ylpropyl;
(z') R¹ is hydrogen and R² is selected from 2-morpholin-4-ylethyl, 3-morpholin-4-ylpropyl, 3-piperidin-1-ylpropyl, 2-piperidin-1-ylethyl, 2-pyrrolidin-1-ylethyl, 3-pyrrolidin-1-ylpropyl and 4-methyl-piperazin-1-yl;
(aa) R¹ and R² are both hydrogen;
(bb) R¹ and R² are both (1-6C)alkyl (particularly (1-3C)alkyl);
(cc) R¹ and R² are both methyl;
(dd) R³ and R⁴ are independently selected from hydrogen and (1-6C)alkyl;
   wherein the (1-6C)alkyl groups are optionally substituted by one or more groups (for example 1 or 2), which may be the same or different, selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more groups (for example 1 or 2), which may be the same or different, selected from (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
(ee) R³ and R⁴ are independently selected from hydrogen and (1-6C)alkyl;
   wherein the (1-6C)alkyl groups are optionally substituted by one or more groups (for example 1 or 2), which may be the same or different, selected from fluoro, hydroxy, (1-6C)allcyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl;
(ff) R³ and R⁴ are both hydrogen;
(gg) A is selected from phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl and 1,3,5-triazinyl;
(hh) A is selected from phenyl, pyrrolyl, thienyl, oxazolyl, imidazolyl, pyrazolyl, thiazolyl, isothiazolyl, thiadiazolyl, triazolyl, tetrazolyl, pyridyl, pyridazinyl, pyrimidinyl, pyrazinyl and 1,3,5-triazinyl;
(hh') A is selected from phenyl, oxazolyl, imidazolyl, pyrrolyl, pyrazolyl, thiazolyl, isothiazolyl, thiadiazolyl, pyridyl, pyridazinyl, pyrazinyl and pyrimidyl.
(ii) A is selected from phenyl, thiazolyl, thiadiazolyl, pyridyl and pyrimidinyl;
(jj) A is phenyl;
(jj') A is phenyl or pyridyl
(jj'') A is phenyl or pyridyl, wherein the nitrogen in the pyridyl ring is in the 3-position relateive to the alkyne bond.
(kk) A is phenyl and n is 0;
(kk') A is phenyl or pyridyl and n is 0;
(11) n is 0, 1 or 2 (particularly 0 or 1, more particularly 0);
(mm) n is 1 or 2 and R⁵ is independently selected from cyclopropyl, halo, (1-6C)alkoxy and (1-6C)alkyl, wherein the (1-6C)alkyl and the (1-6C)alkoxy groups are optionally substituted by cyano or one or more (for example 1 or 2) fluoro;
(mm') n is 1 or 2 and R⁵ is independently selected from cyano, cyclopropyl, halo, (1-6C)alkoxy and (1-6C)alkyl, wherein the (1-6C)alkyl and the (1-6C)alkoxy groups are optionally substituted by cyano or one or more (for example 1 or 2) fluoro;
(mm") n is 0 or 1 and when n is 1, R⁵ is (1-4C)alkyl (particularily methyl);
(nn) L is -N(R⁸)C(O)C(R^{a}R^{b})-, wherein R⁸ independently represents hydrogen or (1-6C)alkyl, R^{a} is hydrogen and R^{b} independently represents hydrogen or (1-6C)alkyl (particularly R^{b} is (1-3C)alkyl, such as methyl);
(oo) L is attached meta on ring A with respect to the point of attachment of the ethynyl group;
(pp) L is attached meta on ring A with respect to the point of attachment of the ethynyl group and represents -N(R⁸)C(O)C(R^{a}R^{b})-, -N(R⁸)C(O)N(R⁹)- or -N(R⁸)C(O)O-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl;
(qq) L is attached meta on ring A with respect to the point of attachment of the ethynyl group and represents -N(R⁸)C(O)C(R^{a}R^{b})-, -N(R⁸)C(O)N(R⁹)- or -N(R⁸)C(O)O-, wherein R⁸ and R⁹ independently represent hydrogen or (1-3C)alkyl and wherein R^{a} and R^{b} independently represent hydrogen or (1-3C)alkyl;
(rr) L is attached para on ring A with respect to the point of attachment of the ethynyl group;
(rr') L is attached para on ring A with respect to the point of attachment of the ethynyl group and represents -N(R⁸)C(O)C(R^{a}R^{b})- or -N(R⁸C(O)N(R⁹)-, wherein R⁸and R⁹ independently represent hydrogen or (1-6C)alkyl and wherein R⁸ and R^{b} independently represent hydrogen or (1-6C)alkyl or R^{a} and R^{b} together with the carbon atom to which they are attached represent (3-6C)cycloalkyl;
(ss) L is attached para on ring A with respect to the point of attachment of the ethynyl group arid represents -N(R⁸)C(O)C(R^{a}R^{b})- or -N(R⁸)C(O)N(R⁹)-, wherein R⁸ and R⁹ independently represent hydrogen or (1-3C)alkyl and wherein R^{a} and R^{b} independently represent hydrogen or (1-3C)alkyl;
(tt) A is selected from phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl and 1,3,5-triazinyl (particularly phenyl, thiazolyl, thiadiazolyl, pyridyl and pyrimidinyl);
   n is 0; and
   L is attached meta on ring A with respect to the point of attachment of the ethynyl group and represents -N(R⁸)C(O)C(R^{a}R^{b})-, -N(R⁸)C(O)N(R⁹)- or -N(R⁸)C(Q) O-, wherein R⁸ and R⁹ independently represent hydrogen or (1-6C)alkyl and wherein R^{a} and R^{b} independently represent hydrogen or (1-6C)alkyl or R⁸ and R^{b} together with the carbon atom to which they are attached represent (3-6C)cycloalkyl;
(uu) A is selected from phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl and 1,3,5-triazinyl (particularly phenyl, thiazolyl, thiadiazolyl, pyridyl and pyrimidinyl,
   n is 0; and
   L is attached meta on ring A with respect to the point of attachment of the ethynyl group and represents -N(R⁸)C(O)C(R^{a}R^{b})-, -N(R⁸)C(O)N(R⁹)- or -N(R⁸)C(O)O-, wherein R⁸ and R⁹ independently represent hydrogen or (1-3C)alkyl and wherein R^{a} and R^{b} independently represent hydrogen or (1-3C)alkyl;
(vv) When B is a (3-7C)cycloalkyl ring then B is selected grom cyclopropyl, cyclobutyl, cyclopentyl and cyclohexyl;
(vv') When B is a saturated or partially saturated 3 to 7 membered heterocyclic ring then B is selected from oxetanyl, azetidinyl, thietanyl, pyrrolidinyl, , morpholinyl, 1,3-dioxolanyl, tetrahydrofuranyl, piperidyl, piperazinyl, thiomorpholinyl, tetrahydropyranyl, homopiperazinyl, pyrrolinyl, imidazolinyl, pyrazolinyl, pyranyl, tetrahydropyridinyl, 1,2,4-oxadiazolyl and dihydrothiopyranyl;
(vv") When B is an 8, 9 or 10 membered bicyclic group which optionally contains 1,2,3 or 4 heteroatoms independently selected from N, O and S and which is saturated, partially saturated or aromatic then B is selected from 2,3-dihydro-1H-indenyl, benzodioxinyl, 1,2,3,4-tetrahydronaphthalenyl, 1,2,3,4-tetrahydropentalene, benzofuranyl, 2,3-dihydrobenzofuranyl, benzimidazolyl, benzthienyl, benzthiazolyl, benzisothiazolyl, benzoxazolyl, benzisoxazolyl, pyridoimidazolyl, pyrimidoimidazolyl, quinolinyl, isoquinolinyl, quinoxalinyl, quinazolinyl, phthalazinyl, cinnolinyl, indolyl, and naphthyridinyl.
   or B is a group of the formula: 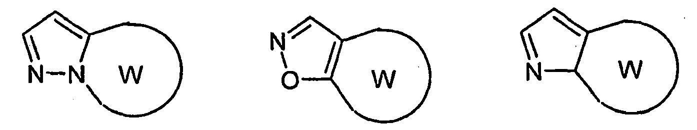 wherein W is a 5-7 membered ring (including the bridging atoms), said W ring comprising carbon atoms or optionally further heteroatoms independently selected from oxygen, nitrogen and sulphur, wherein said bicyclic ring contains no more that 4 heteroatoms in total. Examples of such rings include: pyrazolo[1,5-a]pyridinyl, pyrazolo[1,5-c]pyrimidinyl, pyrazolo[1,5-a][1,3,5]triazinyl, 4,5-dihydropyrazolo[1,5-a]pyridinyl, 4H-pyrazolo[5,1-c][1,4]thiazinyl, 4H-pyrazolo[5,1-c][1,4]oxazinyl, 1,2-benzisoxazolyl, isoxazolo[5,4-b]pyridinyl, isoxazolo[5,4-d]pyrimidinyl, 4H-thiopyrano[3,4-d]isoxazolyl, 4H-pyrano[3,4-d]isoxazolyl, 7aH-indolyl, 7aH-pyrrolo[2,3-b]pyridinyl, 7aH-pyrrolo[2,3-b]pyrimidinyl, 4,7a-dihydrothiopyrano[4,3-b]pyrrolyl and 4,7a-dihydropyrano[4,3-b]pyrrolyl.
(vv"') B is selected from a (4-6C)cycloalkyl ring, a saturated or partially saturated 4 to 6 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
(ww) B is selected from a (4-6C)cycloalkyl ring, a saturated or partially saturated 4 to 6 membered heterocyclic ring, an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
(ww') B is selected from a saturated or partially saturated 4 to 6 membered heterocyclic ring, an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
(ww") B is selected from a saturated or partially saturated 4 to 6 membered heterocyclic ring, or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
(xx) B is selected from cyclopentyl, cyclohexyl, piperidinyl, tetrahydropyranyl, phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazinyl, 2,3-dihydro-1,4-benzodioxinyl and 1,3-benzodioxol-5-yl;
(yy) B is selected from phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazinyl, 2,3-dihydro-1,4-benzodioxinyl and 1,3-benzodioxol-5-yl;
(yy') B is selected from phenyl, piperidinyl, pyridyl, pyrazolyl, isothiazolyl, thiadiazolyl, isoxazolyl, benzodioxolyl or tetrahydropyranyl.
(yy") B is selected from phenyl, 2,3-di-hydro-indenyl, piperidinyl, pyridyl, pyrazolyl, isothiazolyl, thiadiazolyl, isoxazolyl or benzodioxinyl;
(yy"') B is selected from phenyl, 2,3-di-hydro-indenyl, piperidinyl, pyridyl, pyrazolyl, isothiazolyl, thiadiazolyl, isoxazolyl, benzodioxinyl, benzodioxolyl or tetrahydropyranyl.
(zz) B is selected from phenyl, isoxazolyl, isothiazolyl, thiadiazolyl, pyridyl and pyrazolyl;
(aaa) Bis selected from phenyl, isoxazolyl, thiadiazolyl and pyrazolyl;
(aaa') B is selected from isoxazolyl, thiadiazolyl and pyrazolyl;
(aaa") B is selected from isoxazolyl and pyrazolyl;
(bbb) B is phenyl;
(ccc) B is isoxazolyl;
(ddd) B is pyrazolyl;
(eee) B is thiadiazolyl;
(fff) B is a (3-7C)cycloalkyl ring (particularly a (4-6C)cycloalkyl ring);
(ggg) B is a saturated or partially saturated 3 to 7 (particularly 4 to 6) membered heterocyclic ring that contains one or two heteroatoms (particularly one heteroatom) selected from oxygen and nitrogen;
(hhh) B is a 8, 9 or 10 membered bicyclic group which optionally contains 1, 2 or 3 (particularly 1 or 2) heteroatoms independently selected from N and O and which is saturated, partially saturated or aromatic;
(iii) A is phenyl;
   n is 0; and
   B is selected from a (4-6C)cycloalkyl ring, a saturated or partially saturated 4 to 6 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl or a 8, 9 or 10 membered bicyclic group which optionally contains 1, 2, 3 or 4 heteroatoms independently selected from N, O and S and which is saturated, partially saturated or aromatic;
(jjj) A is phenyl;
   n is 0; and
   B is selected from phenyl, isoxazolyl, thiadiazolyl and pyrazolyl;
(kkk) A is selected from phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl and 1,3,5-triazinyl;
   n is 0;
   L is attached meta on ring A with respect to the point of attachment of the ethynyl group and represents -N(R⁸)C(O)C(R^{a}R^{b})-, -N(R⁸)C(O)N(R⁹)- or -N(R⁸)C(O)O-, wherein R⁸ and R⁹ independently represent hydrogen or (1-6C)alkyl and wherein R⁸ and R^{b} independently represent hydrogen or (1-6C)alkyl or R^{a} and R^{b} together with the carbon atom to which they are attached represent (3-6C)cycloalkyl; and
   B is selected from phenyl, isoxazolyl, thiadiazolyl and pyrazolyl;
(III) A is selected from phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl and 1,3,5-triazinyl;
   n is 0;
   L is attached meta on ring A with respect to the point of attachment of the ethynyl group and represents -N(R⁸)C(O)C(R^{a}R^{b})- -N(R⁸)C(O)N(R⁹)- or N(R⁸)C(O)O-, wherein R⁸ and R⁹ independently represent hydrogen or (1-3C)alkyl and wherein R^{a} and R^{b} independently represent hydrogen or (1-3C)alkyl; and
   B is selected from phenyl, isoxazolyl, thiadiazolyl and pyrazolyl;
(mmm) m is 0, 1 or 2 (particularly 1 or 2);
(nnn) m is 1;
(ooo) m is 2;
(ppp) R⁶ is selected from halo, cyano, a (3-7C)cycloalkyl ring or -N(R^{c})C(O)(1-6C)alkyl in which R^{c} is hydrogen or (1-6C)alkyl; or R⁶ is selected from (1-6C)alkyl or (1-6C)alkoxy, wherein the (1-6C)alkyl and the (1-6C)alkoxy groups are optionally substituted by one or more groups (for example 1 or 2), which may be the same or different, selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring; (qqq) R⁶ is selected from halo, cyano, a (3-7C)cycloalkyl ring or -N(R^{c}C(O)(1-6C)alkyl in which R^{c} is hydrogen or (1-6C)alkyl; or R⁶ is selected from (1-6C)alkyl or (1-6C)alkoxy, wherein the (1-6C)alkyl and the (1-6C)alkoxy groups are optionally substituted by one or more groups (for example 1 or 2), which may be the same or different, selected from cyano, fluoro, hydroxy and amino (particularly fluoro);
(rrr) R⁶ is selected from halo, cyano, a (3-7C)cycloalkyl ring or -N(R^{c})C(O)(1-6C)alkyl in which R^{c} is hydrogen or (1-3C)alkyl; or R⁶ is selected from (1-4C)alkyl or (1-4C)alkoxy, wherein the (1-4C)alkyl and the (1-4C)alkoxy groups are optionally substituted by one or more groups (for example 1 or 2), which may be the same or different, selected from cyano, fluoro, hydroxy and amino (particularly fluoro);
(sss) R⁶ is selected from fluoro, chloro, cyano, acetylamino, methyl, ethyl, propyl, isopropyl, butyl, tert-butyl, trifluoromethyl, cyclopropyl, methoxy, ethoxy, propoxy and butoxy;
(sss') R⁶ is independently selected from (1-6C)alky or a (3-7C)cycloalkyl, particularily independently selected from (1-4C)alkyl or a (3-5C)cycloalkyl ring, more particularily independently selected from methyl, ethyl, propyl, isopropyl, see-butyl, tert-butyl, cyclopropyl or cyclobutyl.
(sss") R⁶ is independently selected from halo, cyano, oxo, (3-7C)cycloalkyl, a saturated 3 to 7 membered heterocyclic ring (optionally substituted by (1-4C)alkyl), -N(R^{c})C(O(1-6C)alkyl wherein R^{c} is hydrogen or (1-6C)alkyl (particularily (1-4C)alkyl), (1-6C)alkyl (optionally substituted by up to three groups independently selected from halo, particularily fluoro) or (1-6C)alkoxy wherein the saturated 3 to 7 membered heterocyclic ring is particularily selected from piperazinyl.
(sss"') R⁶ is independently selected from halo, trifluoromethyl, cyano, methyl, isopropyl, tert-butyl, methoxy, acetylamino, oxo, cyclopropyl or 4-methyl-piperazin-l-yl.
(ttt) B is selected from cyclopentyl, cyclohexyl, piperidinyl, tetrahydropyranyl, phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazinyl, 2,3-dihydro-1,4-benzodioxinyl and 1,3-benzodioxol-5-yl;
   m is 1 or 2; and
   R⁶ is independently selected from halo, cyano, a (3-7C)cycloalkyl ring or -N(R^{c})C(O)(1-6C)alkyl in which R^{c} is hydrogen or (1-6C)alkyl; or R⁶ is selected from (1-6C)alkyl or (1-6C)alkoxy, wherein the (1-6C)alkyl and the (1-6C)alkoxy groups are optionally substituted by one or more groups (for example 1 or 2), which may be the same or different, selected from cyano, fluoro, hydroxy and amino (particularly fluoro);
(uuu) B is selected from cyclopentyl, cyclohexyl, piperidinyl, tetrahydropyranyl, phenyl, furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazinyl, 2,3-dihydro-1,4-benzodioxinyl and 1,3-benzodioxol-5-yl;
   m is 1 or 2; and
   R⁶ is independently selected from fluoro, chloro, cyano, acetylamino, methyl, ethyl, propyl, isopropyl, butyl, tert-butyl, trifluoromethyl, cyclopropyl, methoxy, ethoxy, propoxy and butoxy;
(vvv) B is selected from phenyl, isoxazolyl, isothiazolyl, thiadiazolyl, pyrazolyl and pyridyl;
   m is 1 or 2; and
   R⁶ is independently selected from halo, cyano, a (3-7C)cycloalkyl ring or -N(R^{c})C(O)(1-6C)alkyl in which R^{c} is hydrogen or (1-6C)alkyl; or R⁶ is selected from (1-6C)alkyl or (1-6C)alkoxy, wherein the (1-6C)alkyl and the (1-6C)alkoxy groups are optionally substituted by one or more groups (for example 1 or 2), which may be the same or different, selected from cyano, fluoro, hydroxy and amino (particularly fluoro);
(www) B is selected from phenyl, isoxazolyl, isothiazolyl, thiadiazolyl, pyrazolyl and pyridyl;
   m is 1 or 2; and
   R⁶ is independently selected from fluoro, chloro, cyano, acetylamino, methyl, ethyl, propyl, isopropyl, butyl, tert-butyl, trifluoromethyl, cyclopropyl, methoxy, ethoxy, propoxy and butoxy;
(xxx) B is phenyl;
   m is 1 or 2; and
   R⁶ is independently selected from fluoro, chloro, cyano, acetylamino, trifluoromethyl, cyclopropyl, methoxy, ethoxy, propoxy and butoxy;
(yyy) B is phenyl;
   m is 1 or 2; and
   R⁶ is independently selected from fluoro and trifluoromethyl;
(zzz) B is isoxazolyl;
   m is 1 or 2; and
   R⁶ is independently selected from fluoro, chloro, cyano, acetylamino, methyl, ethyl, propyl, isopropyl, butyl, tert-butyl, trifluoromethyl, cyclopropyl, methoxy, ethoxy, propoxy and butoxy;
(aaaa) B is isoxazolyl;
   m is 1 or 2; and
   R⁶ is independently selected from methyl, ethyl, propyl, isopropyl, butyl, tert-butyl (particularly methyl and tert-butyl, more particularly tert-butyl);
(bbbb) B is pyrazolyl;
   m is 1 or 2; and
   R⁶ is independently selected from fluoro, chloro, cyano, acetylamino, methyl, ethyl, propyl, isopropyl, butyl, tert-butyl, trifluoromethyl, cyclopropyl, methoxy, ethoxy, propoxy and butoxy;
(bbbb') B is pyrazolyl;
   m is 1 or 2; and
   R⁶ is independently selected from methyl, ethyl, propyl, isopropyl, butyl, tert-butyl (particularly methyl and tert-butyl, more particularly tert-butyl);
(cccc) B is thiadiazolyl;
   m is 1 or 2; and
   R⁶ is independently selected from fluoro, chloro, cyano, acetylamino, methyl, ethyl, propyl, isopropyl, butyl, tert-butyl, trifluoromethyl, cyclopropyl, methoxy, ethoxy, propoxy and butoxy;
(cccc') B is thiadiazolyl;
   m is 1 or 2; and
   R⁶ is independently selected from methyl; ethyl, propyl, isopropyl, butyl, tert-butyl (particularly methyl and tert-butyl, more particularly tert-butyl);
(dddd) Ring B-R⁶ wherein m is 0, 1 or 2 is selected from cyclopropyl, cyclobutyl, cyclopentyl, cyclohexyl, cycloheptyl, phenyl, 2-fluorophenyl, 3-fluorophenyl, 4-fluorophenyl, 2,5-difluorophenyl, 3,4-difluorophenyl, 3,5-difluorophenyl, 2-(trifluoromethyl)phenyl, 3-(trifluoromethyl)phenyl, 4-(trifluoromethyl)phenyl, 2-fluoro-5-(trifluoromethyl)phenyl, 2-chlorophenyl, 3-chlorophenyl, 4-chlorophenyl, 2,5-dichlorophenyl, 3,4-dichlorophenyl, 3,5-dichlorophenyl, 2-methoxyphenyl, 3-methoxyphenyl, 4-methoxyphenyl, 2-cyanophenyl, 3-cyanophenyl, 4-cyanophenyl, 2-acetamidophenyl, 3-acetamidophenyl, 4-acetamidophenyl, 5-tert-butyl-1,3,4-thiadiazol-2-yl, 5-methyl-1,3,4-thiadiazol-2-yl, 5-ethyl-1,3,4-thiadiazol-2-yl, 5-isopropyl-1,3,4-thiadiazol-2-yl, 5-(trifluoromethyl)-1,3,4-thiadiazol-2-yl, 4-tert-butyl-1,3-thiazol-2-yl, 5-cyclopropyl-1,3,4-thiadiazol-2-yl, 1-methyl-3-cyclopropyl-pyrazol-5-yl, 1-tert-butyl-3-cyclopropyl-pyrazol-5-yl, 3-methylisothiazol-5-yl, 3-methylisoxazol-5-yl, 5-methylisoxazol-3-yl, 5-tert-butylisoxazol-3-yl, 4-(trifluoromethyl)pyridin-2-yl, 2-oxopiperidin-3-yl, 2,2-dimethyltetrahydro-2H-pyran-4-yl, 2,3-dihydro-1,4-benzodioxinyl and 1,3-benzodioxol-5-yl; and
(eeee) Ring B-R⁶ wherein m is 1 or 2 is selected from 2-fluorophenyl, 3-fluorophenyl, 4-fluorophenyl, 2,5-difluorophenyl, 3,4-difluorophenyl, 3,5-difluorophenyl, 2-(trifluoromethyl)phenyl, 3-(trifluoromethyl)phenyl, 4-(trifluoromethyl)phenyl, 2-fluoro-5-(trifluoromethyl)phenyl, 2-chlorophenyl, 3-chlorophenyl, 4-chlorophenyl, 2,5-dichlorophenyl, 3,4-dichlorophenyl, 3,5-dichlorophenyl, 2-methoxyphenyl, 3-methoxyphenyl, 4-methoxyphenyl, 2-cyanophenyl, 3-cyanophenyl, 4-cyanophenyl, 2-acetamidophenyl, 3-acetamidophenyl, 4-acetamidophenyl, 5-tert-butyl-1,3,4-thiadiazol-2-yl, 5-methyl-1,3,4-thiadiazol-2-yl, 5-ethyl-1,3,4-thiadiazol-2-yl, 5-isopropyl-1,3,4-thiadiazol-2-yl, 5-(trifluoromethyl)-1,3,4-thiadiazol-2-yl, 4-tert-butyl-thiazol-2-yl, 5-cyclopropyl-1,3,4-thiadiazol-2-yl, 1-methyl-3-cyclopropyl-pyrazol-5-yl, 1-tert-butyl-3-cyclopropyl-pyrazol-5-yl, 3-methylisothiazol-5-yl, 3-methylisoxazol-5-yl, 5-methylisoxazol-3-yl, 5-tert-butylisoxazol-3-yl; 4-(trifluoromethyl)pyridin-2-yl, 2-oxopiperidin-3-yl and 2,2-dimethyltetrahydro-2H-pyran-4-yl.

A particular embodiment of the compounds of the Formula I is a compound of the Formula Ia: 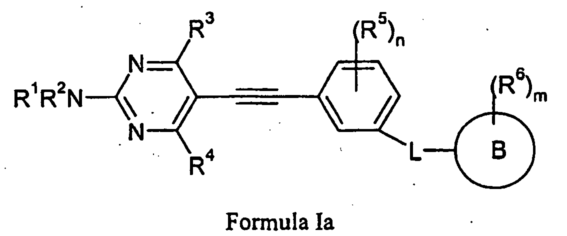 wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6, or a 5 or 6 membered heteroaryl ring, or **R¹ and R²** together with the nitrogen atom to which they are attached represent a saturated or partially saturated 3 to 7 membered heterocyclic ring optionally containing another hetero atom selected from N or O;
   wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or-N(R^{d}C(O)(1-6C)alkyl in which R^{d} is hydrogen or (1-6C)alkyl, or a saturated or partially saturated 3 to 7 membered heterocyclic ring or a 5 or 6 membered heteroaryl ring, wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or -N(R^{d})C(O)(1-6C)alkyl groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and the (1-6C)alkoxy groups are optionally substituted by one or more groups independently selected from hydroxy, amino, mono(1-6C)alkylamino or di-[(1-6C)alkyl]amino;
   and wherein any heterocyclic and heteroaryl rings within R¹ and/or R² are optionally independently substituted by one or more of the following: (1-4C)alkyl, (1-4C)alkoxy, (1-4C)alkoxy(1-4)alkyl, hydroxy, amino, mono(1-6C)alkylamino or di-[(1-6C)alkyl]amino, a saturated or partially saturated 3 to 7 membered heterocyclic ring, or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
**R³ and R⁴** are independently selected from hydrogen, (1-6C)alkyl or (1-6C)alkoxy,
   wherein the (1-6C)alkyl and the (1-6C)alkoxy groups are optionally substituted by one or more groups independently selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
   or one of **R³ and R⁴** is as defined above and the other represents a group **-NR¹R²** as defined above;
**R⁵** is selected from cyclopropyl, cyano, halo, (1-6C)alkoxy or (1-6C)alkyl, wherein the (1-6C)alkyl and (1-6C)alkoxy groups are optionally substituted by cyano or by one or more fluoro;
n is 0, 1, 2 or 3;
**L** represents -C(R^{a}R^{b}C(O)N(R⁹)-, -N(R⁸)C(O)C(R⁸R^{b})-, -N(R⁸)C(O)N(R⁹)-, -N(R⁸)C(O)O-, or -OC(O)-N(R⁹)-, wherein R⁸ and R⁹ independently represent hydrogen or (1-6C)alkyl and wherein R^{a} and R^{b} independently represent hydrogen or (1-6C)alkyl or R⁸ and R^{b} together with the carbon atom to which they are attached represent (3-6C)cycloalkyl;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
R⁶ is selected from one of the following 2 groups:
   (i) **R⁶** is selected from halo, cyano, oxo, a (3-7C)cycloalkyl ring, a saturated or partially saturated 3 to 7 membered heterocyclic ring and -N(R^{c})C(O)(1-6C)alkyl in which R^{c} is hydrogen or (1-6C)alkyl;
      or **R⁶** is selected from (1-6C)alkyl, -S(O)ₚ-(1-6C)alkyl wherein p is 0, 1 or 2, or (1-6C)alkoxy,
      wherein the (1-6C)alkyl, -S(O)ₚ-(1-6C)alkyl and the (1-6C)alkoxy groups are optionally substituted by one or more groups independently selected from: cyano, fluoro, hydroxy, (1-6C)alkoxy, amino, mono(1-6C)alkylamino, di(1-6C)alkylamino, a (3-7C)cycloalkyl ring or a saturated or partially saturated 3 to 7 membered heterocyclic ring; and
      wherein the (3-7C)cycloalkyl ring and saturated or partially saturated 3 to 7 membered heterocyclic ring are optionally independently substituted by one or more groups selected from (1-6C)alkyl;
   (ii) **R⁶** is selected from halo, cyano, a (3-7C)cycloalkyl ring, a saturated or partially saturated 3 to 7 membered heterocyclic ring or an -N(R^{c})C(O)(1-6C)alkyl in which R^{c} is hydrogen or (1-6C)alkyl; or
      **R⁶** is selected from (1-6C)alkyl or (1-6C)alkoxy, wherein the alkyl and the alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring; and
**m** is 0, 1, 2 or 3;
and when B is a (3-7C)cycloalkyl ring or a saturated or partially saturated 3 to 7 membered heterocyclic ring or a saturated or partially saturated 8, 9 or 10 membered bicyclic group, the rings and the bicyclic group optionally bears 1 or 2 oxo or thioxo substituents;
   and salts thereof, particularily pharmaceutically acceptable salts thereof.

Another particular embodiment of the compounds of the Formula I is a compound of the Formula Ib: 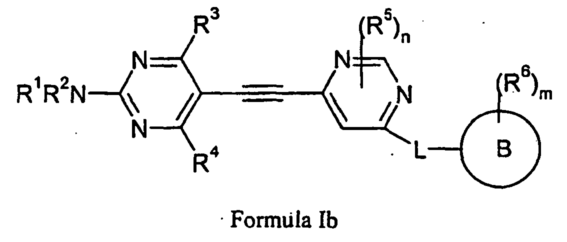 wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6, or a 5 or 6 membered heteroaryl ring, or **R¹ and R²** together with the nitrogen atom to which they are attached represent a saturated or partially saturated 3 to 7 membered heterocyclic ring optionally containing another hetero atom selected from N or O;
   wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or-N(R^{d})C(O)(1-6C)alkyl in which R^{d} is hydrogen or (1-6C)alkyl, or a saturated or partially saturated 3 to 7 membered heterocyclic ring or a 5 or 6 membered heteroaryl ring, wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or -N(R^{d})C(O)(1-6C)alkyl groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and the (1-6C)alkoxy groups are optionally substituted by one or more groups independently selected from hydroxy, amino, mono(1-6C)alkylamino or di-[(1-6C)alkyl]amino;
   and wherein any heterocyclic and heteroaryl rings within R¹ and/or R² are optionally independently substituted by one or more of the following: (1-4C)alkyl, (1-4C)alkoxy, (1-4C)alkoxy(1-4C)alkyl, hydroxy, amino, mono(1-6C)alkylamino or di-[(1-6C)alkyl]amino, a saturated or partially saturated 3 to 7 membered heterocyclic ring, or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
**R³ and R⁴** are independently selected from hydrogen, (1-6C)alkyl or (1-6C)alkoxy,
   wherein the (1-6C)alkyl and the (1-6C)alkoxy groups are optionally substituted by one or more groups independently selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
   or one of **R³ and R⁴** is as defined above and the other represents a group **-NR¹R²** as defined above;
**R⁵** is selected from cyclopropyl, cyano, halo, (1-6C)alkoxy or (1-6C)alkyl, wherein the (1-6C)alkyl and (1-6C)alkoxy groups are optionally substituted by cyano or by one or more fluoro;
**n** is 0, 1 or 2;
**L** represents -C(R^{a}R^{b})C(O)N(R⁹), -N(R⁸)C(O)C(R^{a}R^{b})-, -N(R⁸)C(O)N(R⁹)-, -N(R⁸)C(O)O-, or -OC(O)-N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
R⁶ is selected from one of the following 2 groups:
   (i) **R⁶** is selected from halo, cyano, oxo, a (3-7C)cycloalkyl ring, a saturated or partially saturated 3 to 7 membered heterocyclic ring and -N(R^{c})C(O)(1-6C)alkyl in which R^{c} is hydrogen or (1-6C)alkyl;
      or **R⁶** is selected from (1-6C)alkyl, -S(O)ₚ-(1-6C)alkyl wherein p is 0, 1 or 2, or (1-6C)alkoxy,
      wherein the (1-6C)alkyl, -S(O)ₚ-(1-6C)alkyl and the (1-6C)alkoxy groups are optionally substituted by one or more groups independently selected from: cyano, fluoro, hydroxy, (1-6C)alkoxy, amino, mono(1-6C)alkylamino, di(1-6C)alkylamino, a (3-7C)cycloalkyl ring or a saturated or partially saturated 3 to 7 membered heterocyclic ring; and
      wherein the (3-7C)cycloalkyl ring and saturated or partially saturated 3 to 7 membered heterocyclic ring are optionally independently substituted by one or more groups selected from (1-6C)alkyl;
   (ii) **R⁶** is selected from halo, cyano, a (3-7C)cycloalkyl ring, a saturated or partially saturated 3 to 7 membered heterocyclic ring or -N(R^{c})C(O)(1-6C)alkyl in which R^{c} is hydrogen or (1-6C)alkyl; or
      **R⁶** is selected from (1-6C)alkyl or (1-6C)alkoxy, wherein the alkyl and the alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring; and
**m** is 0, 1, 2 or 3;
and when B is a (3-7C)cycloalkyl ring or a saturated or partially saturated 3 to 7 membered heterocyclic ring or a saturated or partially saturated 8, 9 or 10 membered bicyclic group, the rings and the bicyclic group optionally bears 1 or 2 oxo or thioxo substituents;
   and salts thereof, particularily pharmaceutically acceptable salts thereof.

Another particular embodiment of the compounds of the Formula I is a compound of the Formula Ic: 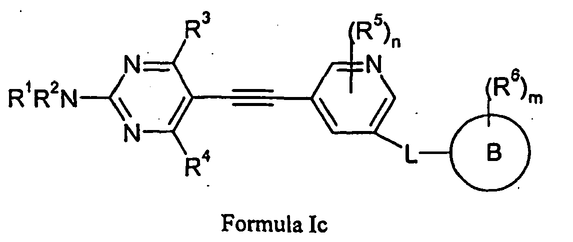 wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6, or a 5 or 6 membered heteroaryl ring, or **R¹ and R²** together with the nitrogen atom to which they are attached represent a saturated or partially saturated 3 to 7 membered heterocyclic ring optionally containing another hetero atom selected from N or O;
   wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or-N(R^{d})C(O)(1-6C)alkyl in which R^{d} is hydrogen or (1-6C)alkyl, or a saturated or partially saturated 3 to 7 membered heterocyclic ring or a 5 or 6 membered heteroaryl ring, wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or N(R^{d})C(O)(1-6C)alkyl groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and the (1-6C)alkoxy groups are optionally substituted by one or more groups independently selected from hydroxy, amino, mono(1-6C)alkylamino or di-[(1-6C)alkyl]amino;
   and wherein any heterocyclic and heteroaryl rings within R¹ and/or R² are optionally independently substituted by one or more of the following: (1-4C)alkyl, (1-4C)alkoxy, (1-4C)alkoxy(1-4C)alkyl, hydroxy, amino, mono(1-6C)alkylamino or di-[(1-6C)alkyl]amino, a saturated or partially saturated 3 to 7 membered heterocyclic ring, or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
**R³ and R⁴** are independently selected from hydrogen, (1-6C)alkyl or (1-6C)alkoxy,
   wherein the (1-6C)alkyl and the (1-6C)alkoxy groups are optionally substituted by one or more groups independently selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
   or one of **R³ and R⁴** is as defined above and the other represents a group **-NR¹R²** as defined above;
**R⁵** is selected from cyclopropyl, cyano, halo, (1-6C)alkoxy or (1-6C)alkyl, wherein the (1-6C)alkyl and (1-6C)alkoxy groups are optionally substituted by cyano or by one or more fluoro;
**n** is 0, 1, 2 or 3;
**L** represents -C(R^{a}R^{b})C(O)N(R⁹)-, -N(R⁸)C(O)C(R^{a}R^{b})-, -N(R⁸)C(O)N(R⁹)-, -N(R⁸)C(O)O-, or -OC(O)-N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
R⁶ is selected from one of the following 2 groups:
   (i) **R⁶** is selected from halo, cyano, oxo, a (3-7C)cycloalkyl ring, a saturated or partially saturated 3 to 7 membered heterocyclic ring and N(R^{c})C(O)(1-6C)alkyl in which R^{c} is hydrogen or (1-6C)alkyl;
      or **R⁶** is selected from (1-6C)alkyl, -S(O)ₚ-(1-6C)alkyl wherein p is 0, 1 or 2, or (1-6C)alkoxy,
      wherein the (1-6C)alkyl, -S(O)ₚ-(1-6C)alkyl and the (1-6C)alkoxy groups are optionally substituted by one or more groups independently selected from: cyano, fluoro, hydroxy, (1-6C)alkoxy, amino, mono(1-6C)alkylamino, di(1-6C)alkylamino, a (3-7C)cycloalkyl ring or a saturated or partially saturated 3 to 7 membered heterocyclic ring; and wherein the (3-7C)cycloalkyl ring and saturated or partially saturated 3 to 7 membered heterocyclic ring are optionally independently substituted by one or more groups selected from (1-6C)alkyl;
   (ii) **R⁶** is selected from halo, cyano, a (3-7C)cycloalkyl ring, a saturated or partially saturated 3 to 7 membered heterocyclic ring or -N(R^{c})C(O)(1-6C)alkyl in which R^{c} is hydrogen or (1-6C)alkyl; or
      **R⁶** is selected from (1-6C)alkyl or (1-6C)alkoxy, wherein the alkyl and the alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring; and
**m** is 0, 1, 2 or 3;
and when B is a (3-7C)cycloalkyl ring or a saturated or partially saturated 3 to 7 membered heterocyclic ring or a saturated or partially saturated 8, 9 or 10 membered bicyclic group, the rings and the bicyclic group optionally bears 1 or 2 oxo or thioxo substituents;
   and salts thereof, particularily pharmaceutically acceptable salts thereof.

Another particular embodiment of the compounds of the Formula I is a compound of the Formula Id: 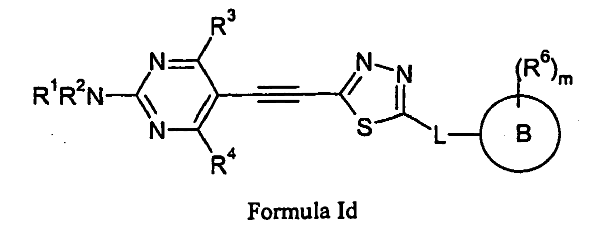 wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6, or a 5 or 6 membered heteroaryl ring, or **R¹ and R²** together with the nitrogen atom to which they are attached represent a saturated or partially saturated 3 to 7 membered heterocyclic ring optionally containing another hetero atom selected from N or O;
   wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl or-N(R^{d})C(O)(1-6C)alkyl in which R^{d} is hydrogen or (1-6C)alkyl, or a saturated or partially saturated 3 to 7 membered heterocyclic ring or a 5 or 6 membered heteroaryl ring, wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or N(R^{d})C(O)(1-6C)alkyl groups are optionally substituted by one or more hydroxy groups;
   wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and the (1-6C)alkoxy groups are optionally substituted by one or more groups independently selected from hydroxy, amino, mono(1-6C)alkylamino or di-[(1-6C)alkyl]amino;
   and wherein any heterocyclic and heteroaryl rings within R¹ and/or R² are optionally independently substituted by one or more of the following: (1-4C)alkyl, (1-4C)alkoxy, (1-4C)alkoxy(1-4C)alkyl, hydroxy, amino, mono(1-6C)alkylamino or di-[(1-6C)alkyl]amino, a saturated or partially saturated 3 to 7 membered heterocyclic ring, or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
   and provided that when R¹ and/or R² is a (1C)alkanoyl group, then the (1C)alkanoyl is not substituted by fluoro or hydroxy;
**R³ and R⁴** are independently selected from hydrogen, (1-6C)alkyl or (1-6C)alkoxy,
   wherein the (1-6C)alkyl and the (1-6C)alkoxy groups are optionally substituted by one or more groups independently selected from fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, (1-4C)alkoxy, hydroxy, amino, mono(1-6C)alkylamino or di-[(1-6C)alkyl]amino or a saturated or partially saturated 3 to 7 membered heterocyclic ring;
   or one **of R³** and **R⁴** is as defined above and the other represents a group **-NR¹R²** as defined above;
**L** represents -C(R^{a}R^{b})C(O)N(R⁹), -N(R⁸)C(O)C(R^{a}R^{b})-, -N(R⁸)C(O)N(R⁹)-, -N(R⁸)C(O)O-, or -OC(O)-N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, **O** and S and which is saturated, partially saturated or aromatic;
**R⁶** is selected from one of the following 2 groups:
   (i) **R⁶** is selected from halo, cyano, oxo, a (3-7C)cycloalkyl ring, a saturated or partially saturated 3 to 7 membered heterocyclic ring and N(R^{c})C(O)(1-6C)alkyl in which R^{c} is hydrogen or (1-6C)alkyl;
      or **R⁶** is selected from (1-6C)alkyl, -S(O)ₚ-(1-6C)alkyl wherein p is 0, 1 or 2, or (1-6C)alkoxy,
      wherein the (1-6C)alkyl, -S(O)ₚ-(1-6C)alkyl and the (1-6C)alkoxy groups are optionally substituted by one or more groups independently selected from: cyano, fluoro, hydroxy, (1-6C)alkoxy, amino, mono(I-6C)alkylamino, di(1-6C)alkylamino, a (3-7C)cycloalkyl ring or a saturated or partially saturated 3 to 7 membered heterocyclic ring; and
      wherein the (3-7C)cycloalkyl ring and saturated or partially saturated 3 to 7 membered heterocyclic ring are optionally independently substituted by one or more groups selected from (1-6C)alkyl;
   (ii) **R⁶** is selected from halo, cyano, a (3-7C)cycloalkyl ring, a saturated or partially saturated 3 to 7 membered heterocyclic ring or -N(R^{c})C(O)(1-6C)alkyl in which R^{c} is hydrogen or (1-6C)alkyl; or
      **R⁶** is selected from (1-6C)alkyl or (1-6C)alkoxy, wherein the alkyl and the alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring; and
**m** is 0, 1, 2 or 3;
and when B is a (3-7C)cycloalkyl ring or a saturated or partially saturated 3 to 7 membered heterocyclic ring or a saturated or partially saturated 8, 9 or 10 membered bicyclic group, the rings and the bicyclic group optionally bears 1 or 2 oxo or thioxo substituents;
   and salts thereof, particularily pharmaceutically acceptable salts thereof.

Specific compounds of the present invention are one or more of the following:
*N*-{3-[(2-aminopyrimidin-5-yl)ethynyl]phenyl}-*N*'-[2-fluoro-5-(trifluoromethyl)phenyl]urea
N-{3-[(2-aminopyrimidin-5-yl)ethynyl]phenyl}-N'-[2-(trifluoromethyl)phenyl]urea
N-{3-[(2-aminopyrimidin-5-yl)ethynyl]phenyl}-N'-[4-(trifluoromethyl)phenyl]urea
N-{3-[(2-aminopyrimidin-5-yl)ethynyl]phenyl}-N'-(2-fluorophenyl)urea
N-{3-[(2-aminopyrimidin-5-yl)ethynyl]phenyl}-N'-(3-fluorophenyl)urea
N-{3-[(2-aminopyrimidin-5-yl)ethynyl]phenyl} N'-(4-fluorophenyl)urea
N-{3-[(2-aminopyrimidin-5-yl)ethynyl]phenyl}-N'-(3-methoxyphenyl)urea
N-{3-[(2-aminopyrimidin-5-yl)ethynyl]phenyl}-N'-(2,5-difluorophenyl)urea
N-{3-[(2-aminopyrimidin-5-yl)ethynyl]phenyl}-N'-1,3-benzodioxol-5-ylurea
N-{3-[(2-aminopyrimidin-5-yl)ethynyl]phenyl}-N'-[3-(trifluoromethyl)phenyl]urea
N-{3-[(2-aminopyrimidin-5-yl)ethynyl]phenyl}-N'-(2-methoxyphenyl)urea
N-{3-[(2-aminopyrimidin-5-yl)ethynyl]phenyl}-N'-(4-methoxyphenyl)urea
N-{3-[(2-aminopyrimidin-5-yl)ethynyl]phenyl}-N'-(3,4-difluorophenyl)urea
N-{3-[(2-aminopyrimidin-5-yl)ethynyl]phenyl}-N'-(3-cyanophenyl)urea
N-{3-[(2-aminopyrimidin-5-yl)ethynyl]phenyl}-N'-(3-chlorophenyl)urea
N-{3-[(2-aminopyrimidin-5-yl)ethynyl]phenyl}-N'-cyclopentylurea
N-{3-[(2-aminopyrimidin-5-yl)ethynyl]phenyl}-N'-(3,5-difluorophenyl)urea
N-{3-[(2-aminopyrimidin-5-yl)ethynyl]phenyl} N'-(5-tert-butyl-1,3,4-thiadiazol-2-yl)urea
N-{3-[(2-aminopyrimidin-5-yl)ethynyl]phenyl}-N'-(3-methylisoxazol-5-yl)urea
N-(3-{[({3-[(2-aminopyrimidin-5-yl)ethynyl]phenyl}amino)carbonyl]amino}phenyl) cetamide
N-{3-[(2-aminopyrimidin-5-yl)ethynyl]phenyl}-N'-[4-(trifluoromethyl)pyridin-2-yl]urea
N-{3-[(2-aminopyrimidin-5-yl)ethynyl]phenyl}-N'-(5-tert-butylisoxazol-3-yl)urea
Phenyl {3-[(2-aminopyrimidin-5-yl)ethynyl]phenyl}carbamate
N-{3-[(2-aminopyrimidin-5-yl)ethynyl]phenyl}-N'-(2-oxopiperidin-3-yl)urea
N-(5-tert-butylisoxazol-3-yl)-N'-(3-{[2-(methylamino)pyrimidin-5-yl]ethynyl}phenyl)urea
N-(5-tert-butylisoxazol-3-yl)-N'-(3-{[2-(dimethylamino)pyrimidin-5-yl]ethynyl}phenyl)urea
N-(5-tert-butylisoxazol-3-yl)-N'-[3-({2-[(2-morpholin-4-ylethyl)amino]pyrimidin-5-yl}ethynyl)phenyl]urea
N-(5-tert-butylisoxazol-3-yl)-N'-[3-({2-[(3-morpholin-4-ylpropyl)amino]pyrimidin-5-yl}ethynyl)phenyl]urea
N-(5-tert-butylisoxazol-3-yl)-N'-[3-({2-[(2-methoxyethyl)amino]pyrimidin-5-yl}ethynyl)phenyl]urea
N-(5-tert-butylisoxazol-3-yl)-N'-{3-[(2-{[3-(1H-imidazol-1-yl)propyl]amino}pyrimidin-5-yl)ethynyl]phenyl}urea
N-(5-tert-butylisoxazol-3-yl)-N'-[3-({2-[(3-methoxypropyl)amino]pyrimidin-5-yl}ethynyl)phenyl]urea
N-(5-tert-butylisoxazol-3-yl)-N'-[3-({2-[(2-hydroxyethyl)amino]pyrimidin-5-yl}ethynyl)phenyl]urea
N-(S-tert-butylisoxazol-3-yl)-N'-[3-({2-[(2-pyrrolidin-1-ylethyl)amino]pyrimidin-5-yl}ethynyl)phenyl]urea
N-(5-tert-butylisoxazol-3-yl)-N'-[3-({2-[(3-pyrrolidin-1-ylpropyl)amino]pyrimidin-5-yl}ethynyl)phenyl]urea
N-[3-({2-[(2-aminoethyl)amino]pyrimidin-5-yl}ethynyl)phenyl]-N'-(5-tert-butylisoxazol-3-yl)urea
N-[3-({2-[(3-aminopropyl)amino]pyrimidin-5-yl}ethynyl)phenyl]-N'-(5-tert-butylisoxazol-3-yl)urea
N-(5-tert-butylisoxazol-3-yl)-N'-{3-[(2-{ [2-(dimethylamino)ethyl]amino}pyrimidin-5-yl)ethynyl]phenyl}urea
N-(5-tert-butylisoxazol-3-yl)-N'-{3-[(2-{[3-(dimethylamino)propyl]amino}pyrimidin-5-yl)ethynyl]phenyl}urea
N-2-(5-{[3-({[(5-tert-butylisoxazol-3-yl)amino]carbonyl}amino)phenyl]ethynyl}pyrimidin-2-yl)glycinamide
N-3-(5-{[3-({[(5-tert-butylisoxazol-3-yl)amino]carbonyl}amino)phenyl]ethynyl}pyrimidin-2-yl)-beta-alaninamide
N-(5-tert-butylisoxazol-3-yl)-N'-{3-[(2-{[2-(1H-imidazol-4-yl)ethyl]amino}pyrimidin-5-yl)ethynyl]phenyl}urea
N-(5-tert-butylisoxazol-3-yl)-N'-[3-({2-[(2-pyridin-2-ylethyl)amino]pyrimidin-5-yl}ethynyl)phenyl]urea
N-(5-tert-butylisoxazol-3-yl)-N'-{3-[(2-{[3-(isopropylamino)propyl]amino}pyrimidin-5-yl)ethynyl]phenyl}urea
N-(5-tert-butylisoxazol-3-yl)-N'-{3-[(2-{[3-(4-methylpiperazin-1-yl)propyl]amino}pyrimidin-5-yl)ethynyl]phenyl}urea
N-(5-tert-butylisoxazol-3-yl)-N'-[3-({2-[(2-pyridin-4-ylethyl)amino]pyrimidin-5-yl}ethynyl)phenyl]urea
N-(5-tert-butylisoxazol-3-yl)-N'-[3-({2-[(3-piperidin-1-ylpropyl)amino]pyrimidin-5-yl}ethynyl)phenyl]urea
N-(5-methylisoxazol-3-yl)-N'-[3-({2-[(2-pyrrolidin-1-ylethyl)amino]pyrimidin-5-yl}ethynyl)phenyl]urea
N-(5-tert-butyl-1,3,4-thiadiazol-2-yl)-N'-[3-({2-[(2-pyrrolidin-1-ylethyl)amino]pyrimidin-5-yl}ethynyl)phenyl]urea
N-(3-methylisothiazol-5-yl)-N'-[3-({2-[(2-pyrrolidin-1-ylethyl)amino]pyrimidin-5-yl}ethynyl)phenyl]urea
N-(3-fluorophenyl)-N'-[3-({2-[(2-pyrrolidin-1-ylethyl)amino]pyrimidin-5-yl}ethynyl)phenyl]urea
N-(4-methoxyphenyl)-N'-[3-({2-[(2-pyrrolidin-1-ylethyl)amino]pyrimidin-5-yl}ethynyl)phenyl]urea
N-(2-fluorophenyl)-N'-[3-({2-[(2-pyrrolidin-1-ylethyl)amino]pyrimidin-S-yl}ethynyl)phenyl]urea
N-(2,5-difluorophenyl)-N'-[3-({2-[(2-pyrrolidin-1-ylethyl)amino]pyrimidin-5-yl}ethynyl)phenyl]urea
N-(3,4-difluorophenyl)-N'-[3-({2-[(2-pyrrolidin-1-ylethyl)amino]pyrimidin-5-yl}ethynyl)phenyl]urea
N-[2-fluoro-5-(trifluoromethyl)phenyl]-N'-[3-({2-[(2-pyrrolidin-1-ylethyl)amino]pyrimidin-5-yl}ethynyl)phenyl]urea
N-[3-({2-[(2-pyrrolidin-1-ylethyl)amino]pyrimidin-5-yl}ethynyl)phenyl]-N'-[4-(trifluoromethyl)phenyl]urea
N-1,3-benzodioxol-5-yl-N'-[3-({2-[(2-pyrrolidin-1-ylethyl)amino]pyrimidin-5-yl}ethynyl)phenyl]urea
N-(4-fluorophenyl)-N'-[3-({2-[(2-pyrrolidin-1-ylethyl)amino]pyrimidin-5-yl}ethynyl)phenyl]urea
N-(3-chlorophenyl)-N'-[3-({2-[(2-pyrrolidin-1-ylethyl)amino]pyrimidin-5-yl}ethynyl)phenyl]urea
N-(5-methylisoxazol-3-yl)-N'-[3-({2-[(2-morpholin-4-ylethyl)amino]pyrimidin-5-yl}ethynyl)phenyl]urea
N-(5-tert-butyl-1,3,4-thiadiazol-2-yl)-N'-[3-({2-[(2-morpholin-4-ylethyl)amino]pyrimidin-5-yl}ethynyl)phenyl]urea
N-[2-fluoro-5-(trifluoromethyl)phenyl]-N'-[3-({2-[(2-morpholin-4-ylethyl)amino]pyrimidin-5-yl}ethynyl)phenyl]urea
N-(5-methylisoxazol-3-yl)-N'-[3-({2-[(3-morpholin-4-ylpropyl)amino]pyrimidin-5-yl}ethynyl)phenyl]urea
N-(5-tert-butyl-1,3,4-thiadiazol-2-yl)-N'-[3-({2-[(3-morpholin-4-ylpropyl)amino]pyrimidin-5-yl}ethynyl)phenyl]urea
N-[2-fluoro-5-(trifluoromethyl)phenyl]-N'-[3-({2-[(3-morpholin-4-ylpropyl)amino]pyrimidin-5-yl}ethynyl)phenyl]urea
N-(5-methylisoxazol-3-yl)-N'-[4-({2-[(2-pyrrolidin-1-ylethyl)amino]pyrimidin-5-yl}ethynyl)phenyl]urea
N-(5-tert-butylisoxazol-3-yl)-N'-[4({2-[(2-pyrrolidin-1-ylethyl)amino]pyrimidin-5-yl}ethynyl)phenyl]urea
N-(5-tert-butyl-1,3,4-thiadiazol-2-yl)-N'-[4-({2-[(2-pyrrolidin-1-ylethyl)amino]pyrimidin-5-yl}ethynyl)phenyl]urea
N-[2-fluoro-5-(trifluoromethyl)phenyl]-N'-[4-({2-[(2-pyrrolidin-1-ylethyl)amino]pyrimidin-5-yl}ethynyl)phenyl]urea
N-(5-{[3-({[(5-tert-butylisoxazol-3-yl)amino]carbonyl}amino)phenyl]ethynyl}pyrimidin-2-yl)-2-(2-methoxyethoxy)acetamide
N-{6-[(2-aminopyrimidin-5-yl)ethynyl]pyridin-2-yl}-N'-(5-tert-butylisoxazol-3-yl)urea
N-{2-[(2-aminopyrimidin-5-yl)ethynyl]pyridin-4-yl}-N'-(5-tert-butylisoxazol-3-yl)urea
N-{5-[(2-aminopyrimidin-5-yl)ethynyl]-1,3-thiazol-2-yl}-N'-[2-fluoro-5-(trifluoromethyl)phenyl]urea
N-{5-[(2-aminopyrimidin-5-yl)ethynyl]-1,3,4-thiadiazol-2-yl}-N'-[2-fluoro-5-(trifluoromethyl)phenyl]urea
*N*-{5-[(2-aminopyrimidin-5-yl)ethynyl]-1,3-thiazol-2-yl}-*N*'-(5-*tert*-butylisoxazol-3-yl)urea
N-{3-[(2-aminopyrimidin-5-yl)ethynyl]phenyl}-2-(2-methoxyphenyl)acetamide
N-{3-[(2-aminopyrimidin-5-yl)ethynyl]phenyl}-2-phenylacetamide
N-{3-[(2-aminopyrimidin-5-yl)ethynyl]phenyl}-2-(3-methoxyphenyl)acetamide
N-{3-[(2-aminopyrimidin-5-yl)ethynyl]phenyl}-2-[3-(trifluoromethyl)phenyl]acetamide
N-{3-[(2-aminopyrimidin-5-yl)ethynyl]phenyl}-2-[4-(trifluoromethyl)phenyl]acetamide
N-{3-[(2-aminopyrimidin-5-yl)ethynyl]phenyl}-2-(3-methylisoxazol-5-yl)acetamide
N-{4-[(2-aminopyrimidin-5-yl)ethynyl]phenyl}-2-(2-methoxyphenyl)acetamide
N-{4-[(2-aminopyrimidin-5-yl)ethynyl]phenyl}-2-(3-methylisoxazol-5-yl)acetamide
N-{3-[(2-aminopyrimidin-5-yl)ethynyl]phenyl}-N'-(2,2-dimethyltetrahydro-2H-pyran-4-yl)urea
N-{6-[(2-aminopyrimidin-5-yl)ethynyl]pyrimidin-4-yl}-N'-(5-tert-butylisoxazol-3-yl)urea
N'-{3-[(2-aminopyrimidin-5-yl)ethynyl]phenyl}-N-(5-tert-butylisoxazol-3-yl)-N-methylurea and salts thereof, particularity pharmaceutically acceptable salts thereof.

Further specific compounds of the present invention are one or more of the following:
N-{3-[(2-aminopyrimidin-5-yl)ethynyl]phenyl}-N'-(1-tert-butyl-3-cyclopropyl-1H-pyrazol-5-yl)urea
N-{3-[(2-aminopyrimidin-5-yl)ethynyl]phenyl}-N'-(5-methylisoxazol-3-yl)urea
N-{5-[(2-aminopyrimidin-5-yl)ethynyl]pyridin-3-yl}-N'-(5-tert-butylisoxazol-3-yl)urea
N-[3-({2-[(3-piperidin-1-ylpropyl)amino]pyrimidin-5-yl}ethynyl)phenyl]-N'-[4-(trifluoromethyl)pyridin-2-yl]urea
N-(5-tert-butyl-1,3,4-thiadiazol-2-yl)-N'-[3-({2-[(3-piperidin-1-ylpropyl)amino]pyrimidin-5-yl}ethynyl)phenyl]urea
N-(3-methylisoxazol-5-yl)-N'-[3₋({2-[(3-piperidin-1-ylpropyl)amino]pyrimidin-5-yl}ethynyl)phenyl]urea
N-(2-methoxyphenyl)-N'-[3-({2-[(3-piperidin-1-ylpropyl)amino]pyrimidin-5-yl}ethynyl)phenyl]urea
N-(3-fluorophenyl)-N'-[3-({2-[(3-piperidin-1-ylpropyl)amino]pyrimidin-5-yl}ethynyl)phenyl]urea
N'-(4-[(2-aminopyrimidin-5-yl)ethynyl]phenyl)-N-(5-tert-butylisoxazol-3-yl)-N-methylurea
N-{3-[(2-aminopyrimidin-5-yl)ethynyl]phenyl}-N'-(3-cyclopropyl-1-methyl-1H-pyrazol-5-yl)urea
N-{3-[(2-aminopyrimidin-5-yl)ethynyl]phenyl}-N'-phenylurea
N-[3-({2-[(4-aminobutyl)amino]pyrimidin-5-yl}ethynyl)phenyl]-N'-(5-tert-butylisoxazol-3-yl)urea
N-(5-tert-butylisoxazol-3-yl)-N'-[3-({2-[(2-piperidin-1-ylethyl)amino]pyrimidin-5-yl}ethynyl)phenyl]urea
N-(5-tert-butylisoxazol-3-yl)-N'-{3-[(2-{[2-(isopropylamino)ethyl]amino}pyrimidin-5-yl)ethynyl]phenyl} urea
N-(5-tert-butylisoxazol-3-yl)-N'-{3-[(2-{[2-(2-hydroxyethoxy)ethyl]amino}pyrimidin-5-yl)ethynyl]phenyl}urea
N-{3-[(2-aminopyrimidin-5-yl)ethynyl]phenyl}-N'-(5-cyclopropyl-1,3,4-thiadiazol-2-yl)urea
N-{3-[(2-aminopyrimidin-5-yl)ethynyl]phenyl}-N'-[5-(trifluoromethyl)-1,3,4-thiadiazol-2-yl]urea
N-{2-[(5-{[3-({[(5-tert-butylisoxazol-3-yl)amino]carbonyl}amino)phenyl]ethynyl}pyrimidin-2-yl)amino]ethyl}-2-hydroxyacetamide
N-(5-tert-butylisoxazol-3-yl)-N'-{3-[(2-{[4-(dimethylamino)butyl]amino}pyrimidin-5-yl)ethynyl]phenyl}urea
N'-{3-[(2-aminopyrimidin-5-yl)ethynyl]phenyl}-N-methyl-N-[5-(trifluoromethyl)-1,3,4-thiadiazol-2-yl]urea
N-phenyl-N'-[3-({2-[(3-piperidin-1-ylpropyl)amino]pyrimidin-5-yl}ethynyl)phenyl]urea
N-(5-methylisoxazol-3-yl)-N'-[3-({2-[(3-piperidin-1-ylpropyl)amino]pyrimidin-5-yl}ethynyl)phenyl]urea
N-{3-[(2-aminopyrimidin-5-yl)ethynyl]phenyl}-N'-(5-tert-butylisoxazol-3-yl)-N-methylurea
N-(5-tert-butylisoxazol-3-yl)-N'-{3-[(2-{[2-(dimethylamino)-1-methylethyl]amino}pyrimidin-5-yl)ethynyl]phenyl}urea
phenyl {3-[(2-{[3-(dimethylamino)propyl]amino}pyrimidin-5-yl)ethynyl]phenyl}carbamate
N-{3-[(2-aminopyrimidin-5-yl)ethynyl]phenyl}-N'-(3-tert-butyl-1-methyl-1H-pyrazol-5-yl)urea
N-{3-[(2-aminopyrimidin-5-yl)ethynyl]phenyl}-N'-(4-tert-butyl-1,3-thiazol-2-yl)urea
N-{3-[(2-aminopyrimidin-5-yl)ethynyl]phenyl}-N'-(5-methyl-1,3,4-thiadiazol-2-yl)urea
N-{3-[(2-aminopyrimidin-5-yl)ethynyl]phenyl}-N'-(5-ethyl-1,3,4-thiadiazol-2-yl)urea
N-{3-[(2-aminopyrimidin-5-yl)ethynyl]phenyl}-N'-(5-isopropyl-1,3,4-thiadiazol-2-yl)urea
N-{3-[(2-{[3-(dimethylamino)propyl]amino}pyrimidin-5-yl)ethynyl]phenyl}-N'-(5-methylisoxazol-3-yl)urea
N-{3-[(2-{[3-(dimethylamino)propyl]amino}pyrimidin-5-yl)ethynyl]phenyl}-N'-phenylurea
N-{3-[(2-aminopyrimidin-5-yl)ethynyl]phenyl}-N'-(5-tert-butylisoxazol-3-yl)urea;
N-{5-[(2-aminopyrimidin-5-yl)ethynyl]pyridin-3-yl}-N'-(5-tert-butylisoxazol-3-yl)urea;
N-{3-[(2-aminopyrimidin-5-yl)ethynyl]phenyl}-N'-(3-tert-butyl-1-methyl-1H-pyrazol-5-yl)urea;
N-(3-tert-butyl-1-methyl-1H-pyrazol-5-yl)-N'-[3-({2-[(3-morpholin-4-ylpropyl)amino]pyrimidin-5-yl}ethynyl)phenyl]urea;
N-(3-tert-butyl-1-methyl-1H-pyrazol-5-yl)-N'-[3-({2-[(2-morpholin-4-ylethyl)amino]pyrimidin-5-yl}ethynyl)phenyl]urea;
and salts thereof, particularily pharmaceutically acceptable salts thereof.
A compound of the Formula I, or a pharmaceutically-acceptable salt thereof, may be prepared by any process known to be applicable to the preparation of chemically-related compounds. Such processes, when used to prepare a compound of the Formula I are provided as a further feature of the invention and are illustrated by the following representative process variants. Necessary starting materials may be obtained by standard procedures of organic chemistry. The preparation of such starting materials is described in conjunction with the following representative process variants and within the accompanying Examples. Alternatively necessary starting materials are obtainable by analogous procedures to those illustrated which are within the ordinary skill of an organic chemist.

According to a further aspect of the present invention provides a process for preparing a compound of formula I or a pharmaceutically acceptable salt thereof (wherein R¹, R², R³, R⁴, R⁵, R⁶, R⁷, R⁸, R⁹, R¹⁰, R¹¹ L , ring A and ring B, n and m are, unless otherwise specified, as defined in formula I) as described schematically below.
Process (a) For compounds of the formula I wherein L is -N(R⁸)C(O)N(H)-, the reaction of a compound of the formula II: 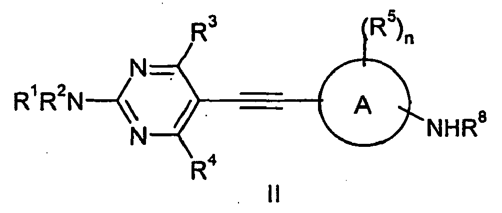 wherein R¹, R², R³, R⁴, R⁵, R⁸, n and A have any of the meanings defined hereinbefore except that any functional group is protected if necessary, with an isocyanate of the formula IV: 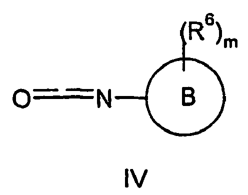 wherein R⁶, m and B have any of the meanings defined hereinbefore except that any functional group is protected if necessary;
or
Process(b) For compounds of the formula I wherein L is -N(R⁸)C(O)N(H)-, the reaction of a compound of the formula II as defined above with an aryl carbamate of the formula III: 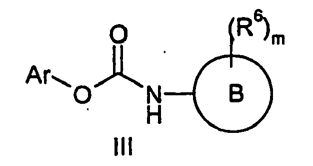 wherein Ar is a suitable aryl group, for example phenyl, and R⁶, m and B have any of the meanings defined hereinbefore except that any functional group is protected if necessary; or
Process (c) For compounds of the formula I wherein L is N(R⁸)C(O)-O-, the reaction of a compound of the formula II as defined above with a compound of the formula XI: 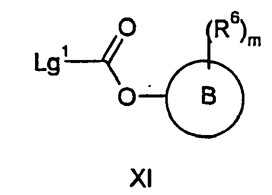 wherein Lg¹ is a suitable displaceable group for example halogeno (such as fluoro, chloro or bromo) and R⁶, m and B have any of the meanings defined hereinbefore except that any functional group is protected if necessary;
or
Process (d) For compounds of the formula I wherein L is N(R⁸)C(O)C(R^{a}R^{b}), the reaction of a compound of the formula II as defined above with a compound of the formula IX: 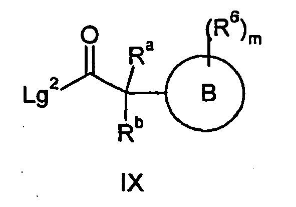 wherein Lg² is a suitable displaceable group for example hydroxy, halogeno (such as fluoro, chloro or bromo), R^{x}-C(O)-O- or R^{x}-O- (wherein R^{x} is a suitable alkyl or aryl group) and R⁶, R^{a}, R^{b}, m and B have any of the meanings defined hereinbefore except that any functional group is protected if necessary;
or
Process(e) For compounds of the formula I wherein L is -N(R⁸)C(O)N(H)-, the reaction of a compound of the formula II as defined above with a trichloroacetylamine of the formula XIII: 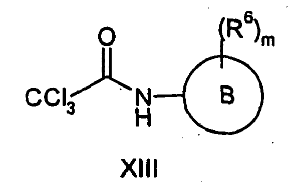 wherein R⁶, m and B have any of the meanings defined hereinbefore except that any functional group is protected if necessary;
or
Process(f) For compounds of the formula I wherein L is -C(R^{a}R^{b})C(O)N(R⁹)-, the reaction of a compound of the formula XIV: 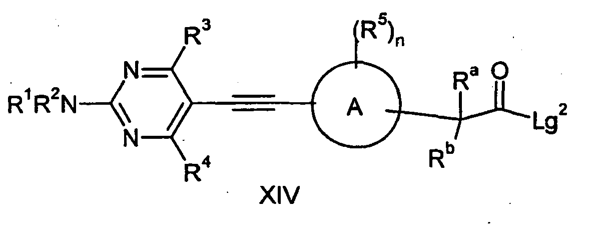 wherein Lg² is a suitable displaceable group as described above and R¹, R², R³, R⁴, R⁵, R^{a}, R^{b}, n and A have any of the meanings defined hereinbefore except that any functional group is protected if necessary, with an amine of the formula XV: 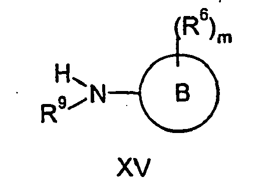 wherein R⁶, R⁹, m and B have any of the meanings defined hereinbefore except that any functional group is protected if necessary;
or
Process(g) The reaction of a compound of the formula XVI: 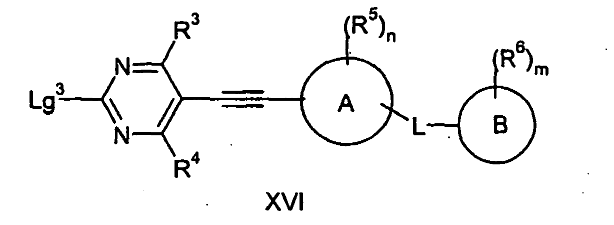 wherein Lg³ is a suitable displaceable group for example halogeno (such as fluoro, chloro, bromo or iodo), methyl sulfonyl, methylthio or aryloxy (such as phenoxy) and R³, R⁴, R⁵, R⁶, n, m, A, B and L have any of the meanings defined hereinbefore except that any functional group is protected if necessary, with an amine of the formula HNR¹R², wherein R¹ and R² have any of the meanings defined hereinbefore except that any functional group is protected if necessary;
or
Process(h) The reaction of a compound of the formula XVII: 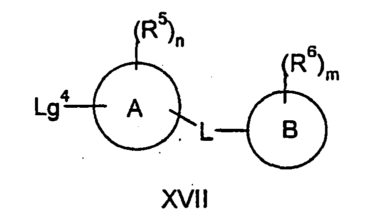 wherein Lg⁴ is a suitable displaceable group for example halogeno (such as chloro, bromo or iodo) or a sulfonyloxy group (such as trifluoromethylsulfonyloxy) and R⁵, R⁶, n, m, A, B and L have any of the meanings defined hereinbefore except that any functional group is protected if necessary, with an alkyne of the formula XVIII: 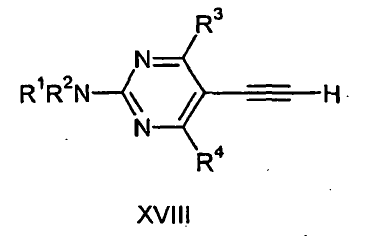 wherein R¹, R², R³ and R⁴ have any of the meanings defined hereinbefore except that any functional group is protected if necessary;
or
Process (i) For compounds of the formula I wherein L is -N(H)C(O)N(R⁹)-, the reaction of an isocyanate of the formula XIX: 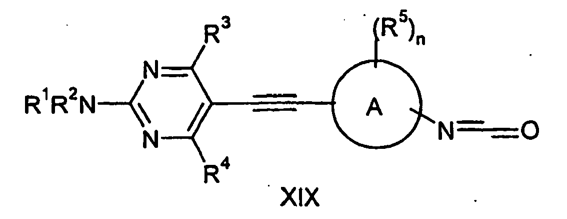 wherein R¹, R², R³, R⁴, R⁵, n and A have any of the meanings defined hereinbefore except that any functional group is protected if necessary, with an amine of the formula XV as defined above;
or
Process (j) For compounds of the formula I wherein L is -N(H)C(O)N(R⁹)-, the reaction of a compound of the formula XX: 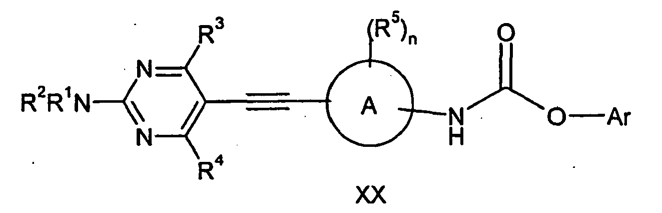 wherein Ar is a suitable aryl group, for example phenyl, and R¹, R², R³, R⁴, R⁵, n and A have any of the meanings defined hereinbefore except that any functional group is protected if necessary, with an amine of the formula XV as defined above.
and thereafter if necessary:
i) converting a compound of the Formula (I) into another compound of the Formula (I);
ii) removing any protecting groups;
iii) forming a salt.

### Reaction Conditions for Process (a)

The reaction of process (a) is conveniently carried out in the presence of a suitable inert solvent or diluent, for example a halogenated solvent such as dichloromethane, chloroform or carbon tetrachloride, an ether such,as tetrahydrofuran or 1,4-dioxane, an amine such as pyridine or a dipolar aprotic solvent such as N,N-dimethylformamide or N,N-dimethylacetamide. The reaction is conveniently carried out at a temperature in the range, for example, from ambient temperature to about 60°C, preferably at or near ambient temperature.

### Reaction Conditions for Process (b)

The reaction of process (b) is conveniently carried out in the presence of a suitable base. A suitable base is, for example, an organic amine base such as pyridine or a trialkylamine (such as triethylamine or diisopropylethylamine).

The reaction of process (b) is conveniently carried out in the presence of a suitable inert solvent or diluent, for example an ether such as tetrahydrofuran or 1,4-dioxane or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from ambient temperature to about 120°C, preferably from about 80°C to about 100°C.

Conveniently, this reaction may also be performed by heating the reactants in a sealed vessel using a suitable heating apparatus such as a microwave heater.

### Reaction Conditions for Process (c)

The reaction of process (c) is conveniently carried out in the presence of a suitable base. A suitable base is, for example, an organic amine base such as pyridine or a trialkylamine (such as triethylamine or diisopropylethylamine) or, for example, an alkali or alkaline earth metal carbonate such as sodium carbonate or potassium carbonate.

The reaction of process (c) is conveniently carried out in the presence of a suitable inert solvent or diluent, for example a halogenated solvent such as dichloromethane, chloroform or carbon tetrachloride or an ether such as tetrahydrofuran or 1,4-dioxane. The reaction is conveniently carried out at a temperature in the range, for example, from about - 10°C to about 30°C, preferably at or near 0°C.

### Reaction Conditions for Process (d)

When Lg² is hydroxy, the reaction of process (d) is conveniently carried out in the presence of a suitable coupling agent. A suitable coupling agent is, for example, a suitable peptide coupling agent, for example O-(7-azabenzotriazol-1-yl)-N,N,N',N'-tetramethyluronium hexafluorophosphate (HATU) or a suitable carbodiimide such as dicyclohexylcarbodiimide (DCC) or carbonyldiimidazole (CDI), optionally in the presence of a catalyst such as dimethylaminopyridine or hydroxybenzotriazole.

When Lg² is any suitable displaceable group as described above, the reaction of process (d) may conveniently be carried out in the presence of a suitable base. A suitable base is, for example, an organic amine base such as, for example, pyridine, 2,6-lutidine, collidine, 4-dimethylaminopyridine, triethylamine, diisopropylethylamine, N-methylmorpholine or diazabicyclo[5.4.0]undec-7-ene. Another, suitable base is, for example, an alkali or alkaline earth metal carbonate, for example sodium carbonate, potassium carbonate, caesium carbonate or calcium carbonate.

The reaction of process (d) is conveniently carried out in the presence of a suitable inert solvent or diluent, for example an ester such as ethyl acetate, a halogenated solvent such as dichloromethane, chloroform or carbon tetrachloride, an ether such as tetrahydrofuran or 1,4-dioxane, an aromatic solvent such as toluene or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from about 0°C to about 120°C, preferably at or near ambient temperature.

### Reaction Conditions for Process (e)

The reaction of process (e) is conveniently carried out in the presence of a suitable base. A suitable base is, for example, an organic amine base such as pyridine or a trialkylamine (such as triethylamine or diisopropylethylamine).

The reaction of process (e) is conveniently carried out in the presence of a suitable inert solvent or diluent, for example an ether such as tetrahydrofuran or 1,4-dioxane or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from ambient temperature to about 120°C, preferably from about 100°C to about 120°C.

Conveniently, this reaction may also be performed by heating the reactants in a sealed vessel using a suitable heating apparatus such as a microwave heater.

### Reaction Conditions for Process (f)

The reaction of process (f) is conveniently carried out under the conditions as described above for process (d).

### Reaction Conditions for Process (g)

The reaction of process (g) is conveniently carried out in the presence of a catalytic amount of a suitable acid. A suitable acid is, for example, hydrogen chloride.

The reaction of process (g) may conveniently be carried out in the absence or the presence of a suitable inert solvent or diluent. A suitable inert solvent or diluent, when used, is for example an alcohol such as ethanol, isopropanol or butanol or a dipolar aprotic solvent such as acetonitrile, N,N-dimethylformamide, N,N-dimethylacetamide, N-methylpyrrolidin-2-one or dimethylsulfoxide. The reaction is conveniently carried out at a temperature in the range, for example, from ambient temperature to about 120°C, preferably from about 80°C to about 90°C.

### Reaction Conditions for Process (h)

The reaction of process (h) is conveniently carried out in the presence of a suitable palladium catalyst, optionally in combination with a suitable copper catalyst. A suitable palladium catalyst is, for example, bis(triphenylphosphine)palladium dichloride, [1,1'-bis(diphenylphosphino)ferrocene] palladium dichloride or tetrakis(triphenylphosphine)palladium(0). A suitable copper catalyst is, for example, copper (I) iodide.

The reaction of process (h) is conveniently carried out in the presence of a suitable base. A suitable base is, for example, an organic amine base, such as trialkylamine (for example triethylamine) or tetramethylguanidine.

The reaction of process (h) may conveniently be carried out in the absence or the presence of a suitable inert solvent or diluent, for example an ester such as ethyl acetate, an ether such as tetrahydrofuran or 1,4-dioxane or a dipolar aprotic solvent such as N,N-dimethylformamide, N,N-dimethylacetamide, N-methylpyrrolidin-2-one or dimethylsulfoxide. The reaction is conveniently carried out at a temperature in the range, for example, from about -20°C to about 100°C.

### Reaction Conditions for Process (i)

The reaction of process (i) is conveniently carried out under the conditions as described above for process (a).

### Reaction Conditions for Process (j)

The reaction of process (j) is conveniently carried out under the conditions as described above for process (b).

### Starting Materials for Process (a)

Compounds of the formula II may be obtained by conventional procedures. For example, compounds of the formula II may be obtained by reaction of a pyrimidine of the formula VI with an alkyne of the formula VII as illustrated in *Reaction Scheme 1*: 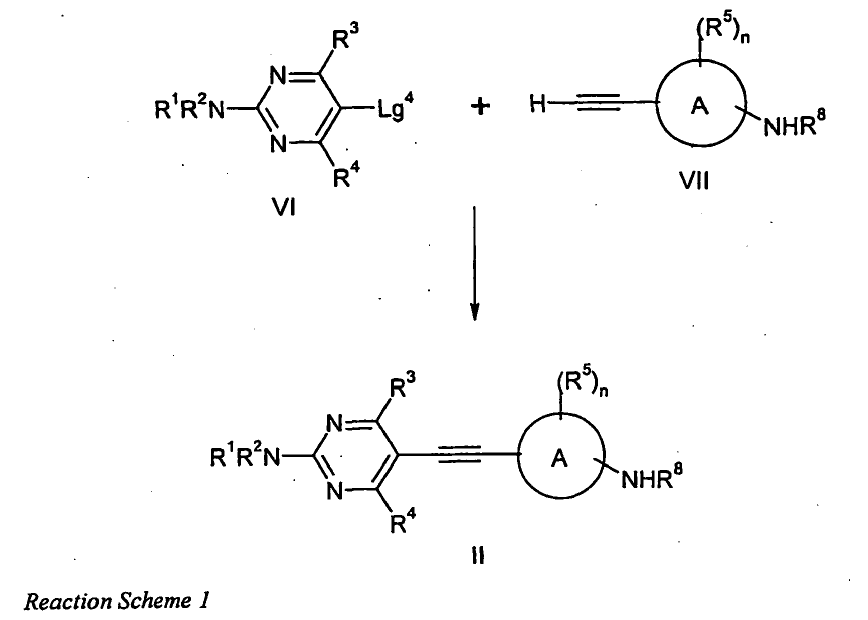 wherein Lg⁴ is a suitable displaceable group as described above and R¹, R², R³, R⁴, R⁵, R⁸, n and A have any of the meanings defined hereinbefore except that any functional group is protected if necessary.

The reaction of *Reaction Scheme 1* is conveniently carried out under the conditions as described above for process (h).

Alternatively, compounds of the formula II may be obtained by reaction of a pyrimidine of the formula VI with a protected alkyne of the formula VIa and then with an amine of the formula VIb as illustrated in *Reaction Scheme 2*: 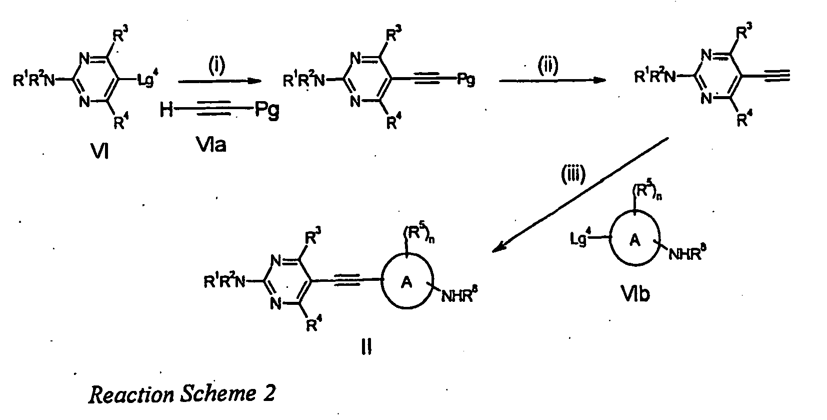 wherein Lg⁴ in the compounds of the formulae VI and VIb are each a suitable displaceable group as described above, Pg is a suitable protecting group, for example a trialkylsilyl group, such as trimethylsilyl or tert-butyldimethylsilyl or Me₂(OH)C- and R¹, R², R³, R⁴, R⁵, R⁸, n and A have any of the meanings defined hereinbefore except that any functional group is protected if necessary.

Step (i) of *Reaction Scheme 2* is the coupling of a protected alkyne of the formula VIa to a pyrimidine of the formula VI. Step (i) is carried out under conditions as described above for process (h). Step (ii) of *Reaction Scheme 2* is the deprotection of the alkyne under basic or acidic conditions to provide an unprotected alkyne. A person skilled in the art would readily be able to select the appropriate conditions for deprotection in step (ii). Step (iii) of *Reaction Scheme 2* is the coupling of the alkyne to an amine of the formula VIb. Step (iii) of *Reaction Scheme 2* is carried out under conditions as described above for process (h).

Alternatively, compounds of the formula II may be obtained by reaction of a compound of the formula VIc, wherein Lg³ is a suitable displaceable group as described above and R³, R⁴, R⁵, R⁸, n and A have any of the meanings defined hereinbefore except that any functional group is protected if necessary, with an amine of the formula HNR¹R² using reaction conditions as described above for process (g).

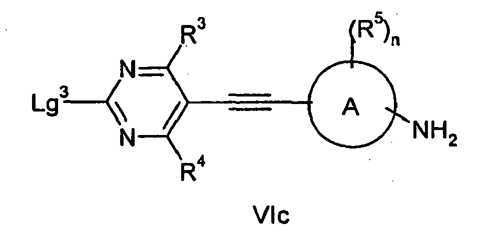

The starting materials of the formulae VI, VII, VIa, VIb and VIc and the amine HNR¹R² are commercially available or they are known in the literature, or they can be prepared by standard processes known in the art.

Isocyanates of the formula IV are commercially available or they are known in the literature, or they can be prepared by standard processes known in the art. For example, as the skilled person would appreciate, the isocyanates can conveniently be prepared from the corresponding acids or acid chlorides via a Curtis reaction with for example azide or diphenylphosphoryl azide. Alternatively, the isocyanates can conveniently be prepared by reaction of the corresponding amine with phosgene or a phosgene equivalent, for example triphosgene, diphosgene or N,N'-carbonyldiimidazole (March J., Adv. Org. Chem., 4th edition, 1992, page 1290, Wiley Interscience).

### Starting Materials for Process (b)

Compounds of the formula II may be obtained by conventional procedures as discussed above.

Aryl carbamates of the formula III are commercially available or they are known in the literature, or they can be prepared by standard processes known in the art. For example, the aryl carbamates can be prepared by reaction of an amine of the formula V with an arylchloroformate as illustrated in *Reaction Scheme 3*: 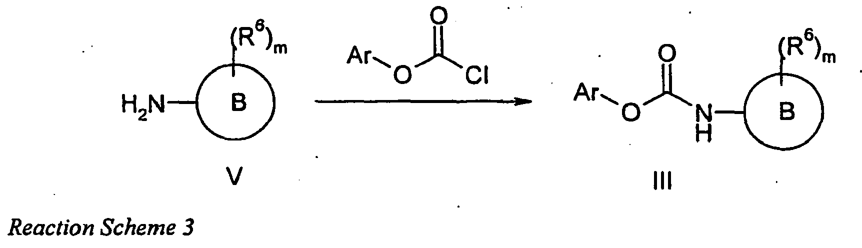 wherein R⁶, m, B and Ar have any of the meanings defined hereinbefore except that any functional group is protected if necessary.

The reaction of *Reaction Scheme 3* is conveniently carried out in the presence of a suitable base. A suitable base is, for example, an organic amine base such as pyridine or a trialkylamine (such as triethylamine).

The reaction is conveniently carried out in the presence of a suitable inert solvent or diluent, for example an ether such as tetrahydrofuran or 1,4-dioxane. The reaction is conveniently carried out at a temperature in the range, for example, from about -20°C to about 100°C, preferably at or near 0°C.

The starting material of the formula V and the arylchloroformate are commercially available or they are known in the literature, or they can be prepared by standard processes known in the art.

### Starting Materials for Process (c)

Compounds of the formula II may be obtained by conventional procedures as discussed above.

Compounds of the formula XI are commercially available or they are known in the literature, or they can be prepared by standard processes known in the art.

### Starting Materials for Process (d)

Compounds of the formula II may be obtained by conventional procedures as discussed above.

Compounds of formula IX are commercially available or they are known in the literature, or they can be prepared by standard processes known in the art.

### Starting Materials for Process (e)

Compounds of the formula II may be obtained by conventional procedures as discussed above.

Trichloroacetylamines of the formula XIII are commercially available or they are known in the literature, or they can be prepared by standard processes known in the art.

### Starting Materials for Process (f)

Compounds of the formula XIV may be obtained by conventional procedures as discussed above.

Amines of the formula XV are commercially available or they are known in the literature, or they can be prepared by standard processes known in the art.

### Starting Materials for Process (g)

As the skilled person would appreciate, compounds of the formula XVI can be prepared using similar processes to those described above using the appropriate starting materials, for example wherein the starting materials carry an, optionally protected, group Lg³ in place of the -NR¹R² group.

Amines of the formula HNR¹R² are commercially available or they are known in the literature, or they can be prepared by standard processes known in the art.

### Starting Materials for Process (h)

Compounds of formula XVII are commercially available or they are known in the literature, or as the skilled person would appreciate they can be prepared using similar processes to those described above using the appropriate starting materials. For example, compounds of the formula XVII wherein L is -N(R⁸)C(O)N(H)- may conveniently be obtained by reaction of an amine of the formula XVIIa with an aryl carbamate of the formula XVIIb as illustrated in *Reaction Scheme 4*: 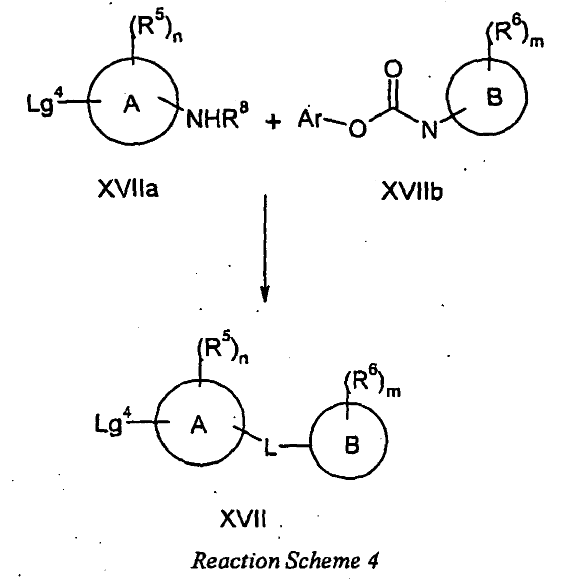 wherein Lg⁴ is a suitable displaceable group as described above, L is -N(R⁸)C(O)N(H)- and R⁵, R⁶, R⁸, n, m, A and B have any of the meanings defined hereinbefore except that any functional group is protected if necessary.

The reaction of *Reaction Scheme 4* is conveniently carried out under the conditions as described above for process (b).

The starting materials of the formulae XVIIa and XVIIb are commercially available or they are known in the literature, or they can be prepared by standard processes known in the art.

Alkynes of the formula XVIII are commercially available or as the skilled person would appreciate they can be prepared using similar processes to those described above using the appropriate starting materials. For example, compounds of the formula XVIII may conveniently be obtained by reaction of a pyrimidine of the formula XVIIIa: 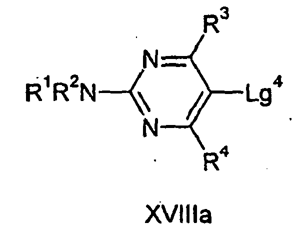 wherein Lg⁴ is a suitable displaceable group as described above and R¹, R², R³ and R⁴ have any of the meanings defined hereinbefore except that any functional group is protected if necessary, with trimethylsilylacetylene or 2-methyl-3-butyn-2-ol conveniently under the conditions as described above for process (h), followed by the removal of the protecting group using standard procedures known in the art.

### Starting Materials for Process (i)

As the skilled person would appreciate, isocyanates of the formula XIX can conveniently be prepared from the corresponding acids or acid chlorides via a Curtis reaction for example with azide or diphenylphosphoryl azide. Alternatively, the isocyanates can conveniently be prepared by reaction of the corresponding amine with phosgene or a phosgene equivalent, for example diphosgene, diphosgene or N,N'-carbonyldiimidazole (March J., Adv. Org. Chem., 4th edition, 1992, page 1290, Wiley Interscience).

Amines of the formula XV are commercially available or they are known in the literature, or they can be prepared by standard processes known in the art.

### Starting Materials for Process (i)

Compounds of formula XX are commercially available or they are known in the literature, or as the skilled person would appreciate they can be prepared using similar processes to those described above using the appropriate starting materials.

Amines of the formula XV are commercially available or they are known in the literature, or they can be prepared by standard processes known in, the art.

Compounds of the formula I can be converted into further compounds of the formula I using standard procedures conventional in the art.

Examples of the types of conversion reactions that may be used include introduction of a substituent by means of an aromatic substitution reaction or of a nucleophilic substitution reaction, reduction of substituents, alkylation of substituents and oxidation of substituents. The reagents and reaction conditions for such procedures are well known in the chemical art.

Particular examples of aromatic substitution reactions include the introduction of an alkyl group using an alkyl halide and Lewis acid (such as aluminium trichloride) under Friedel Crafts conditions; and the introduction of a halogeno group. Particular examples of nucleophilic substitution reactions include the introduction of an alkoxy group or of a monoalkylamino group, a dialkyamino group or a N-containing heterocycle using standard conditions. Particular examples of reduction reactions include the reduction of a carbonyl group to a hydroxy group with sodium borohydride or of a nitro group to an amino group by catalytic hydrogenation with a nickel catalyst or by treatment with iron in the presence of hydrochloric acid with heating.

An example of a suitable conversion reaction is the conversion of a carbamate compound of the formula I wherein R¹, R², R³, R⁴, R⁵, n and A are as defined in claim 1, L is N(H)C(O)-O- and B is an optionally substituted phenyl group to a compound of the formula I wherein L is N(H)C(O)N(H) and R¹, R², R³, R⁴, R⁵, n, B and A are as defined in claim 1. Such a conversion may be achieved using standard procedures, for example by reaction of the carbamate with an appropriate amine, for example under conditions as described above for process (b).

Another example of a suitable conversion reaction is the conversion of a compound of the formula I wherein R², R³, R⁴, R⁵, R⁶, n, m, A, B and L are as defined in claim 1 and R¹ and/or R² is hydrogen to a compound of the formula I wherein R¹ and/or R² is, for example, an optionally substituted (1-6C)alkoxycarbonyl group. Such a conversion may be achieved using standard procedures, for example by substitution of one or both of the hydrogen atoms R¹ and/or R² for a desired, optionally substituted (1-6C)alkoxycarbonyl group.

Certain compounds of Formula I are capable of existing in stereoisomeric forms. It will be understood that the invention encompasses all geometric and optical isomers of the compounds of formula I and mixtures thereof including racemates. Tautomers and mixtures thereof also form an aspect of the present invention.

Isomers may be resolved or separated by conventional techniques, e.g. chromatography or fractional crystallisation. Enantiomers may be isolated by separation of a racemic or other mixture of the compounds using conventional techniques (e.g. chiral High Performance Liquid Chromatography (HPLC)). Alternatively the desired optical isomers may be made by reaction of the appropriate optically active starting materials under conditions which will not cause racemisation, or by derivatisation, for example with a homochiral acid followed by separation of the diastereomeric derivatives by conventional means (e.g. HPLC, chromatography over silica) or may be made with achiral starting materials and chiral reagents. All stereoisomers are included within the scope of the invention.

The compounds of the invention may be isolated from their reaction mixtures using conventional techniques.

It will be appreciated that in some of the reactions mentioned herein it may be necessary/desirable to protect any sensitive groups in the compounds. The instances where protection is necessary or desirable and suitable methods for protection are known to those skilled in the art. Conventional protecting groups may be used in accordance with standard practice (for illustration see T.W. Green, Protective Groups in Organic Synthesis, John Wiley and Sons, 1991). Thus, if reactants include groups such as amino, carboxy or hydroxy it may be desirable to protect the group in some of the reactions mentioned herein. Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower", as in, for example, lower alkyl, signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned are, of course, within the scope of the invention.

It will also be appreciated that certain of the various ring substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group. Particular examples of modifications include the reduction of a nitro group to an amino group by for example, catalytic hydrogenation with a nickel catalyst or treatment with iron in the presence of hydrochloric acid with heating; oxidation of alkylthio to alkylsulfinyl or alkylsulfonyl.

It is believed that certain intermediate compounds of Formulae II, XIV, XVI, XIX, XX and VIc are novel and are herein claimed as another aspect of the present invention.

### Biological Assays

The following assays can be used to measure the effects of the compounds of the present invention as Tie2 inhibitors in vitro and as inhibitors of Tie2 autophosphorylation in whole cells.

### a. In vitro receptor tyrosine kinase inhibition assay

To test for inhibition of Tie2 receptor tyrosine kinase, compounds are evaluated in a non-cell based protein kinase assay by their ability to inhibit the protein kinase enzyme phosphorylation of a tyrosine containing polypeptide substrate in an ELISA based microtitre plate assay. In this particular case, the assay was to determine the IC₅₀, for three different recombinant human tyrosine kinases Tie2, KDR and Flt.

To facilitate production of the tyrosine kinases, recombinant receptor genes were produced using standard molecular biology cloning and mutagenesis techniques. These recombinant proteins fragments encoded within these genes consist of only the intracellular portion C-terminal portion of the respective receptor, within which is found the kinase domain. The recombinant genes encoding the kinase domain containing fragments were cloned and expressed in standard baculovirus/Sf21 system (or alternative equivalent).

Lysates were prepared from the host insect cells following protein expression by treatment with ice-cold lysis buffer (20mM N-2-hydroxyethylpiperizine-N'-2-ethanesulphonic acid (HEPES) pH7.5, 150 mM NaCl, 10% glycerol, 1% Triton X-100, 1.5 mM MgCl₂, 1 mM ethylene glycol-bis (β-aminoethyl ether) N',N',N',N'- tetraacetic acid (EGTA), plus protease inhibitors and then cleared by centrifugation.
Tie2, KDR and Flt1 lysates were stored in aliquots at -80 °C.

Constitutive kinases activity of these recombinant proteins was determined by their ability to phosphorylate a synthetic peptide (made up of a random co-polymer of Glutamic Acid, Alanine and Tyrosine in the ratio of 6:3:1). Specifically, Nunc Maxisorb^{™} 96-well immunoplates were coated with 100 microlitres of synthetic peptide Sigma P3899 (1mg/ml stock solution in PBS diluted 1:500 in PBS prior to plate coating) and incubated at 4 °C overnight. Plates were washed in 50 mM HEPES pH 7.4 at room temperature to remove any excess unbound synthetic peptide.

Tie2, KDR or Flt1 activities were assessed by incubation of the appropriate freshly diluted lysates (1:200, 1:400 and 1:1000 respectively) in peptide coated plates for 60 minutes (Tie2) or 20 minutes for (KDR, Flt) at room temperature in100 mM HEPES pH 7.4 , adenosine trisphosphate (ATP) at 5 micromolar (or Km concentration for the respective enzyme, 10 mM MnCl₂, 0.1 mM Na₃VO₄, 0.2 mM DL-dithiothreitol (DTT), 0.1% Triton X-100 together with the test compound(s) in dissolved in DMSO (final concentration of 2.5%) with final compound concentrations ranging from 0.05 micromolar -100 micromolar. Reactions were terminated by the removal of the liquid components of the assay followed by washing of the plates with PBS-T (phosphate buffered saline with 0.5% Tween 20) or an alternative equivalent wash buffer.

The immobilised phospho-peptide product of the reaction was detected by immunological methods. Firstly, plates were incubated for 4 hours at room temperature with murine monoclonal anti-phosphotyrosin -HRP (Horseradish Peroxidase) conjugated antibodies (4G10 from Upstate Biotechnology UBI 16-105). Following extensive washing with PBS-T, HRP activity in each well of the plate was measured colorimetrically using 22'-Azino-di-[3-ethylbenzthiazoline sulfonate (6)] diammonium salt crystals ABTS (Sigma P4922 - prepared as per manufactures instructions) as a substrate incubated for 30-45 minutes to allow colour development, before 100ul of 1M H₂SO₄ was added to stop the reaction.

Quantification of colour development and thus enzyme activity was achieved by the measurement of absorbance at 405nm on a Molecular Devices ThermoMax microplate reader. Kinase inhibition for a given compound was expressed as an IC₅₀ value. This was determined by calculation of the concentration of compound that was required to give 50% inhibition of phosphorylation in this assay. The range of phosphorylation was calculated from the positive (vehicle plus ATP) and negative (vehicle minus ATP) control values.

### b. Cellular Tie2 autophosphorylation assay

This assay is based on measuring the ability of compounds to inhibit autophosphorylation of the Tie2 receptor which normally leads to the production of "activated" receptor that in turn initiates the particular signal transduction pathways associated with the receptor function.

Autophosphorylation can be achieved by a number of means. It is known that expression of recombinant kinase domains in baculoviral systems can lead to the production of phosphorylated and activated receptor. It is also reported that over expression of receptors in recombinant cell lines can itself lead to receptor autophosphorylation in the absence of the ligand (Heldin C-H. 1995 Cell : 80, 213-223; Blume-J. P, Hunter T. 2001 Nature: 411, 355-65). Furthermore, there are numerous literature examples in which chimaeric receptors have been constructed. In these cases the natural, external cell surface domain of the receptor has been replaced with that of a domain which is known to be readily dimerised via the addition of the appropriate ligand (e.g. TrkA-Tie2/NGF ligand (Marron, M.B., et al., 2000 Journal of Biological Chemistry : 275:39741-39746) or C-fms-Tie-1/CSF-1 ligand (Kontos, C.D., et al., 2002 Molecular and Cellular Biology : 22, 1704-1713). Thus when the chimaeric receptor expressed in a host cell line and the respective ligand is added, this induces autophosphorylation of the chimeric receptor's kinase domain. This approach has the advantage of often allowing a known (and often easily obtained) ligand to be used instead of having to identify and isolate the natural ligand for each receptor of interest.

Naturally if the ligand is available one can use natural cell lines or primary cells which are known to express the receptor of choice and simply stimulate with ligand to achieve ligand induced phosphorylation. The ability of compounds to inhibit autophosphorylation of the Tie2 receptor, which is expressed for example in EA.hy926/B3 cells (supplied by J. McLean/ B. Tuchi, Univ.of N. Carolina at Chapel Hill, CB- 4100, 300 Bynum Hall, Chapel Hill, N.C. 27599-41000, USA) or primary HUVEC (human umbilical vein endothelial cells - available from various commercial sources), can measured by this assay.

Natural Ang1 ligand can be isolated using standard purification technology from either tumour cell supernatants or alternatively the Ang1 gene can be cloned and expressed recombinantly using stand molecular biology techniques and expression systems. In this case one can either attempt to produce the ligand either in its native state or as recombinant protein which for example may have been genetically engineered to contain additional of purification tags (eg. polyhistidine peptides, antibody Fc domains) to facilitate the process.

Using the ligand stimulation of either EA.hy926B3 or HUVEC cellular Tie2 receptor as the example, a Ang1 ligand stimulated cellular receptor phosphorylation assay can be constructed which can be used to analyse to determine the potential of compounds to inhibit this process. For example EA.hy926/B3 cells were grown in the appropriate tissue culture media plus 10% foetal calf serum (FCS) for two days in 6 well plates starting with an initial seeding density of 5x10⁵ cells/well. On the third day the cells were serum starved for a total of 2 hours by replacing the previous media with media containing only 1% FCS. After 1 hour 40 minutes of serum starvation the media was removed and replace with 1 ml of the test compound dilutions (compound dilutions made in serum starvation media yet keeping the DMSO concentration below 0.8%). After 1.5 hours of serum starvation orthovanidate was added to a final concentration of 0.1 mM for the final 10 minutes of serum starvation.

Following a total of 2 hours of serum starvation, the ligand plus orthovandiate was added to stimulate autophosphorylation of the cellular Tie2 receptor (ligand can be added either as purified material diluted in serum starvation media or non-purified cell supernatant containing ligand e.g. when recombinantly expressed mammalian cells).
After 10 minutes incubation at 37°C with the ligand, the cells were cooled on ice washed with approximately 5mls with cold PBS containing 1 mM orthovanadate, after which 1 ml of ice cold lysis buffer ((20 mM Tris pH 7.6,150 mM NaCl, 50 mM NaF, 0.1 % SDS, 1% NP40, 0.5 % DOC, 1 mM orthovanadate, 1 mM EDTA, 1 mM PMSF, 30 µl / ml Aprotinin, 10 µg/ml Pepstatin, 10 µg/ml Leupeptin) was added the cells and left on ice for 10- 20 minutes. The lysate was removed and transferred to a 1.5 ml Eppendorf tube and centrifuged for 3 minutes at 13000 rpm at 4 °C. 800 µl of each lysate was transferred to fresh 2 ml Eppendorf tubes for the immuno-precipitation. 3 mg = 15 µl of anti-phospho-tyrosine antibody (Santa Cruz PY99 -sc-7020) was added to the lysates and left to incubate for 2 hours at 4 °C. 600 µl washed MagnaBind beads (goat anti-mouse IgG, Pierce 21354) were added to the lysates and the tubes left to rotate over night at 4°C.

Samples were treated for 1 minute in the magnet before carefully removing the lysis supernatant. 1 ml of lysis buffer was then added to the beads and this step repeated twice more. The beads were suspended in 25 µl of 94 °C hot 2 x Laemmli loading buffer plus betamercaptoethanol and left to stand for 15 minutes at room temperature.

The beads were removed by exposing the tubes for 1 minutes in the magnet, and the total liquid separated from the beads from each immuno-precipitate loaded onto Polyacrylamide/SDS protein gels (pre-cast 4-12 % BisTris NuPAGE / MOPS 12 well gels from Novex). Protein gels were run at 200 V and then blotted onto NC membrane for 1hours30 minutes at 50 V / 250 mA. All blots were treated with 5% Marvel in PBS-Tween for1 hour at room temperature to reduce non-specific binding of the detection antibody. A rabbit anti-Tie2 (Santa Cruz sc-324) was added in a 1:500 dilution in 0.5 % Marvel / PBS-Tween and left to incubate overnight at 4 °C. The blots were rigorously washed with PBS-Tween before adding the goat anti rabbit -POD conjugate (Dako P0448) at a 1:5000 dilution in 0.5 % Marvel / PBS-Tween. The antibody was left on for 1 hour at room temperature before subsequently washing the blots with PBS-Tween. The western blots of the various immuno-precipitated samples were developed the blots with LumiGLO (NEB 7003). And transferred to an X-Ray cassette and films exposed for 15 sec /30 sec and 60 sec. The relative strength of the protein band which pertains to the phosphorylated Tie2 receptor was evaluated using a FluorS BioRad image analyser system. The percentage phosphorylation for each test compound dilution series was determined from which IC₅₀ values were calculated by standard methods using the appropriate control samples as reference.

Although the pharmacological properties of the compounds of the Formula I vary with structural change as expected, in general activity possessed by compounds of the Formula I, may be demonstrated at the following concentrations or doses in one or more of the above tests (a) and (b):-
1(a):- IC₅₀ in the range, for example, < 100µM;
Test (b):- IC₅₀ in the range, for example, < 50µM;

By way of example, Table A illustrates the activity of representative compounds according to the invention. Column 2 of Table A shows IC₅₀ data from Test (a) for the inhibition of Tie2 receptor tyrosine kinase *in vitro* and column 3 shows IC₅₀ data from Test (b) for the inhibition of autophosphorylation of Tie2 receptor tyrosine kinase.

**Table A**

| Example Number | IC₅₀ (µM) Test (a): Inhibition of Tie2 receptor tyrosine kinase *in vitro* | IC₅₀ (µM) Test (b): Inhibition of autophosphorylation of Tie2 receptor tyrosine kinase |
|---|---|---|
| 19 | 19.871 | 0.337 |
| 33 | 5.700 | 2.592 |
| 48 | 74.949 | 3.287 |

In the following section references to a compound of formula I, refer also to other sub-groups of the invention as described above, for example would also apply, amongst other sub-groups of the invention, to compounds of formula Ia, Ib, Ic and Id.

According to a further aspect of the invention there is provided a pharmaceutical composition which comprises a compound of the Formula I, or a pharmaceutically acceptable salt thereof, as defined hereinbefore in association with a pharmaceutically-acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a compound of the Formula I will naturally vary according to the nature and severity of the conditions, the age and sex of the animal or patient and the route of administration, according to well known principles of medicine.

In using a compound of the Formula I for therapeutic or prophylactic purposes it will generally be administered so that a daily dose in the range, for example, 0.1 mg/kg to 75 mg/kg body weight is received, given if required in divided doses. In general lower doses will be administered when a parenteral route is employed. Thus, for example, for intravenous administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration is however preferred, particularly in tablet form. Typically, unit dosage forms will contain about 0.5 mg to 0.5 g of a compound of this invention.

The compounds according to the present invention as defined herein are of interest for, amongst other things, their antiangiogenic effect. The compounds of the invention are expected to be useful in the treatment or prophylaxis of a wide range of disease states associated with undesirable or pathological angiogenesis, including cancer, diabetes, psoriasis, rheumatoid arthritis, Kaposi's sarcoma, haemangioma, lymphoedema, acute and chronic nephropathies, atheroma, arterial restenosis, autoimmune diseases, acute inflammation, excessive scar formation and adhesions, endometriosis, dysfunctional uterine bleeding and ocular diseases with retinal vessel proliferation. Cancer may affect any tissue and includes leukaemia, multiple myeloma and lymphoma. In particular such compounds of the invention are expected to slow advantageously the growth of primary and recurrent solid tumours of, for example, the colon, breast, prostate, lungs and skin.

We believe that the antiangiogenic properties of the compounds according to the present invention arise from their Tie2 receptor tyrosine kinase inhibitory properties. Accordingly, the compounds of the present invention are expected be useful to produce a Tie2 inhibitory effect in a warm-blooded animal in need of such treatment. Thus the compounds of the present invention may be used to produce an antiangiogenic effect mediated alone or in part by the inhibition of Tie2 receptor tyrosine kinase.

More particularly the compounds of the invention are expected to inhibit any form of cancer associated with Tie2. For example, the growth of those primary and recurrent solid tumours which are associated with Tie2, especially those tumours which are significantly dependent on Tie2 receptor tyrosine kinase for their growth and spread.

According to a further aspect of the invention there is provided a compound of the Formula I, or a pharmaceutically-acceptable salt thereof, as defined hereinbefore, for use as a medicament.

According to another aspect of the invention, there is provided the use of a compound of the formula I, or a pharmaceutically acceptable salt thereof, as defined hereinbefore, in the manufacture of a medicament for use as a Tie2 receptor tyrosine kinase inhibitor in a warm-blooded animal such as man.

According to another aspect of the invention, there is provided the use of a compound of the formula I, or a pharmaceutically acceptable salt thereof, as defined hereinbefore, in the manufacture of a medicament for use in the production of an anti-angiogenic effect in a warm-blooded animal such as man.

According to another aspect of the invention, there is provided the use of a compound of the formula I, or a pharmaceutically acceptable salt thereof, as defined hereinbefore in the manufacture of a medicament for use in the treatment of cancers in a warm-blooded animal such as man.

According to another aspect of the invention, there is provided the use of a compound of the formula I, or a pharmaceutically acceptable salt thereof, as defined hereinbefore in the manufacture of a medicament for use in the treatment of a cancer selected from leukaemia, breast, lung, colon, rectal, stomach, prostate, bladder, pancreas, ovarian, lymphoma, testicular, neuroblastoma, hepatic, bile duct, renal cell, uterine, thyroid and skin cancer in a warm-blooded animal such as man.

According to another aspect of the invention there is provided a method of inhibiting Tie2 receptor tyrosine kinase in a warm-blooded animal, such as man, in need of such treatment, which comprises administering to said animal an effective amount of a compound of the formula I, or a pharmaceutically acceptable salt thereof, as defined hereinbefore.

According to another aspect of the invention there is provided a method for producing an anti-angiogenic effect in a warm-blooded animal, such as man, in need of such treatment, which comprises administering to said animal an effective amount of a compound of the formula I, or a pharmaceutically acceptable salt thereof, as defined hereinbefore.

According to another aspect of the invention there is provided a method of treating cancers in a warm-blooded animal, such as man, in need of such treatment, which comprises administering to said animal an effective amount of a compound of the formula I, or a pharmaceutically acceptable salt thereof, as defined hereinbefore.

According to another aspect of the invention there is provided a method of treating a cancer selected from leukaemia, breast, lung, colon, rectal, stomach, prostate, bladder, pancreas, ovarian, lymphoma, testicular, neuroblastoma, hepatic, bile duct, renal cell, uterine, thyroid or skin cancer, in a warm-blooded animal, such as man, in need of such treatment, which comprises administering to said animal an effective amount of a compound of the formula I, or a pharmaceutically acceptable salt thereof, as defined hereinbefore.

According to another aspect of the invention there is provided a compound of the formula I, or a pharmaceutically acceptable salt thereof, as defined hereinbefore, for use in inhibiting Tie2 receptor tyrosine kinase in a warm-blooded animal, such as man.

According to an another aspect of the invention there is provided a compound of the formula I, or a pharmaceutically acceptable salt thereof, as defined hereinbefore, for use in producing an anti-angiogenic effect in a warm-blooded animal, such as man.

According to another aspect of the invention there is provided a compound of the formula I, or a pharmaceutically acceptable salt thereof, as defined hereinbefore, for use in the treatment of cancer.

According to another aspect of the invention there is provided a compound of the formula I, or a pharmaceutically acceptable salt thereof, as defined hereinbefore, for use in the treatment of a cancer selected from leukaemia, breast, lung, colon, rectal, stomach, prostate, bladder, pancreas, ovarian, lymphoma, testicular, neuroblastoma, hepatic, bile duct, renal cell, uterine, thyroid or skin cancer.

As hereinbefore mentioned it is further expected that a compound of the present invention will possess activity against other diseases mediated by undesirable or pathological angiogenesis including psoriasis, rheumatoid arthritis, Kaposi's sarcoma, haemangioma, lymphoedema, acute and chronic nephropathies, atheroma, arterial restenosis, autoimmune diseases, acute inflammation, excessive scar formation and adhesions, endometriosis, dysfunctional uterine bleeding and ocular diseases with retinal vessel proliferation.

The anti-angiogenic activity defined herein may be applied as a sole therapy or may involve, in addition to a compound of the invention, one or more other substances and/or treatments. Such conjoint treatment may be achieved by way of the simultaneous, sequential or separate administration of the individual components of the treatment. In the field of medical oncology it is normal practice to use a combination of different forms of treatment to treat each patient with cancer. In medical oncology the other component(s) of such conjoint treatment in addition to the cell cycle inhibitory treatment defined hereinbefore may be: surgery, radiotherapy or chemotherapy. Such chemotherapy may include one or more of the following categories of anti-tumour agents:
(i) anti-invasion agents (for example metalloproteinase inhibitors like marimastat and inhibitors of urokinase plasminogen activator receptor function);
(ii) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside and hydroxyurea, or, for example, one of the preferred antimetabolites disclosed in European Patent Application No. 562734 such as (25)-2-{o-fluoro-p-[N-{2,7-dimethyl-4-oxo-3,4-dihydroquinazolin-6-ylmethyl)-N-(prop-2-ynyl)amino]benzamido}-4-(tetrazol-5-yl)butyric acid); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(iii) cytostatic agents such as antioestrogens (for example tamoxifen, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrazole, vorazole and exemestane) and inhibitors of 5 α-reductase such as finasteride;
(iv) inhibitors of growth factor function, for example such inhibitors include growth factor antibodies, growth factor receptor antibodies, farnesyl transferase inhibitors, tyrosine kinase inhibitors and serine/threonine kinase inhibitors, for example inhibitors of the epidermal growth factor family (for example the EGFR tyrosine kinase inhibitors N-(3-chloro-4-fluorophenyl)-7-methoxy-6-(3-morpholinopropoxy)quinazolin-4-amine (ZD 1839), N-(3-ethynylphenyl)-6,7-bis(2-methoxyethoxy)quinazolin-4-amine (CP 358774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents that work by different mechanisms to those defined hereinbefore, such as those which inhibit vascular endothelial growth factor such as the compounds disclosed in International Patent Applications WO 97/22596, WO 97/30035, WO 97/32856 and WO 98/13354 and those that work by other mechanisms (for example linomide, inhibitors of integrin αvβ3 function and angiostatin);
(vi) biotherapeutic therapeutic approaches for example those which use peptides or proteins (such as antibodies or soluble external receptor domain constructions) which either sequest receptor ligands, block ligand binding to receptor or decrease receptor signalling (e.g. due to enhanced receptor degradation or lowered expression levels)
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

According to this aspect of the invention there is provided a pharmaceutical product comprising a compound of the Formula I as defined hereinbefore and an additional anti-tumour substance as defined hereinbefore for the conjoint treatment of cancer.

In addition to their use in therapeutic medicine, the compounds of Formula I and their pharmaceutically acceptable salts, are also useful as pharmacological tools in the development and standardisation of in vitro and *in vivo* test systems for the evaluation of the effects of inhibitors of cell cycle activity in laboratory animals such as cats, dogs, rabbits, monkeys, rats and mice, as part of the search for new therapeutic agents.

The invention will now be illustrated by the following non limiting examples in which, unless stated otherwise:
(i) temperatures are given in degrees Celsius (°C); operations were carried out at room or ambient temperature, that is, at a temperature in the range of 18-25 °C;
(ii) organic solutions were dried over anhydrous magnesium sulfate; evaporation of solvent was carried out using a rotary evaporator under reduced pressure (600-4000 Pascals; 4.5-30 mmHg) with a bath temperature of up to 60 °C;
(iii) chromatography means flash chromatography on silica gel; thin layer chromatography (TLC) was carried out on silica gel plates;
(iv) in general, the course of reactions was followed by TLC and / or analytical LC-MS, and reaction times are given for illustration only;
(v) final products had satisfactory proton nuclear magnetic resonance (NMR) spectra and/or mass spectral data;
(vi) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 300 MHz using perdeuterio dimethyl sulphoxide (DMSO-d₆) as solvent unless otherwise indicated; the following abbreviations have been used: s, singlet; d, doublet; t, triplet; q, quartet; m, multiplet; b, broad;
(viii) chemical symbols have their usual meanings; SI units and symbols are used;
(ix) solvent ratios are given in volume:volume (v/v) terms; and
(x) mass spectra (MS) were run with an electron energy of 70 electron volts in the chemical ionization (CI) mode using a direct exposure probe; where indicated ionization was effected by electron impact (EI), fast atom bombardment (FAB) or electrospray (ESP); values for m/z are given; generally, only ions which indicate the parent mass are reported; and unless otherwise stated, the mass ion quoted is MH⁺;
(xi) unless stated otherwise compounds containing an asymmetrically substituted carbon and/or sulphur atom have not been resolved;
(xii) where a synthesis is described as being analogous to that described in a previous example the amounts used are the millimolar ratio equivalents to those used in the previous example;
(xvi) the following abbreviations have been used:

| | |
|---|---|
| AcOH | Acetic acid |
| AIBN | 2,2'-Azobisisobutyronitrile |
| DCM | Dichloromethane |
| DIPEA | Diisopropylethylamine |
| DMA | *N,N* Dimethylacetamide |
| DMF | *N,N* Dimethylformamide |
| DMSO | Dimethylsulfoxide |
| DMTMM | 4-(4,6-Dimethoxy-1,3,5-triazin-2-yl)-4-methylmorpholin-4-ium chloride |
| dppf | 1,1'-Bis(diphenylphosphino)ferrocene |
| EtOAc | Ethylacetate |
| HATU | O-(7-Azabenzotriazol-1-yl)-N,N,N',N'-tetramethyluronium hexafluorophosphate |
| *^{t}*PrMgCl | Isopropylmagnesium chloride |
| LDA | Lithium diisopropylamide |
| LHMDS | Lithium bis(trimethylsilyl) amide |
| *m*-CPBA | *meta*-Chloroperbenzoic acid |
| MeOH | Methanol |
| MeCN | Acetonitrile |
| MCX | Mixed cation exchange resin |
| MTBE | Methyl *tert*-butyl ether |
| LCMS | Liquid Chromatograpy - Mass Spectrometry |
| NMP | 1-Methyl-2-pyrrolidinone |
| PhTosMIC | α-Tosylbenzyl isocyanide |
| POCl₃ | Phosphorus oxychloride |
| RPHPLC | Reversed phase high peformance liquid chromatography |
| TFA | Trifluoroacetic acid |
| THF | Tetrahydrofuran |

xvii) where a synthesis is described as leading to an acid addition salt (e.g. HCl salt), no comment is made on the stoichiometry of this salt. Unless otherwise stated, all NMR data is reported on free-base material, with isolated salts converted to the free-base form prior to characterisation.

### Example 1

### N-{3-[(2-aminopyrimidin-5-yl)ethynyl]phenyl}-N'-[2-fluoro-5-(trifluoromethyl)phenyl]urea

5-[(3-aminophenyl)ethynyl]pyrimidin-2-amine (105 mg) was stirred in THF and 2-fluoro-5-trifluoromethylphenyl isocyanate (123 mg) was added dropwise. After 30 min, methylethylenediamine-polystyrene (200 mg) was added and stirring continued for 30 min. The reaction mixture was filtered and concentrated to give a gray solid which was purified by flash chromatography on silica using 0-10% MeOH in DCM as eluent to give the title compound as a yellow solid (166 mg, 80%);
¹H NMR (DMSO-d₆) 7.13 (bs, 2H), 7.16-7.19 (m, 1H), 7.32-7.44 (m, 3H), 7.50-7.54 (m, 1H), 7.80 (bs, 1H); 8.47 (s, 2H), 8.61-8.64 (m, 1H), 8.94-8.95 (m, 1H), 9.28 (s, 1H);
MS m/e MH⁺ 416.

### Preparation of Intermediate

### 5-[(3-aminophenyl)ethynyl]pyrimidin-2-amine

2-Amino-5-iodopyrimidine (2.21 g), bis(triphenylphosphine)palladium dichloride (350 mg) and copper(I) iodide (40 mg) were stirred in DMF (100 mL)-triethylamine (20 mL) and degassed with nitrogen for 10 min. 3-Ethynyl aniline (1.29 g) was added and the mixture heated to 95 °C for 2 hours. The solvent was evaporated and the residue was purified by trituration with DCM (20 mL) to give the title compound as a brown solid (1.25 g, 60%);
¹HNMR (DMSO-d₆) 5.21 (bs, 2H), 6.58-6.70 (m, 3H), 7.03-7.07 (m, 3H), 8.40 (s, 2H);
MS m/e MH⁺ 211.
Examples 2 to 17 were prepared by an analogous method to Example 1 but purified by trituration from methanol.

### Example 2

### N-{3-[(2-aminopyrimidin-5-yl)ethynyl]phenyl}-N'-[2-(trifluoromethyl)phenyl]urea

Starting Materials: Intermediate 1 and 2-trifluoromethylphenyl isocyanate.
¹H NMR (DMSO-d₆) 7.13-7.16 (m, 3H), 7.29-7.35 (m, 3H), 7.64-7.72 (m, 2H), 7.78 (s, 1H), 7.95 (d, 1H), 8.13 (s, 1H), 8.45 (s, 2H), 9.47 (s, 1H);
MS m/e MH⁺ 398.

### Example 3

### N-{3-[(2-aminopyrimidin-5-yl)ethynyl]phenyl}-N'-[4-(trifluoromethyl)phenyl]urea

Starting Materials: Intermediate 1 and 4-trifluoromethylphenyl isocyanate.
¹H NMR (DMSO-d₆) 7.14-7.17 (m, 3H), 7.32-7.41 (m, 2H), 7.64-7.71 (m, 4H), 7.75 (s, 1H), 8.46 (s, 2H), 8.92 (s, 1H), 9.16 (s, 1H);
MS m/e MH⁺ 398.

### Example 4

### N-{3-[(2-aminopyrimidin-5-yl)ethynyl]phenyl}-N'-(2-fluorophenyl)urea

Starting Materials: Intermediate 1 and 2-fluorophenyl isocyanate.
¹H NMR (DMSO-d₆)·7.00-7.08 (m, 1H), 7.14-7.29 (m, 5H), 7.34 (d, 2H), 7.76 (s, 1H), 8.15 (td, 1H), 8.46 (s, 2H), 8.59 (s, 1H), 9.18 (s, 1H);
MS m/e MH⁺+MeCN 389.

### Example 5

### N-{3-[(2-aminopyrimidin-5-yl)ethynyl]phenyl}-N'-(3-fluorophenyl)urea

Starting Materials: Intermediate 1 and 3-fluorophenyl isocyanate.
¹H NMR (DMSO-d₆) 6.81 (td, 1H), 7.12-7.16 (m, 4H); 7.29-7.39 (m, 3H), 7.50 (dt, 1H), 7.74 (d, 1H), 8.46 (s, 2H), 8.85 (s, 1H), 8.96 (s, 1H);
MS m/e MH⁺+MeCN 389.

### Example 6

### N-{3-[(2-aminopyrimidin-5-yl)ethynyl]phenyl}-N'-(4-fluorophenyl)urea

Starting Materials: Intermediate 1 and 4-fluorophenyl isocyanate.
¹HNMR (DMSO-d₆) 7.11-7.17 (m, 5H); 7.30-7.38 (m, 2H), 7.46-7.51 (m, 2H), 7.73 (s, 1H), 8.45 (s, 2H), 8.75 (s, 1H), 8.77 (s, 1H);
MS m/e MH⁺+MeCN 389.

### Example 7

### N-{3-[(2-aminopyrimidin-5-yl)ethynyl]phenyl}-N'-(3-methoxyphenyl)urea

Starting Materials: Intermediate 1 and 3-methoxyphenyl isocyanate.
¹HNMR (DMSO-d₆) 3.76 (s, 3H), 6.58 (dd, 1H), 6.94-6.97 (m, 1H); 7.11-7.23 (m, 5H), 7.30-7.37 (m, 2H), 7.75 (s, 1H), 8.46 (s, 2H), 8.73 (s, 1H), 8.76 (s, 1H);
MS m/e MH⁺+MeCN 401.

### Example 8

### N-{3-[(2-aminopyrimidin-5-yl)ethynyl]phenyl}-N'-(2,5-difluorophenyl)urea

Starting Materials: Intermediate 1 and 2,5-difluorophenyl isocyanate.
¹H NMR (DMSO-d₆) 6.81-6.89 (m, 1H), 7.14-7.18 (m, 3H), 7.27-7.38 (m, 3H), 7.76 (s, 1H), 8.02-8.08 (m, 1H), 8.46 (s, 2H), 8.81 (s, 1H), 9.25 (s, 1H);
MS m/e MH⁺+MeCN 407.

### Example 9

### N-{3-[(2-aminopyrimidin-5-yl)ethynyl]phenyl}-N'-1,3-benzodioxol-5-ylurea

Starting Materials: Intermediate 1 and 3,4-methylenedioxyphenyl isocyanate.
¹H NMR (DMSO-d₆) 5.99 (s, 2H), 6.77-6.86 (m, 2H), 7.09-7.13 (m, 3H), 7.21-7.22 (m, 1H), 7.28-7.36 (m, 2H), 7.72 (s, 1H), 8.45 (s, 2H), 8.60 (s, 1H), 8.71 (s, 1H);
MS m/e MH⁺+MeCN 415.

### Example 10

### N-{3-[(2-aminopyrimidin-5-yl)ethynyl] phenyl}-N'-[3-(trifluoromethyl)phenyl]urea

Starting Materials: Intermediate 1 and 3-trifluoromethylphenyl isocyanate.
¹H NMR (DMSO-d₆) 7.14-7.16 (m, 3H), 7.31-7.40 (m, 3H), 7.51-7.61 (m, 2H), 7.77 (s, 1H), 8.05 (s, 1H), 8.46 (s, 2H), 8.91 (s, 1H), 9.10 (s, 1H);
MS m/e MH⁺+MeCN 439.

### Example 11

### N-{3-[(2-aminopyrimidin-5-yl)ethynyl]phenyl}-N'-(2-methoxyphenyl)urea

Starting Materials: Intermediate 1 and 2-methoxyphenyl isocyanate.
¹H NMR (DMSO-d₆) 3.90 (s, 3H), 6.89-7.11 (m, 3H), 7.11-7.13 (m, 3H), 7.32-7.34 (m, 2H), 7.77 (s, 1H), 8.14 (dd, 1H), 8.26 (s, 1H), 8.46 (s, 2H), 9.43 (s, 1H);
MS m/e MH⁺ 360.

### Example 12

### N-{3-[(2-aminopyrimidin-5-yl)ethynyl]phenyl}-N'-(4-methoxyphenyl)urea

Starting Materials: Intermediate 1 and 4-methoxyphenyl isocyanate.
¹H NMR (DMSO-d₆) 3.74 (s, 3H), 6.88-6.90 (m, 2H); 7.09-7.13 (m, 3H), 7.28-7.39 (m, 4H), 7.74 (s, 1H), 8.45 (s, 2H), 8.52 (s, 1H), 8.69 (s, 1H);
MS m/e MH⁺+MeCN 401.

### Example 13

### N-{3-[(2-aminopyrimidin-5-yl)ethynyl]phenyl}-N'-(3,4-difluorophenyl)urea

Starting Materials: Intermediate 1 and 3,4-difluorophenyl isocyanate.
¹H NMR (DMSO-d₆) 7.13-7.15 (m, 4H), 7.31-7.41 (m, 3H), 7.64-7.73 (m, 2H), 8.45 (s, 2H), 8.86 (s, 1H), 8.95 (s, 1H);
MS m/e MH⁺+MeCN 407.

### Example 14

### N-{3-[(2-aminopyrimidin-5-yl)ethynyl]phenyl}-N'-(3-cyanophenyl)urea

Starting Materials: Intermediate 1 and 3-cyanophenyl isocyanate.
¹H NMR (DMSO-d₆) 7.14-7.16 (m, 3H), 7.32-7.55 (m, 4H), 7.69-7.75 (m, 2H), 8.00 (s, 1H), 8.46 (s, 2H), 8.96 (s, 1H), 9.08 (s, 1H);
MS m/e MH⁺ 355.

### Example 15

### N-{3-[(2-aminopyrimidin-5-yl)ethynyl]phenyl}-N'-(3-chlorophenyl)urea

Starting Materials: Intermediate 1 and 3-chlorophenyl isocyanate.
¹H NMR (DMSO-d₆) 7.03-7.06 (m, 1H), 7.13-7.15 (m, 3H), 7.27-7.39 (m, 4H), 7.74-7.75 (m, 2H), 8.46 (s, 2H), 8.87 (s, 1H), 8.95 (s, 1H);
MS m/e MH⁺+MeCN 405.

### Example 16

### N-{3-[(2-aminopyrimidin-5-yl)ethynyl]phenyl}-N'-cyclopentylurea

Starting Materials: Intermediate 1 and cyclopentyl isocyanate.
¹H NMR (DMSO-d₆) 1.36-1.44 (m, 2H), 1.53-1.68 (m, 4H), 1.81-1.91 (m, 2H), 3.95 (sextet, 1H), 6.21 (d, 1H), 7.02-7.04 (m, 1H), 7.12 (s, 2H), 7.24-7.26 (m, 2H), 7.68 (s, 1H), 8.36 (s, 1H), 8.44 (s, 2H);
MS m/e MH⁺ 322.

### Example 17

### N-{3-[(2-aminopyrimidin-5-yl)ethynyl]phenyl}-N'-(3,5-difluorophenyl)urea

Starting Materials: Intermediate 1 and 3,5-difluorophenyl isocyanate.
¹H NMR (DMSO-d₆) 6.77-6.85 (m, 1H), 7.14-7.25 (m, 5H), 7.31-7.40 (m, 2H), 7.74 (s, 1H), 8.46 (s, 2H), 8.96 (s, 1H), 9.14 (s, 1H);
MS m/e MH⁺+MeCN 407.

### Example 18

### N-{3-[(2-aminopyrimidin-5-yl)ethynyl]phenyl}-N'-(5-tert-butyl-1,3,4-thiadiazol-2-yl)urea

5-[(3-aminophenyl)ethynyl]pyrimidine-2-amine (Intermediate 1) (50.0 mg), triethylamine (0.04 mL) and phenyl (5-tert-butyl-1,3,4-thiadiazol-2-yl)carbamate (Intermediate 2) (79.0 mg) in THF (2 mL) were irradiated under microwave conditions (CEM explorer, 80°C, 50W) for 20 min. The reaction mixture was concentrated *in vacuo,* purification by flash chromatography on silica using 1-10% MeOH in DCM as eluent gave the title compound as a solid (35 mg, 37%);
¹H NMR (DMSO-d₆) 1.38 (s, 9H), 7.08-7.19 (m, 3H), 7.33-7.37 (t, 1H), 7.37-7.45 (m, 1H), 7.75 (s, 1H), 8.43 (s, 2H), 9.10 (bs, 1H), 10.89 (bs, 1H);
MS m/e MH⁺ 394.

### Intermediate 2

### Phenyl (5-tert-butyl-1,3,4-thiadiazol-2-yl)carbamate

Phenylchloroformate (0.6 mL) was added dropwise to 2-amino-5-tert-butyl-1,3,4-thiadiazole (0.5 g) and pyridine (0.51 mL) in THF (40 mL) at 0°C. After 2 hour, the reaction mixture was quenched with H₂O (10 mL) and extracted with EtOAc (3x 10 mL). The combined organics were dried (MgSO₄) filtered and concentrated *in vacuo.* Purification by flash chromatography on silica using 20-50% EtOAc in isohexane gave the title compound as a yellow solid (0.819 mg, 93%);
¹H NMR (DMSO-d₆) 1.38 (s, 9H), 7.22-7.28 (m, 3H), 7.41-7.44 (m, 2H);
MS m/e MH⁺ 278.

Intermediates 3 to 7 were prepared by an analogous method to Intermediate 2 using phenylchloroformate and the appropriate heterocyclic amine.

### Intermediate 3

### Phenyl (3-methylisoxazol-5-yl)carbamate

Starting Materials: Phenylchloroformate and 5-amino-3-methyl isoxazole.
¹H NMR (DMSO-d₆) 2.17 (s, 3H), 5.93 (s, 1H), 7.21-7.30 (m, 3H), 7.41-7.46 (m, 2H), 11.79 (bs, 1H);
MS m/e MH⁺ 219.

### Intermediate 4

### Phenyl (5-tert-butylisoxazol-3-yl)carbamate

Starting Materials: Phenylchloroformate and 3-amino-5-tert-butylisoxazole.
¹HNMR (DMSO-d₆) 1.28 (s, 9H), 6.42 (s, 1H), 7.18-7.26 (m, 3H), 7.39-7.45 (m, 2H), 11.13, (bs, 1H);
MS m/e MH⁺ 261.

### Intermediate 5

### Phenyl [4-(trifluoromethyl)pyridin-2-yl]carbamate

Starting Materials: Phenylchloroformate and 2-amino-4-trifluoromethylpyridine.
¹H NMR (DMSO-d₆) 7.22-7.30 (m, 3H), 7.41-7.46 (m, 3H), 8.11 (s, 1H), 8.59-8.61 (d, 1H), 11.23 (bs, 1H);
MS m/e MH⁺ 283.

### Intermediate 6

### Phenyl [3-(acetylamino)phenyl]carbamate

Starting Materials: Phenylchloroformate and 3-aminoacetanilide.
¹H NMR (DMSO-d₆) 2.01 (s, 3H), 7.17-7.30 (m, 6H), 7.38-7.44 (m, 2H), 7.77 (s, 1H), 9.90 (bs, 1H), 10.16 (bs, 1 H);
MS m/e MH⁺ 271.

### Intermediate 7

### Phenyl (3-methylisothiazol-5-yl)carbamate

Starting Materials: Phenylchloroformate and 3-methyl-5-aminoisothiazole.
¹H NMR (DMSO-d₆) 2.30 (s, 3H), 6.68 (s, 1H), 7.25-7.31 (m, 3H), 7.41-7.46 (m, 2H), 11.90 (bs, 1H);
MS m/e MH⁺ 235.

Examples 19 and 20 were prepared by an analogous method to Example 18 (using the appropriate starting materials), except purification was by reverse phase HPLC, gradient H₂O:MeCN (0-70%).

### Example 19

### N-{3-[(2-aminopyrimidin-5-y1)ethynyl]phenyl}-N'-(3-methylisoxazol-5-yl)urea

Starting Materials: Intermediate 1 and Intermediate 3.
¹H NMR (DMSO-d₆) 2.16 (s, 3H), 5.96 (s, 1H), 7.02-7.18 (m, 3H), 7.28-7.39 (m, 2H), 7.70 (s, 1H), 8.41 (s, 2H), 8.92 (s, 1H), 10.15 (s, 1H);
MS m/e MH⁺ 335.

### Example 20

### N-(3-{[({3-[(2-aminopyrimidin-5-yl)ethynyl]phenyl}amino)carbonyl]amino}phenyl)acetamide

**Starting Materials: Intermediate 1 and Intermediate 6.**
¹H NMR (DMSO-d₆) 2.02 (s, 3H), 7.04-7.11 (m, 3H), 7.14-7.18 (m, 3H), 7.28-7.36 (m, 2H), 7.71 (s, 1H), 7.78 (s, 1H), 8.41 (s, 2H), 8.66 (s, 1H), 8.75 (s, 1H), 9.86 (s, 1H);
MS m/e MH⁺ 387.

### Example 21

### N-{3-[(2-aminopyrimidin-5-yl)ethynyl] phenyl}-N'-[4-(trifluoromethyl)pyridin-2-yl] urea

Example 21 was prepared by an analogous method to Example 18 using Intermediate 1 and Intermediate 5. Purification was by trituration with DCM/MeOH.
¹H NMR (DMSO-d₆) 7.11 (s, 2H), 7.14-7.19 (d, 1H), 731-7.43 (m, 3H), 7.78 (s, 1H), 8.04 (s, 1H), 8.43 (s, 2H), 8.52-8.57 (d, 1H), 9.73 (s, 1H), 9.84 (s, 1H);
MS m/e MH⁺ 399.

### Example 22

### N-{3-[(2-aminopyrimidin-5-yl)ethynyl]phenyl}-N'-(5-tert-butylisoxazol-3-yl)urea

Example 22 was prepared by an analogous method to Example 18 using Intermediate 1 and Intermediate 4. Purification was by trituration with THF.
¹H NMR (DMSO-d₆) 1.32 (s, 9H), 6.53 (s, 1H), 7.14-7.18 (m, 3H), 7.31-7.36 (m, 2H), 7.74 (s, 1H), 8.45 (s, 2H), 9.04 (s, 1H), 9.62 (s, 1H);
MS m/e MH⁺ 377.

### Example 23

### Phenyl {3-[(2-aminopyrimidin-5-yl)ethynyl]phenyl}carbamate

Example 23 was prepared by an analogous method to Intermediate 2 using Intermediate 1 and phenylchloroformate. Purification was by trituration with DCM.
¹H NMR (DMSO-d₆) 7.15 (s, 2H), 7.20-7.32 (m, 4H), 7.36-7.55 (m, 4H), 7.69 (s, 1H), 8.45 (s, 2H), 10.37 (s, 1H);
MS m/e MH⁺ 331.

### Example 24

### N-{3-[(2-aminopyrimidin-5-yl)ethynyl]phenyl}-N'-(2-oxopiperidin-3-yl)urea

Phenyl {3-[(2-aminopyrimidin-5-yl)ethynyl]phenyl}carbamate (Example 23) (50 mg), 3-amino-piperidin-2-one hydrochloride (46 mg) and triethylamine (0.06 mL) in THF (2 mL) were heated at 80°C for 24 hours. The reaction mixture was concentrated *in vacuo* and the solid triturated with water then diethyl ether, dried under vacuum at 60°C to give the title compound as a beige solid (41 mg, 77%);
¹H NMR (DMSO-d₆) 1.49-1.62 (m, 1H), 1.71-1.82 (m, 2H), 2.16-2.28 (m, 1H), 3.10-3.18 (m, 2H), 3.95-4.04 (m, 1H), 6.42-6.48 (d, 2H), 6.98-7.04 (m, 1H), 7.09 (s, 2H), 7.20-7.31 (m, 2H), 7.65 (s, 2H), 8.41 (s, 2H), 8.85 (s, 1H);
MS m/e MH⁺ 351.

### Example 25

### N-(5-tert-butylisoxazol-3-yl)-N'-(3-{[2-(methylamino)pyrimidin-5-yl]ethynyl}phenyl)urea

### N-(5-tert-butylisoxazol-3-yl)-N'-(3-[(2-chloropyrimidin-5-yl)ethynyl]phenyl)urea

(Intermediate 9) (94 mg) was stirred in methylamine (33% wt. solution in ethanol) (5 mL) and hydrogen chloride (1.0M solution in diethyl ether) (0.25 mL) was added dropwise. The reaction mixture was stirred at 80°C for 3 hours. The solvent was evaporated and the residue was purified by trituration with diethyl ether (20 mL) to give the title compound as a white solid (50 mg, 54%);
¹H NMR (DMSO-d₆) 1.30 (s, 9H), 2.84 (d, 3H), 6.49 (s, 1H), 7.10-7.16 (m, 1H), 7.30-7.36 (m, 2H), 7.57 (q, 1H), 7.72 (s, 1H), 8.42-8.53 (m, 2H), 8.89 (s, 1H), 9.48 (bs, 1H);
MS m/e MH⁺ 391.

### Intermediate 8

### {3-[(2-chloropyrimidin-5-yl)ethynyl]phenyl}amine

Palladium (10 wt.%) on activated carbon (1.5 g) was added to a stirred solution of 5-bromo-2-chloropyrimidine (12.76 g) and 3-ethynyl aniline (9.28 g) in DIPEA (120 mL) under an inert atmosphere. The reaction mixture was stirred at 80°C for 4 hours. The reaction mixture was filtered through diatomaceous earth and washed with DCM. The filtrate was purified by flash chromatography on silica using 0-30% EtOAc in DCM as eluent. The resultant solid was triturated with ether to give the title compound as a cream solid (4.28 g, 28%);
¹H NMR (DMSO-d₆) 5.31 (s, 2H), 6.64 (dd, 1H), 6.69-6.76 (m, 2H), 7.08 (dd, 1H), 8.94 (s, 2H);
MS m/e (MH+ MeCN)⁺ 271.

### Intermediate 9

### N-(5-tert-butylisoxazol-3-yl)-N'-{3-[(2-chloropyrimidin-5-yl)ethynyl]phenyl}urea

Phenyl (5-tert-butylisoxazol-3-yl)carbamate (Intermediate 4) (526 mg) was added to a stirred solution of {3-[(2-chloropyrimidin-5-yl)ethynyl]phenyl}amine (Intermediate 8) (387 mg) and triethylamine (0.28 mL) in THF (10 mL). The reaction mixture was heated at 75°C for 4 hours. The solvent was evaporated and the residue was purified by trituration with ether (20 mL) to give the title compound as a white solid (520 mg, 78%);
¹H NMR (DMSO-d₆) 1.28 (s, 9H), 6.49 (s, 1H), 7.23-7.27 (m, 1H), 7.36-7.41 (m, 2H), 7.88 (s, 1H), 8.96 (s, 1H), 9.00 (s, 2H), 9.58 (s, 1H);
MS m/e MH⁺ 396.

Examples 26 to 31 were prepared by an analogous method to Example 25 (using the appropriate starting materials).

### Example 26

### N-(5-tert-butylisoxazol-3-yl)-N'-(3-{[2-(dimethylamino)pyrimidin-5-yl]ethynyl}phenyl)urea

Starting Materials: Intermediate 9 and dimethylamine.
¹H NMR (DMSO-d₆) 1.29 (s, 9H), 3.15 (s, 6H), 6.49 (s, 1H), 7.12-7.16 (m, 1H), 7.30-7.34 (m, 2H), 7.74 (s, 1H), 8.52 (s, 2H), 8.88 (s, 1H), 9.54 (s, 1H);
MS m/e MH⁺ 405.

### Example 27

### N-(5-tert-butylisoxazol-3-yl)-N'-[3-({2-[(2-morpholin-4-ylethyl)amino]pyrimidin-5-yl}ethynyl)phenyl]urea

Starting Materials: Intermediate 9 and 4-(2-aminoethyl)morpholine.
¹H NMR (DMSO-d₆) 1.29 (s, 9H), 2.37-2.41 (m, 4H), 2.44-2.51 (m, 2H + DMSO), 3.39-3.46 (m, 2H), 3.53-3.58 (m, 4H), 6.48 (s, 1H), 7.11-7.16 (m, 1H), 7.30-7.34 (m, 2H), 7.50 (t, 1H), 7.72 (s, 1H), 8.48 (s, 2H), 8.89 (s, 1H);
MS m/e MH⁺ 490.

### Example 28

### N-(5-tert-butylisoxazol-3-yl)-N'-[3-({2-[(3-morpholin-4-ylpropyl)amino] pyrimidin-5-yl}ethynyl)phenyl]urea

Starting Materials: Intermediate 9 and 4-(3-aminopropyl)morpholine.
¹H NMR (DMSO-d₆) 1.29 (s, 9H), 1.69 (m, 2H), 2.29-2.35 (m, 6H), 3.28-3.37 (m, 2H), 3.54-3.57 (m, 4H), 6.48 (s, 1H), 7.11-7.15 (m, 1H), 7.30-7.34 (m, 2H), 7.69 (t, 1H), 7.73 (s, 1H), 8.46 (bs, 2H), 8.88 (s, 1H);
MS m/e MH⁺ 504.

### Example 29

### N-(5-tert-butylisoxazol-3-yl)-N'-[3-({2-[(2-methoxyethyl)amino]pyrimidin-5-yl}ethynyl)phenyl]urea

Starting Materials: Intermediate 9 and 2-methoxyethylamine.
¹H NMR (DMSO-d₆) 1.29 (s, 9H), 3.24 (s, 3H), 3.45-3.49 (m, 4H), 6.49 (s, 1H), 7.12-7.16 (m, 1H), 7.31-7.35 (m, 2H), 7.61-7.66 (m, 1H), 7.73 (s, 1H), 8.47 (s, 2H), 8.88 (s, 1H), 9.55 (s, 1H);
MS m/e MH⁺ 435.

### Example 30

### N-(5-tert-butylisoxazol-3-yl)-N'-{3-[(2-{[3-(1H-imidazol-1-yl)propyl]amino}pyrimidin-5 yl)ethynyl]phenyl}urea

Starting Materials: Intermediate 9 and 1-(3-aminopropyl)imidazole.
¹H NMR (DMSO-d₆) 1.29 (s, 9H), 1.96 (qt, 2H), 3.21-3.32 (m, 2H + H₂O), 4.02 (t, 2H), 6.48 (s, 1H), 6.88 (s, 1H), 7.12-7.16 (m, 1H), 7.19 (s, 1H), 7.30-7.33 (m, 2H), 7.62 (s, 1H), 7.73 (s, 1H), 7.77 (t, 1H), 8.48 (s, 2H), 8.92 (s, 1H);
MS m/e MH⁺ 485.

### Example 31

### N-(5-tert-butylisoxazol-3-yl)-N'-[3-({2-[(3-methoxypropyl)amino]pyrimidin-5-yl}ethynyl)phenyl]urea

Starting Materials: Intermediate 9 and 3-methoxypropylamine.
¹H NMR (DMSO-d₆) 1.29 (s, 9H), 1.75 (m, 2H), 3.21 (s, 3H), 3.30-3.39 (m, 4H), 6.49 (s, 1H), 7.12-7.16 (m, 1H), 7.30-7.33 (m, 2H), 7.66 (t, 1H), 7.72 (s, 1H), 8.47 (s, 2H), 8.94 (s, 1H);
MS m/e M-H⁺ 447.

Examples 32 to 46 were prepared by an analogous method to Example 25 (using the appropriate starting materials). Purification was by flash chromatography on silica using 1-12% MeOH/NH₃ in DCM as eluent. The resultant solid was then triturated with ether.

### Example 32

### N-(5-tert-butylisoxazol-3-yl)-N'-[3-({2-[(2-hydroxyethyl)amino]pyrimidin-5-yl}ethynyl)phenyl]urea

Starting Materials: Intermediate 9 and 2-aminoethanol.
¹H NMR (DMSO-d₆) 1.29 (s, 9H), 3.32-3.40 (m, 2H), 3.48-3.56 (m, 2H), 4.67 (t, 1H), 6.49 (s, 1H), 7.12-7.16 (m, 1H), 7.30-7.32 (m, 2H), 7.54 (t, 1H), 7.72 (s, 1H), 8.45 (bs, 2H), 8.88 (s, 1H), 9.54 (s, 1H);
MS m/e MH⁺ 421.

### Example 33

### N-(5-tert-butylisoxazol-3-yl)-N'-[3-({2-[(2-pyrrolidin-1-ylethyl)amino]pyrimidin-5-yl}ethynyl)phenyl]urea

Starting Materials: Intermediate 9 and 1-(2-aminoethyl)pyrrolidine.
¹H NMR (DMSO-d₆) 1.29 (s, 9H), 1.63-1.72 (m, 4H), 2.42-2.55 (m, 4H+DMSO), 2.55-2.62 (m, 2H), 3.37-3.46 (m, 2H). 6.49 (s, 1H), 7.12-7.17 (m, 1H), 7.28-7.35 (m, 2H), 7.51 (t, 1H), 7.72 (s, 1H), 8.46 (s, 2H), 8.89 (s, 1H), 9.55 (s, 1H);
MS m/e MH⁺ 474.

### Example 34

### N-(5-tert-butylisoxazol-3-yl)-N'-[3-({2-[(3-pyrrolidin-1-ylpropyl)amino]pyrimidin-5-yl}ethynyl)phenyl]urea

Starting Materials: Intermediate 9 and 1-(3-aminopropyl)pyrrolidine.
¹H NMR (DMSO-d₆) 1.29 (s, 9H), 1.64-1.75 (m, 6H), 2.36-2.46 (m, 6H), 3.27-3.37 (m, 2H), 6.49 (s, 1H), 7.11-7.16 (m, 1H), 7.30-7.34 (m, 2H), 7.65-7.74 (m, 2H), 8.46 (s, 2H), 8.88 (s, 1H), 9.54 (s, 1H);
MS m/e MH⁺ 488.

### Example 35

### N-[3-({2-[(2-aminoethyl)amino]pyrimidin-5-yl}ethynyl)phenyl]-N'-(5-tert-butylisoxazol-3-yl)urea

Starting Materials: Intermediate 9 and ethylenediamine.
¹H NMR (DMSO-d₆) 1.29 (s, 9H), 2.68 (t, 2H), 3.24-3.36 (m, 2H+ H₂O), 6.49 (s, 1H), 7.11-7.16 (m, 1H), 7.31-7.35 (m, 2H), 7.61 (t, 1H), 7.73 (s, 1H), 8.46 (s, 2H), 8.92 (s, 1H);
MS m/e MH⁺ 420.

### Example 36

### N-[3-({2-[(3-aminopropyl)amino]pyrimidin-5-yl}ethynyl)phenyl]-N'-(5-tert-butylisoxazol-3-yl)urea

Starting Materials Intermediate 9 and 1-(3-diaminopropane).
¹H NMR (DMSO-d₆) 1.29 (s, 9H); 1.60 (m, 2H), 2.60 (t, 2H), 3.22-3.39 (m, 2H+ H₂O), 6.49 (s, 1H), 7.10-7.15 (m, 1H), 7.29-7.36 (m, 2H), 7.66-7.74 (m, 2H), 8.62 (s, 2H), 8.99 (s, 1H);
MS m/e MH⁺ 434.

### Example 37

### N-(5-tert-butylisoxazol-3-yl)-N'-{3-[(2-{[2-(dimethylamino)ethyl]amino}pyrimidin-5-yl)ethynyl]phenyl}urea

Starting Materials: Intermediate 9 and 1-(2-dimethylaminoethylamine).
¹H NMR (DMSO-d₆) 1.29 (s, 9H), 2.16 (s, 6H), 2.40 (t, 2H), 3.39 (dd, 2H), 6.49 (s, 1H), 7.12-7.16 (m, 1H), 7.31-7.34 (m, 2H), 7.46 (t, 1H), 7.72 (s, 1H), 8.46 (s, 2H), 8.88 (s, 1H), 9.55 (s, 1H);
MS m/e MH⁺ 448.

### Example 38

### N-(5-tert-butylisoxazol-3-yl)-N'-{3-[(2-{[3-(dimethylamino)propyl]amino}pyrimidin-5-yl)ethynyl]phenyl}urea

Starting Materials: Intermediate 9 and 1-(3-dimethylaminopropylamine).
¹H NMR (DMSO-d₆) 1.29 (s, 9H), 1.65 (m, 2H), 2.11 (s, 6H), 2.24 (t, 2H), 3.25-3.35 (m, 2H+ H₂O), 6.49 (s, 1H), 7.10-7.16 (m, 1H), 7.30-7.34 (m, 2H), 7.68 (t, 1H), 7.72 (s, 1H), 8.45 (s, 2H), 8.89 (s, 1H), 9.55 (s, 1H);
MS m/e MH⁺ 462.

### Example 39

### N-2-(5-{[3-({[(5-tert-butylisoxazol-3-yl)amino]carbonyl}amino)phenyl]ethynyl}pyrimidin-2-yl)glycinamide

Starting Materials: Intermediate 9 and glycinamide.
¹H NMR (DMSO-d₆) 1.29 (s, 9H), 3.85 (d, 2H), 6.49 (s, 1H), 6.96 (bs, 1H), 7.12-7.17 (m, 1H), 7.30-7.36 (m, 3H), 7.64 (t, 1H), 7.73 (s, 1H), 8.49 (s, 2H), 8.89 (s, 1H), 9.55 (s, 1H);
MS m/e MH⁺ 456.

### Example 40

### N-3-(5-{[3-({[(5-tert-butylisoxazol-3-yl)amino]carbonyl}amino)phenyl]ethynyl}pyrimidin-2-yl)-beta-alaninamide

Starting Materials: Intermediate 9 and beta-alaninamide.
¹H NMR (DMSO-d₆) 1.29 (s, 9H), 2.35 (t, 2H), 3.49 (dd, 2H), 6.49 (s, 1H), 6.79 (bs, 1H), 7.12-7.16 (m, 1H), 7.31-7.3 4 (m, 3H). 7.59 (t, 1H), 7.73 (s, 1H), 8.47 (s, 2H), 8.88 (s, 1H), 9.55 (s, 1H);
MS m/e MH⁺ 448.

### Example 41

### N-(5-tert-butylisoxazol-3-yl)-N'-{3-[(2-{[2-(1H-imidazol-4-yl)ethyl]amino}pyrimidin-5-yl)ethynyl]phenyl}urea

Starting Materials: Intermediate 9 and histamine.
¹H NMR (DMSO-d₆) 1.29 (s, 9H), 2.76 (t, 2H), 3.52 (dd, 2H), 6.49 (s, 1H), 6.80 (s, 1H), 7.14 (t, 1H), 7.30-7.34 (m, 2H), 7.51 (s, 1H), 7.70-7.74 (m, 2H), 8.47 (s, 2H), 8.88 (s, 1H), 9.55 (s, 1H);
MS m/e MH⁺ 471.

### Example 42

### N-(5-tert-butylisoxazol-3-yl)-N'-[3-({2-[(2-pyridin-2-ylethyl)amino]pyrimidin-5-yl}ethynyl)phenyl]urea

Starting Materials: Intermediate 9 and 2-(2-aminoethyl)pyridine.
¹H NMR (DMSO-d₆) 1.29 (s, 9H), 3.00 (t, 2H), 3.66 (dd, 2H), 6.50 (s, 1H), 7.11-7.29 (m, 3H), 7.30-7.34 (m, 2H), 7.65-7.76 (m, 3H), 8.44-8.51 (m, 3H), 8.88 (s, 1H), 9.55 (s, 1H);
MS m/e MH⁺ 482.

### Example 43

### N-(5-tert-butylisoxazol-3-yl)-N'-{3-[(2-{[3-(isopropylamino)propyl]amino}pyrimidin-5-yl)ethynyl]phenyl}urea

Starting Materials: Intermediate 9 and N-isopropyl-1,3-propanediamine.
¹H NMR (DMSO-d₆) 0.95 (d, 6H), 1.29 (s, 9H), 1.63 (m, 2H), 2.46-2.56 (m, 2H+DMSO), 2.66 (m, 1H), 3.26-3.38 (m, 2H+H₂O), 6.49 (s, 1H), 7.10-7.16 (m, 1H), 7.30-7.34 (m, 2H), 7.70-7.74 (m, 2H), 8.45 (s, 2H), 8.89 (s, 1H);
MS m/e MH⁺ 476.

### Example 44

### N-(5-tert-butylisoxazol-3-yl)-N'-{3-[(2-{[3-(4-methylpiperazin-1-yl)propyl]amino}pyrimidin-5-yl)ethynyl] phenyl}urea

Starting Materials: Intermediate 9 and 1-(3-aminopropyl)-4-methylpiperazine.
¹H NMR (DMSO-d₆) 1.29 (s, 9H), 1.66 (m, 2H), 2.13 (s, 3H), 2.34-2.36 (m, 10H), 3.26-3.35 (m, 2H+H₂O), 6.49 (s, 1H), 7.11-7.16 (m; 1H), 7.30-7.34 (m, 2H), 7.67 (t, 1H), 7.72 (s, 1H), 8.45 (s, 2H), 8.88 (s, 1H), 9.55 (s, 1H);
MS m/e MH⁺ 517.

### Example 45

### N-(5-tert-butylisoxazol-3-yl)-N'-[3-({2-[(2-pyridin-4-ylethyl)amino]pyrimidin-5-yl}ethynyl)phenyl]urea

Starting Materials: Intermediate 9 and 4-(2-aminoethyl)pyridine.
¹H NMR (DMSO-d₆) 1.29 (s, 9H), 2.87 (t, 2H), 3.58 (q, 2H), 6.49 (s, 1H), 7.11-7.16 (m, 1H), 7.22-7.27 (m, 2H), 7.30-7.34 (m, 2H). 7.72 (s, 1H), 7.76 (t, 1H), 8.42-8.49 (m, 4H), 8.88 (s, 1H), 9.55 (s, 1H);
MS m/e MH⁺ 482.

### Example 46

### N-(5-tert-butylisoxazol-3-yl)-N'-[3-({2-[(3-piperidin-1-ylpropyl)amino]pyrimidin-5-yl}ethynyl)phenyl]urea

Starting Materials: Intermediate 9 and 1-(3-aminopropyl)piperidine.
¹H NMR (DMSO-d₆) 1.29 (s, 9H), 1.33-1.47 (m, 2H), 1.44-1.53 (m, 4H), 1.67 (m, 2H), 2.27-2.37 (m, 6H), 3.24-3.35 (m, 2H+H₂O), 6.49 (s, 1H), 7.10-7.16 (m, 1H), 7.27-7.35 (m, 2H), 7.68-7.74 (m, 2H), 8.45 (s, 2H), 8.93 (s, 1H), 9.58 (s, 1H);
MS m/e MH⁺ 502.

### Example 47

### N-(5-methylisoxazol-3-yl)-N'-[3-({2-[(2-pyrrolidin-1-ylethyl)amino]pyrimidin-5-yl}ethynyl)phenyl]urea

Phenyl (3-methylisoxazol-5-yl)carbamate (Intermediate 3) (102 mg) was added to a stirred solution of 5-[(3-aminophenyl)ethynyl]-*N*-(2-pyrrolidin-1-ylethyl)pyrimidin-2-amine (Intermediate 10) (120 mg) and triethylamine (0.065 mL) in THF (10 mL). The reaction mixture was heated at 80°C for 3 hours. The solvent was evaporated and the product was purified by flash chromatography on silica using 0-10% MeOH/NH₃ in DCM as eluent. The resultant solid was triturated with ether to give the title compound as a white solid (121 mg, 72%);
¹H NMR (DMSO-d₆) 1.63-1.70 (m, 4H), 2.16 (s, 3H), 2.44-2.52 (m, 4H+DMSO), 2.57 (t, 2H), 3.37-3.45 (m, 2H), 5.95 (s, 1H), 7.13-7.17 (m, 1H), 7.29-7.39 (m, 2H), 7.53 (t, 1H), 7.69 (s, 1H), 8.46 (s, 2H), 8.93 (s, 1H), 10.12 (s, 1H);
MS m/e MH⁺ 432.

### Intermediate 10

### 5-[(3-aminophenyl)ethynyl]-N-(2-pyrrolidin-1-ylethyl)pyrimidin-2-amine

{3-[(2-chloropyrimidin-5-yl)ethynyl]phenyl}amine (Intermediate 8) (726 mg) and 1-(2-aminoethyl)-pyrrolidine (3.6 g) were stirred in acetonitrile (10 mL) and hydrogen chloride (1.0M solution in diethyl ether) (3.80 mL) was added dropwise. The reaction mixture was stirred and heated at 80°C for 3 hours. The solvent was evaporated and the product was purified by flash chromatography on silica using 0-10% MeOH/NH₃ in DCM as eluent to give the title compound as an off white solid (738 mg, 76%);
¹H NMR (DMSO-d₆) 1.63-1.69 (m, 4H), 2.42-2.52 (m, 4H+DMSO), 2.56 (m, 2H), 3.40 (m, 2H), 5.19 (s, 2H), 6.53 (dd, 1H), 6.61 (d, 1H), 6.67 (s, 1H), 7.01 (t, 1H), 7.46 (t, 1H), 8.41 (s, 2H);
MS m/e MH⁺ 308.

Intermediates 11 and 12 were prepared by an analogous method to Intermediate 10 by using Intermediate 8 and the appropriate amine.

### Intermediate 11

### 5-[(3-aminophenyl)ethynyl]-N-(2-morpholin-4-ylethyl)pyrimidin-2-amine

Starting Materials: Intermediate 8 and 1-(2-aminoethyl)morpholine.
¹H NMR (DMSO-d₆) 2.36-2.52 (m, 6H+DMSO), 3.41 (m, 2H), 3.55 (m, 4H), 5.19 (s, 2H), 6.54-6.57 (m, 1H), 6.61 (d, 1H), 6.67 (s, 1H), 7.01 (t, 1H), 7.43 (t, 1H), 8.41 (s, 2H);
MS m/e MH⁺ 324.

### Intermediate 12

### 5-[(3-aminophenyl)ethynyl]-N-(3-morpholin-4-ylpropyl)pyrimidin-2-amine

Starting Materials: Intermediate 8 and 1-(3-aminopropyl)morpholine.
¹H NMR (DMSO-d₆) 1.67 (m, 2H), 2.28-2.36 (m, 6H), 3.27-3.35 (m, 2H+H₂O), 3.53-3.58 (m, 4H), 5.19 (s, 2H), 6.54-6.58 (m, 1H), 6.60 (d, 1H), 6.66 (s, 1H), 7.01 (t, 1H), 7.62 (t, 1H), 8.66 (s, 2H);
MS m/e MH⁺ 338.

Examples 48 and 49 were prepared by an analogous method to Example 47, using Intermediate 10 with the appropriate phenyl carbamate.

### Example 48

### N-(5-tert-butyl-1,3,4-thiadiazol-2-yl)-N'-[3-({2-[(2-pyrrolidin-1-ylethyl)amino] pyrimidin-5-yl}ethynyl)phenyl]urea

Starting Materials: Intermediate 10 and Intermediate 2.
¹H NMR (DMSO-d₆) 1.38 (s, 9H), 1.65-1.73 (m, 4H), 2.46-2.58 (m, 4H+DMSO), 2.64 (t, 2H), 3.43 (m, 2H), 7.13 (d, 1H), 7.32 (t, 1H), 7.49-7.57 (t, 2H), 7.82 (s, 1H), 8.47 (s, 2H), 9.66 (bs, 1H), 11.40 (bs, 1H);
MS m/e MH⁺ 491.

### Example 49 .

### N-(3-methylisothiazol-5-yl)-N'-[3-({2-[(2-pyrrolidin-1-ylethyl)amino]pyrimidin-5-yl}ethynyl)phenyl]urea

Starting Materials: Intermediate 10 and Intermediate 7.
¹H NMR (DMSO-d₆) 1.63-1.70 (m, 4H), 2.28 (s, 3H), 2.44-2.52 (m, 4H+DMSO), 2.58 (t, 2H), 3.42 (m, 2H), 6.66 (s, 1H), 7.13-7.18 (m, 1H), 7.29-7.41 (m, 2H), 7.53 (t, 1H), 7.72 (s, 1H), 8.47 (s, 2H), 9.20 (s, 1H), 10.39 (s, 1H);
MS m/e MH⁺ 448.

Examples 50 to 56 were prepared by an analogous method to Example 1 by using Intermediate 10 with the appropriate isocyanate.

### Example 50

### N-(3-fluorophenyl)-N'-[3-({2-[(2-pyrrolidin-1-ylethyl)amino]pyrimidin-5 yl}ethynyl)phenyl]urea

Starting Materials: Intermediate 10 and 3-fluorophenylisocyanate.
¹H NMR (DMSO-d₆) 1.63-1.70 (m, 4H), 2.43-2.52 (m, 4H+DMSO), 2.57 (t, 2H), 3.37-3.46 (m, 2H), 6.74-6.82 (m, 1H), 7.11 (d, 2H), 7.25-7.36 (m, 3H), 7.44-7.55 (m, 2H), 7.72 (s, 1H), 8.46 (s, 2H), 8.83 (s, 1H), 8.94 (s, 1H);
MS m/e MH⁺ 445.

### Example 51

### N-(4-methoxyphenyl)-N'-[3-({2-[(2-pyrrolidin-1-ylethyl)amino]pyrimidin-5-yl}ethynyl)phenyl]urea

Starting Materials: Intermediate 10 and 4-methoxyphenylisocyanate.
¹H NMR (DMSO-d₆) 1.63-1.69 (m, 4H), 2.42-2.52 (m, 4H+DMSO), 2.57 (t, 2H), 3.41 (m, 2H), 3.71 (s, 3H), 6.83-6.89 (m, 2H), 7.04-7.10 (m, 1H), 7.24-7.38 (m, 4H), 7.51 (t, 1H), 7.71 (s, 1H), 8.43-8.51 (m, 3H), 8.67 (s, 1H);
MS m/e MH⁺ 457.

### Example 52

### N-(2-fluorophenyl)-N'-[3-({2-[(2-pyrrolidin-1-ylethyl)amino]pyrimidin-5-yl}ethynyl)phenyl]urea

Starting Materials: Intermediate 10 and 2-fluorophenylisocyanate.
¹H NMR (DMSO-d₆) 1.61-1.71 (m, 4H), 2.43-2.52 (m, 4H+DMSO), 2.57 (t, 2H), 3.38-3.46 (m, 2H), 6.96-7.05 (m, 1H), 7.09-7.32 (m, 5H), 7.51 (t, 1H), 7.74 (s, 1H), 8.11 (m, 1H), 8.47 (s, 2H), 8.56 (s, 1H), 9.13 (s, 1H);
MS m/e MH⁺ 445.

### Example 53

### N-(2,5-difluorophenyl)-N'-[3-({2-[(2-pyrrolidin-1-ylethyl)amino]pyrimidin-5-yl}ethynyl)phenyl]urea

Starting Materials: Intermediate 10 and 2,5-difluorophenylisocyanate.
¹H NMR (DMSO-d₆) 1.63-1.70 (m, 4H), 2.44-2.52 (m, 4H+DMSO), 2.57 (t, 2H), 3.41 (m, 2H), 6.78-6.86 (m, 1H), 7.11-7.16 (m, 1H), 7.24-7.34 (m, 3H), 7.52 (t, 1H), 7.74 (s, 1H), 8.02 (m, 1H), 8.47 (s, 2H), 8.78 (s, 1 H), 9.22 (s, 1H);
MS m/e MH⁺ 463.

### Example 54

### N-(3,4-difluorophenyl)-N'-[3-({2-[(2-pyrrolidin-1-ylethyl)amino]pyrimidin-5-yl}ethynyl)phenyl]urea

Starting Materials: Intermediate 10 and 3,4-difluorophenylisocyanate.
¹H NMR (DMSO-d₆) 1.63-1.71 (m, 4H), 2.42-2.53 (m, 4H+DMSO), 2.57 (t, 2H), 3.40 (m, 2H), 7.09-7.16 (m, 2H), 7.27-7.37 (m, 3H), 7.51 (t, 1H), 7.60-7.72 (m, 2H), 8.47 (s, 2H), 8.83 (s, 1H), 8.93 (s, 1H);
MS m/e MH⁺ 463.

### Example 55

### N-[2-fluoro-5-(trifluoromethyl)phenyl]-N'-[3-({2-[(2-pyrrolidin-1-ylethyl)amino]pyrimidin-5-yl}ethynyl)phenyl]urea

Starting Materials: Intermediate 10 and 2-fluoro-5-trifluoromethylphenylisocyanate.
¹H NMR (DMSO-d₆) 1.63-1.71 (m, 4H), 2.45-2.52 (m, 4H+DMSO), 2.58 (t, 2H), 3.37-3.45 (m, 2H), 7.12-7.16 (m, 1H), 7.26-7.56 (m, 5H), 7.77 (s, 1H), 8.47 (s, 2H), 8.59 (dd, 1H), 8.92 (s, 1H), 9.27 (s, 1H);
MS m/e MH⁺ 513.

### Example 56

### N-[3-({2-[(2-pyrrolidin-1-ylethyl)amino]pyrimidin-5-yl}ethynyl)phenyl]-N'-[4-(trifluoromethyl)phenyl]urea

Starting Materials: Intermediate 10 and 4-trifluoromethylphenylisocyanate.
¹H NMR (DMSO-d₆) 1.64-1.72 (m, 4H), 2.41-2.53 (m, 4H+DMSO), 2.57 (t, 2H), 3.41 (m, 2H), 7.12 (d, 1H), 7.29-7.39 (m, 2H), 7.52 (t, 1H), 7.60-7.68 (m, 4H), 7.72 (s, 1H), 8.46 (s, 2H), 8.88 (s, 1H), 9.12 (s, 1H);
MS m/e MH⁺ 495.

Examples 57 to 59 were prepared by an analogous method to Example 1 using Intermediate 10 with the appropriate isocyanate, except purification was by trituration with hot methanol.

### Example 57

### N-1,3-benzodioxol-5-yl-N'-[3-({2-[(2-pyrrolidin-1-ylethyl)amino]pyrimidin-5-yl}ethynyl)phenyl]urea

Starting Materials: Intermediate 10 and (3,4-methylenedioxy)phenyl isocyanate.
¹H NMR (DMSO-d₆) 1.62-1.71 (m, 4H), 2.42-2.53 (m, 4H+DMSO), 2.57 (t, 2H), 3.41 (m, 2H), 5.96 (s, 2H), 6.72-6.84 (m, 2H), 7.05-7.11 (m, 1H), 7.17-7.20 (m, 1H), 7.25-7.34 (m, 2H), 7.51 (t, 1H), 7.70 (s, 1H), 8.42-8.49 (s, 2H), 8.58 (s, 1H), 8.69 (s, 1H);
MS m/e MH⁺ 471.

### Example 58

### N-(4-fluorophenyl)-N'-[3-({2-[(2-pyrrolidin-1-ylethyl)amino]pyrimidin-5-yl}ethynyl)phenyl]urea

Starting Materials: Intermediate 10 and 4-fluorophenyl isocyanate.
¹H NMR (DMSO-d₆) 1.62-1.70 (m, 4H), 2.42-2.52 (m, 4H+DMSO), 2.57 (t, 2H), 3.41 (m, 2H), 7.06-7.16 (m, 3H), 7.26-7.36 (m, 2H), 7.40-7.55 (m, 3H), 7.71 (s, 1H), 8.46 (s, 2H), 8.71-8.77 (m, 2H);
MS m/e MH⁺ 445.

### Example 59

### N-(3-chlorophenyl)-N'-[3-({2-[(2-pyrrolidin-1-ylethyl)amino]pyrimidin-5-yl}ethynyl)phenyl] urea

Starting Materials: Intermediate 10 and 3-chlorophenyl isocyanate.
¹H NMR (DMSO-d₆) 1.63-1.70 (m, 4H), 2.43-2.52 (m, 4H+DMSO), 2.57 (t, 2H), 3.41 (m, 2H), 7.02 (dt, 1H), 7.11 (d, 1H), 7.22-7.37 (m, 4H), 7.52 (t, 1H), 7.69-7.74 (m, 2H), 8.46 (s, 2H), 8.85 (s, 1H), 8.92 (s, 1H);
MS m/e MH⁺ 461.

### Example 60

### N-(5-methylisoxazol-3-yl)-N'-[3-({2-[(2-morpholin-4-ylethyl)amino]pyrimidin-5-yl}ethynyl)phenyl]urea

Example 60 was prepared by an analogous method to Example 47 using Intermediate 11 and Intermediate 3.
¹H NMR (DMSO-d₆) 2.16 (s, 3H), 2.36-2.52 (m, 6H+DMSO), 3.43 (m, 2H), 3.52-3.58 (m, 4H), 5.95 (s, 1H), 7.12-7.18 (m, 1H), 7.29-7.38 (m, 2H), 7.49 (t, 1H), 7.70 (s, 1H), 8.46 (s, 2H), 8.92 (s, 1H), 10.12 (s, 1H);
MS m/e MH⁺ 448.

### Example 61

### N-(5-tert-butyl-1,3,4-thiadiazol-2-yl)-N'-[3-({2-[(2-morpholin-4-ylethyl)amino]pyrimidin-5-yl}ethynyl)phenyl] urea

Example 61 was prepared by an analogous method to Example 47, using Intermediate 11 and Intermediate 2. Purification was by trituration with methanol.
¹HNMR (DMSO-d₆) 1.38 (s, 9H), 2.36-2.52 (m, 6H+DMSO), 3.43 (m, 2H), 3.53-3.58 (m, 4H), 7.15 (d, 1H), 7.33 (t, 1H), 7.39-7.45 (m, 1H), 7.49 (t, 1H), 7.76 (s, 1H), 8.47 (s, 2H), 9.16 (s, 1H);
MS m/e MH⁺ 507.

### Example 62

### N-[2-fluoro-5-(trifluoromethyl)phenyl]-N'-[3-({2-[(2-morpholin-4-ylethyl)amino]pyrimidin-5-yl}ethynyl)phenyl]urea

Example 62 was prepared by an analogous method to Example 1, using Intermediate 11 and 2-fluoro-5-trifluoromethylphenyl isocyanate.
¹H NMR (DMSO-d₆) 2.36-2.52 (m, 6H+DMSO), 3.43 (m, 2H), 3.52-3.58 (m, 4H), 7.14 (d, 1H), 7.26-7.34 (m, 2H), 7.35-7.42 (m, 1H), 7.45-7.53 (m, 2H), 7.78 (s, 1H), 8.47 (s, 2H), 8.59 (dd, 1H), 8.91 (s, 1H), 9.25 (s, 1H);
MS m/e MH⁺ 529.

Examples 63 and 64 were prepared by an analogous method to Example 47 except using Intermediate 12 in place of Intermediate 10 with the appropriate phenylcarbamate.

### Example 63

### N-(5-methylisoxazol-3-yl)-N'-[3-({2-[(3-morpholin-4-ylpropyl)amino] pyrimidin-5-yl}ethynyl)phenyl]urea

Starting Materials: Intermediate 12 and Intermediate 3.
¹H NMR (DMSO-d₆) 1.68 (m, 2H), 2.16 (s, 3H), 2.29-2.37 (m, 6H), 3.26-3.37 (m, 2H+H₂O), 3.53-3.58 (m, 4H), 5.95 (s, 1H), 7.13-7.17 (m, 1H), 7.29-7.38 (m, 2H), 7.66-7.72 (m, 2H), 8.46 (s, 2H), 8.93 (s, 1H), 10.12 (s, 1H);
MS m/e MH⁺ 462.

### Example 64

### N-(5-tert-butyl-1,3,4-thiadiazol-2-yl)-N'-[3-({2-[(3-morpholin-4-ylpropyl)amino]pyrimidin-5-yl}ethynyl)phenyl]urea

Starting Materials: Intermediate 12 and Intermediate 2.
¹H NMR (DMSO-d₆) 1.38 (s, 9H), 1.68 (m, 2H), 2.29-2.39 (m, 6H), 3.26-3.37 (m, 2H+H₂O), 3.53-3.58 (m, 4H), 7.15 (d, 1H), 7.33 (t, 1H), 7.40-7.45 (m, 1H), 7.68 (t, 1H), 7.76 (s, 1H), 8.46 (s, 2H), 9.21 (s, 1H);
MS m/e MH⁺ 521.

### Example 65

### N-[2-fluoro-5-(trifluoromethyl)phenyl]-N'-[3-({2-[(3-morpholin-4-ylpropyl)amino]pyrimidin-5-yl}ethynyl)phenyl]urea

Example 65 was prepared by an analogous method to Example 1 using Intermediate 12 and 2-fluoro-5-trifluoromethylphenyl isocyanate.
¹H NMR (DMSO-d₆) 1.68 (m, 2H), 2.29-2.37 (m, 6H), 3.25-3.37 (m, 2H+H₂O), 3.53-3.58 (m, 4H), 7.14 (dt, 1H), 7.26-7.34 (m, 2H), 7.35-7.42 (m, 1H), 7.45-7.53 (m, 1H), 7.68 (t, 1H), 7.78 (s, 1H), 8.47 (s, 2H), 8.59 (dd, 1H), 8.91 (d, 1H), 9.25 (s, 1H);
MS m/e MH⁺ 543.

### Example 66

### N-(5-methylisoxazol-3-yl)-N'-[4-({2-[(2-pyrrolidin-1-ylethyl)amino]pyrimidin-5-yl}ethynyophenyl]urea

Example 66 was prepared by an analogous method to Example 47 using Intermediate 14 and Intermediate 3.
¹H NMR (DMSO-d₆) 1.66-1.70 (m, 4H), 2.18 (s, 3H), 2.46-2.52 (m, 4H+DMSO), 2.59 (t, 2H), 3.37-3.45 (m, 2H), 5.97 (s, 1H), 7.43-7.53 (m, 5H), 8.44 (s, 2H), 9.02 (s, 1H), 10.11 (s, 1H);
MS m/e MH⁺ 432.

### Intermediate 13

### {4-[(2-chloropyrimidin-5-yl)ethynyl]phenyl}amine

Intermediate 13 was prepared by an analogous method to Intermediate 8, except using 4-ethynylaniline in place of 3-ethynylaniline.
¹H NMR (DMSO-d₆) 5.72 (s, 2H), 6.57 (d, 2H), 7.23 (d, 2H), 8.85 (s, 2H);
MS m/e (M+CH₃CN)⁺ 271.

### Intermediate 14

### 5-[(4-aminophenyl)ethynyl]-N-(2-pyrrolidin-1-ylethyl)pyrimidin-2-amine

Intermediate 14 was prepared by an analogous method to Intermediate 10, by using Intermediate 13 in place of Intermediate 8.
¹H NMR (DMSO-d₆) 1.62-1.69 (m, 4H), 2.42-2.51 (m, 4H+DMSO), 2.56 (m, 2H), 3.39 (m, 2H), 5.49 (s, 2H), 6.52 (d, 2H), 7.14 (d, 2H), 7.35 (t, 1H), 8.35 (s, 2H);
MS m/e MH⁺ 308.

### Example 67

### N-(5-tert-butylisoxazol-3-yl)-N'-[4-({2-[(2-pyrrolidin-1-ylethyl)amino] pyrimidin-5-yl}ethynyl)phenyl]urea

Example 67 was prepared by an analogous method to Example 47 but using Intermediate 14 and Intermediate 4.
¹H NMR (DMSO-d₆) 1.30 (s, 9H), 1.65-1.71 (m, 4H), 2.44-2.53 (m, 4H+DMSO), 2.59 (m, 2H), 3.42 (m, 2H), 6.51 (s, 1H), 7.42-7.52 (m, 5H), 8.44 (s, 2H), 8.98 (s, 1H), 9.55 (s, 1H);
MS m/e MH⁺ 474.

### Example 68

### N-(5-tert-butyl-1,3,4-thiadiazol-2-yl)-N'-[4-({2-[(2-pyrrolidin-1-ylethyl)amino]pyrimidin-5-yl}ethynyl)phenyl]urea

Example 68 was prepared by an analogous method to Example 47 but using Intermediate 14 and Intermediate 2. Purification was by trituration with ether and methanol.
¹H NMR (DMSO-d₆) 1.40 (s, 9H), 1.67-1.72 (m, 4H), 2.48-2.56 (m, 4H+DMSO), 2.63 (t, 2H), 3.40-3.47 (m, 2H), 7.45 (d, 2H), 7.49 (t, 1H), 7.61 (d, 2H), 8.45 (s, 2H), 9.55 (bs, 1H), 11.45 (bs, 1H);
MS m/e MH⁺ 491.

### Example 69

### N-[2-fluoro-5-(trifluoromethyl)phenyl]-N'-[4-({2-[(2-pyrrolidin-1-ylethyl)amino]pyrimidin-5-yl}ethynyl)phenyl]urea

Example 69 was prepared by an analogous method to Example 1 but using Intermediate and 2-fluoro-5-trifluoromethylphenyl isocyanate.
¹H NMR (DMSO-d₆) 1.63-1.70 (m, 4H), 2.45-2.51 (m, 4H+DMSO), 2.56 (t, 2H), 3.36-3.45 (m, 2H), 7.36-7.53 (m, 7H), 8.43 (s, 2H), 8.59 (dd, 1H), 8.93 (s, 1H), 9.34 (s, 1H);
MS m/e MH⁺ 513.

### Example 70

### N-(5-{[3-({[(5-tert-butylisoxazol-3-yl)amino]carbonyl}amino)phenyl]ethynyl}pyrimidin-2-yl)-2-(2-methoxyethoxy)acetamide

N-{3-[(2-aminopyrimidin-5-yl)ethynyl]phenyl}-N'-(5-tert-butylisoxazol-3-yl)urea (Example 22) (100 mg) and 2-(2-methoxyethoxy)acetic acid (71 mg) were stirred in pyridine (8 mL) and phosphorous oxychloride (0.050 mL) was added dropwise. The reaction mixture was stirred at 25 °C for 1 hour. The solvent was evaporated and the residue was purified by reverse phase HPLC using 0-100% MeCN in water (0.2% TFA) as eluent to give the title compound as an off-white solid. (15 mg, 12%);
¹H NMR (CDCl₃) 1.29 (s, 9H), 3.41 (s, 3H), 3.54-3.59 (m, 2H), 3.71-3.76 (m, 2H), 4.16 (s, 2H), 5.84 (s, 1H), 7.18-7.29 (m, 2H), 7.46-7.51 (m, 1H), 7.71 (s, 1H), 7.82 (bs, 1H), 8.67 (s, 2H), 9.28 (bs, 1H), 9.59 (s, 1H);
MS m/e MH⁺ 493.

### Example 71

### N-{6-[(2-aminopyrimidin-5-yl)ethynyl]pyridin-2-yl}-N'-(5-tert-butylisoxazol-3-yl)urea

PdCl₂dppf (37 mg) was added to a degassed solution of *N*-(5-*tert*-butylisoxazol-3-yl)-*N'*-(6-iodopyridin-2-yl)urea (Intermediate 16) (550 mg), 5-ethynylpyrimidin-2-amine (Intermediate 18) (119 mg), CuI (4 mg) and Et₃N (5 mL) in DMF (20 mL). The reaction was stirred at ambient temperature under an inert atmosphere for 24 hours. The solvent was evaporated *in vacuo* and the residue diluted with DCM (30 mL) and water (20 mL). The organic phase was separated and then washed with brine (20 mL), dried (MgSO₄), filtered and concentrated. Purification by flash chromatography on silica using 0-10% MeOH in DCM as the eluent followed by reverse phase HPLC, gradient H₂O:MeCN (0-70%) gave the title compound as a beige solid (28 mg, 7%);
¹H NMR (DMSO-d₆) 1.33 (s, 9H), 6.56 (s, 1H), 7.26 (s, 1H), 7.28 (s, 2H), 7.67 (d, 1H), 7.83 (t, 1H), 8.49 (s, 2H), 9.67 (s, 1H), 10.53 (s, 1H);
MS m/e MH⁺ 378.
**Intermediate 15**

### 6-iodopyridin-2-amine

2-Bromo-6-aminopyridine (519 mg), CuI (29 mg), NaI (899 mg) and N,N-dimethylethylenediamine (26 mg) in dioxane (5 mL) were heated at 110 °C under an inert atmosphere for 6 hours. Concentrated NH₃ (8 mL) then water (10 mL) was added. The product was then extracted with DCM (3 x 15 mL), dried (MgSO₄), filtered and concentrated. The product was purified by flash chromatography on silica using 0-10% MeOH in DCM as the eluent to give the title compound as a beige solid (530 mg, 80%);
¹H NMR (CDCl₃) 4.60 (s, 2H), 6.41-6.44 (m, 1H), 7.00-7.08 (m, 2H);
MS m/e MH⁺ 221.

### Intermediate 16

### N-(5-tert-butylisoxazol-3-yl)-N'-(6-iodopyridin-2-yl)urea

Intermediate 16 was prepared by an analogous method to Example 24 using Intermediate 15 and Intermediate 2. Purification was by flash chromatography on silica using 0-10% methanol in dichloromethane as the eluent.
MS m/e MH⁺ 378.

### Intermediate 17

### 5-[(trimethylsilyl)ethynyl]pyrimidin-2-amine

PdCl₂dppf (146 mg) was added to a solution of 2-amino-5-iodopyrimidine (221 mg), trimethylsilylacetylene (491 mg), CuI (57 mg) and DIPEA (259 mg) in EtOAc (5 mL) at -20°C under an inert atmosphere. The reaction was allowed to warm to ambient temperature and stirred for 6 hours. The reaction mixture was diluted with water (10 mL). The organic layer was separated, dried (MgSO₄), filtered and concentrated. The crude product was used directly without further purification (191 mg, 100%);
¹H NMR (CDCl₃); 0.26 (s, 9H), 5.19 (bs, 2H), 8.39 (s, 2H);
MS m/e MH⁺+MeCN 233.

### Intermediate 18

### 5-ethynylpyrimidin-2-amine

K₂CO₃ (276 mg) was added to a solution of 5-[(trimethylsilyl)ethynyl]pyrimidin-2-amine (Intermediate 17) (191 mg) in MeOH (40 mL):water (20 mL). The reaction mixture was allowed to stir at ambient temperature under an inert atmosphere for 24 hours then neutralised with HCl (1M). The reaction mixture was then concentrated and the resultant residue dissolved in DCM (30 mL). The DCM phase was washed with water (15 mL), brine (15 mL), dried (MgSO₄), filtered and concentrated. The crude product was used directly without further purification (119 mg, 100%);
¹H NMR (CDCl₃); 3.19 (s, 1H), 5.26 (bs, 2H), 8.41 (s, 2H);
MS m/e NH⁺+MeCN 161.

### Example 72

### N-{2-[(2-aminopyrimidin-5-yl)ethynyl]pyridin-4-yl}-N'-(5-tert-butylisoxazol-3-yl)urea

Example 72 was prepared by an analogous method to Example 71 but using Intermediate 20 and Intermediate 18 and without RPHPLC purification.
¹H NMR (DMSO-d₆) 1.32 (s, 9H), 6.54 (s, 1H), 7.24 (s, 2H), 7.24-7.36 (m, 1H), 7.77 (s, 1H), 8.39-8.50 (m, 3H), 9.28 (s, 1H), 9.82 (s, 1H);
MS m/e MH⁺ 378.

### Intermediate 19

### 2-iodopyridin-4-amine

Intermediate 19 was prepared by an analogous method to Intermediate 15 but using 2-bromo-4-aminopyridine in place of 2-bromo-6-aminopyridine.
MS m/e MH⁺ 221.

### Intermediate 20

### N-(5-tert-butylisoxazol-3-yl)-N'-(2-iodopyridin-4-yl)urea

Intermediate 20 was prepared by an analogous method to Example 24 using Intermediate 2 and Intermediate 19. Purification was by flash chromatography on silica using 0-10% methanol in dichloromethane as the eluent.
MS m/e MH⁺ 387.

### Example 73

### N-{5-[(2-aminopyrimidin-5-yl)ethynyl]-1,3-thiazol-2-yl}-N'-[2-fluoro-5-(trifluoromethyl)phenyl]urea

A mixture of 2-amino-5-ethynylpyrimidine (Intermediate 18) (60 mg), *N*-(5-bromo-1,3-thiazol-2-yl)-*N*'-[2-fluoro-5-(trifluoromethyl)phenyl]urea (Intermediate 21) (192 mg), 1,1,3,3-tetramethylguanidine (69 mg), and copper (I) iodide (5 mg) in dry DMF (1.5 mL) was stirred and degassed with nitrogen. Tetrakis(triphenylphosphine)palladium(0) (58 mg) was added and the mixture heated at 80°C for 2.5 hours. The mixture was cooled, stirred, and diluted with water (15 mL). The solid formed was filtered off and dried. Purification by flash chromatography on silica using 0-30% MeOH in DCM as eluent, then trituration with DCM gave the title compound as a solid (60 mg, 28%);
¹H NMR (DMSO-d₆) 7.16(s, 2H), 7.45-7.60 (m, 2H), 7.70 (s, 1H), 8.42 (s, 2H), 8.53 (m, 1H), 9.23 (bs, 1H), 11.15 (bs, 1H);
MS m/e MH⁺ 423.

### Intermediate 21

### N-(5-bromo-1,3-thiazol-2-yl)-N'-[2-fluoro-5-(trifluoromethyl)phenyl]urea

2-Fluoro-5-(trifluoromethyl)phenyl isocyanate (1.02 g) was added to a stirred solution of 2-amino-5-bromothiazole (0.90 g) in dry DCM (20 mL) at ambient temperature. After 17 hours the precipitate was filtered off, washed with DCM then isohexane, and dried to give the title compound (1.38 g, 71%);
¹H NMR (DMSO-d₆) 7.45-7.60 (m, 3H), 8.50 (m, 1H), 9.20 (bs, 1H), 11.05 (bs, 1H);
MS m/e MH⁺ 384, 386 (1 x Br).

### Example 74

### N-{5-[(2-aminopyrimidin-5-yl)ethynyl]-1,3,4-thiadiazol-2-yl}-N'-[2-fluoro-5-(trifluoromethyl)phenyl]urea

Example 74 was prepared by an analogous method to Example 73 but using Intermediate 22 and Intermediate 18.
¹H NMR (DMSO-d₆) 7.36 (s, 2H), 7.50-7.60 (m, 2H), 8.48 (m, 1H), 8.53 (s, 2H), 9.30 (bs, 1H), 11.50 (bs, 1H);
MS m/e MH⁺ 424.

### Intermediate 22

### N-(5-bromo-1,3,4-thiadiazol-2-yl)-N'-[2-fluoro-5-(trifluoromethyl)phenyl]urea

2-Fluoro-5-(trifluoromethyl)phenyl isocyanate (1.02 g) was added to a stirred solution of 2-amino-5-bromo-1,3,4-thiadiazole {Eur.J.Med.Chem.Chim.Ther. (1975) 121} (0.90 g) in dry THF (50 mL) at 50°C. After 17 hours at ambient temperature the solvent was evaporated and the residue was triturated with 1:1 isohexane / DCM. The solid formed was filtered off, washed with isohexane, and dried to give the title compound (1.90 g, 98%);
¹H NMR (DMSO-d₆) 7.50-7.60 (m, 2H), 8.43 (m, 1H), 9.25 (bs, 1H), 11.50 (bs, 1H);
MS m/e MH⁺ 385, 387 (1 x Br).

### Example 75

### N-{5-[(2-aminopyrimidin-5-yl)ethynyl]-1,3-thiazol-2-yl}-N'-(5-tert-butylisoxazol-3-yl)urea

Example 75 was prepared by an analogous method to Example 73 but using Intermediate 23 and Intermediate 18.
¹H NMR (DMSO-d₆) 1.30 (s, 9H), 6.55 (s, 1H), 7.15 (s, 2H), 7.69 (s, 1H), 8.43 (s, 2H), 9.82 (bs, 1H), 10.80 (bs, 1H);
MS m/e (M-H)⁻ 382.

### Intermediate 23

### N-(5-bromo-1,3-thiazol-2-yl)-N'-(5-tert-butylisoxazol-3-yl)urea

Triethylamine (1.3 mL) was added to a stirred mixture of phenyl (5-*tert*-butylisoxazol-3-yl)carbamate (Intermediate 4) (702 mg) and 2-amino-5-bromothiazole (483 mg) in 1,4-dioxane (20 mL) and heated at 90°C for 1 hour. The solvent was evaporated and the residue was purified by flash chromatography on silica using 0-50% EtOAc in DCM as eluent to give the title compound as a solid (205 mg, 20%);
¹H NMR (DMSO-d₆) 1.30 (s, 9H), 6.52 (s, 1H), 7.51 (s, 1H), 9.77 (bs, 1H), 10.73 (bs, 1H); MS m/e MH⁺ 345, 347 (1 x Br).

### Example 76

### N-{3-[(2-aminopyrimidin-5-yl)ethynyl]phenyl}-2-(2-methoxyphenyl)acetamide

A solution of 2-methoxyphenylacetic acid (50 mg) in DMF (1 mL) was added to HATU (120 mg) and polymer supported DIPEA (Argonaut Technologies, 3.9 mmolg⁻¹, 300 mg). The mixture was shaken for 5 min. A solution of 5-[(3-aminophenyl)ethynyl]pyrimidine-2-amine (Intermediate 1) (68 mg) in DMF (1 mL) was added and agitation continued overnight. The resin was removed by filtration, and the solvent was added dropwise to water (10 mL) with shaking. The resulting solid was sonicated in fresh water (10 mL), filtered and dried to give the title compound (32 mg, 30%);
¹H NMR (DMSO-d₆) 3.63 (s, 2H), 3.76 (s, 3H), 6.89 (t, 1H), 6.97 (d, 1H), 7.10 (s, 2H), 7.15 (d, 1H), 7.18-7.27 (m, 2H), 7.32 (t, 1H), 7.51 (d, 1H), 7.83 (s, 2H), 8.41 (s, 1H);
MS m/e MH⁺ 359.

Examples 77 to 81 were prepared by an analogous method to Example 76 by using the appropriate acid.

### Example 77

### N-{3-[(2-aminopyrimidin-5-yl)ethynyl]phenyl}-2-phenylacetamide

Starting Materials: Intermediate 1 and phenylacetic acid.
¹H NMR (DMSO-d₆) 3.67 (s, 2H), 7.12 (bs, 2H), 7.18-7.20 (m, 1H), 7.22-7.28 (m, 1H), 7.33-7.37 (m, 6H), 7.52-7.54 (m, 1H), 7.85-7.86 (m, 1H), 8.44 (s, 2H);
MS m/e MH⁺ 329.

### Example 78

### N-{3-[(2-aminopyrimidin-5-yl)ethynyl]phenyl}-2-(3-methoxyphenyl)acetamide

Starting Materials: Intermediate 1 and 3-methoxyphenylacetic acid.
¹H NMR (DMSO-d₆) 3.61 (s, 2H), 3.74 (s, 3H), 6.81 (d, 1H), 6.91 (s, 2H), 7.11 (s, 2H), 7.12-7.27 (m, 2H), 7.33 (t, 1H), 7.50 (d, 1H), 7.83 (s, 1H), 8.41 (s, 2H), 10.22 (s, 1H);
MS m/e MH⁺ 359.

### Example 79

### N-{3-[(2-aminopyrimidin-5-yl)ethynyl]phenyl}-2-[3-(trifluoromethyl)phenyl]acetamide

Starting Materials: Intermediate 1 and 3-trifluoromethylphenylacetic acid.
¹H NMR (DMSO-d₆) 3.79 (s, 2H), 7.11 (s, 2H), 7.17 (d, 1H), 7.33 (t, 1H), 7.49 (d, 1H), 7.52-7.66 (m, 3H), 7.69 (s, 1H), 7.83 (s, 1H), 8.41 (s, 2H), 10.31 (s, 1H);
MS m/e MH⁺ 397.

### Example 80

### N-{3-[(2-aminopyrimidin-5-yl)ethynyl]phenyl}-2-[4-(trifluoromethyl)phenyl]acetamide

Starting Materials: Intermediate 1 and 4-trifluoromethylphenylacetic acid.
¹H NMR (DMSO-d₆) 3.77 (s, 2H), 7.11 (s, 2H), 7.17 (d, 1H), 7.33 (t, 1H), 7.49 (d, 1H), 7.55 (d, 2H), 7.69 (d, 2H), 7.82 (s, 1H), 8.41 (s, 2H), 10.32 (s, 1H);
MS m/e MH⁺ 397.

### Example 81

### N-{3-[(2-aminopyrimidin-5-yl)ethynyl]phenyl}-2-(3-methylisoxazol-5-yl)acetamide

Starting Materials: Intermediate 1 and 3-methyl-5-isoxazoleacetic acid.
¹H NMR (DMSO-d₆) 2.20 (s, 3H), 3.89 (s, 2H), 6.27 (s, 1H), 7.11 (s, 2H), 7.19 (d, 1H), 7.35 (t, 1H), 7.48 (d, 1H), 7.81 (s, 1H), 8.42 (s, 2H), 10.36 (s, 1H);
MS m/e MH⁺ 334.

### Intermediate 24

### 5-[(4-Aminophenyl)ethynyl]pyrimidin-2-amine

A mixture of 2-amino-5-iodopyrimidine (1.10 g), 4-ethynylaniline (0.82 g), 1,1,3,3-tetramethylguanidine (0.81 g), and copper (I) iodide (9.5 mg) in dry DMF (3.0 mL) was stirred and degassed with nitrogen. Tetrakis(triphenylphosphine)palladium(0) (115 mg) was added and the mixture heated at 60 °C for 2.5 hours. The solvent was evaporated and the residue was triturated with DCM. The solid formed was filtered off and washed with water then dissolved in 1:1 DCM / MeOH, filtered then evaporated. The solid obtained was triturated with ether and dried to give the title compound (0.67 g, 63%);
¹H NMR (DMSO-d₆) 5.50 (s, 2H), 6.55 (d, 2H), 6.95 (s, 2H), 7.15 (d, 2H), 8.35 (s, 2H);
MS m/e MH⁺ 211.

Examples 82 to 84 were prepared by an analogous method to Example 76 but using Intermediate 24 in place of Intermediate 1 with the appropriate acid.

### Example 82

### N-{4-[(2-aminopyrimidin-5-yl)ethynyl]phenyl}-2-(2-methoxyphenyl)acetamide

Starting Materials: Intermediate 24 and 2-methoxyphenylacetic acid.
¹H NMR (DMSO-d₆) 3.64 (s, 2H), 3.76 (s, 3H), 6.89 (t, 1H), 6.97 (d, 1H), 7.06 (2H), 7.18-7.28 (m, 2H), 7.42 (d, 2H), 7.63 (d, 2H), 8.38 (s, 2H), 10.19 (s, 1H);
MS m/e MH⁺ 359.

### Example 83

### N-{4-[(2-aminopyrimidin-5-yl)ethynyl]phenyl}-2-(3-methylisoxazol-5-yl)acetamide

Starting Materials: Intermediate 24 and 3-methyl-5-isoxazoleacetic acid.
¹H NMR (DMSO-d₆) 2.20 (s, 3H), 3.90 (s, 2H), 6.26 (s, 1H), 7.07 (s, 2H), 7.44 (d, 2H), 7.63 (d, 2H), 8.39 (s, 2H), 10.42 (s, 1H);
MS m/e MH⁺ 334.

### Example 84

### N-{3-[(2-aminopyrimidin-5-yl)ethynyl]phenyl}-N'-(2,2-dimethyltetrahydro-2H-pyran-4-yl)urea

Example 84 was prepared by an analogous method to Example 24 by using the compound prepared in Example 23 and 2,2-dimethyltetrahydro-2H-pyran-4-ylamine.
¹H NMR (DMSO-d₆) 1.11-1.31 (m, 2H), 1.14 (s, 3H), 1.17 (s, 3H), 1.72-1.79 (dd, 2H), 3.52-3.67 (m, 2H), 3.72-3.88 (m, 1H), 6.05-6.11 (d, 1H), 6.98-7.04 (m, 1H), 7.09 (s, 2H), 7.18-7.27 (m, 2H), 7.65 (s, 1H), 8.40 (s, 2H), 8.42 (s, 1H);
MS m/e MH⁺ 366.

### Example 85

### N-{6-[(2-aminopyrimidin-5-yl)ethynyl]pyrimidin-4-yl}-N'-(5-tert-butylisoxazol-3-yl)urea

Example 85 was prepared by an analogous method to Example 71 by using Intermediate 26 and Intermediate 18 and without RPHPLC purification.
¹H NMR (DMSO-d₆) 1.33 (s, 9H), 6.58 (s, 1H), 7.39 (s, 2H), 7.87 (d, 1H), 8.56 (s, 2H), 8.81 (d, 1H), 9.90 (s, 1H), 10.34 (s, 1H);
MS m/e MH⁺ 379.

### Intermediate 25

### 6-iodopyrimidin-4-amine

6-Chloropyrimidin-4-ylamine (450 mg) was added in portions to HI (57% wt. aq., 20 mL) at 0 °C. The reaction mixture was stirred for 30 minutes at 0 °C and then at ambient temperature for 18 hours. The reaction mixture was treated with NaHCO₃ (sat. aq.) until pH8 was achieved and then the product extracted with EtOAc (2 x 30 mL). The combined organics were washed with NaOH (2M, aq.), dried (MgSO₄), filtered and then concentrated. The crude product was used directly without further purification (500 mg, 65%);
¹H NMR (CDCl₃); 6.90 (s, 1H), 7.03 (s, 2H), 8.05 (s, 1H);
MS m/e MH⁺ 221.

### Intermediate 26

### N-(5-tert-butylisoxazol-3-yl)-N'-(6-iodopyrimidin-4-yl)urea

Intermediate 26 was prepared by an analogous method to Example 24 except by using Intermediate 4 and Intermediate 25. Purification was by flash chromatography on silica using 0-10% MeOH in DCM as the eluent.
MS m/e MH⁺ 388.

### Example 86

### N'-{3-[(2-aminopyrimidin-5-yl)ethynyl]phenyl}-N-(5-tert-butylisoxazol-3-yl)-N-methylurea

Example 86 was prepared by an analogous method to Example 24 by using the compound prepared in Example 23 and Intermediate 28.
¹H NMR (DMSO-d₆) 9.48 (s, 1H), 8.43 (s, 2H), 7.73 (s, 1H), 7.49 (d, 1H), 7.33 (t, 1H), 7.17 (d, 1H), 6.54 (s, 1H), 5.39 (br s, 3H), 3.37 (s, 3H), 1.30 (s, 9H);
MS m/e MH⁺ 391.

### Intermediate 27

### 5-tert-butylisoxazol-3-ylformamide

A solution of *p*-nitrophenyl formate (19.0 g) and 5-*tert*-butylisoxazol-3-amine (14.0 g) in MeCN (100 mL) was stirred at ambient temperature for 16 hours. Aqueous sodium hydrogen carbonate solution (50% saturated, 100 mL) was added and the mixture extracted into DCM. The combined organics were washed with 50% saturated sodium carbonate solution then water and concentrated *in vacuo* to give the title compound as a colourless solid (16.3 g);
¹H NMR (DMSO-d₆, major rotamer) 11.07 (s, br, 1H), 8.27 (s, 1H), 6.56 (s, 1H), 1.28 (s, 9H); MS m/e MH⁺ 169.

### Intermediate 28

### 5-tert-Butyl-N-methylisoxazol-3-amine

A solution of lithium aluminium hydride in THF (2.0M, 35.4 mL) was added over 40 minutes to a solution of 5-*tert*-butylisoxazol-3-ylformamide (Intermediate 27) (11.9 g) in THF (100 mL) at 0°C. The reaction mixture was warmed to ambient temperature and stirred for 30 minutes. Water (2.7 mL), 2N sodium hydroxide solution (2.7 mL) and water (8.1 mL) were added. The reaction mixture was filtered, the filter cake washed with THF and the filtrate concentrated *in vacuo.* Purification by flash chromatography on silica (35-50% EtOAc in isohexane) gave the title compound as a colourless oil (3.60 g);
¹H NMR (CDCl₃) 5.44 (s, 1H), 3.77 (s, br, 1H), 2.89 (d, 3H), 1.29 (s, 3H);
MS m/e MH⁺ 155.

### Example 87

### N-{3-[(2-aminopyrimidin-5-yl)ethynyl]phenyl}-N'-phenylurea

5-[(3-aminophenyl)ethynyl]pyrimidin-2-amine (Intermediate 1) (200 mg) was stirred in THF (5 mL) and phenyl isocyanate (0.114 mL) was added dropwise. The reaction mixture was concentrated *in vacuo* and the solid triturated with ether, dried under vacuum at 60°C to give the title compound as a brown solid (86 mg, 27%);
¹H NMR (DMSO-d₆) 6.92-7.00 (m, 1H), 7.09 (bs, 3H), 7.23-7.37 (m, 4H), 7.40-7.47 (m, 2H), 7.71 (s, 1H), 8.43 (s, 2H), 8.73 (s, 1H), 8.80 (s, 1H);
MS m/e MH⁺ 330.

### Example 88

### N-{3-[(2-aminopyrimidin-5-yl)ethynyl]phenyl}-N'-(1-tert-butyl-3-cyclopropyl-1H-pyrazol-5-yl)urea

Phenyl {3-[(2-aminopyrimidin-5-yl)ethynyl]phenyl}carbamate (Example 23) (100 mg), triethylamine (0.06 mL) and 3-cyclopropyl-1-methyl-1*H*-pyrazol-5-amine (60.0 mg) in THF (3 mL) were irradiated under microwave conditions (CEM explorer, 80°C, 50W) for 60 min. The reaction mixture was concentrated *in vacuo,* purification by flash chromatography on silica using 1-10% MeOH in DCM as eluent to give the title compound as a cream solid (46 mg, 37%);
¹H NMR (DMSO-d₆) 0.52-0.59 (m, 2H), 0.76-0.85 (m, 1H), 1.52 (s, 9H), 1.71-1.82 (m, 1H), 5.80 (s, 1H), 7.05-7.08 (m, 1H), 7.09 (bs, 2H), 7.21-7.35 (m, 2H), 7.70 (s, 1H), 7.90 (s, 1H), 8.40 (s, 2H), 8.94 (bs, 1H);
MS m/e MH⁺ 416.

### Example 89

### N-{3-[(2-aminopyrimidin-5-yl)ethynyl]phenyl}-N'-(5-methyl-1,3,4-thiadiazol-2-yl)urea

Phenyl {3-[(2-aminopyrimidin-5-yl)ethynyl]phenyl}carbamate (Example 23) (200 mg), 2 amino-5-methyl 1,3,4-thiadiazole (105 mg) and triethylamine (0.12 mL) in THF (5 mL) were heated at 60°C for 24 hours. The reaction mixture was concentrated *in vacuo* and the solid triturated with ether then MeOH, dried under vacuum at 60°C to give the title compound as a solid (170 mg, 80%);
¹H NMR (DMSO-d₆)2.5 (s, 3H), 7.10 (bs, 2H), 7.12-7.19 (m, 1H), 7.29-7.37 (m, 1H), 7.39-7.44 (m, 1H), 7.72 (bs, 1H), 8.43 (s, 2H), 9.15 (bs, 1H);
MS m/e MH⁺ 352.

**The following Examples were made in a similar way to Example 89 by using the appropriate amine in place of 2-amino-5-methyl-1,3,4-thiadiazole**

### Example 90

### N-{3-[(2-aminopyrimidin-5-yl)ethynyl]phenyl}-N'-(5-ethyl-1,3,4-thiadiazol-2-yl)urea

SM: Phenyl {3-[(2-aminopyrimidin-5-yl)ethynyl]phenyl}carbamate (Example 23), 2-Amino-5-ethyl-1,3,4-thiadiazole
¹H NMR (DMSO-d₆) 1:25-1.31 (t, 3H), 2.88-2.98 (q, 2H), 7.12 (bs, 2M, 7.13-7.17 (m, 1H), 7.18-7.25 (m, 1H), 7.73 (bs, 1H), 8.43 (s, 2M, 9.15 (bs, 1H);
MS m/e MH⁺ 365.

### Example 91

### N-{3-[(2-aminopyrimidin-5-yl)ethynyl]phenyl}-N'-(5-isopropyl-1,3,4-thiadiazol-2-yl)urea

SM: Phenyl {3-[(2-aminopyrimidin-5-yl)ethynyl]phenyl}carbamate (Example 23), 2-Amino-5-Isopropyl-1,3,4-thiadiazole
¹H NMR (DMSO-d₆) 1.29-1.35 (d, 6H), 3.20-3.30 (m, 1H), 7.10 (bs, 2H), 7.12-7.19 (m, ¹H), 7.29-7.37 (m, 1H), 7.38-7.44(m, 1H), 7.73 (s, 1H), 8.42 (s, 2H), 9.14 (s, 1H);
MS m/e MH⁺ 380.

**The following compound was made in a similar way to Example 89 except that the purification was by RPHPLC, gradient 0-90% MeCN/ H₂O:**

### Example 92

### N-{3-[(2-aminopyrimidin-5-yl)ethynyl]phenyl}-N'-(4-tert-butyl-1,3-thiazol-2-yl)urea

SM: Phenyl {3-[(2-aminopyrimidin-5-yl)ethynyl]phenyl}carbamate (Example 23), 2-Amino-4-tert-butylthiazole
¹H NMR (DMSO-d₆) 1.24 (s, 9H), 6.64 (s, 1H), 7.13-7.18 (m, 1H), 7.32-7.36 (m, 2H), 7.72 (s, 1H), 8.46 (s, 2H), 8.91 (bs, 1H);
MS m/e MH⁺ 393.

**The following Example was made in a similar way to Example 89 except that toluene was used as solvent instead of THF and purification was by trituration with ether:**

### Example 93

### N-{3-[(2-aminopyrimidin-5-yl)ethynyl]phenyl}-N'-(5-methylboxazol-3-yl)urea

SM: Phenyl {3-[(2-aminopyrimidin-5-yl)ethynyl]phenyl}carbamate (Example 23), 3-Amino-5-methylisoxazole
¹H NMR (DMSO-d₆) 2.36 (s, 3H), 6.52 (s, 1H), 7.07-7.17 (m, 3H), 7.28-7.39 (m, 2H), 7.69 (s, 1H), 8.42 (s, 2H), 8.92 (bs, 1H), 9.51 (bs, 1H);
MS m/e MH⁺ 335.

**The following Example was made in a similar way to Example 93 except it was purified by trituration with MeOH**

### Example 94

### N-{3-[(2-aminopyrimidin-5-yl)ethynyl]phenyl}-N'-[5-(trifluoromethyl)-1,3,4-thiadiazol-2-yl] u rea

SM: Phenyl {3-[(2-aminopyrimidin-5-yl)ethynyl]phenyl}carbamate (Example 23), 2-Amino-5-trifluoromethyl-1,3,4-thiadiazole
¹H NMR (DMSO-d₆) 7.12 (bs, 2H), 7.19-7.25 (m, 1H), 7.31-7.47 (m, 2H), 7.72 (s, 1H), 8.42 (s, 2H), 9.30 (bs, 1H);
MS m/e MH⁺ 406.

**The following Example was made in a similar way to Example 93 except that it was purified by trituration with MeOH then ether followed by RPHPLC gradient 0-70% MeCN / H₂O:**

### Example 95

### N'-{3-[(2-aminopyrimidin-5-yl)ethynyl]phenyl}-N-methyl-N-[5-(trifluoromethyl)-1,3,4-thiadiazol-2-yl]urea

SM: Phenyl {3-[(2-aminopyrimidin-5-yl)ethynyl]phenyl}carbamate (Example 23), N-2-Methyl-5-trifluoromethyl-1,3,4-thiadiazol-2-amine
¹H NMR (DMSO-d₆) 3.82 (s, 3H), 7.13 (bs, 2H), 7.24-7.29 (m, 1H), 7.37-7.44 (m, 1H), 7.52-7.50 (m, 1H), 7.75 (s, 1H), 8.43 (s, 2H), 9.66 (bs, 1H);
MS m/e MH⁺ 420.

**The following Example was made in a similar way to Example 93 except that it was purified by trituration with ether then flash chromatography on silica using 1 to 10% MeOH in DCM as eluent:**

### Example 96

### N-{3-[(2-aminopyrimidin-5-yl)ethynyl]phenyl}-N'-(5-cyclopropyl-1,3,4-thiadiazol-2-yl)urea

SM: Phenyl {3-[(2-aminopyrimidin-5-yl)ethynyl]phenyl}carbamate (Example 23), 2-Amino-5-cyclopropylthiadiazole
¹H NMR (DMSO-d₆) 0:88-1.00 (m, 2H, 1.07-1.17 (m, 2H), 2.24-2.37 (m, 1H), 7.11 (s, 2H), 7.13-7.18 (m, 1H), 7.29-7.36 (m, 1H), 7.38-7.43 (m, 1H), 7.72 (s, 1H), 8.43 (s, 2H), 9.10 (bs, 1H);
MS m/e MH⁺ 378.

### Example 97

### N-phenyl-N'-[3-({2-[(3-piperidin-1-ylpropyl)amino]pyrimidin-5-yl}ethynyl)phenyl]urea

Phenylchloroformate (0.13 mL) was added dropwise to a stirred solution of 5-[(3-aminophenyl)ethynyl]-N-(3-piperidin-1-ylpropyl)pyrimidin-2-amine (Intermediate 29) (318 mg) and pyridine (0.155 mL) in THF (8 mL) at 0°C. The reaction mixture was stirred and allowed to warm to ambient temperature over 2 hours. The solvent was evaporated and the resultant crude gum dissolved in THF (10 mL) and triethylamine (0.16 mL). Aniline (0.2 mL) was added and the reaction mixture heated at 75°C for 16 hours. The solvent was evaporated and the product was purified by flash chromatography on silica using 1-12% MeOH/NH₃ in DCM as eluent. The resultant product was triturated with MeOH to give the title compound as an off-white solid (208 mg, 48%);
¹H NMR (DMSO-d6) 1.31 - 1.41 (m, 2H), 1.42 - 1.53 (m, 4H), 1.66 (quintet, 2H), 2.22 - 2.36 (m, 6H), 3.22 - 3.38 (m, 2H), 6.97 (t, 1H), 7.06 - 7.12 (m, 1H), 7.23 - 7.34 (m, 4H), 7.41 - 7.48 (m, 2H), 7.67 - 7.76 (m, 2H), 8.40 - 8.51 (m, 2H), 8.70 (s, 1H), 8.77 (s, 1H);
MS m/e MH⁺ 455.

### Intermediate 29

### 5-[(3-aminophenyl)ethynyl]-N-(3-piperidin-1-ylpropyl)pyrimidin-2-amine

{3-[(2-Chloropyrimidin-5-yl)ethynyl]phenyl}amine (Intermediate 8) (1.5 g) and 1-amino-3-(N-piperidino)propane (4.6 g) were stirred in MeCN (20 mL) and hydrogen chloride (1.0M solution in ether) (7.80 mL) was added dropwise. The reaction mixture was stirred and heated at 80°C for 1 hour. The solvent was evaporated and the product was purified by flash chromatography on silica using 0-10% MeOH/NH₃ in DCM as eluent to give the title compound as an off-white solid (1.67 g, 76%);
¹H NMR (DMSO-d6) 1.30 - 1.42 (m, 2H), 1.42 - 1.53 (m, 4H), 1.66 (quintet, 2H), 2.18 - 2.39 (m, 6H), 3.24 - 3.35 (m, 2H), 5.19 (s, 2H), 6.52 - 6.64 (m, 2H), 6.64 - 6.69 (m, 1H), 7.01 (t, 1H), 7.64 (t, 1H), 8.40 (s, 2H),
MS m/e MH⁺ 336.

**The following Example was made in a similar way to Example 97:**

### Example 98

### N-(5-methylisoxazol-3-yl)-N'-[3-({2-[(3-piperidin-1-ylpropyl)amino]pyrimidin-5-yl}ethynyl)phenyl]urea

SM: 5-[(3-aminophenyl)ethynyl]-N-(3-piperidin-1-ylpropyl)pyrimidin-2-amine (Intermediate 29), 3-amino-5-methylisoxazole
¹H NMR (DMSO-d6) 1.31 - 1.41 (m, 2H), 1.42 - 1.53 (m, 4H), 1.66 (quintet, 2H), 2.24 - 2.32 (m, 6H), 2.36 (s, 3H), 3.27 - 3.36 (m, 2H), 6.51 (s, 1H), 7.10 - 7.15 (m, 1H), 7.29 - 7.35 (m, 2H), 7.68 - 7.75 (m, 2H), 8.45 (s, 2H), 8.89 (m, 1H), 9.50 (s, 1H);
MS m/e MH⁺ 460.

### Example 99

### N-[3-({2-[(3-piperidin-1-ylpropyl)amino]pyrimidin-5-yl}ethynyl)phenyl]-N'-[4-(trifluoromethyl)pyridin-2-yl]urea

Phenyl [4-(trifluoromethyl)pyridin-2-yl]carbamate (Intermediate 5) (201 mg) was added to a stirred solution of 5-[(3-aminophenyl)ethynyl]-N-(3-piperidin-1-ylpropyl)pyrimidin-2-amine (Intermediate 29) (200 mg) and triethylamine (0.100 mL) in THF (10 mL). The reaction mixture was heated at 80°C for 2 hours. The solvent was evaporated and the residue was triturated with ether to give the title compound as a white solid (299 mg, 95%);
¹H NMR (DMSO-d6) 1.31-1.42 (m, 2H), 1.43 - 1.53 (m, 4H), 1.66 (quintet, 2H), 2.13 - 2.38 (m, 6H), 3.24 - 3.36 (m, 2H), 7.16 (d, 1H), 7.30 - 7.42 (m, 3H), 7.70 (t, 1H), 7.78 (s, 1H), 8.04 (s, 1H), 8.42 - 8.50 (m, 2H), 8.54 (d, 1H), 9.72 (s, 1H), 9.84 (s, 1H);
MS m/e MH⁺ 524.

**The following Examples were made in a similar way to Example 99 by using the appropriate phenyl carbamate:**

### Example 100

### N-(5-tert-butyl-1,3,4-thiadiazol-2-yl)-N'-[3-({2-[(3-piperidin-1-ylpropyl)amino]pyrimidin-5-yl}ethynyl)phenyl]urea

SM: 5-[(3-aminophenyl)ethynyl]-N-(3-piperidin-1-ylpropyl)pyrimidin-2-amine (Intermediate 29), Phenyl (5-tert-butyl-1,3,4-thiadiazol-2-yl)carbamate (Intermediate 2)
¹H NMR (DMSO-d6) 1.34 - 1.44 (m, 11H), 1.44 - 1.56 (m, 4H), 1.69 (quintet, 2H), 2.32 - 2.44 (m, 6H), 3.21 - 3.39 (m, 2H), 7.13 (d, 1H), 7.32 (t, 1H), 7.53 (d, 1H), 7.70 (t, 1H), 7.83 (s, 1H), 8.46 (s, 2H);
MS m/e MH⁺ 519.

### Example 101

### N-(3-methylisoxazol-5-yl)-N'-[3-({2-[(3-piperidin-1-ylpropyl)amino] pyrimidin-5-yl}ethynyl)phenyl]urea

SM: 5-[(3-aminophenyl)ethynyl]-N-(3-piperidin-1-ylpropyl)pyrimidin-2-amine (Intermediate 29), Phenyl (3-methylisoxazol-5-yl)carbamate (Intermediate 3)
¹H NMR (DMSO-d6) 1.31-1.42 (m, 2H), 1.42 1.55 (m, 4H), 1.67 (quintet, 2H), 2.16 (s, 3H), 2.24 - 2.37 (m, 6H), 3.25 - 3.36 (m, 2H), 5.95 (s, 1H), 7.11 - 7.19 (m, 1H), 7.28 - 7.40 (m, 2H), 7.67 - 7.75 (m, 2H), 8.46 (s, 2H), 8.93 (s, 1H), 10.09 (s, 1H);
MS m/e MH⁺ 460.

### Example 102

### N-(2-methoxyphenyl)-N'-[3-({2-[(3-piperidin-1-ylpropyl)amino]pyrimidin-5-yl}ethynyl)phenyl]urea

2-Methoxyphenyl isocyanate (107 mg) was added to a stirred solution of 5-[(3-aminophenyl)ethynyl]-N-(3-piperidin-1-ylpropyl)pyrimidin-2-amine (Intermediate 29) (200 mg) in THF (10 mL). The reaction mixture was heated at 80°C for 2 hours. The solvent was evaporated and the product was purified by flash chromatography on silica using 1-12% MeOH/NH₃ in DCM as eluent and the product was triturated with ether to give the title compound as a white solid (108 mg, 37%);
¹H NMR (DMSO-d6) 1.32 - 1.42 (m, 2H), 1.43 - 1.53 (m, 4H), 1.67 (quintet, 2H), 2.24 - 2.34 (m, 6H), 3.22 - 3.36 (m, 2H), 3.88 (s, 3H), 6.85 - 7.05 (m, 3H), 7.05 - 7.12 (m, 1H), 7.27 - 7.32 (m, 2H), 7.69 (t, 1H), 7.75 (s, 1H), 8.11 (dd, 1H), 8.23 (s, 1H), 8.46 (s, 2H), 9.40 (s, 1H);
MS m/e MH⁺ 485.

**The following Example was made in a similar way to Example 102:**

### Example 103

### N-(3-fluorophenyl)-N'-[3-({2-[(3-piperidin-1-ylpropyl)amino]pyrimidin-5-yl}ethynyl)phenyl]urea

SM: 5-[(3-aminophenyl)ethynyl]-N-(3-piperidin-1-ylpropyl)pyrimidin-2-amine (Intermediate 29), 3-fluorophenylisocyanate
¹H NMR (DMSO-d6) 1.31 - 1.42 (m, 2H), 1.42 - 1.54 (m, 4H), 1.67 (quintet, 2H), 2.21 - 2.37 (m, 6H), 3.25 - 3.35 (m, 2H), 6.73 - 6.83 (m, 1H), 7.08 - 7.15 (m, 2H), 7.24 - 7.35 (m, 3H), 7.43 - 7.52 (m, 1H), 7.66 - 7.74 (m, 2H), 8.46 (s, 2H), 8.83 (s, 1H), 8.94 (s, 1H);
MS m/e MH⁺ 473.

**The following Examples were made in a similar manner to Example 25 by reacting N-(5-*tert*-butylisoxazol-3-yl)-*N*'-{3-[(2-chloropyrimidin-5-yl)ethynyl]phenyl}urea (Intermediate 9) with the appropriate amine.**

### Example 104

### N-[3-({2-[(4-aminobutyl)amino]pyrimidin-5-yl}ethynyl)phenyl]-N'-(5-tert-butylisoxazol-3-yl)urea

SM: *N*-(5-*tert*-butylisoxazol-3-yl)-*N*'-{3-[(2-chloropyrimidin-5-yl)ethynyl]phenyl}urea (Intermediate 9), 1,4-diaminobutane
¹H NMR (DMSO-d6) 1.29 (s, 9H), 1.32 - 1.45 (m, 2H), 1.46 - 1.60 (m, 2H), 2.46 - 2.58 (m, 2H), 3.21 - 3.38 (m, 4H), 6.49 (s, 1H), 7.11 - 7.15 (m,1H), 7.28 - 7.35 (m, 2H), 7.65 - 7.75 (m, 2H), 8.45 (s, 2H), 8.97 (s, 1H);
MS m/e MH⁺ 448.

### Example 105

### N-(5-tert-butylisoxazol-3-yl)-N'-[3-({2-[(2-piperidin-1-ylethyl)amino]pyrimidin-5-yl}ethynyl)phenyl]urea

SM: *N*-(5-*tert-*butylisoxazol-3-yl)-*N*'-{3-[(2-chloropyrimidin-5-yl)ethynyl]phenyl}urea (Intermediate 9), (2-piperidin-1-ylethyl)amine
¹H NMR (DMSO-d6) 1.29 (s, 9H), 1.32 - 1.42 (m, 2H), 1.42 - 1.55 (m, 4H), 2.31 - 2.55 (m, 6H), 3.36 - 3.46 (m, 2H), 6.49 (s, 1H), 7.12 - 7.16 (m, 1H), 7.29 - 7.35 (m, 2H), 7.44 (t, 1H), 7.72 (s, 1H), 8.46 (s, 2H), 8.90 (s, 1H), 9.55 (s, 1H)
MS m/e MH⁺ 488.

### Example 106

### N-(5-tert-butylisoxazol-3-yl)-N'-{3-[(2-{[2-(isopropylamino)ethyl]amino}pyrimidin-5-yl)ethynyl]phenyl}urea

SM: *N*-(5-*tert*-butylisoxazol-3-yl)-*N*'-{3-[(2-chloropyrimidin-5-yl)ethynyl]phenyl}urea (Intermediate 9), *N*-isopropylethane-1,2-diamine
¹H NMR (DMSO-d6) 0.96 (d, 6H), 1.29 (s, 9H), 2.61 - 2.78 (m, 3H), 3.32 - 3.40 (m, 2H), 6.49 (s, 1H), 7.11 - 7.16 (m, 1H), 7.30 - 7.35 (m, 2H), 7.56 (t, 1H), 7.72 (s, 1H), 8.46 (s, 2H), 8.90 (s, 1H), 9.57 (s, 1H)
MS m/e MH⁺ 462.

### Example 107

### N-(5-tert-butylisoxazol-3-yl)-N'-{3-[(2-{[2-(2-hydroxyethoxy)ethyl]amino}pyrimidin-5-yl)ethynyl]phenyl}urea

SM: *N*-(5-*tert*-butylisoxazol-3-yl)-*N*'-{3-[(2-chloropyrimidin-5-yl)ethynyl]phenyl}urea (Intermediate 9), 2-(2-aminoethoxy)ethanol
¹H NMR (DMSO-d6) 1.29 (s, 9H), 3.39 - 3.59 (m, 8H), 4.57 (t, 1H), 6.49 (s, 1H), 7.11-7.17 (m, 1H), 7.29 - 7.35 (m, 2H), 7.62 (t, 1H), 7.73 (s, 1H), 8.47 (s, 2H), 8.88 (s, 1H), 9.54 (s, 1H);
MS m/e MH⁺ 465.

### Example 108

### N-(5-tert-butylisoxazol-3-yl)-N'-{3-[(2-{[4-(dimethylamino)butyl]amino}pyrimidin-5-yl)ethynyl]phenyl}urea

SM: *N*-(5-*tert*-butylisoxazol-3-yl)-*N*'-{3-[(2-chloropyrimidin-5-yl)ethynyl]phenyl}urea (Intermediate 9), *N*,*N*-dimethylbutane-1,4-diamine
¹H NMR (DMSO-d6) 1.29 (s, 9H), 1.34 - 1.61 (m, 4H), 2.10 (s, 6H), 2.19 (t, 2H), 3.22 - 3.34 (m, 2H), 6.49 (s, 1H), 7.10 - 7.16 (m, 1H), 7.32 (d, 2H), 7.66 - 7.74 (m, 2H), 8.45 (s, 2H), 8.89 (s, 1H), 9.5 (s, 1H);
MS m/e MH⁺ 476.

### Example 109

### N-(5-tert-butylisoxazol-3-yl)-N'-{3-[(2-{[2-(dimethylamino)-1-methylethyl]amino}pyrimidin-5-yl)ethynyl]phenyl}urea

SM: *N*-(5-*tert*-butylisoxazol-3-yl)-*N'*-{3-[(2-chloropyrimidin-5-yl)ethynyl]phenyl}urea (Intermediate 9), *N*¹,*N*¹-dimethylpropane-1,2-diamine
¹H NMR (DMSO-d6) 1.12 (d, 3H), 1.29 (s, 9H), 2.14 (s, 6H), 2.16 - 2.23 (m, 1H), 2.31 - 2.41 (m, 1H), 4.12 (quintet, 1H), 6.49 (s, 1H), 7.10 - 7.16 (m, 1H), 7.32 (d, 2H), 7.39 (d, 1H), 7.71 (s, 1H), 8.45 (s, 2H), 8.88 (s, 1H), 9.54 (s, 1H);
MS m/e MH⁺ 462.

### Example 110

### N-(5-tert-butylisoxazol-3-yl)-N'-[3-({2-[(1-methyl-2-morpholin-4-ylethyl)amino]pyrimidin-5-yl}ethynyl)phenyl]urea

SM: *N*-(5-*tert*-butylisoxnol-3-yl)-*N*'-{3-[(2-chloropyrimidin-5-yl)ethynyl]phenyl}urea (Intermediate 9), (1-methyl-2-morpholin-4-ylethyl)amine (Intermediate 30)
¹H NMR (DMSO-d6) 1.13 (d, 3H), 1.29 (s, 9H), 2.21 - 2.30 (m, 1H), 2.34 - 2.45 (m, 5H), 3.47 - 3.55 (m, 4H), 4.19 (quintet, 1H), 6.49 (s, 1H), 7.09 - 7.17 (m, 1H), 7.28 - 7.35 (m, 2H), 7.41 (d, 1H), 7.72 (s, 1H), 8.44 (s, 2H), 8.87 (s, 1H), 9.54 (s, 1H);
MS m/e MH⁺ 504.

### Intermediate 30

### (1-methyl-2-morpholin-4-ylethyl)amine

2-(1,3-Dioxo-1,3-dihydro-2*H*-isoindol-2-yl)propanal (J. Org. Chem. 1953, 18,297) (3.0 g), morpholine (1.41 g) and acetic acid (0.87 mL) were dissolved in DCM (60 mL) and stirred at ambient temperature for 1 hour. Afterwhich sodium cyanoborohydride (1.4 g) was added and the reaction mixture was stirred for 2 hours at ambient temperature. Water was then added and the mixture extracted with EtOAc, the organics were washed with water and saturated brine then dried (MgSO₄) and concentrated *in vacuo.* Purification by flash chromatography on silica using 1-3% MeOH in DCM as eluent gave a residue which was dissolved in methylamine (33% in absolute ethanol, 20 mL) and heated at 40°C for 4 hours. The solvent was evaporated and the residue dissolved in ether and filtered, the filtrate was concentrated *in vacuo* to give the title compound as a yellow crystalline solid. (0.7 g);
¹H NMR (DMSO-d6) 0.90 (d, 3H), 2.04 (d, 2H), 2.21 - 2.32 (m, 2H), 2.32 - 2.44 (m, 2H), 2.87 - 2.98 (m, 1H), 3.51 - 3.59 (m, 4H).

### Example 111

### N-(5-tert-butylisoxazol-3-yl)-N'-{3-[(2-{[(1-glycoloylpyrrolidin-2-yl)methyl]amino}pyrimidin-5-yl)ethynyl]phenyl}urea

HATU (310 mg) was added to a stirred solution of *N*-(5-*tert*-butylisoxazol-3-yl)-*N*'-[3-({2-[(pyrrolidin-2-ylmethyl)amino]pyrimidin-5-yl}ethynyl)phenyl]urea (Intermediate 32) (250 mg), glycolic acid (62 mg) and DIPEA (283 µL) in DMA (5 mL) and the reaction mixture was stirred at ambient temperature for 2 hours. The solvent was evaporated and the residue was purified by flash chromatography on silica using 1-10% MeOH in DCM as eluent and the residue was triturated with ether to give the title compound as a white solid (200 mg, 71 %); ¹H NMR (DMSO-d₆ @ 373K) 1.32 (s, 9H), 1.80 -1.99 (m, 4H), 3.32 - 3.68 (m, 4H), 4.00 - 4.09 (m, 2H), 4.23 (m, 1H), 6.45 (s, 1H), 7.15 (d, 1H), 7.27-7.44 (m, 3H), 7.68 (s, 1H), 8.44 (s, 2H), 8.75 (s, 1H), 9.24 (s, 1H);
MS m/e MH⁺ 518.

### Intermediate 31

### N-(5-tert-butylisoxazol-3-yl)-N'-{3-[(2-{[(1-{tert-butoxycarbonyl}pyrrolidin-2-yl)methyl]amino}pyrimidin-5-yl)ethynyl]phenyl}urea

*N*-(5-*tert*-Butylisoxazol-3-yl)-*N*'-{3-[(2-chloropyrimidin-5-yl)ethynyl]phenyl}urea (Intermediate 9) (1.5 g) and (+/-)-*tert*-butyl 2-(aminomethyl)pyrrolidine-1-carboxylate (2.52 g) were stirred in MeCN (30 mL) and hydrogen chloride (1.0M solution in ether) (0.68 mL) was added dropwise. The reaction mixture was stirred and heated at 75°C for 24 hours. The solvent was evaporated and the product was purified by flash chromatography on silica using 1-8% MeOH in DCM as eluent to give mixed fractions which were re-purified by flash chromatography on silica using 0-100% EtOAc in DCM as eluent to give the title compound as a white solid (1.78 g, 84%);
¹H NMR (DMSO-d6) 1.29 (s, 9H), 1.39 (s, 9H), 1.72 - 1.88 (m, 4H), 3.17 - 3.34 (m, 3H), 3.44 - 3.58 (m, 1H), 3.88 - 4.01 (m, 1H), 6.49 (s, 1H), 7.11 - 7.17 (m, 1H), 7.29 - 7.35 (m, 2H), 7.65 - 7.77 (m, 2H), 8.44 (s, 2H), 8.88 (s, 1H), 9.54 (s, 1H)
MS m/e MH⁺ 560.

### Intermediate 32

### N-(5-tert-butylisoxazol-3-yl)-N'-[3-({2-[(pyrrolidin-2-ylmethyl)amino]pyrimidin-5-yl}ethynyl)phenyl]urea

tert-Butyl-2-{[(5-{[3-({[(5-tert-butylisoxazol-3-yl)amino]carbonyl}amino)phenyl]ethynyl}pyrimidin-2-yl)amino]methyl}pyrrolidine-1-carboxylate (Intermediate 31) was dissolved in DCM (40 mL) and TFA (10 mL). The reaction mixture was stirred at ambient temperature for 2 hours. The solvent was evaporated and the residue was dissolved in DCM and washed with saturated sodium bicarbonate solution, saturated brine and dried (MgSO₄). The solvent was evaporated and the residue was triturated with ether/MeOH to give the title compound as an off-white solid (1.38 g, 94%);
¹H NMR (DMSO-d₆ + d₄ acetic acid) 1.28 (s, 9H), 1.60 - 1.72 (m, 1H), 1.83 - 1.95 (m, 2H), 1.97 - 2.11 (m, 1H), 3.08 - 3.25 (m, 2H), 3.55 - 3.62 (m, 2H), 3.63 - 3.76 (m, 1H), 6.48 (s, 1H), 7.12 (d, 1H), 7.26 - 7.41 (m, 2H), 7.74 - 7.78 (m, 1H), 8.53 (s, 2H);
MS m/e MH⁺ 460.

**The following Example was prepared in an similar way to Example 111.**

### Example 112

### N-(5-tert-butylisoxazol-3-yl)-N'-(3-{[2-({[1-(N,N-dimethylglycyl)pyrrolidin-2-yl]methyl}amino)pyrimidin-5-yl]ethynyl}phenyl)urea

SM: *N*-(5-*tert*-butylisoxazol-3-yl)-*N*'-[3-({2-[(pyrrolidin-2-ylmethyl)amino]pyrimidin-5-yl}ethynyl)phenyl]urea (Intermediate 32), *N*,*N*-dimethylglycine
¹H NMR (DMSO-d₆ @ 373K) 1.32 (s, 9H), 1.76-1.96 (m, 4H), 2.25 (s, 6H), 2.89 - 2.97 (m, 1H), 3.02 - 3.09 (m, 1H), 3.30 - 3.58 (m, 4H), 4.25 - 4.32 (m, 1H), 6.45 (s, 1H), 7.15 (d, 1H), 7.27- 7.39 (m, 2H), 7.68 (s, 1H), 8.44 (s, 2H), 8.75 (s, 1H), 9.24 (s, 1H);
MS m/e MH⁺ 545.

### Example 113

### N-(5-tert-butylisoxazol-3-yl)-N'-[3-({2-[(2-piperazin-1-ylethyl)amino]pyrimidin-5-yl}ethynyl)phenyl]urea

*N*-(5-*tert*-Butylisoxazol-3-yl)-*N*'-{3-[(2-chloropyrimidin-5-yl)ethynyl]phenyl}urea (Intermediate 9) (0.22 g) and *tert*-butyl 4-(2-aminoethyl)-piperazine-1-carboxylate (0.38 g) were stirred in MeCN (10 mL) and hydrogen chloride (1.0M solution in ether) (0.11 mL) was added dropwise. The reaction mixture was stirred and heated at 70°C for 4 hours. The solvent was evaporated and the residue was dissolved in DCM (20 mL) and TFA (10 mL). The reaction mixture was stirred at ambient temperature for 2 hours, the solvent was evaporated and the product was purified by flash chromatography on silica using 1-20% (7N NH₃ in MeOH) in DCM as eluent. The product was triturated with ether to give the title compound as an off-white solid (274 mg, 99%);
¹H NMR (DMSO-d6) 1.29 (s, 9H), 2.45-2.57 (m, 2H), 2.57 - 2.65 (m, 4H), 3.03 - 3.10 (m, 4H), 3.39 - 3.49 (m, 2H), 6.49 (s, 1H), 7.10 - 7.16 (m, 1H), 7.29 - 7.35 (m, 2H), 7.51 (t, 1H), 7.75 (s, 1H), 8.40 - 8.52 (m, 3H), 8.99 (s, 1H), 9.62 (s, 1H);
MS m/e MH⁺ 489.

**The following Example was made in similar manner to Example 113:**

### Example 114

### N-(5-tert-butylisoxazol-3-yl)-N'-[3-({2-[(3-piperazin-1-ylpropyl)amino]pyrimidin-5-yl}ethynyl)phenyl]urea

SM: *N*-(5-*tert*-Butylisoxazol-3-yl)-*N*'-{3-[(2-chloropyrimidin-5-yl)ethynyl]phenyl}urea (Intermediate 9), tert-butyl 4-(3-aminopropyl)piperazine-1-carboxylate (Intermediate 34)
¹H NMR (DMSO-d6) 1.29 (s, 9H), 1.63-1.74 (m, 2H), 2.36 - 2.42 (m, 2H), 2.51 - 2.77 (m, 4H), 3.03 - 3.10 (m, 4H), 3.23 - 3.37 (m, 4H), 6.49 (s, 1H), 7.10 - 7.16 (m, 1H), 7.31 (d, 2H), 7.67 (t, 1H), 7.73 (s, 1H), 8.36 - 8.49 (m, 3H), 8.98 (s, 1H), 9.61 (s, 1H);
MS m/e MH⁺ 503.

### Intermediate 33

### tert-Butyl 4-[3-(1,3-dioxo-1,3-dihydro-2H-isoindol-2-yl)propyl]piperazine-1-carboxylate

N-(3-Bromopropyl)phthalimide (1.37 g), tert-butyl 1-piperazinecarboxylate (1 g), sodium iodide (1.53 g) and potassium carbonate (1.41 g) were dissolved in DMA (10 mL) and stirred at ambient temperature for 2 hours. The solvent was evaporated and the residue was dissolved in DCM and filtered. The filtrate was purified by flash chromatography on silica using 50-80% EtOAc in DCM as eluent to give the title compound as a colourless crystalline solid (1.7 g, 89%);
¹HNMR (DMSO-d6) 1.35 (s, 9H), 1.52 (m, 2H), 2.16 - 2.22 (m, 4H), 2.30 (t, 2H), 3.07 - 3.13 (m, 4H), 3.63 (t, 2H), 7.79 - 7.88 (m, 4H);
MS m/e MH⁺ 374

### Intermediate 34

### tert-butyl 4-(3-aminopropyl)piperazine-1-carboxylate

*tert*-Butyl 4-[3-(1,3-dioxo-1,3-dihydro-2*H*-isoindol-2-yl)propyl]piperazine-1-carboxylate (Intermediate 33) (1.7 g) was dissolved in methylamine (33% in absolute ethanol, 20 mL) and heated at 40°C for 4 hours. The solvent was evaporated and the residue was dissolved in ether and filtered. The filtrate was then evaporated to give the title compound as a colourless oil.
¹H NMR (DMSO-d6) 1.38 (s, 9H), 1.42-1.51 (m, 2H), 2.22 - 2.36 (m, 6H), 2.48-2.57 (m, 2H), 3.23 - 3.32 (m, 4H).

### Example 115

### N-(5-tert-butylisoxazol-3-yl)-N-methyl-N'-[3-({2-[(2-morpholin-4-ylethyl)amino]pyrimidin-5-yl}ethynyl)phenyl]urea

N-(5-tert-Butylisoxazol-3-yl)-N'-{3-[(2-chloropyrimidin-5-yl)ethynyl]phenyl}-N-methylurea (Intermediate 36) (0.25 g) and 4-(2-aminoethyl)morpholine (240 mg) were stirred in MeCN (10 mL) and hydrogen chloride (1.0M solution in ether) (0.67 mL) was added dropwise. The reaction mixture was stirred and heated at 75°C for 2 hours. The solvent was evaporated and the product was purified by flash chromatography on silica using 5-8% MeOH/NH₃ in DCM as eluent and the residue was triturated with ether to give the title compound as a white solid 195 mg, 63%);
¹H NMR (DMSO-d6) 1.30 (s, 9H), 2.36 - 2.42 (m, 4H), 2.42 - 2.52 (m, 2H), 3.37 (s, 3H), 3.39 - 3.47 (m, 2H), 3.55 (t, 4H), 6.54 (s, 1H), 7.15 - 7.20 (m, 1H), 7.33 (t, 1H), 7.45 - 7.51 (m, 2H), 7.71 - 7.75 (m, 1H), 8.46 (s, 2H), 9.48 (s, 1H);
MS m/e MH⁺ 504.

### Intermediate 35

### phenyl {3-[(2-chloropyrimidin-5-yl)ethynyl]phenyl}carbamate

Phenylchloroformate (1.02 g) was added dropwise to a stirred solution of {3-[(2-chloropyrimidin-5-yl)ethynyl]phenyl}amine (Intermediate 8) (1 g) and pyridine (0.7 mL) in THF (50 mL) at 0°C. The reaction mixture was stirred and allowed to warm to ambient temperature over 2 hours. The residue was partitioned between ethyl acetate and water and the organics were washed with water, saturated brine and dried over magnesium sulfate. The solvent was evaporated to give the title compound as a yellow solid (1.5 g, 98%);
¹H NMR (DMSO-d6) 7.19 - 7.32 (m, 4H), 7.33 - 7.50 (m, 3H), 7.54 - 7.62 (m, 1H), 7.79 (s, 1H), 8.99 (s, 2H), 10.42 (s, 1H);
MS m/e MH⁺ 350.

### Intermediate 36

### N-(5-tert-butylisoxazol-3-yl)-N'-{3-[(2-chloropyrimidin-5-yl)ethynyl]phenyl}-N-methylurea

5-*tert*-Butyl-*N*-methylisoxazol-3-amine (Intermediate 28) (1.34 g) was added to a stirred solution of phenyl {3-[(2-chloropyrimidin-5-yl)ethynyl]phenyl}carbamate (Intermediate 35) (2.03 g) in THF (120 mL) and triethylamine (1.21 mL). and the reaction mixture was heated at 65°C for 4 days. The solvent was evaporated and the product was purified by flash chromatography on silica using 20-40% EtOAc in DCM as eluent. The solvent was evaporated to give the title compound as an off-white crystalline solid (1.0 g, 42%);
MS m/e MH⁺ 410.

**The following Examples were made in a similar way to Example 115 by using the appropriate amine in place of 4-(2-aminoethyl)morpholine.**

### Example 116

### N-(5-tert-butylisoxazol-3-yl)-N-methyl-N'-[3-({2-[(3-morpholin-4-ylpropyl)amino]pyrimidin-5-yl}ethynyl)phenyl]urea

SM: N-(5-tert-butylisoxazol-3-yl)-N'-{3-[(2-chloropyrimidin-5-yl)ethynyl]phenyl}-N-methylurea (Intermediate 36), 4-(3-aminopropyl)morpholine
¹H NMR (DMSO-d6) 1.30 (s, 9H), 1.68 (quintet, 2H), 2.28 - 2.37 (m, 6H), 3.27 - 3.36 (m, 2H), 3.37 (s, 3H), 3.52 - 3.58 (m, 4H), 6.54 (s, 1H), 7.17 (d, 1H), 7.33 (t, 1H), 7.45 - 7.52 (m, 1H), 7.67 (t, 1H), 7.73 (s, 1H), 8.45 (s, 2H), 9.48 (s, 1H)
MS m/e MH⁺ 518.

### Example 117

### N-(5-tert-butylisoxazol-3-yl)-N-methyl-N'-[3-({2-[(3-piperidin-1-ylpropyl)amino]pyrimidin-5-yl}ethynyl)phenyl]urea

SM: N-(5-tert-butylisoxazol-3-yl)-N'-{3-[(2-chloropyrimidin-5-yl)ethynyl]phenyl}-N-methylurea (Intermediate 36), (3-piperidin-1-ylpropyl)amine
¹H NMR (DMSO-d6) 1.30 (s, 9H), 1.33 -1.42 (m, 2H), 1.42 - 1.54 (m, 4H), 1.67 (quintet, 2H), 2.24 - 2.36 (m, 6H), 3.24 - 3.35 (m, 2H), 3.37 (s, 3H), 6.54 (s, 1H), 7.17 (d, 1H), 7.33 (t, 1H), 7.45 - 7.52 (m, 1H), 7.66 - 7.75 (m, 2H), 8.45 (s, 2H), 9.48 (s, 1H);
MS m/e MH⁺ 516.

### Example 118

### N-(3-tert-butyl-1-methyl-1H-pyrazol-5-yl)-N'-[3-({2-[(3-piperidin-1-ylpropyl)amino]pyrimidin-5-yl}ethynyl)phenyl]urea

N-(3-tert-Butyl-1-methyl-1H-pyrazol-5-yl)-N'-{3-(2-chloropyrimidin-5-yl)ethynyl]phenyl}urea (Intermediate 37) (0.3 g) and (3-piperidin-1-ylpropyl)amine (523 mg) were stirred in MeCN (10 mL) and hydrogen chloride (1.0M solution in ether) (0.88 mL) was added dropwise. The reaction mixture was stirred and heated at 75°C for 2 hours. The solvent was evaporated and the product was purified by flash chromatography on silica using 6-8% methanolic NH₃ in DCM as eluent and the residue was triturated with ether to give the title compound as an off-white solid (195 mg, 50%);
¹HNMR (DMSO-d6) 1.20 (s, 9H), 1.31 - 1.42 (m, 2H), 1.42 - 1.53 (m, 4H), 1.59 - 1.73 (m, 2H), 2.23 - 2.35 (m, 6H), 3.23 - 3.35 (m, 2H), 3.59 (s, 3H), 6.04 (s, 1H), 7.06 - 7.14 (m, 1H), 7.27 - 7.36 (m, 2H), 7.69 (t, 1H), 7.74 (s, 1H), 8.44 (s, 2H), 8.51 (s, 1H), 8.96 (s, 1H);
MS m/e MH⁺ 515.

### Intermediate 37

### N-(3-tert-butyl-1-methyl-1H-pyrazol-5-yl)-N'-{3-[(2-chloropyrimidin-5-yl)ethynyl]phenyl}urea

5-Amino-3-tert-butyl-1-methylpyrazole (1.32 g) was added to a stirred solution of phenyl {3-[(2-chloropyrimidin-5-yl)ethynyl]phenyl}carbamate (Intermediate 35) (2.0 g) in THF (120 mL) and triethylamine (1.2 mL) and the reaction mixture was heated at 65°C for 8 hours. The solvent was evaporated and the residue was triturated with ether/DCM to give the title compound as an off-white solid (1.5 g, 64%);
MS m/e MH⁺ 409.

**The following Examples were made in a similar way to Example 118 by using the appropriate amine in place of (3-piperidin-1-ylpropyl)amine.**

### Example 119

### N-(3-tert-butyl-1-methyl-1H-pyrazol-5-yl)-N'-[3-({2-[(3-morpholin-4-ylpropyl)amino]pyrimidin-5-yl}ethynyl)phenyl]urea

SM: N-(3-tert-butyl-1-methyl-1H-pyrazol-5-yl)-N'-{3-[(2-chloropyrimidin-5-yl)ethynyl]phenyl}urea (Intermediate 37), 4-(3-aminopropyl)morpholine
¹H NMR (DMSO-d6) 1.20 (s, 9H), 1.68 (quintet, 2H), 2.27 - 2.37 (m, 6H), 3.24 - 3.39 (m, 2H), 3.52 - 3.58 (m, 4H), 3.59 (s, 3H), 6.04 (s, 1H), 7.07 - 7.14 (m, 1H), 7.27 - 7.35 (m, 2H), 7.67 (t, 1H), 7.74 (s, 1H), 8.45 (s, 2H), 8.51 (s, 1H), 8.96 (s, 1H);
MS m/e MH⁺ 517.

### Example 120

**N-(3-tert-butyl-1-methyl-1H-pyrazol-5-yl)-N'-[3-({2-[(2-morpholin-4-ylethyl)amino]pyrimidin-5-yl}ethynyl)phenyl]urea**
SM: N-(3-tert-butyl-1-methyl-1H-pyrazol-5-yl)-N'-{3-[(2-chloropyrimidin-5-yl)ethynyl]phenyl}urea (Intermediate 37), 4-(2-aminoethyl)morpholine ¹H NMR (DMSO-d6) 1.20 (s, 9H), 2.37 - 2.42 (m, 6H), 3.37 - 3.47 (m, 2H), 3.52 - 3.58 (m, 4H), 3.59 (s, 3H), 6.04 (s, 1H), 7.07 - 7.13 (m, 1H), 7.27 - 7.34 (m, 2H), 7.48 (t, 1H), 7.74 (s, 1H), 8.46 (s, 2H), 8.50 (s, 1H), 8.96 (s, 1H);
MS m/e MH⁺ 503.

### Example 121

### N-(3-tert-butyl-1-methyl-1H-pyrazol-5-yl)-N'-{3-[(2-{[2-(dimethylamino)ethyl]amino}pyrimidin-5-yl)ethynyl]phenyl}urea

SM: N-(3-tert-butyl-1-methyl-1H-pyrazol-5-yl)-N'-{3-[(2-chloropyrimidin-5-yl)ethynyl]phenyl}urea (Intermediate 37), *N*,*N*-dimethylethane-1,2-diamine
¹H NMR (DMSO-d6) 1.20 (s, 9H), 2.16 (s, 6H), 2.40 (t, 2H), 3.34 - 3.44 (m, 2H), 3.59 (s, 3H), 6.04 (s, 1H), 7.07 - 7.13 (m, 1H), 7.28 - 7.33 (m, 2H), 7.45 (t, 1H), 7.74 (s, 1H), 8.45 (s, 2H), 8.51 (s, 1H), 8.96 (s, 1H);
MS m/e MH⁺ 461.

### Example 122

### N-(3-tert-butyl-1-methyl-1H-pyrazol-5-yl)-N'-{3-[(2-{[2-(isopropylamino)ethyl]amino}pyrimidin-5-yl)ethynyl]phenyl}urea

SM: N-(3-tert-butyl-1-methyl-1H-pyrazol-5-yl)-N'-{3-[(2-chloropyrimidin-5-yl)ethynyl]phenyl}urea (Intermediate 37), *N*-isopropylethane-1,2-diamine
¹H NMR (DMSO-d6) 0.95 (d, 6H), 1.20 (s, 9H), 2.62 - 2.75 (m, 3H), 3.31 - 3.41 (m, 2H), 3.59 (s, 3H), 6.04 (s, 1H), 7.07 - 7.13 (m, 1H), 7.27 - 7.34 (m, 2H), 7.55 (t, 1H), 7.74 (s, 1H), 8.45 (s, 2H), 8.52 (s, 1H), 8.97 (s, 1H)
MS m/e MH⁺ 475.

### Example 123

### N-(3-cyclopropyl-1-methyl-1H-pyrazol-5-yl)-N'-[3-({2-[(3-piperidin-1-ylpropyl)amino]pyrimidin-5-yl}ethynyl)phenyl]urea

N-{3-[(2-Chloropyrimidin-5-yl)ethynyl]phenyl}-N'-(3-cyclopropyl-1-methyl-1H-pyrazol-5-yl)urea (Intermediate 38) (0.25 g) and 1-amino-3-(N-piperidino)propane (0.5 mL) were stirred in MeCN (10 mL) and hydrogen chloride (1.0M solution in ether) (0.76 mL) was added dropwise. The reaction mixture was stirred and heated at 75°C for 2 hours. The solvent was evaporated and the product was purified by flash chromatography on silica using 1-10% MeOH/NH₃ in DCM as eluent and the residue was triturated with ether to give the title compound as an off white solid (210 mg, 66%);
¹H NMR (DMSO-d6) 0.54 - 0.62 (m, 2H), 0.74 - 0.83 (m, 2H), 1.31 - 1.42 (m, 2H), 1.42 - 1.54 (m, 4H), 1.60 - 1.82 (m, 3H), 2.23 - 2.34 (m, 6H), 3.22 - 3.37 (m, 2H), 3.56 (s, 3H), 5.87 (s, 1H), 7.07 - 7.13 (m, 1H), 7.26 - 7.34 (m, 2H), 7.65 - 7.74 (m, 2H), 8.44 (s, 2H), 8.52 (s, 1H), 8.92 (s, 1H);
MS m/e MH⁺ 499.

### Intermediate 38

### N-{3-[(2-chloropyrimidin-5-yl)ethynyl]phenyl}-N'-(3-cyclopropyl-1-methyl-1H-pyrazol-5-yl)urea

5-Amino-3-cyclopropyl-1-methylpyrazole (0.88 g) was added to a stirred solution of phenyl {3-[(2-chloropyrimidin-5-yl)ethynyl]phenyl}carbamate (Intermediate 35) (1.5 g) in THF (100 mL) and triethylamine (0.9 mL) and the reaction mixture was heated at 80°C for 8 hours. The solvent was evaporated and the residue was triturated with ether/DCM to give the title compound as an off-white solid (1.1 g, 65%);
¹H NMR (DMSO-d6) 0.54 - 0.62 (m, 2H), 0.75 - 0.84 (m, 2H), 1.70 - 1.82 (m, 1H), 3.56 (s, 3H), 5.87 (s, 1H), 7.17 - 7.26 (m, 1H), 7.34 - 7.43 (m, 2H), 7.86 (s, 1H), 8.55 (s, 1H), 8.94 - 9.05 (m, 3H);
MS m/e MH⁺ 393.

**The following Examples were made in a similar way to Example 123 by using the appropriate amine in place of (3-piperidin-1-ylpropyl)amine.**

### Example 124

### N-(3-cyclopropyl-1-methyl-1H-pyrazol-5-yl)-N'-[3-({2-[(3-morpholin-4-ylpropyl)amino]pyrimidin-5-yl}ethynyl)phenyl]ureaSM: N-{3-[(2-chloropyrimidin-5-yl)ethynyl]phenyl}-N'-(3-cyclopropyl-1-methyl-1H-pyrazol-5-yl)urea (Intermediate 38), 4-(3-aminopropyl)morpholine

¹H NMR (DMSO-d6) 0.54 - 0.62 (m, 2H), 0.74 - 0.84 (m, 2H), 1.62 - 1.82 (m, 3H), 2.27 - 2.43 (m, 6H), 3.22 - 3.38 (m, 2H), 3.50 - 3.61 (m, 7H), 5.87 (s, 1H), 7.07 - 7.13 (m, 1H), 7.26 - 7.35 (m, 2H), 7.67 (t, 1H), 7.72 (s, 1H), 8.45 (s, 2H), 8.54 (s, 1H), 8.94 (s, 1H);
MS m/e MH⁺ 501.

### Example 125

### N-(3-cyclopropyl-1-methyl-1H-pyrazol-5-yl)-N'-[3-({2-[(2-morpholin-4-ylethyl)amino]pyrimidin-5-yl}ethynyl)phenyl]urea

SM: N-{3-[(2-chloropyrimidin-5-yl)ethynyl]phenyl}-N'-(3-cyclopropyl-1-methyl-1H-pyrazol-5-yl)urea (Intermediate 38), 4-(2-aminoethyl)morpholine
¹H NMR (DMSO-d6) 0.54 - 0.62 (m, 2H), 0.75 - 0.83 (m, 2H), 1.71 - 1.82 (m, 1H), 2.36 - 2.42 (m, 4H), 2.42 - 2.52 (m, 2H), 3.38 - 3.47 (m, 2H), 3.51 - 3.59 (m, 7H), 5.87 (s, 1H), 7.08 - 7.13 (m, 1H), 7.27 - 7.35 (m, 2H), 7.48 (t, 1H), 7.72 (s, 1H), 8.45 (s, 2H), 8.52 (s, 1H), 8.92 (s, 1H);
MS m/e MH⁺ 487.

### Example 126

### N-(5-tert-butylisoxazol-3-yl)-N'-(3-{[2-({2-[(2-hydroxy-1-oxo-ethyl)amino]ethyl}amino)-pyrimidin-5-yl]ethynyl}phenyl)urea

HATU (343 mg) was added to a stirred solution of N-[3-({2-[(2-aminoethyl)amino]pyrimidin-5-yl}ethynyl)phenyl]-N'-(5-tert-butylisoxazol-3-yl)urea) (Example 35) (252 mg), glycolic acid (69 mg) and DIPEA (0.31 mL) in DMA (5 mL) and the reaction mixture stirred at ambient temperature for 2 hours. The solvent was evaporated and the residue was purified by flash chromatography on silica using 1-12% MeOH/NH₃ in DCM as eluent and the residue was triturated with ether to give the title compound as an off-white solid (225 mg, 79%);
¹H NMR (DMSO-d6) 1.29 (s, 9H), 3.25 - 3.35 (m, 2H), 3.35 - 3.44 (m, 2H), 3.78 (d, 2H), 5.43 (t, 1H), 6.49 (s, 1H), 7.10 - 7.18 (m, 1H), 7.32 (d, 2H), 7.68 (t, 1H), 7.73 (s, 1H), 7.84 - 7.94 (m, 1H), 8.47 (s, 2H), 8.88 (s, 1H), 9.54 (s, 1H);
MS m/e MH⁺ 478.

### Example 127

### N-(5-tert-butylisoxazol-3-yl)-N'-(3-{[2-({3-[(2-hydroxyethyl)amino]propyl}amino)pyrimidin-5-yl]ethynyl}phenyl)urea

Titanium(IV) isopropoxide (342 mg) was added to a stirred solution of N-[3-({2-[(3-aminopropyl)amino]pyrimidin-5-yl}ethynyl)phenyl]-N'-(5-tert-butylisoxazol-3-yl)urea (Example 36) (260 mg) and (tert-butyldimethylsilyl-oxy)acetaldehyde (105 mg) in DCM (10 mL) and the reaction mixture was stirred at ambient temperature for 1 hour. Sodium borohydride (20 mg) was added then MeOH (1.5 mL) and the reaction was stirred for 10 min and then quenched with water (10 mL). The reaction mixture was filtered, the organics were washed with water, saturated brine and dried (MgSO₄). The solvent was evaporated and the residue dissolved in THF (10 mL) and tetrabutylammonium fluoride 1.0M in THF (1.0 mL) was added and the reaction mixture stirred at ambient temperature for 2 hours. The reaction mixture was purified by flash chromatography on silica using 15-20% MeOH/NH₃ in DCM as eluent and the residue was triturated with ether to give the title compound as an off-white solid (65 mg, 23%);
¹HNMR (DMSO-d6) 1.29 (s, 9H), 1.60 - 1.74 (m, 2H), 2.54 - 2.64 (m, 4H), 3.23 - 3.40 (m, 2H), 3.40- 3.49 (m, 2H), 4.39 - 4.54 (m, 1H), 6.49 (s, 1H), 7.09 - 7.18 (m, 1H), 7.32 (d, 2H), 7.67 - 7.75 (m, 2H), 8.45 (s, 2H), 8.90 (s, 1H);
MS m/e MH⁺ 478.

### Example 128

### Phenyl 3-[(2-{[3-(dimethylamino)propyl]amino}pyrimidin-5-yl)ethynyl]phenylcarbamate

*N*'-{5-[(3-aminophenyl)ethynyl]pyrimidin-2-yl}-*N*,*N*-dimethylpropane-1,3-diamine (Intermediate 39) (1.5 g) and pyridine (0.41 mL) were dissolved in THF (200 mL) and cooled to 0°C. Phenyl chloroformate (0.89 mL) was added dropwise and the solution allowed to warm to ambient temperature over one hour. The reaction mixture was concentrated *in vacuo* and the residue dissolved in ethyl acetate, washed with aqueous sodium carbonate solution, water and brine, concentrated *in vacuo* and triturated with ether to give the title compound as a white solid (2.28 g).
¹H NMR (DMSO-d₆) 1.60-1.79 (m, 2H), 2.12 (s, 6H), 2.26 (t, 2H), 3.30-3.40 (m, 2H), 7.15-7.51 (m, 8H), 7.66-7.70 (m, 2H), 8.45 (s, 2H), 10.33 (s, 1H);
MS m/e MH⁺ 416.

### Intermediate 39

### N'-{5-[(3-aminophenyl)ethynyl]pyrimidin-2-yl}-N,N-dimethylpropane-1,3-diamine

3-[(2-Chloropyrimidin-5-yl)ethynyl]aniline (Intermediate 8) (2.23 g), *N*,*N*-dimethylpropane-1,3-diamine (6.11 mL) and 1.0 M HCl in ether (11.7 mL) were dissolved in MeCN (10 ml) and heated at reflux for 4 hours. Concentration *in vacuo* and purification by flash chromatography on silica using 1-10% (10% 7N NH3 in MeOH) in DCM as eluent gave the title compound as a beige solid (2.70 g, 94%);
¹H NMR (DMSO-d₆) 1.59-1.69 (m, 2H), 2.11 (s, 6H), 2.24 (t, 2H), 3.30 (dt, 2H), 5.19 (s, 2H), 6.56 (dd, 1H), 6.61 (d, 1H), 6.67 (s, 1H), 7.01 (t, 1H), 7.62 (t, 1H), 8.40 (s, 2H);
MS m/e MH⁺ 296.

### Example 129

### N-{3-[(2-{[3-(dimethylamino)propyl]amino}pyrimidin-5-yl)ethynyl]phenyl}-N'-phenylurea

Triethylamine (0.12 mL) was added to a solution of phenyl 3-[(2-{[3-(dimethylamino)propyl]amino}pyrimidin-5-yl)ethynyl]phenylcarbamate (Example 128) (300 mg) and aniline (0.08 mL) in THF (10 mL). The reaction mixture was heated to 50°C for 18 hours and then concentrated *in vacuo.* Trituration from ether gave the title compund (251 mg) as a solid;
¹H NMR (DMSO-d₆) 1.63-1.70 (m, 2H), 2.14 (s, 6H), 2.17 (t, 2H), 3.34 (dt, 2H), 6.99 (t, 1H), 7.11 (d, 1H), 7.12-7.36 (m, 4H), 7.46 (d, 2H), 7.68 (t, 1H), 7.74 (s, 1H), 8.47 (s, 2H), 8.71 (s, 1H), 8.77 (s, 1H);
MS m/e MH⁺ 415.

**The following Examples were made in a similar way to Example 129 but were purified by flash chromatography on silica using 1-10% (10% 7N NH3 in MeOH) in DCM as eluent.**

### Example 130

### N-{3-[(2-{[3-(dimethylamino)propyl]amino}pyrimidin-5-yl)ethynyl]phenyl}-N'-(5-methylisoxazol-3-yl)urea

SM: phenyl 3-[(2-{[3-(dimethylamino)propyl]amino}pyrimidin-5-yl)ethynyl]phenylcarbamate (Example 128) and 5-methyl-3-aminoisoxazole..
¹H NMR (DMSO-d₆) 1.63-1.70 (m, 2H), 2.14 (s, 6H), 2.27 (t, 2H), 2.38 (s, 3H), 3.34 (dt, 2H), 6.54 (s, 1H), 7.11-7.17 (m, 1H), 7.30-7.38 (m, 2H), 7.66-7.71 (m, 1H), 7.76 (s, 1H), 8.48 (s, 2H), 8.85 (s, 1H), 8.91 (s, 1H);
MS m/e MH⁺ 420.

### Example 131

### N-(5-tert-butylisoxazol-3-yl)-N'-{3-[(2-{[3-(dimethylamino)propyl]amino}pyrimidin-5-yl)ethynyl]phenyl}-N-methylurea

SM: phenyl 3-[(2-{[3-(dimethylamino)propyl]amino}pyrimidin-5-yl)ethynyl]phenylcarbamate (Example 128) and 5-*tert*-butyl-*N*-methylisoxazol-3-amine (Intermediate 28).
¹H NMR (CDCl₃) 1.46 (s, 9H), 1.83-1.93 (m, 2H), 2.35 (s, 6H), 2.53 (t, 2H), 3.47 (s, 3H), 3.60 (dt, 2H), 5.94 (s, 1H), 6.35 (t, 1H), 7.28 (d, 1H), 7.36 (t, 1H), 7.62 (d, 1H), 7.81 (s, 1H), 8.49 (s, 2H), 10.39 (s, 1H);
MS m/e MH⁺ 476.

**The following Example was made in a similar way to Example 24 but purified using RPHPLC (H2O:MeCN 0-90%).**

### Example 132

### N'-{4-[(2-aminopyrimidin-5-yl)ethynyl]phenyl}-N-(5-tert-butylisoxazol-3-yl)-N-methylurea

SM: phenyl {4-[(2-aminopyrimidin-5-yl)ethynyl]phenyl}carbamate (Intermediate 40), 5*-tert-*butyl-*N*-methylisoxazol-3-amine (Intermediate 28)
¹H NMR (DMSO-d₆) 1.29 (s, 9H), 3.37 (s, 3H), 6.52 (s, 1H), 7.43 (d, 2H), 7.56 (d, 2H), 8.40 (s, 2H), 9.57 (s, 1H);
MS m/e MH⁺ 391.

### Intermediate 40

### Phenyl {4-[(2-aminopyrimidin-5-yl)ethynyl]phenyl}carbamate

Phenyl chloroformate (0.90 mL) was added to a stirred solution of 5-[(4-aminophenyl)ethynyl]pyrimidin-2-amine (Intermediate 24) (1.0 g) and pyridine (0.77 mL) in THF (75 mL). The reaction mixture was quenched with water (10 mL) and concentrated *in vacuo,* the solid filtered and washed with water followed by ether, dried under vacuum at 60°C to give the title compound as a beige solid (1.43 g, 91%);
¹H NMR (DMSO-d₆) 7.07 (bs,2H), 7.20-7.29 (m, 4H), 7.38-7.47 (m, 5H), 7.53 (s, 1H), 7.56 (s, 1H), 8.39 (s, 2H), 10.84 (bs, 1H);
MS m/e MH⁺ 331.

### Example 133

### N-{3-[(2-aminopyrimidin-5-yl)ethynyl]phenyl}-N'-(5-tert-butylisoxazol-3-yl)-N-methylurea

A solution of 5-{[3-(methylamino)phenyl]ethynyl}pyrimidin-2-amine (Intermediate 44) (91 mg) and phenyl (5-tert-butylisoxazol-3-yl)carbamate (Intermediate 4) (115 mg) in THF (6 mL) and triethylamine (0.06 mL) was heated to 60°C for 2 days. Additional phenyl (5-tert-butylisoxazol-3-yl)carbamate (Intermediate 4) (110 mg) and triethylamine (0.1 mL) was added and heating continued for 7 days. Concentration *in vacuo* and trituration with ether gave the title compound (115 mg, 73%);
¹H NMR (DMSO-d₆) 1.27 (s, 9H), 3.28 (s, 3H), 6.47 (s, 1H), 7.12 (s, 2H), 7.29-7.43 (m, 4H), 8.41,(s, 2H), 9.37 (s, 1H);
MS m/e MH⁺ 391.

### Intermediate 41

### tert-butyl 3-ethynylphenylcarbamate

3-aminophenylacetylene (10.5 mL) and di-*tert*butyldicarbonate (44.0 g) in THF (250 mL) were stirred for 48 hours, concentrated *in vacuo,* dissolved in DCM, washed with water and concentrated *in vacuo.* Purification by flash chromatography on silica using 0-50% DCM in isohexane as eluent gave the title compound as a yellow oil (20.0 g, 92%);
¹H NMR (CDCl₃) 1.52 (s, 9H), 3.04 (s, 1H), 6.45 (s, br, 1H), 7.15 (d, 1H), 7.23 (t, 1H), 7.35 (d, 1H), 7.52 (s, 1H);
MS m/e (M-CH₃)H⁺ 203.

### Intermediate 42

### tert-butyl (N-methyl)-3-ethynylphenylcarbamate

Sodium hydride (60% in mineral oil, 1.0 g) was added portionwise to a solution of *tert*-butyl 3-ethynylphenylcarbamate (Intermediate 41) (4.50 g) and iodomethane (1.80 mL) in THF (75 mL) and stirred for 5 hours. Water (100 mL) was added and the mixture extracted into ether (3 x 100 mL). The combined organics were washed with water and concentrated *in vacuo.* Purification by flash chromatography on silica using 0-10% EtOAc in isohexane as eluent gave the title compound as a yellow oil (4.02 g, 84%);
¹H NMR (CDCl₃) 1.45 (s, 9H), 3.06 (s, 1H), 3.25 (s, 3H), 7.22-7.31 (m, 3H), 7.36 (s, 1H);
MS m/e (M-CH₃)H⁺ 217.

### Intermediate 43

### tert-butyl (N-methyl)-3-[(2-aminopyrimidin-5-yl)ethynyl]phenylcarbamate

Triethylamine (1.0 mL) was added to a degassed solution of 2-amino-5-iodopyrimidine (275 mg), *tert*-butyl 3-ethynylphenyl(methyl)carbamate (Intermediate 42) (298 mg) PdCl₂(PPh₃)₂ (17 mg) and cuprous iodide (1.25 mg) in DMF.(5.0 mL). The mixture was heated to 60°C for 4 hours and concentrated *in vacuo.* Purification by flash chromatography on silica using 0-10% MeOH in DCM as eluent gave the title compound as a yellow solid (344 mg, 86%).
¹H NMR (CDCl₃) 1.47 (s, 9H), 3.27 (s, 1H), 5.22 (s, br, 2H), 7.23-7.31 (m, 3H), 7.39 (s, 1H), 8.45 (s, 2H);
MS m/e MH⁺ 325.

### Intermediate 44

### 5-{[3-(methylamino)phenyl]ethynyl}pyrimidin-2-amine

A solution of *tert*-butyl 3-[(2-aminopyrimidin-5-yl)ethynyl]phenyl(methyl)carbamate (Intermediate 43) (317 mg) in DCM (10 mL) and TFA (1 mL) was heated to 40°C for 3 hours, cooled to ambient temperature, diluted with DCM (50 mL), washed with 50% saturated aqueous sodium carbonate and concentrated *in vacuo.* Purification by flash chromatography on silica using 50% EtOAc in isohexanes as eluent gave the title compound as a yellow solid (91 mg, 42%).
¹H NMR (CDCl₃) 2.66 (d, 3H), 5.77 (q, 2H), 6.53 (d, 1H), 6.60 (s, 1H), 6.64 (d, 1H), 7.05-7.11 (m, 3H), 8.38 (s, 2H);
MS m/e MH⁺ 225.

### Example 134

### N-{5-[(2-aminopyrimidin-5-yl)ethynyl]pyridin-3-yl}-N'-(5-tert-butylisoxazol-3-yl)urea

Triethylamine (4.8 mL) was added to a solution of 5-[(5-aminopyridin-3-yl)ethynyl]pyrimidin-2-amine (Intermediate 48) (2.7 g) and phenyl (5-*tert*-butylisoxazol-3-yl)carbamate (Intermediate 4) (4.33 g) in 1,4-dioxane (100 mL) under an inert atmosphere. The reaction was heated at 80°C for 24 hours. The solvent was evaporated and the product purified by flash chromatography on silica using 0-10% MeOH in DCM as the eluent to give the title compound as a beige solid (2.33 g, 48%);
¹H NMR (DMSO-d₆) 1.32 (s, 9H), 6.53 (s, 1H), 7.21 (s, 2H), 8.15 (t, 1H), 8.36 (d, 1H), 8.49 (s, 2H), 8.54 (d, 1H), 9.09 (s, 1H), 9.77 (s,1H);
MS m/e MH⁺ 378.

### Intermediate 45

### tert-butyl (5-bromopyridin-3-yl)carbamate

Triethylamine (7 mL) followed by DPPA (10.9 mL) was added to a solution of 5-bromonicotinic acid (10.1 g) and t-BuOH (7.1 mL) in toluene (100 mL) and the reaction heated at reflux under an inert atmosphere for 1.5 hours. The reaction mixture was diluted with EtOAc (100 mL) and water (100 mL). The organic layer was separated, washed with NaHCO₃ (3 x 50 mL), dried (MgSO₄) filtered and concentrated *in vacuo.* The product was purified by flash chromatography on silica using 0-4% EtOAc in DCM as the eluent to give the title compound as a beige solid (9.82 g, 72%);
¹H NMR (DMSO-d₆) 1.50 (s, 9H), 8.19 (t, 1H), 8.30 (d, 1H), 8.57 (d, 1H), 9.80 (s, 1H);
MS m/e MH⁺ 273/275.

### Intermediate 46

### tert-Butyl (5-iodopyridin-3-yl)carbamate

*tert*-Butyl (5-bromopyridin-3-yl)carbamate (Intermediate 45) (14.9 g), CuI (520 mg), NaI (16.35 g) and N,N-dimethylethylenediamine (481 mg) in dioxane (300 mL) were heated at 110°C under an inert atmosphere for 24 hours. The reaction mixture was concentrated *in vacuo* to approx 100 mL and then water (400 mL) was added. The resultant solid was filtered, dissolved in DCM, dried (MgSO4) filtered and concentrated to afford the title compound as a beige solid (15.18 g, 87%);
MS m/e MH⁺ 321.

### Intermediate 47

### tert-butyl {5-[(2-aminopyrimidin-5-yl)ethynyl]pyridin-3-yl}carbamate

PdCl₂dppf (907 mg) was added to a degassed solution of *tert*-butyl (5-iodopyridin-3-yl)carbamate (Intermediate 46) (7.94 g), 5-ethynylpyrimidin-2-amine (Intermediate 18) (3.7 g), CuI (94 mg) and Et₃N (63 mL) in DMF (250 mL). The reaction was allowed to stir at ambient temperature under an inert atmosphere for 24 hours. Silica was added to the reaction mixture and then solvent was evaporated *in vacuo.* The preabsorbed product was purified by flash chromatography on silica using 0-10% MeOH in DCM as the eluent followed by an aqueous wash and then dried *in vacuo* to give the title compound as a beige solid (5.3 g, 69%);
¹H NMR (DMSO-d₆) 1.51 (s, 9H), 7.20 (s, 2H), 8.05 (s, 1H), 8.31 (s, 1H), 8.48 (s, 2H), 8.57 (s, 1H), 9.74 (s, 1H);
MS m/e (M-H⁺)- 310.

### Intermediate 48

### 5-[(5-aminopyridin-3-yl)ethynyl] pyrimidin-2-amine

TFA (25 mL) was added to a solution of *tert*-butyl {5-[(2-aminopyrimidin-5-yl)ethynyl]pyridin-3-yl}carbamate (Intermediate 47) (2.92 g) in DCM (150 mL) under an inert atmosphere. The reaction was allowed to stir at ambient temperature for 3 hours. It was then diluted with water (100 mL) and the DCM removed *in vacuo.* The aqueous solution was neutralised with NaHCO₃ and the resultant precipitate was filtered, washed with water and then dried *in vacuo* to give the title compound (2.00 g, 66%);
¹H NMR (DMSO-d₆) 5.49 (s, 2H), 7.00 (s, 1H), 7.88 (s, 2H), 8.44 (s, 2H);
MS m/e MH⁺ 212.

### Example 135

### N-{5-[(2-aminopyrimidin-5-yl)ethynyl]pyridin-3-yl}-N'-(3-tert-butyl-1-methyl-1H-pyrazol-5-yl)urea

Triethylamine (0.41 mL) was added to a solution of 5-[(5-aminopyridin-3-yl)ethynyl]pyrimidin-2-amine (Intermediate 48) (250 mg) and phenyl (3-*tert*-butyl-1-methyl-1*H*-pyrazol-5-yl)carbamate (Intermediate 49) (388 mg) in dioxane (10 mL) under an inert atmosphere. The reaction was heated at 80°C for 24 hours. The solvent was evaporated and the product purified by flash chromatography on silica using 0-10% MeOH in DCM as the eluent to afford the title compound as a beige solid (105 mg, 23%);
¹H NMR (DMSO-d₆) 1.23 (s, 9H), 3.63 (s, 3H), 6.08 (s, 1H), 7.20 (s, 2H), 8.16 (t, 1H), 8.33 (d, 1H), 8.48 (s, 2H), 8.54 (d, 1H), 8.74 (s, 1H), 9.23 (s, 1H);
MS m/e MH⁺ 391.

### Intermediate 49

### Phenyl (3-tert-butyl-1-methyl-1H-pyrazol-5-yl)carbamate

A suspension of 3-*tert*-butyl-1-methyl-1*H*-pyrazol-5-amine (1.92 g) and sodium hydrogencarbonate (1.26 g) in THF (40 mL) was cooled (ice-bath) then phenyl chloroformate (1.73 mL) added dropwise under an inert atmosphere. After 20 mins additional sodium hydrogencarbonate (0.3 g) and phenyl chloroformate (0.6 mL) was added and the reaction mixture warmed to ambient temperature. After stirring for a total of 4 hours sat. aq. ammonium chloride was added, then the mixture was extracted with EtOAc (2x), organics dried (MgSO₄), filtered and concentrated *in vacuo* to give a yellow oil. Trituration with 1:1 ether/iso-hexane then filtration and drying under high vacuum gave the title compound as a colourless solid (2.52g, 74%);
¹H NMR (DMSO-d₆ 1.21 (s, 9H), 3.64 (s, 3H), 6.03 (s, 1H), 7.16-7.29 (m, 3H), 7.37-7.45 (m, 2H);
MS m/e MH⁺ 274.

### Example 136

### N-{5-[(2-aminopyrimidin-5-yl)ethynyl]pyridin-3-yl}-N'-(3-cyclopropyl-1-methyl-1H-pyrazol-5-yl)urea

Triethylamine (0.41 mL) was added to a solution of 5-[(5-aminopyridin-3-yl)ethynyl]pyrimidin-2-amine (Intermediate 48) (250 mg) and phenyl (3-cyclopropyl-1-methyl-1*H*-pyrazol-5-yl)carbamate (Intermediate 50) (365 mg) in dioxane (10 mL) under an inert atmosphere. The reaction was heated at 80°C for 6 hours. The precipitate was filtered, washed with dioxane and then dried *in vacuo* to afford the title compound as a beige solid (258 mg, 58%);
¹H NMR (DMSO-d₆) 0.59 - 0.64 (m, 2H), 0.79 - 0.86 (m, 2H), 1.75 -1.84 (m, 1H), 3.59 (s, 3H), 5.91 (s, 1H), 7.20 (s, 2H), 8.14 (s, 1H), 8.34 (s, 1H), 8.48 (s, 2H), 8.54 (s, 1H), 8.74 (s, 1H), 9.16 (s, 1H);
MS m/e MH⁺ 375.

### Intermediate 50

### Phenyl (3-cyclopropyl-1-methyl-1H-pyrazol-5-yl)carbamate

A suspension of 3-cyclopropyl-1-methyl-1*H*-pyrazol-5-amine (1.63 g) and sodium hydrogencarbonate (1.2 g) in THF (40 mL) was cooled (ice-bath) then phenyl chloroformate (1.57 mL) added dropwise under an inert atmosphere. After 30 mins water was added, extracted with EtOAc (2x), organics dried (MgSO₄), filtered and concentrated *in vacuo* to give a yellow oil. Purification by flash chromatography on silica using 0-80% EtOAc in DCM as eluent gave the title compound as a colourless oil which solidified on standing (1.6g, 52%); ¹H NMR (DMSO-d₆) 0.54-0.62 (m, 2H), 0.75-0.83 (m, 2H), 1.71-1.81 (m, 1H), 3.62 (s, 3H), 5.87 (s, 1H), 7.08-7.28 (m, 3H), 7.37-7.45 (m, 2H);
MS m/e MH⁺ 258.

### Example 137

### N-{5-[(2-aminopyrimidin-5-yl)ethynyl]-1,3-thiazol-2-yl}-N'-phenylurea

A mixture of 2-amino-5-ethynylpyrimidine (Intermediate 18) (119 mg), N-(5-bromo-1,3-thiazol-2-yl)-N'-phenylurea (Intermediate 52) (298 mg), 1,1,3,3-tetramethylguanidine (138 mg), and copper (I) iodide (10 mg) in anhydrous DMF (3 mL) was stirred and degassed with nitrogen. Tetrakis(triphenylphosphine)palladium(0) (116 mg) was added and the mixture heated at 60°C for 3 hours. The mixture was concentrated, cooled, stirred, and diluted with water (20 mL). The solid formed was filtered off and dried. Purification by flash chromatography on silica using 0-40% MeOH in DCM as eluent, then trituration with DCM gave the title compound as a solid (66 mg, 19%);
¹H NMR (DMSO-d₆) 7.05 (t, 1H), 7.14 (s, 2H), 7.33 (t, 2H), 7.50 (d, 2H), 7.66 (s, 1H), 8.42 (s, 2H), 9.01 (s, 1H), 10.70 (bs, 1H);
MS m/e MH⁺ 337.

### Intermediate 51

### Phenyl (5-bromo-1,3-thiazol-2-yl)carbamate

2-Amino-5-bromothiazole (6.27 g) was stirred with pyridine (3.22 mL) in anhydrous DCM (120 mL) under an inert atmosphere and cooled on ice. Phenyl chloroformate (4.4 mL) in DCM (20 mL) was added dropwise then stirred for 2 hours. The mixture was concentrated then diluted with isohexane and water. The solid formed was filtered off, washed with water then 3:1 isohexane:DCM, and dried at ambient temperature. The solid was taken up in THF (400 mL), dried (MgSO4) and the solvent evaporated to give the product (9.5 g, 88%).
¹H NMR (DMSO-d₆) 7.27 (m, 3H), 7.44 (m, 2H), 7.52 (s, 1H);
MS m/e MH⁺ 301, 299 (1 x Br).

### Intermediate 52

### N-(5-bromo-1,3-thiazol-2-yl)-N'-phenylurea

Phenyl (5-bromo-1,3-thiazol-2-yl)carbamate (Intermediate 51) (1.79 g) was stirred with aniline (0.55 g) and triethylamine (1.0 mL) in anhydrous 1,4-dioxane (10 mL) at 80°C under an inert atmosphere for 1 hour. Concentration, then purification by flash chromatography on silica using 0-100% EtOAc in DCM, then 0-10% MeOH in DCM as eluent gave the product as a solid (0.9 g, 50%);
¹H NMR (DMSO-d₆) 7.03 (t, 1H), 7.30 (t, 2H), 7.45 (m, 3H), 8.90 (s, 1H), 10.69 (bs, 1H); MS m/e MH⁺ 298, 300 (1 x Br).

**Examples 138 to 142 were made in a similar way to Example 137 by using the appropriate bromo-urea intermediate in place of Intermediate 52.**

### Example 138

### N-{5-[(2-aminopyrimidin-5-yl)ethynyl]-1,3-thiazol-2-yl}-N'-(2,2-dimethyltetrahydro-2H-pyran-4-yl)urea

SM: 2-amino-5-ethynylpyrimidine (Intermediate 18), *N*-(5-bromo-1,3-thiazol-2-yl)-*N*-(2,2-dimethyltetrahydro-2*H*-pyran-4-yl)urea (Intermediate 53)
¹H NMR (DMSO-d₆) 1.15 (s, 3H), 1.19 (s, 3H), 1.25 (m, 2H), 1.79 (m, 2H), 3.63 (m, 2H), 3.86 (m, 1H), 6.48 (d, 1H), 7.12 (s, 2H), 7.59 (s, 1H), 8.40 (s, 2H), 10.53 (bs, 1H);
MS m/e MH⁺ 373.

### Intermediate 53

### N-(5-bromo-1,3-thiazol-2-yl)-N'-(2,2-dimethyltetrahydro-2H-pyran-4-yl)urea

Prepared in a similar manner to Intermediate 52 from phenyl (5-bromo-1,3-thiazol-2-yl)carbamate (Intermediate 51) and (2,2-dimethyltetrahydro-2*H*-pyran-4-yl)amine
¹H NMR (DMSO-d₆) 1.13 (s, 3H), 1.17 (s, 3H), 1.25 (m, 2H), 1.75 (m, 2H), 3.60 (m, 2H), 3.82 (m, 1H), 6.41 (d, 1H), 7.35 (s, 1H), 10.42 (bs, 1H);
MS m/e MH⁺ 334, 336 (1 x Br).

### Example 139

### N-{5-[(2-aminopyrimidin-5-yl)ethynyl]-1,3-thiazol-2-yl}-N'-(3-cyclopropyl-1-methyl-1H-pyrazol-5-yl)urea

SM: 2-amino-5-ethynylpyrimidine (Intermediate 18), *N*-(5-bromo-1,3-thiazol-2-yl)-N-(3-cyclopropyl-1-methyl-1*H*-pyrazol-5-yl)urea (Intermediate 54)
¹H NMR (DMSO-d₆) 0.61 (m, 2H), 0.81 (m, 2H), 1.80 (m, 1H), 3.57 (s, 3H), 5.93 (s, 1H), 7.13 (s, 2H), 7.67 (s, 1H), 8.41 (s, 2H), 8.88 (bs, 1H), 11.00 (bs, 1H);
MS m/e MH⁺ 381.

### Intermediate 54

### N-(5-bromo-1,3-thiazol-2-yl)-N'-(3-cyclopropyl-1-methyl-1H-pyrazol-5-yl)urea

Phenyl (5-bromo-1,3-thiazol-2-yl)carbamate (Intermediate 51) (748 mg) was stirred with 3-cyclopropyl-1-methyl-1*H*-pyrazol-5-amine (343 mg) and triethylamine (0.42 mL) in anhydrous THF (5 mL) at 60°C under an inert atmosphere for 3 hours. Concentration, then purification by flash chromatography on silica using 0-100% EtOAc in DCM, then 0-10% MeOH in DCM as eluent gave the product as a solid (500 mg, 58%);
¹H NMR (DMSO-d₆) 0.59 (m, 2H), 0.80 (m, 2H), 1.77 (m, 1H), 3.54 (s, 3H), 5.89 (s, 1H), 7.44 (s, 1H), 8.80 (bs, 1H), 10.97 (bs, 1H);
MS m/e MH⁺ 342, 344 (1 x Br).

### Example 140

### N-{5-[(2-aminopyrimidin-5-yl)ethynyl]-1,3-thiazol-2-yl}-N'-(3-tert-butyl-1-methyl-1H-pyrazol-5-yl)urea

SM: 2-amino-5-ethynylpyrimidine (Intermediate 18), *N*-(5-bromo-1,3-thiazol-2-yl)-*N*'-(3-*tert-*butyl-1-methyl-1*H*-pyrazol-5-yl)urea (Intermediate 55)
¹H NMR (DMSO-d₆) 1.22 (s, 9H), 3.61 (s, 3H), 6.12 (s, 1H), 7.15 (s, 2H), 7.67 (s, 1H), 8.41 (s, 2H), 8.83 (bs, 1H), 11.05 (bs, 1H);
MS m/e MH⁺ 397.

### Intermediate 55

### N-(5-bromo-1,3-thiazol-2-yl)-N'-(3-tert-butyl-1-methyl-1H-pyrazol-5-yl)urea

Prepared in a similar manner to Intermediate 54 using phenyl (5-bromo-1,3-thiazol-2-yl)carbamate (Intermediate 51) and 3-*tert*-butyl-1-methyl-1*H*-pyrazol-5-amine;
¹H NMR (DMSO-d₆) 1.20 (s, 9H), 3.58 (s, 3H), 6.07 (s, 1H), 7.45 (s, 1H), 8.78 (bs, 1H), 10.94 (bs, 1H);
MS m/e (M-H)-356, 358 (1 x Br).

### Example 141

### N-{5-[(2-aminopyrimidin-5-yl)ethynyl]-1,3,4-thiadiazol-2-yl}-N'-phenylurea

SM: 2-amino-5-ethynylpyrimidine (Intermediate 18), *N*-(5-bromo-1,3,4-thiadiazol-2-yl)-*N-*phenylurea (Intermediate 57). Further purified by trituration from MeOH then DCM;
¹H NMR (DMSO-d₆) 7.06 (t, 1H), 7.34 (m, 4H), 7.50 (d, 2H), 8.53 (s, 2H), 9.12 (bs, 1H), 11.23 (bs, 1H);
MS m/e MH⁺ 338.

### Intermediate 56

### Phenyl (5-bromo-1,3,4-thiadiazol-2-yl)carbamate

Phenyl chloroformate (4.4 mL) in DCM (20 mL) was added dropwise to a stirred solution of 2-amino-5-bromo-1,3,4-thiadiazole {Eur.J.Med.Chem.Chim.Ther. (1975) 121} (6.3 g) in pyridine (100 mL) cooled on an ice bath. After 3 hours the mixture was concentrated then diluted with water (400 mL). The solid formed was filtered off and dried at ambient temperature *in vacuo.* Purification by flash chromatography on silica using acetone as eluent, then trituration with ether/iso-hexane gave the product as a solid (5.3 g, 51%);
¹H NMR (DMSO-d₆) 7.29 (m, 3H), 7.44 (m, 2H), 13.10 (bs, 1H);
MS m/e MH⁺ 300, 302 (1 x Br).

### Intermediate 57

### N-(5-bromo-1,3,4-thiadiazol-2-yl)-N'-phenylurea

Phenyl (5-bromo-1,3,4-thiadiazol-2-yl)carbamate (Intermediate 56) (600 mg) was stirred with aniline (186 mg) and triethylamine (0.5 mL) in anhydrous THF (5 mL) at 60°C under an inert atmosphere for 3 hours. Concentration, then purification by flash chromatography on silica using 0-100% EtOAc in DCM as eluent gave the product as a solid (300 mg, 50%);
¹H NMR (DMSO-d₆) 7.05.(t, 1H), 7.31 (t, 2H), 7.45 (d, 2H), 9.04 (bs, 1H), 11.23 (bs, 1H); MS m/e (M-H)⁻ 297, 299 (1 x Br).

### Example 142

### N-{5-[(2-aminopyrimidin-5-yl)ethynyl]-1,3,4-thiadiazol-2-yl}-N'-(2,2-dimethyltetrahydro-2H-pyran-4-yl)urea

SM: 2-amino-5-ethynylpyrimidine (Intermediate 18), *N*-(5-bromo-1,3,4-thiadiazol-2-yl)-*N'-*(2,2-dimethyltetrahydro-2*H*-pyran-4-yl)urea (Intermediate 58) (335 mg). Final trituration from MeOH.
¹H NMR (DMSO-d₆) 1.15 (s, 3H), 1.19 (s, 3H), 1.29 (m, 2H), 1.78 (m, 2H), 3.62 (m, 2H), 3.87 (m, 1H), 6.63 (bs, 1H), 7.33 (s, 2H), 8.52 (s, 2H), 10.95 (bs, 1H);
MS m/e MH⁺ 374.

### Intermediate 58

### N-(5-bromo-1,3,4-thiadiazol-2-yl)-N'-(2,2-dimethyltetrahydro-2H-pyran-4-yl)urea

Prepared in a similar manner to Intermediate 57 from Phenyl (5-bromo-1,3,4-thiadiazol-2-yl)carbamate (Intermediate 56) (600 mg) and (2,2-dimethyltetrahydro-2*H*-pyran-4-yl)amine (258 mg) to give a solid (500 mg, 74%);
¹H NMR (DMSO-d₆) 1.13 (s, 3H), 1.16 (s, 3H), 1.29 (m, 2H), 1.73 (m, 2H), 3.60 (m, 2H), 3.84 (m, 1H), 6.60 (bs, 1H), 10.97 (bs, 1H);
MS m/e MH⁺ 335, 337 (1 x Br).

**The following Examples were made in a similar way to Example 89 by reacting the appropriate amino heterocycle with Example 23.**

### Example 143

### N-{3-[(2-aminopyrimidin-5-yl)ethynyl]phenyl}-N'-(1,3-dimethyl-1H-pyrazol-5-yl)urea

SM: Phenyl {3-[(2-aminopyrimidin-5-yl)ethynyl]phenyl}carbamate (Example 23), 5-amino-1,3-dimethylpyrazole.
¹H NMR (DMSO-d₆) 2.08 (s, 3H), 3.57 (s, 3H), 5.95 (s, 1H). 7.10 (s, 2H), 7.12 (t, 1H), 7.27 - 7.36 (m, 2H), 7.71 (s, 1H), 8.42 (s, 2H), 8.54 (s, 1H), 8.92 (s, 1H);
MS m/e MH⁺ 348.

### Example 144

### N-{3-[(2-aminopyrimidin-5-yl)ethynyl]phenyl}-N'-[5-(ethylthio)-1,3,4-thiadiazol-2-yl] urea

SM: Phenyl {3-[(2-aminopyrimidin-5-yl)ethynyl]phenyl}carbamate (Example 23), 2-Amino-5-(Ethylthio)-1,3,4-Thiadiazole
¹H NMR (DMSO-d₆) 1.34 (t, 3H), 3.19 (q, 2H), 7.11 (s, 2H), 7.12 (d, 1H), 7.31 - 7.39 (m, 2H), 7.71 (s, 1H), 8.43 (s, 2H), 9.10 (s, 1H), 11.08 (s, 1H);
MS m/e MH⁺ 398.

### Example 145

### N-{3-[(2-aminopyrimidin-5-yl)ethynyl]phenyl}-N'-(3-cyclopropyl-1-methyl-1H-pyrazol-5-yl)urea

Phenyl (3-cyclopropyl-1-methyl-1*H*-pyrazol-5-yl)carbamate (Intermediate 50) (220 mg), 5-[(3-aminophenyl)ethynyl]pyrimidin-2-amine (Intermediate 1) (163 mg) and triethylamine (0.22 mL) in THF (10 mL) were heated at 70°C for 1.5 hours under an inert atmosphere. The reaction mixture was cooled, filtered and washed with ether to give a dark brown solid. Purification by flash chromatography on silica using 8% MeOH in DCM as eluent gave the product as an off-white solid (200 mg, 67%);
¹H NMR (DMSO-d₆) 0.52-0.60 (m, 2H), 0.71-0.82 (m, 2H), 1.69-1.81 (m, 1H), 3.55 (s, 3H), 5.87 (s, 1H), 7.04-7.12 (m, 3H), 7.24-7.37 (m; 2H), 7.71 (bs, 1H), 8.42 (s, 2H), 8.53 (bs, 1H), 8.92 (bs, 1H);
MS m/e MH⁺ 374.

**The following Examples were prepared in similar manner to Example 145;**

### Example 146

### N-{3-[(2-aminopyrimidin-5-yl)ethynyl]phenyl}-N'-(3-tert-butyl-methyl-1H-pyrazol-5-yl)urea

SM: phenyl (3-*tert*-butyl-1-methyl-1*H*-pyrazol-5-yl)carbamate (Intermediate 49), 5-[(3-aminophenyl)ethynyl]pyrimidin-2-amine (Intermediate 1);
¹H NMR (DMSO-d₆) 0.52-0.60 (m, 2H), 1.21 (s, 9H), 3.59 (s, 3H), 6.04 (s, 1H), 7.09 (bs, 3H), 7.26-7.33 (m, 2H), 7.74 (s, 1H), 8.41 (s, 2H), 8.50 (bs, 1H), 8.95 (bs, 1H);
MS m/e MH⁺ 374.

### Example 147

### N-{3-[(2-aminopyrimidin-5-yl)ethynyl-phenyl]-N'-(1-tert-butyl-1H-pyrazol-4-yl)urea

SM: phenyl (1-*tert*-butyl-1*H*-pyrazol-4-yl)carbamate (Intermediate 59), 5-[(3-aminophenyl)ethynyl]pyrimidin-2-amine (Intermediate 1);
¹H NMR (DMSO-d₆) 1.47 (s, 9H), 7.03-7.11 (m, 3H), 7.21-7.33 (m, 2H), 7.40 (s, 1H), 7.73 (s, 1H), 7.80 (s, 1H), 8.34 (s, 1H), 8.42 (s, 2H), 8.69 (s, 1H);
MS m/e MH⁺ 376.

### Intermediate 59

### Phenyl (1-tert-butyl-1H-pyrazol-4-yl)carbamate

Prepared in a similar manner to phenyl (5-tert-butyl-1,3,4-thiadiazol-2-yl)carbamate (Intermediate 2) using 1-*tert*-butyl-1*H*-pyrazol-4-amine (DE 3332270) in place of 2-amino-5-tert-butyl-1,3,4-thiadiazole;
¹H NMR (DMSO-d₆) 1.47 (s, 9H), 7.13-7.25 (m, 3H), 7.34-7.42 (m, 3H), 7.72 (s, 1H), 9.89 (s, 1H);
MS m/e MH⁺ 260.

### Example 148

### N-{3-[(2-aminopyrimidin-5-yl)ethynyl]phenyl}-N'-(3-isopropyl-1-methyl-1H-pyrazol-5-yl)urea

Prepared in similar manner to Example 89 by replacing 2-amino-5-methyl-1,3,4-thiadiazole with 3-isopropyl-1-methyl-1*H*-pyrazol-5-amine (JP 59065089) and reacting with phenyl {3-[(2-aminopyrimidin-5-yl)ethynyl]phenyl}carbamate (Example 23);
¹H NMR (DMSO-d₆) 1.14 (d, 6H), 2.68-2.82 (m, 1H), 3.57 (s, 3H), 5.98 (s, 1H), 7.05-7.12 (m, 3H), 7.26-7.34 (m, 2H), 7.72 (s, 1H), 8.41 (s, 2H). 8.53 (s, 1H), 8.95 (s, 1H);
MS m/e MH⁺ 376.

### Example 149

### N-{3-[(2-aminopyrimidin-5-yl)ethyny]phenyl}-N'-(5-isopropyl-1,3,4-oxadiazol-2-yl)urea

Prepared in similar manner to Example 89 by replacing 2-amino-5-methyl-1,3,4-thiadiazole with 5-isopropyl-1,3,4-oxadiazol-2-amine (WO 9932106) and reacting with phenyl {3-[(2-aminopyrimidin-5-yl)ethynyl]phenyl}carbamate (Example 23). Purification was by trituration with DMF and washing with ether;
¹H NMR (DMSO-d₆) 1.26 (d, 6H), 3.05-3.13 (m, 1H), 7.05-7.18 (m, 3H), 7.26-7.35 (m, 1H), 7.39-7.47 (m, 1H), 7.74 (bs, 1H), 8.42 (s, 2H), 9.61 (s, 1H);
MS m/e MH⁺ 364.

### Example 150

### N-{3-[(2-aminopyrimidin-5-yl)ethynyl]phenyl}-N'-(1-ethyl-1H-pyrazol-3-yl)urea

Prepared in similar manner to Example 89 by replacing 2-amino-5-methyl-1,3,4-thiadiazole with 1-ethyl-1*H*-pyrazol-3-amine (Intermediate 60) and reacting with phenyl {3-[(2-aminopyrimidin-5-yl)ethynyl]phenyl}carbamate (Example 23);
¹H NMR (DMSO-d₆) 1.32 (t, 3H), 4.01 (q, 2H), 6.18 (d, 1H), 7.09 (bs, 3H), 7.26-7.33 (m, 2H), 7.56 (d, 1H), 7.73 (s, 1H), 8.43 (s, 2H), 8.99 (s, 1H), 9.05 (s, 1H);
MS m/e MH⁺ 348.

### Intermediate 60

### 1-ethyl-1H-pyrazol-3-amine

2-Chloro acrylonitrile (2.67 mL) was added at ambient temperature to a stirred solution of *N-*ethylhydrazine oxalate (5.00 g), K₂CO₃ (9.20 g) in H₂O (50 mL). The reaction was warmed to 45°C for 4 hours before cooling back to ambient temperature. The aqueous layer was then extracted with EtOAc (6 x 30 mL) and the combined organic layers were dried (MgSO₄), filtered and evaporated to give the title compound (3.00 g, 81%) as a colourless oil;
¹H NMR (CDCl₃) 1.42 (t, 3H), 3.58 (br. s, 2H), 3.98 (q, 2H), 5.59 (d, 1H), 7.16 (d, 1H).

### Example 151

### N-{3-[(2-aminopyrimidin-5-yl)ethynyl]phenyl}-N'-(1-isopropyl-1H-pyrazol-3-yl)urea

Prepared in similar manner to Example 89 by replacing 2-amino-5-methyl-1,3,4-thiadiazole with 1-isopropyl-1*H*-pyrazol-3-amine (Intermediate 61) and reacting with phenyl {3-[(2-aminopyrimidin-5-yl)ethynyl]phenyl}carbamate (Example 23);
¹H NMR (DMSO-d₆) 1.38 (d, 6H), 4.36 (sep, 1H), 6.16 (d, 1H), 7.08 (bs, 3H), 7.26-7.31 (m, 1H), 7.59 (d, 1H), 7.72 (s, 1H), 8.42 (s, 2H), 9.03 (s, 1H);
MS m/e MH⁺ 362.

### Intermediate 61

### 1-isopropyl-1H-pyrazol-3-amine

2-Chloro acrylonitrile (3.41 mL) was added at ambient temperature to a stirred solution of *N-*isopropylhydrazine hydrochloride (4.71 g), K₂CO₃ (11.77 g) in H₂O (50 mL). The reaction was warmed to 45°C for 4 hours before cooling back to ambient temperature. The aqueous layer was then extracted with EtOAc (5 x 30 mL) and the combined organic layers were dried (MgSO₄), treated with activated charcoal, filtered and evaporated. Purification by flash chromatography on silica using 2:1 EtOAc:hexanes to EtOAc as eluent gave the title compound (3.08 g, 58%) as a 6:1 mixture of title compound to regioisomeric product as an oil;
¹H NMR (CDCl₃): 1.42 (m, 6H), 3.58 (br. s, 2H), 4.25 (sept, 1H), 5.58 (d, 1H), 7.15 (d, 1H).

### Example 152

### N-{3-[(2-aminopyrimidin-5-yl)ethynyl]phenyl}-N'-[3-fluoro-5-(4-methylpiperazin-1-yl)phenyl]urea

Phenyl, {3-[(2-aminopyrimidin-5-yl)ethynyl]phenyl}carbamate (Example 23) (601 mg), [3-fluoro-5-(4-methylpiperazin-1-yl)phenyl]amine (Intermediate 63) (400 mg) and triethylamine (0.51 mL) were stirred in THF (12 mL) under an inert atmosphere at 60°C for 16 hours. After cooling, the resultant precipitate was filtered, washed with THF (10 mL) and dried under high vacuum to afford the title compound (350 mg, 43%);
¹H NMR (DMSO-d₆) 2.21 (s, 3H), 2.43 (t, 4H), 3.12 (t, 4H), 6.34 (d, 1H), 6.71-6.82 (m, 2H), 7.10 (s, 3H), 7.27-7.36 (m, 2H), 7.71 (s, 1H), 8.42 (s, 2H), 8.75 (d, 2H);
MS m/e MH⁺ 446.

### Intermediate 62

### 1-(3-fluoro-5-nitrophenyl)-4-methylpiperazine

A solution of 3,5-difluoronitrobenzene (1.13 mL) and 1-methylpiperazine (2.21 mL) in DMSO (17 mL) was stirred at 100°C under an inert atmosphere for 16 hours. The reaction was cooled, water (150 mL) added then stirred for 30 mins. The resultant precipitate was isolated by filtration, washed with water (4x 30 mL) and dried to give the title compound as a bright yellow solid (2.07 g, 86%);
¹H NMR (DMSO-d₆) 2.21 (s, 3H), 2.42 (t, 4H), 3.25-3.31 (m, obscured by water), 7.23 (d, 1H), 7.33 (d, 1H), 7.50, s, 1H);
MS m/e MH⁺ 240.

### Intermediate 63

### [3-fluoro-5-(4-methylpiperazin-1-yl)phenyl]amine

10% Platinum on activated carbon (100 mg) was added to a solution of 1-(3-fluoro-5-nitrophenyl)-4-methylpiperazine (Intermediate 62 (1.00g) in EtOAc (10 mL) under an inert atmosphere. The reaction was then placed under an atmosphere of hydrogen and stirred vigorously for 16 hours. The reaction vessel was purged with nitrogen, the reaction mixture filtered through diatomaceous earth and washed with EtOAc (20 mL). The filtrate was evaporated *in vacuo* and dried under high vacuum to give the title compound as a brown gum which crystallized on standing (804 mg, 92%);
¹H NMR (DMSO-d₆) 1.86 (s, 3H), 2.67 (t, 4H), 3.41 (t, 4H), 4.77 (s, 2H), 6.91 (d, 1H), 6.78 (d, 1H), 7.60 (s, 1H);
MS m/e MH⁺ 210.

### Example 153

### N-{3-[(2-Aminopyrimidin-5-yl)ethynyl]-4-methylphenyl}-N'-(3-tert-butyl-1-methyl-1H pyrazol-5-yl)urea

Triethylamine (0.3 mL) was added to a solution of 5-[(5-amino-2-methylphenyl)ethynyl]pyrimidin-2-amine (Intermediate 64) (200 mg) and phenyl (5-tert-butylisoxazol-3-yl)carbamate (Intermediate 4) (257 mg) in THF (5 mL) and heated to 60°C for 4 hours. The reaction mixture was concentrated *in vacuo* and dissolved in ethyl acetate, washed with water and concentrated *in vacuo.* Trituration from ether and vacuum drying at 60°C gave the title compound as a white solid (176 mg, 53%).
¹H NMR (DMSO-d₆) 1.20 (s, 9H), 2.36 (s, 3H), 3.59 (s, 3H), 6.03 (s, 1H), 7.10 s, 2H), 7.18 8 (d, 1H), 7.23 (dd, 1H), 7.68 (d, 1H), 8.42 (s, 2H), 8.46 (s, 1H), 8.83 (s, 1H);
MS m/e MH⁺ 404.

### Intermediate 64

### 5-[(5-Amino-2-methylphenyl)ethynyl]pyrimidin-2-amine

Triethylamine (8 mL) was added to a degassed solution of 5-ethynylpyrimidin-2-amine (Intermediate 18) (1.19 g), 3-iodo-4-methylaniline (2.6 g), PdCl₂(PPh₃)₂ (100 mg) and CuI (15 mg) in DMF (40 mL) and the mixture heated to 60°C for 90 minutes, cooled and concentrated *in vacuo.* The residue was dissolved in methanol, filtered, and concentrated *in vacuo*. Purification by flash chromatography on silica using 0-10% MeOH in DCM gave the title compound as a pale yellow solid (1.60 g, 70%, contaminated with 0.3 moles of Et₃NHI).
¹H NMR (DMSO-d₆) 2.24 (s, 3H), 4.95 (s, 2H), 6.50 (dd, 1H), 6.65 (d, 1H), 6.91 (s, 1H), 7.05 (s, 2H), 7.94 (s, 2H);
MS m/e MH⁺ 225.

## Claims

1. A compound of the Formula I: 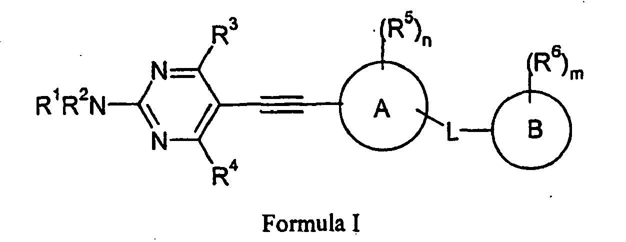 wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, (3-6C)cycloalkyl(CH₂)ₓ- in which x is 0, 1, 2, 3, 4, 5 or 6, or a 5 or 6 membered heteroaryl ring, or R¹ and R² together with the nitrogen atom to which they are attached represent a saturated or partially saturated 3 to 7 membered heterocyclic ring optionally containing another hetero atom selected from N or O;
wherein the (1-6C)alkyl, the (1-6C)alkanoyl and the (3-6C)cycloalkyl groups are optionally substituted by one or more groups independently selected from fluoro, hydroxy, (1-6C)alkyl, (1-6C)alkoxy, (1-6C)alkoxy(1-6C)alkoxy, (1-6C)alkoxy(1-6C)alkoxy(1-6C)alkoxy, amino, mono(1-6C)alkylamino, di-[(1-6C)alkyl]amino, carbamoyl, mono(1-6C)alkylcarbamoyl, di-[(1-6C)alkyl]carbamoyl, N(R^{d})C(O)(1-6C)alkyl in which R^{d} is hydrogen or (1-6C)alkyl, a saturated or partially saturated 3 to 7 membered heterocyclic ring, or a 5 or 6 membered heteroaryl ring,
wherein the (1-6C)alkoxy, (1-6C)alkoxy(1-6C)alkoxy and (1-6C)alkoxy(1-6C)alkoxy(1-6C)alkoxy groups and the (1-6C)alkyl groups of the mono(1-6C)alkylamino, di-[(1-6C)alkyl]amino, mono(1-6C)alkylcarbamoyl, di-[(1-6C)alkyl]carbamoyl and/or -N(R^{d})C(O)(1-6C)alkyl groups are optionally substituted by one or more hydroxy groups;
wherein the phenyl is optionally substituted by one or more groups independently selected from halo, (1-6C)alkyl, (1-6C)alkoxy, amino, mono(1-6C)alkylamino or di-[(1-6C)alkyl]amino, wherein the (1-6C)alkyl and the (1-6C)alkoxy groups are optionally substituted by one or more groups independently selected from hydroxy, amino, mono(1-6C)alkylamino or di-[(1-6C)alkyl]amino;
and wherein any heterocyclic and heteroaryl rings within R¹ and/or R² are optionally independently substituted by one or more of the following: (1-4C)alkyl, (1-4C)alkoxy, (1-4C)alkoxy(1-4C)alkyl, hydroxy, amino, mono(1-6C)alkylamino, di-[(1-6C)alkyl]amino, a saturated or partially saturated 3 to 7 membered heterocyclic ring or -C(O)(CH₂)_{z}Y wherein z is 0, 1, 2 or 3 and Y is selected from hydrogen, hydroxy, (1-4C)alkoxy, amino, mono(1-6C)alkylamino, di-[(1-6C)alkyl]amino or a saturated or partially saturated 3 to 7 membered heterocyclic ring;
and provided that when R¹ and/or R² is a (1C)alkanoyl group, then the (1C)alkanoyl is not substituted by fluoro or hydroxy;
**R³ and R⁴** are independently selected from hydrogen, (1-6C)alkyl or (1-6C)alkoxy, wherein the (1-6C)alkyl and the (1-6C)alkoxy groups are optionally substituted by one or more groups independently selected from: fluoro, hydroxy, (1-6C)alkyl, (1-6C)alkoxy, amino, mono(1-6C)alkylamino, di-[(1-6C)alkyl]amino, carbamoyl, mono(1-6C)alkylcarbamoyl or di-[(1-6C)alkyl]carbamoyl, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)atkyl, (1-4C)alkoxy, hydroxy, amino, mono(1-6C)alkylamino or di-[(1-6C)alkyl]amino or a saturated or partially saturated 3 to 7 membered heterocyclic ring;
or one of **R³** and **R⁴** is as defined above and the other represents a group-NR¹R² as defined above;
A represents an aryl group or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁵** is selected from cyclopropyl, cyano, halo, (1-6C)alkoxy or (1-6C)alkyl, wherein the (1-6C)alkyl and the (1-6C)alkoxy groups are optionally substituted by cyano or by one or more fluoro;
**n** is 0, 1, 2 or 3;
**L** is attached meta or para on ring A with respect to the point of attachment of the ethynyl group and represents -C(R^{a}R^{b})C(O)N(R⁹)-, -N(R⁸)C(O)C(R^{a}R^{b})-, -N(R⁸)C(O)N(R⁹)-, -N(R⁸)C(O)O-, or -OC(O)-N(R⁹)-, wherein R⁸ and R⁹ independently represent hydrogen or (1-6C)alkyl and wherein R^{a} and R^{b} independently represent hydrogen or (1-6C)alkyl or R^{a} and R^{b} together with the carbon atom to which they are attached represent (3-6C)cycloalkyl;
**B** represents a (3-7C)cycloalkyl ring, a saturated or partially saturated 3 to 7 membered heterocyclic ring, an aryl group, a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl, or a 8, 9 or 10 membered bicyclic group which optionally contains 1, 2, 3 or 4 heteroatoms independently selected from N, O and S and which is saturated, partially saturated or aromatic;
**R⁶** is selected from halo, cyano, oxo, a (3-7C)cycloalkyl ring, a saturated or partially saturated 3 to 7 membered heterocyclic ring, and -N(R^{c})C(O)(1-6C)alkyl in which R^{c} is hydrogen or (1-6C)alkyl; or
**R⁶** is selected from (1-6C)alkyl, -S(O)ₚ-(1-6C)alkyl wherein p is 0, 1 or 2, or (1-6C)alkoxy, wherein the (1-6C)alkyl, -S(O)ₚ-(1-6C)alkyl and the (1-6C)alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring; and
wherein the (3-7C)cycloalkyl ring and saturated or partially saturated 3 to 7 membered heterocyclic ring are optionally independently substituted by one or more groups selected from (1-6C)alkyl; and
**m** is 0,1,2 or 3;
and when B is a (3-7C)cycloalkyl ring, a saturated or partially saturated 3 to 7 membered heterocyclic ring or a saturated or partially saturated 8, 9 or 10 membered bicyclic group, the rings and the bicyclic group optionally bear 1 or 2 oxo or thioxo substituents;
and salts thereof.

2. A compound of Formula I according to Claim 1, wherein:
**R⁶** is selected from halo, cyano, a (3-7C)cycloalkyl ring, a saturated or partially saturated 3 to 7 membered heterocyclic ring or an alkanoylamino group -N(R^{c})C(O)(1-6C)alkyl in which R^{c} is hydrogen or (1-6C)alkyl; or
**R⁶** is selected from (1-6C)alkyl or (1-6C)alkoxy, wherein the (1-6C)alkyl and the (1-6C)alkoxy groups are optionally substituted by one or more groups independently selected from cyano, fluoro, hydroxy, (1-6C)alkoxy, amino, mono(1-6C)alkylamino, di-[(1-6C)alkyl]amino, a (3-7C)cycloalkyl ring or a saturated or partially saturated 3 to 7 membered heterocyclic ring;
and salts thereof.

3. A compound of the Formula I according to claim 1, wherein:
**R¹ and R²** are independently selected from hydrogen, (1-6C)alkylsulfonyl, phenyl(CH₂)ᵤ- wherein u is 0, 1, 2, 3, 4, 5 or 6, (1-6C)alkanoyl, (1-6C)alkyl, (1-6C)alkoxycarbonyl, or (3-6C)cycloalkyl(CH₂)ₓ- in which x is 0, 1, 2, 3, 4, 5 or 6 or **R¹ and R²** together with the nitrogen atom to which they are attached represent a saturated or partially saturated 3 to 7 membered heterocyclic ring optionally containing another hetero atom selected from N or O;
wherein the alkyl and the cycloalkyl groups are optionally substituted by one or more groups selected from fluoro, hydroxy, (1-6C)alkyl, (1-6C)alkoxy, amino, mono(1-6C)alkylamino or di-[(1-6C)alkyl]amino, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, hydroxy, amino, mono(1-6C)alkylamino or di-[(1-6C)alkyl]amino or a saturated or partially saturated 3 to 7 membered heterocyclic ring;
and wherein the phenyl is optionally substituted by one or more groups selected from halo, (1-6C)alkyl, (1-6C)alkoxy, amino, mono(1-6C)alkylamino or di-[(1-6C)alkyl]amino, wherein the (1-6C)alkyl or (1-6C)alkoxy are optionally substituted by hydroxy, amino, mono(1-6C)alkylamino or di-[(1-6C)alkyl]amino;
**R³ and R⁴** are independently selected from hydrogen, (1-6C)alkyl or (1-6C)alkoxy wherein the alkyl and the alkoxy groups are optionally substituted by one or more groups selected from fluoro, hydroxy, (1-6C)alkyl, (1-6C)alkoxy, amino, mono(1-6C)alkylamino or di-[(1-6C)alkyl]amino, a saturated or partially saturated 3 to 7 membered heterocyclic ring or a 5 or 6 membered heteroaryl ring, wherein said heterocyclic and heteroaryl rings are optionally independently substituted by one or more of the following: (1-4C)alkyl, hydroxy, amino, mono(1-6C)alkylamino or di-[(1-6C)alkyl]amino or a saturated or partially saturated 3 to 7 membered heterocyclic ring;
or one of **R³** and **R⁴** is as defined above and the other represents a group -NR¹R² as defined above;
**R⁵** is selected from cyano, halo, (1-6C)alkoxy or (1-6C)alkyl optionally substituted by cyano or by one or more fluoro;
**B** represents a (3-7C)cycloalkyl ring, an aryl or a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl or 1,3,5-triazinyl;
**R⁶** is selected from halo, cyano, a saturated or partially saturated 3 to 7 membered heterocyclic ring or an alkanoylamino group -N(R^{c})C(O)(1-6C)alkyl in which R^{c} is hydrogen or (1-6C)alkyl; or **R⁶** is selected from (1-6C)alkyl or (1-6C)alkoxy, wherein the alkyl and the alkoxy groups are optionally substituted by one or more groups selected from cyano, fluoro, hydroxy, (1-6C)alkoxy, amino, mono(1-6C)alkylamino, di-[(1-6C)alkyl]amino, or a saturated or partially saturated 3 to 7 membered heterocyclic ring; and
m is **0, 1, 2 or 3**; and when m is at least 2 then two substituents on adjacent carbon atoms in ring B may together represent a methylenedioxy group;
and wherein A, L and n are as defined in Claim 1.
and salts thereof.

4. A compound according to any one of Claims 1, 2 and 3 wherein A is selected from phenyl, pyridyl, thiazolyl, thiadiazolyl or pyrimidinyl.

5. A compound accordingly to any one of the preceding claims wherein B is selected from phenyl, 2,3-di-hydro-indenyl, piperidinyl, pyridyl, pyrazolyl, isothiazolyl, thiadiazolyl, isoxazolyl, benzodioxinyl, benzodioxolyl or tetrahydropyranyl

6. A compound accordingly to any one of the preceding claims wherein L is selected from -N(R⁸)C(O)N(R⁹)-, -N(R⁸)C(O)O- or -N(R⁸)C(O)CH₂- wherein R⁸ and R⁹ independently represent hydrogen or (1-6C)alkyl.

7. A compound accordingly to any one of the preceding claims wherein R¹ and R² are both hydrogen or R¹ is hydrogen or (1-6C)alkyl and R² is (1-6C)alkyl
wherein (1-6Calkyl) is optionally substituted by hydroxy, amino, mono(1-6C)alkylamino or di(1-6C)alkylamino, carbamoyl, (1-6C)alkoxy, (1-6C)alkoxy(1-6C)alkoxy, -N(R^{d})C(O)(1-6C)alkyl in which R^{d} is hydrogen or (1-6C)alkyl, aryl (particularily phenyl), a saturated or partially saturated 3 to 7 membered heterocyclic ring or a 5 or 6 membered heteroaryl ring;
wherein the (1-6C)alkoxy, mono(1-6C)alkylamino and -N(R^{d})C(O)(1-6C)alkyl groups are optionally substituted by hydroxy;
wherein an aryl ring, a saturated or partially saturated 3 to 7 membered heterocyclic ring or a 5 or 6 membered heteroaryl ring is optionally substituted by (1-4C)alkyl, (1-4C)alkoxy or -C(O)CH₂Y wherein Y is selected from hydroxy or di(1-6C)alkylamino.

8. A compound accordingly to any one of the preceding claims wherein R³ and R⁴ are both hydrogen.

9. A compound accordingly to any one of the preceding claims wherein R⁶ is independently selected from halo, cyano, oxo, (3-7C)cycloalkyl, a saturated 3 to 7 membered heterocyclic ring (optionally substituted by (1-4C)alkyl), -N(R^{c})C(O)(1-bC)alkyl wherein R^{c} is hydrogen or (1-6C)alkyl (particularily (1-4C)alkyl), (1-6C)alkyl (optionally substituted by up to three groups independently selected from halo) or (1-6C)alkoxy and m is selected from 1 or 2.

10. A compound according to Claim 1 selected from:
*N-*{3-[(2-aminopyrimidin-5-yl)ethynyl]phenyl}-*N'*-[2-fluoro-5-(trifluoromethyl)phenyl]urea
N-{3-[(2-aminopyrimidin-5-yl)ethynyl]phenyl}-N'-[2-(trifluoromethyl)phenyl]urea
N-{3-[(2-aminopyrimidin-5-yl)ethynyl]phenyl}-N'-[4-(trifluoromethyl)phenyl]urea
N-{3-[(2-aminopyrimidin-5-yl)ethynyl]phenyl}-N'-(2-fluorophenyl)urea
N-{3-[(2-aminopyrimidin-5-yl)ethynyl]phenyl}-N'-(3-fluorophenyl)urea
N-{3-[(2-aminopyrimidin-5-yl)ethynyl]phenyl}-N'-(4-fluorophenyl)urea
N-{3-[(2-aminopyrimidin-5-yl)ethynyl]phenyl}-N'-(3-methoxyphenyl)urea
N-{3-[(2-aminopyrimidin-5-yl)ethynyl]phenyl}-N'-(2,5-difluorophenyl)urea
N-{3-[(2-aminopyrimidin-5-yl)ethynyl]phenyl }-N'-1,3-benzodioxol-5-ylurea
N-{3-[(2-aminopyrimidin-5-yl)ethynyl]phenyl}-N'-[3-(trifluoromethyl)phenyl]urea
N-{3-[(2-aminopyrimidin-5-yl)ethynyl]phenyl}-N'-(2-methoxyphenyl)urea
N-{3-[(2-aminopyrimidin-5-yl)ethynyl]phenyl}-N'-(4-methoxyphenyl)urea
N-{3-[(2-aminopyrimidin-5-yl)ethynyl]phenyl}-N'-(3,4-difluorophenyl)urea
N-{3-[(2-aminopyrimidin-5-yl)ethynyl]phenyl}-N'-(3-cyanophenyl)urea
N-{3-[(2-aminopyrimidin-5-yl)ethynyl]phenyl}-N'-(3-chlorophenyl)urea
N-{3-[(2-aminopyrimidin-5-yl)ethynyl]phenyl}-N'-cyclopentylurea
N-{3-[(2-aminopyrimidin-5-yl)ethynyl]phenyl}-N'-(3,5-difluorophenyl)urea
N-{3-[(2-anainopyrimidin-5-yl)ethynyl]phenyl}-N'-(5-tert-butyl-1,3,4-thiadiazol-2-yl)urea
N-{3-[(2-aminopyrimidin-5-yl)ethynyl]phenyl}-N'-(3-methylisoxazol-5-yl)urea
N-(3-{[({3-[(2-aminopyrimidin-5-yl)ethynyl]phenyl}amino)carbonyl]amino}phenyl) a cetamide
N-{3-[(2-aminopyrimidin-5-yl)ethynyl]phenyl}-N'-[4-(trifluoromethyl)pyridin-2-yl]urea
N-{3-[(2-aminopyrimidin-5-yl)ethynyl]phenyl}-N'-(5-tert-butylisoxazol-3-yl)urea
Phenyl {3-[(2-aminopyrimidin-5-yl)ethynyl]phenyl}carbamate
N-{3-[(2-aminopyrimidin-5-yl)ethynyl]phenyl}-N'-(2-oxopiperidin-3-yl)urea
N-(5-tert-butylisoxazol-3-yl)-N'-(3-{[2-(methylamino)pyrimidin-5-yl]ethynyl}phenyl)urea
N-(5-tert-butylisoxazol-3-yl)-N'-(3-{[2-(dimethylamino)pyrimidin-5-yl]ethynyl}phenyl)urea
N-(5-tert-butylisoxazol-3-yl)-N'-[3-({2-[(2-morpholin-4-ylethyl)amino]pyrimidin-5-yl}ethynyl)phenyl]urea
N-(5-tert-butylisoxazol-3-yl)-N'-[3-({2-[(3-morpholin-4-ylpropyl)amino]pyrimidin-5-yl}ethynyl)phenyl]urea
N-(5-tert-butylisoxazol-3-yl)-N'-[3-({2-[(2-methoxyethyl)amino]pyrimidin-5-yl}ethynyl)phenyl]urea
N-(5-tert-butylisoxazol-3-yl)-N'-{3-[(2-{[3-(1H-imidazol-1-yl)propyl]amino}pyrimidin-5-yl)ethynyl]phenyl}urea
N-(5-tert-butylisoxazol-3-yl)-N'-[3-({2-[(3-methoxypropyl)amino]pyrimidin-5-yl}ethynyl)phenyl]urea
N-(5-tert-butylisoxazol-3-yl)-N'-[3-({2-[(2-hydroxyethyl)amino]pyrimidin-5-yl}ethynyl)phenyl]urea
N-(5-tert-butylisoxazol-3-yl)-N'-[3-({2-[(2-pyrrolidin-1-ylethyl)amino]pyrimidin-5-yl}ethynyl)phenyl]urea
N-(5-tert-butylisoxazol-3-yl)-N'-[3-({2-[(3-pyrrolidin-1-ylpropyl)amino]pyrimidin-5-yl}ethynyl)phenyl]urea
N-[3-({2-[(2-aminoethyl)amino]pyrimidin-5-yl}ethynyl)phenyl]-N'-(5-tert-butylisoxazol-3-yl)urea
N-[3-({2-[(3-aminopropyl)amino]pyrimidin-5-yl}ethynyl)phenyl]-N'-(5-tert-butylisoxazol-3-yl)urea
N-(5-tert-butylisoxazol-3-yl)-N'-{3-[(2-{[2-(dimethylamino)ethyl]amino}pyrimidin-5-yl)ethynyl]phenyl}urea
N-(5-tert-butylisoxazol-3-yl)-N'-{3-[(2-{[3-(dimethylamino)propyl]amino}pyrimidin-5-yl)ethynyl]phenyl}urea
N-2-(5-{[3-({[(5-tert-butylisoxazol-3-yl)amino]carbonyl}amino)phenyl]ethynyl}pyrimidin-2-yl)glycinamide
N-3-(5-{[3-({[(5-tert-butylisoxazol-3-yl)amino]carbonyl}amino)phenyl]ethynyl}pyrimidin-2-yl)-beta-alaninamide
N-(5-tert-butylisoxazol-3-yl)-N'-{3-[(2-{[2-(1H-imidazol-4-yl)ethyl]amino}pyrimidin-5-yl)ethynyl]phenyl}urea
N-(5-tert-butylisoxazol-3-yl)-N'-[3-({2-[(2-pyridin-2-ylethyl)amino]pyrimidin-5-yl}ethynyl)phenyl]urea
N-(5-tert-butylisoxazol-3-yl)-N'-{3-[(2-{[3-(isopropylamino)propyl]amino}pyrimidin-5-yl)ethynyl]phenyl}urea
N-(5-tert-butylisoxazol-3-yl)-N'-{3-[(2-{[3-(4-methylpiperazin-1-yl)propyl]amino}pyrimidin-5-yl)ethynyl]phenyl}urea
N-(5-tert-butylisoxazol-3-yl)-N'-[3-({2-[(2-pyridin-4-ylethyl)amino]pyrimidin-5-yl}ethynyl)phenyl]urea
N-(5-tert-butylisoxazol-3-yl)-N'-[3-({2-[(3-pipendin-1-ylpropyl)amino]pyrimidin-5-yl}ethynyl)phenyl]urea
N-(5-methylisoxazol-3-yl)-N'-[3-({2-[(2-pyrrolidin-1-ylethyl)amino]pyrimidin-5-yl}ethynyl)phenyl]urea
N-(5-tert-butyl-1,3,4-thiadiazol-2-yl)-N'-[3-({2-[(2-pyrrolidin-1-ylethyl)amino]pyrimidin-5-yl}ethynyl)phenyl]urea
N-(3-methylisothiazol-5-yl)-N'-[3-({2-[(2-pyrrolidin-1-ylethyl)amino]pyrimidin-5-yl}ethynyl)phenyl]urea
N-(3-fluorophenyl)-N'-[3-({2-[(2-pyrrolidin-1-ylethyl)amino]pyrimidin-5-yl}ethynyl)phenyl]urea
N-(4-methoxyphenyl)-N'-[3-({2-[(2-pyrrolidin-1-ylethyl)amino]pyrimidin-5-yl}ethynyl)phenyl]urea
N-(2-fluorophenyl)-N'-[3-({2-[(2-pyrrolidin-1-ylethyl)amino]pyrimidin-5-yl}ethynyl)phenyl]urea
N-(2,5-difluorophenyl)-N'-[3-({2-[(2-pyrrolidin-1-ylethyl)amino]pyrimidin-5-yl}ethynyl)phenyl]urea
N-(3,4-difluorophenyl)-N'-[3-({2-[(2-pyrrolidin-1-ylethyl)amino]pyrimidin-5-yl}ethynyl)phenyl]urea
N-[2-fluoro-5-(trifluoromethyl)phenyl]-N'-[3-({2-[(2-pyrrolidin-1-ylethyl) amino]pyrimidin-5-yl}ethynyl)phenyl]urea
N-[3-({2-[(2-pyrrolidin-1-ylethyl)amino]pyrimidin-5-yl}ethynyl)phenyl]-N'-[4-(trifluoromethyl)phenyl]urea
N-1,3-benzodioxol-5-yl-N'-[3-({2-[(2-pyrrolidin-1-ylethyl)amino]pyrimidin-5-, yl}ethynyl)phenylJurea
N-(4-fluorophenyl)-N'-[3-({2=[(2-pyrrolidin-1-ylethyl)amino]pyrimidin-5-yl}ethynyl)phenyl]urea
N-(3-chlorophenyl)-N'-[3-({2-[(2-pyrrolidin-1-ylethyl)amino]pyrimidin-5-yl}ethynyl)phenyl]urea
N-(5-methylisoxazol-3-yl)-N-[3-({2-[(2-morpholin-4-ylethyl)amino]pyrimidin-5-yl}ethynyl)phenyl]urea
N-(5-tert-butyl-1,3,4-thiadiazol-2-yl)-N'-[3-({2-[(2-morpholin-4-ylethyl)amino]pyrimidin-5-yl}ethynyl)phenyl]urea
N-[2-fluoro-5-(trifluoromethyl)phenyl]-N'-[3-({2-[(2-morpholin-4-ylethyl)amino]pyrimidin-5-yl}ethynyl)phenyl]urea
N-(5-methylisoxazol-3-yl)-N'-[3-({2-[(3-morpholin-4-ylpropyl)amino]pyrimidin-5-yl}ethynyl)phenyl]urea
N-(5-tert-butyl-1,3,4-thiadiazol-2-yl)-N'-[3-({2-[(3-morpholin-4-ylpropyl)amino]pyrimidin-5-yl}ethynyl)phenyl]urea
N-[2-fluoro-5-(trifluoromethyl)phenyl]-N'-[3-({2-[(3-morpholin4-ylpropyl)amino]pyrimidin-5-yl}ethynyl)phenyl]urea
N-(5-methylisoxazol-3-yl)-N'-[4-({2-[(2-pyrrolidin-1-ylethyl)amino]pyrimidin-5-yl}ethynyl)phenyl]urea
N-(5-tert-butylisoxazol-3-yl)-N'-[4-({2-[(2-pyrrolidin-1-ylethyl)amino]pyrimidin-5-yl}ethynyl)phenyl]urea
N-(5-tert-butyl-1,3,4-thiadiazol-2-yl)-N'-[4-({2-[(2-pyrrolidin-1-ylethyl)amino]pyrimidin-5-yl}ethynyl)phenyl]urea
N-[2-fluoro-5-(trifluoromethyl)phenyl]-N'-[4-({2-[(2-pyrrolidin-1-ylethyl)amino]pyrimidin-5-yl} ethynyl)phenyl]urea
N-(5-{[3-({[(5-tert-butylisoxazol-3-yl)amino]carbonyl}amino)phenyl]ethynyl}pyrimidin-2-yl)-2-(2-methoxyethoxy)acetamide
N-{6-[(2-aminopyrimidin-5-yl)ethynyl]pyridin-2-yl}-N'-(5-tert-butylisoxazol-3-yl)urea
N-{2-[(2-aminopyrimidin-5-yl)ethynyl]pyridin-4-yl}-N'-(5-tert-butylisoxazol-3-yl)urea
N-{5-[(2-aminopyrimidin-5-yl)ethynyl]-1,3-thiazol-2-yl}-N'-[2-fluoro-5-(trifluoromethyl)phenyl]urea
N-{5-[(2-aminopyrimidin-5-yl)ethynyl]-1,3,4-thiadiazol-2-yl}-N'-[2-fluoro-5-(trifluoromethyl)phenyl]urea
*N*-{5-[(2-aminopyrimidin-5-yl)ethynyl]-1,3-thiazol-2-yl}-*N*'-(5-*tert*-butylisoxazol-3-yl)urea
N-{3-[(2-aminopyrimidin-5-yl)ethynyl]phenyl}-2-(2-methoxyphenyl)acetamide
N-{3-[(2-aminopyrimidin-5-yl)ethynyl]pheny}-2-phenylacetamide
N-{3-[(2-aminopyrimidin-5-yl)ethynyl]phenyl}-2-(3-methoxyphenyl)acetamide
N-{3-[(2-aminopyrimidin-5-yl)ethynyl]phenyl}-2-[3-(trifluoromethyl)phenyl]acetamide
N-{3-[(2-aminopyrimidin-5-yl)ethynyl]phenyl}-2-[4-(trifluoromethyl)phenyl]acetamide
N-{3-[(2-aminopyrimidin-5-yl)ethynyl]phenyl}-2-(3-methylisoxazol-5-yl)acetamide
N-{4-[(2-aminopyrimidin-5-yl)ethynyl]phenyl}-2-(2-methoxyphenyl)acetamide
N-{4-[(2-aminopyrimidin-5-yl)ethynyl]phenyl}-2-(3-methytisoxazol-5-yl)acetamide
N-(3-[(2-aminopyrimidin-5-yl)ethynyl]phenyl)-N'-(2,2-dimethyttetrahydro-2H-pyran-4-yl)urea
N-{6-[(2-aminopyrimidin-5-yl)ethynyl]pyrimidin-4-yl}-N'-(5-tert-butylisoxazol-3 - yl)urea
N'-{3-[(2-aminopyrimidin-5-yl)ethynyl]phenyl}-N-(5-tert-butylisoxazol-3-yl)-N-methylurea
N-{3-[(2-aminopyrimidin-5-yl)ethynyl]phenyl}-N'-(1-tert-butyl-3-cyclopropyl-1H-pyrazol-5-yl)urea
N-{3-[(2-aminopyrimidin-5-yl)ethynyl]phenyl}-N'-(5-methylisoxazol-3-yl)urea
N-{5-[(2-aminopyrimidin-5-yl)ethynyl]pyridin-3-yl}-N'-(5-tert-butylisoxazol-3-yl)urea
N-[3-({2-[(3-piperidin-1-ylpropyl)amino]pyrimidin-5-yl}ethynyl)phenyl]-N'-[4-(tnfluoromethyl)pyridin-2-yl]urea
N-(5-tert-butyl-1,3,4-thiadiazol-2-yi)-N'-[3-({2-[(3-piperidin-1-ylpropyl)amino]pyrimidin-5-yl}ethynyl)phenyl]urea
N-(3-methylisoxazol-5-yl)-N'-[3-({2-[(3-piperidin-1-ylpropyl)amino]pyrimidin-5-yl}ethynyl)phenyl]urea
N-(2-methoxyphenyl)-N'-[3-({2-[(3-piperidin-1-ylpropyl)amino]pyrimidin-5-yl}ethynyl)phenyl]urea
N-(3-fluorophenyl)-N'-[3-({2-[(3-piperidin-1-ylpropyl)amino]pyrimidin-5-yl}ethynyl)phenyl]urea
N'-{4-[(2-aminopyrimidin-5-yl)ethynyl]phenyl}-N-(5-tert-butylisoxazol-3-yl)-N-methylurea
N-{3-[(2-aminopyrimidin-5-yl)ethynyl]phenyl}-N'-(3-cyclopropyl-1-methyl-1H-pyrazol-5-yl)urea
N-{3-[(2-aminopyrimidin-5-yl)ethynyl]phenyl}-N'-phenylurea
N-[3-({2-[(4-aminobutyl)amino]pyhmidin-5-yl}ethynyl)phenyl]-N'-(5-tert-butylisoxazol-3-yl)urea
N-(5-tert-butylisoxazol-3-yl)-N'-[3-({2-[(2-piperidin-1-ylethyl)amino]pyrimidin-5-yl}ethynyl)phenyl]urea
N-(5-tert-butylisoxazol-3-yl)-N'-{3-[(2-{[2-(isopropylamino)ethyl]amino} pyrimidin-5-yl)ethynyl]phenyl}urea
N-(5-tert-butylisoxazol-3-yl)-N'-{3-[(2-{[2-(2-hydroxyethoxy)ethyl]amino} pyrimidin-5-yl)ethynyl]phenyl}urea
N-{3-[(2-aminopyrimidin-5-yl)ethynyl]phenyl}-N'-(5-cyclopropyl-1,3,4-thiadiazol-2-yl)urea
N-{3-[(2-aminopyrimidin-5-yl)ethynyl]phenyl}-N'-[5-(trifluoromethyl)-1,3,4-thiadiazol-2-yl]urea
N-{2-[(5-{[3-({[(5-tert-butylisoxazol-3-yl)amino]carbonyl}amino)phenyl]ethynyl}pyrimidin-2-yl)amino]ethyl}-2-hydroxyacetamide
N-(5-tert-butylisoxazol-3-yl)-N'-{3-[(2-{[4-(dimethylamino)butyl]amino}pyrimidin-5-yl)ethynyl]phenyl}urea
N'-{3-[(2-aminopyrimidin-5-yl)ethynyl]phenyl}-N-methyl-N-[5-(trifluoromethyl)-1,3,4-thiadiazol-2-yl]urea
N-phenyl-N'-[3-({2-[(3-piperidin-1-ylpropyl)amino]pyrimidin-5-yl}ethynyl)phenyl]urea
N-(5-methylisoxazol-3-yl)-N'-[3-({2-[(3-piperidin-1-ylpropyl)amino]pyrimidin-5-yl}ethynyl)phenyl]urea
N-{3-[(2-aminopyrimidin-5-yl)ethynyl]phenyl}-N'-(5-tert-butylisoxazol-3-yl)-N-methylurea
N-(5-tert-butylisoxazol-3-yl)-N'-{3-[(2-{[2-(dimethylamino)-1-methylethyl]amino}pyrimidin-5-yl)ethynyl]phenyl}urea
phenyl{3-[(2-{[3-(dimethylamino)propyl]amino}pyrimidin-5-yl)ethynyl]phenyl}carbamate
N-{3-[(2-aminopyrimidin-5-yl)ethynyl]phenyl}-N'-(3-tert-butyl-1-methyl-1H-pyrazol-5-yl)urea
N-{3-[(2-aminopyrimidin-5-yl)ethynyl]phenyl}-N'-(4-tert-butyl-1,3-thiazol-2-yl)urea
N-{3-[(2-aminopyrimidin-5-yl)ethynyl]phenyl}-N'-(5-methyl-1,3,4-thiadiazol-2-yl)urea
N-{3-[(2-aminopyrimidin-5-yl)ethynyl]phenyl}-N'-(5-ethyl-1,3,4-thiadiazol-2-yl)urea
N-{3-[(2-aminopyrimidin-5-yl)ethynyl]phenyl}-N'-(5-isopropyl-1,3,4-thiadiazol-2-yl)urea
N-{3-[(2-{[3-(dimethylamino)propyl]amino}pyrimidin-5-yl)ethynyl]phenyl}-N'-(5-methylisoxazol-3-yl)urea
N-(3-[(2-{[3-(dimethylamino)propyl]amino}pyrimidin-5-yl)ethynyl]phenyl)-N'-phenylurea
N-{5-[(2-aminopyrimidin-5-yl)ethynyl]pyridin-3-yl}-N'-(5-tert-butylisoxazol-3-yl)urea;
N-(3-tert-butyl-1-methyl-1H-pyrazol-5-yl)-N'-[3-({2-[(3-morpholin-4-ylpropyl)amino]pyrimidin-5-yl}ethynyl)phenyl]urea;
N-(3-tert-butyl-1-methyl-1H-pyrazol-5-yl)-N'-[3-({2-[(2-morpholin-4-ylethyl)amino]pyrimidin-5-yl}ethynyl)phenyl]urea;
N-(3-tert-butyl-1-methyl-1H-pyrazol-5-yl)-N'-[3-({2-[(3-piperidin-1-ylpropyl)amino]pyrimidin-5-yl}ethynyl)phenyl]urea;
N-(5-tert-butylisoxazol-3-yl)-N'-[3-({2-[(1-methyl-2-morpholin-4-ylethyl)amino]pyrimidin-5-yl}ethynyl)phenyl]urea;
N-(5-tert-butylisoxazol-3-yl)-N'-{3-[(2-{[(1-glycoloylpyrrolidin-2-yl)methyl]amino}pyrimidin-5-yl)ethynyl]phenyl}urea;
N-(5-tert-butylisoxazol-3-yl)-N'-(3-{[2-({[1-(N,N-dimethylglycyl)pyrrolidin-2-yl]methyl}amino)pyrimidin-5-yl]ethynyl}phenyl)urea;
N-(5-tert-butylisoxazol-3-yl)-N'-[3-({2-[(2-piperazin-1-ylethyl)amino]pyrimidin-5-yl}ethynyl)phenyl]urea;
N-(5-tert-butylisoxazol-3-yl)-N'-[3-({2-[(3-piperazin-1-ylpropyl)amino]pyrimidin-5-yl}ethynyl)phenyl]urea;
N-(5-tert-butylisoxazol-3-yl)-N-methyl-N'-[3-({2-[(2-morpholin-4-ylethyl)amino]pyrimidin-5-yl}ethynyl)phenyl]urea;
N-(5-tert-butylisoxazol-3-yl)-N-methyl-N'-[3-({2-[(3-morpholin-4-ylpropyl)amino]pyrimidin-5-yl}ethynyl)phenyl]urea;
N-(5-tert-butylisoxazol-3-yl)-N-methyl-N'-[3-({2-[(3-piperidin-1-ylpropyl)amino]pyrimidin-5-yl}ethynyl)phenyl]urea;
N-(3-tert-butyl-1-methyl-1H-pyrazol-5-yl)-N'-[3-({2-[(3-piperidin-1-ylpropyl)amino]pyrimidin-5-yl}ethynyl)phenyl]urea;
N-(3-tert-butyl-1-methyl-1H-pyrazol-5-yl)-N'-{3-[(2-{[2-(dimethylamino)ethyl]amino}pyrimidin-5-yl)ethynyl]phenyl}urea;
N-(3-tert-butyl-l-methyl-1H-pyrazol-5-yl)-N'-{3-[(2-{[2-(isopropylamino)ethyl]amino}pyrimidin-5-yl)ethynyl]phenyl}urea;
N-(3-cyclopropyl-1-methyl-1H-pyrazol-5-yl)-N'-[3-({2-[(3-piperidin-1-ylpropyl)amino]pyrimidin-5-yl}ethynyl)phenyl]urea;
N-(3-cyclopropyl-1-methyl-1H-pyrazol-5-yl)-N'-[3-({2-[(3-morpholin-4-ylpropyl)amino]pyrimidin-5-yl}ethynyl)phenyl]urea;
N-(3-cyclopropyl-1-methyl-1H-pyrazol-5-yl)-N'-[3-({2-[(2-morpholin-4-ylethyl)amino]pyrimidin-5-yl}ethynyl)phenyl]urea;
N-(5-tert-butylisoxazol-3-yl)-N'-(3-{[2-({2-[(2-hydroxy-1-oxo-ethyl)amino]ethyl}amino)-pyrimidin-5-yl]ethynyl}phenyl)urea;
N-(5-tert-butylisoxazol-3-yl)-N'-(3-{[2-({3-[(2-hydroxyethyl)amino]propyl}amino)pyrimidin-5-yl]ethynyl}phenyl)urea;
N-(5-tert-butylisoxazol-3-yl)-N'-{3-[(2-{[3-(dimethylamino)propyl]amino} pyrimidin-5-yl)ethynyl]phenyl}-N-methylurea;
N-{5-[(2-aminopyrimidin-5-yl)ethynyl]pyridin-3-yl}-N'-(3-tert-butyl-1-methyl-1H-pyrazol-5-yl)urea;
N-{5-[(2-aminopyrimidin-5-yl)ethynyl]pyridin-3-yl}-N'-(3-cyclopropyl-1-methyl-1H-pyrazol-5-yl)urea;
N-{5-[(2-aminopyrimidin-5-yl)ethynyl]-1,3-thiazol-2-yl}-N'-phenylurea;
N-{5-[(2-aminopyrimidin-5-yl)ethynyl]-1,3-thiazol-2-yl}-N'-(2,2-dimethyltetrahydro-2H-pyran-4-yl)urea;
N-{5-[(2-aminopyrimidin-5-yl)ethynyl]-1,3-thiazol-2-yl}-N'-(3-cyclopropyl-1-methyl-1H-pyrazol-5-yl)urea;
N-{5-[(2-aminopyrimidin-5-yl)ethynyl]-1,3-thiazol-2-yl}-N'-(3-tert-butyl-1-methyl-1H-pyrazol-5-yl)urea;
N-{5-[(2-aminopyrimidin-5-yl)ethynyl]-1,3,4-thiadiazol-2-yl}-N'-phenylurea; N-{5-[(2-aminopyrimidin-5-yl)ethynyl]-1,3,4-thiadiazol-2-yl}-N'-(2,2-dimethyltetrahydro-2H-pyran-4-yl)urea;
N-{3-[(2-aminopyrimidin-5-yl)ethynyl]phenyl}-N'-(1,3-dimethyl-1H-pyrazol-5-yl)urea;
N-{3-[(2-aminopyrimidin-5-yl)ethynyl]phenyl}N'-[5-(ethylthio)-1,3,4-thiadiazol-2-yl]urea;
N-{3-[(2-aminopyrimidin-5-yl)ethynyl]phenyl}-N'-(1-tert-butyl-1H-pyrazol-4-yl)urea;
N-{3-[(2-aminopyrimidin-5-yl)ethynyl]phenyl}-N'-(3-isopropyl-1-methyl-1H-pyrazol-5-yl)urea;
N-{3-[(2-aminopyrimidin-5-yl)ethynyl]phenyl}-N'-(5-isopropyl-1,3,4-oxadiazol-2-yl)urea;
N-{3-[(2-aminopyrimidin-5-yl)ethynyl]phenyl}-N'-(1-ethyl-1H-pyrazol-3-yl)urea;
N-{3-[(2-aminopyrimidin-5-yl)ethynyl]phenyl}-N'-(1-isopropyl-1H-pyrazol-3-yl)urea;
N-{3-[(2-aminopyrimidin-5-yl)ethynyl]phenyl}-N'-[3-fluoro-5-(4-methylpiperazin-1-yl)phenyl]urea or
*N*-{3-[(2-Aminopyrimidin-5-yl)ethynyl]-4-methylphenyl}-*N*'(3-*tert*-butyl-1-methyl-1*H*-pyrazol-5-yl)urea.

11. A pharmaceutical composition which comprises a compound of the Formula I, or a pharmaceutically acceptable salt thereof, as defined in claims 1 to 10 in association with a pharmaceutically acceptable diluent or carrier.

12. A compound of the Formula I, or a pharmaceutically acceptable salt thereof, as defined in claims 1 to 10, for use as a medicament.

13. Use of a compound of the Formula I, or a pharmaceutically acceptable salt thereof, as defined in claims 1 to 10, in the manufacture of a medicament for use as a Tie2 receptor tyrosine kinase inhibitor in a warm-blooded animal such as man.

14. Use of a compound of the Formula I, or a pharmaceutically acceptable salt thereof, as defined in claims 1 to 10, in the manufacture of a medicament for use in the production of an anti-angiogenic effect in a warm-blooded animal such as man.

15. A process for preparing a compound of formula I, or salt thereof, as defined in Claim 1, or a pharmaceutically acceptable salt thereof (wherein R¹, R², R³, R⁴, R⁵, R⁶, R⁷, R⁸, R⁹, R¹⁰, R¹¹ L , ring A and ring B, n and m are, unless otherwise specified, as defined in Claim 1) comprising:
(a) For compounds of the formula I wherein L is -N(R⁸)C(O)N(H)-, the reaction of a compound of the formula II: 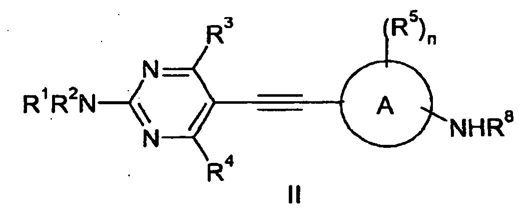 wherein R¹, R², R³, R⁴, R⁵, R⁸, n and A have any of the meanings defined hereinbefore except that any functional group is protected if necessary, with an isocyanate of the formula IV: 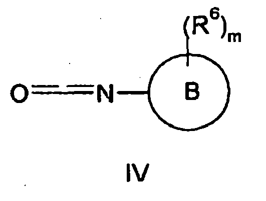 wherein R⁶, m and B have any of the meanings defined hereinbefore except that any functional group is protected if necessary; or
(b) For compounds of the formula I wherein L is -N(R⁸)C(O)N(H)-, the reaction of a compound of the formula II as defined above with an aryl carbamate of the formula III: 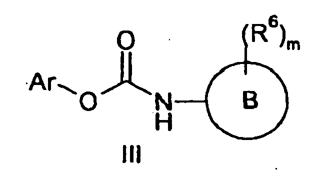 wherein Ar is a suitable aryl group and R⁶, m and B have any of the meanings defined hereinbefore except that any functional group is protected if necessary; or
(c) For compounds of the formula I wherein L is N(R⁸)C(O)-O-, the reaction of a compound of the formula II as defined above with a compound of the formula XI: 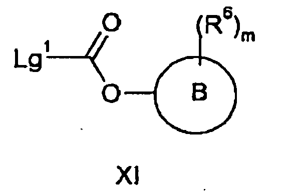 wherein Lg¹ is a suitable displaceable group and R⁶, m and B have any of the meanings defined hereinbefore except that any functional group is protected if necessary; or
(d) For compounds of the formula I wherein L is N(R⁸)C(O)C(R^{a}R^{b}), the reaction of a compound of the formula II as defined above with a compound of the formula IX: 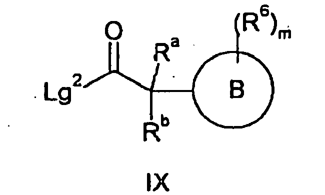 wherein Lg² is a suitable displaceable group, R^{x}-C(O)-O- or R^{x}-O-(wherein R^{x} is a suitable alkyl or aryl group) and R⁶, R^{a}, R^{b}, m and B have any of the meanings defined hereinbefore except that any functional group is protected if necessary; or
(e) For compounds of the formula I wherein L is -N(R⁸)C(O)N(H)-, the reaction of a compound of the formula II as defined above with a trichloroacetylamine of the formula XIII: 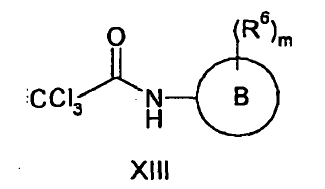 wherein R⁶, m and B have any of the meanings defined hereinbefore except that any functional group is protected if necessary; or
(f) For compounds of the formula I wherein L is -C(R^{a}R^{b})C(O)N(R⁹)-, the reaction of a compound of the formula XIV: 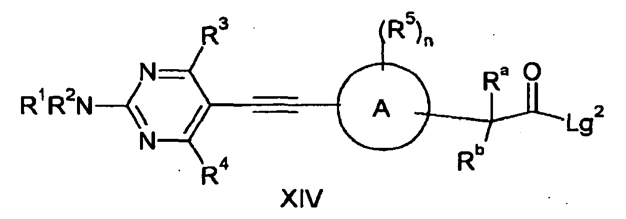 wherein Lg² is a suitable displaceable group as described above and R¹, R², R³, R⁴, R⁵, R^{a}, R^{b}, n and A have any of the meanings defined hereinbefore except that any functional group is protected if necessary, with an amine of the formula XV: 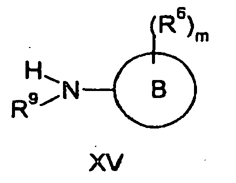 wherein R⁶, R⁹, m and B have any of the meanings defined hereinbefore except that any functional group is protected if necessary; or
(g) The reaction of a compound of the formula XVI: 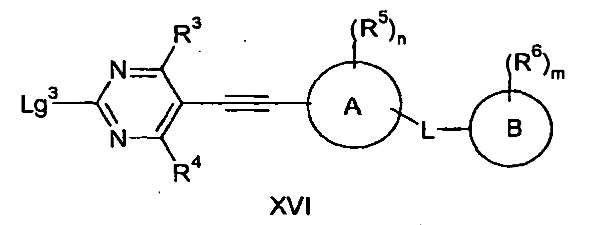 wherein Lg³ is a suitable displaceable group, methyl sulfonyl, methylthio or aryloxy and R³, R⁴, R⁵, R⁶, n, m. A, B and L have any of the meanings defined hereinbefore except that any functional group is protected if necessary, with an amine of the formula HNR¹R², wherein R¹ and R² have any of the meanings defined hereinbefore except that any functional group is protected if necessary; or
(h) The reaction of a compound of the formula XVII: 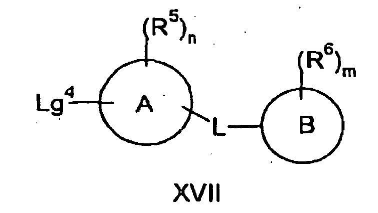 wherein Lg⁴ is a suitable displaceable group or a sulfonyloxy group and R⁵, R⁶, n, m, A, B and L have any of the meanings defined hereinbefore except that any functional group is protected if necessary, with an alkyne of the formula XVIII: 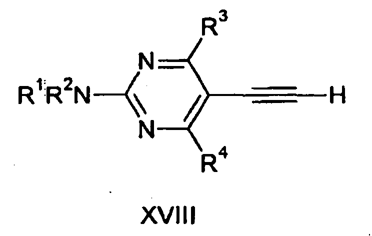 wherein R¹, R², R³ and R⁴ have any of the meanings defined hereinbefore except that any functional group is protected if necessary; or
(i) For compounds of the formula I wherein L is -N(H)C(O)N(R⁹)-, the reaction of an isocyanate of the formula XIX: 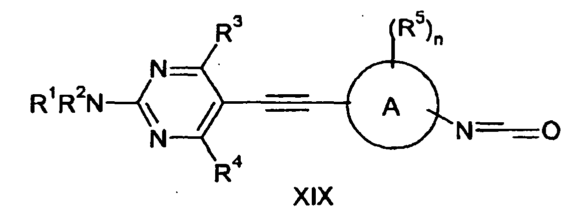 wherein R¹, R², R³, R⁴, R⁵, n and A have any of the meanings defined hereinbefore except that any functional group is protected if necessary, with an amine of the formula XV as defined above; or
(j) For compounds of the formula I wherein L is -N(H)C(O)N(R⁹)-, the reaction of a compound of the formula XX: 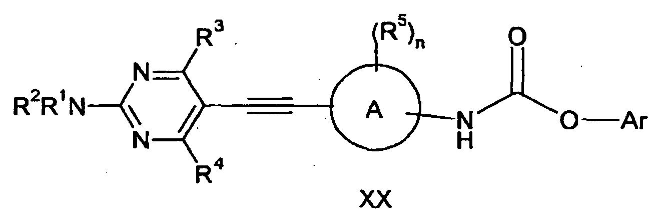 wherein Ar is a suitable aryl group and R¹, R², R³, R⁴, R⁵, n and A have any of the meanings defined hereinbefore except that any functional group is protected if necessary, with an amine of the formula XV as defined above.
and thereafter if necessary:
i) converting a compound of the Formula (I) into another compound of the Formula (I);
ii) removing any protecting groups;
iii) forming a salt.

16. A compound selected from Formulae II, XIV, XVI, XIX and XX as defined in Claim 15, wherein A is a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl, thiadiazolyl, tirzolyl, tetrazolyl, pyridyl, pyridazinyl, pyrimidinyl, pyrazinyl or 1,3,5-triazinyl
or a compound of Formula VIc: 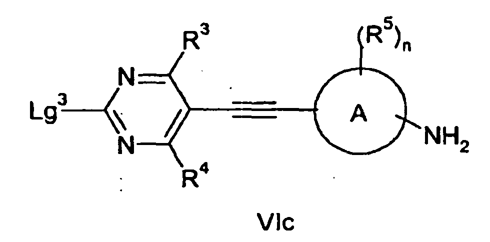 or salt thereof, wherein A is a 5 or 6 membered heteroaryl ring selected from furyl, pyrrolyl, thienyl, oxazolyl, isoxazolyl, imidazolyl, pyrazolyl, thiazolyl, isothiazolyl, oxadiazolyl; thiadiazolyl, triazolyl, tetrazolyl, pyridyl, pyridazinyl, pyrimidinyl, pyrazinyl or 1,3,5-triazinyl and R¹, R², R³, R⁴, R⁵, R⁶, R⁷, R⁸, R⁹, R¹⁰, R¹¹, L, m, ring B and n are as defined in claim 1, and Lg² is hydroxyl or halogeno and Lg³ is halogeno, methylsulphonyl, methylthio or aryloxy.

## Patentansprüche

1. Verbindung der Formel I 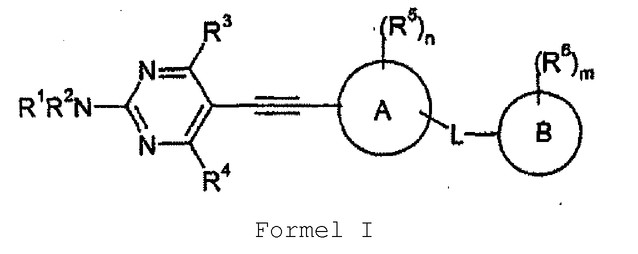 worin:
**R¹ und R²** unabhängig voneinander unter Wasserstoff, C₁₋₆-Alkylsulfonyl, Phenyl (CH₂)ᵤ, worin u für 0, 1, 2, 3, 4, 5 oder 6 steht, C₁₋₆-Alkanoyl, C₁₋₆-Alkyl, C₁₋₆-Alkoxycarbonyl, C₃₋₆-Cycloalkyl (CH₂)ₓ, worin x für 0, 1, 2, 3, 4, 5 oder 6 steht, oder einem 5-oder 6-gliedrigen Heteroarylring ausgewählt sind, oder **R¹ und R²** gemeinsam mit dem Stickstoffatom, an das sie gebunden sind, für einen gesättigten oder teilweise gesättigten 3- bis 7-gliedrigen heterocyclischen Ring, der gegebenenfalls ein unter N oder 0 ausgewähltes anderes Heteroatom enthält, stehen;
worin die C₁₋₆-Alkyl-, die C₁₋₆-Alkanoyl- und die C₃₋₆-Cycloalkylgruppen gegebenenfalls durch eine oder mehrere unabhängig voneinander unter Fluor, Hydroxy, C₁₋₆-Alkyl, C₁₋₆-Alkoxy, C₁₋₆-Alkoxy-C₁₋₆-alkoxy, C₁₋₆-Alkoxy-C₁₋₆-alkoxy-C₁₋₆-alkoxy, Amino, Mono-C₁₋₆-alkylamino, Di (C₁₋₆-alkyl)amino, Carbamoyl, Mono-C₁₋₆-alkylcarbamoyl, Di (C₁₋₆-alkyl) carbamoyl oder -N(R^{d})C(O)-C₁₋₆-Alkyl, worin R^{d} für Wasserstoff oder C₁₋₆-Alkyl steht, oder einem gesättigten oder teilweise gesättigten 3- bis 7-gliedrigen heterocyclischen Ring oder einem 5- oder 6-gliedrigen Heteroarylring ausgewählte Gruppen substituiert sind,
worin die C₁₋₆-Alkoxy-, C₁₋₆-Alkoxy-C₁₋₆-alkoxy-und C₁₋₆-Alkoxy-C₁₋₆-alkoxy-C₁₋₆-alkoxygruppen und die C₁₋₆-Alkylgruppen der Mono-C₁₋₆-alkylamino-, Di (C₁-₆-alkyl) amino-, Mono-C₁₋₆-alkyl-carbamoyl-, Di (C₁₋₆-alkyl) carbamoyl- und/oder -N(R^{d})C(O)-C₁₋₆-Alkylgruppen gegebenenfalls durch eine oder mehrere Hydroxygruppen substituiert sind;
worin das Phenyl gegebenenfalls durch eine oder mehrere unabhängig voneinander unter Halogen, C₁₋₆-Alkyl, C₁₋₆-Alkoxy, Amino, Mono-C₁₋₆-alkylamino oder Di(C₁₋₆-alkyl)amino ausgewählte Gruppen substituiert ist, worin die C₁₋₆-Alkyl-und C₁₋₆-Alkoxygruppen gegebenenfalls durch eine oder mehrere unabhängig voneinander unter Hydroxy, Amino, Mono-C₁₋₆-alkylamino oder Di-(C₁₋₆-alkyl)amino ausgewählte Gruppen substituiert sind;
und worin alle heterocyclischen Ringe und Heteroarylringe in R¹ und/oder R² gegebenenfalls unabhängig voneinander durch eine oder mehrere der folgenden Gruppen substituiert sind: C₁₋₄-Alkyl, C₁₋₄-Alkoxy, C₁₋₄-Alkoxy-C₁₋₄-alkyl, Hydroxy, Amino, Mono-C₁₋₆-alkylamino oder Di-(C₁₋₆-alkyl)amino oder einen gesättigten oder teilweise gesättigten 3- bis 7-gliedrigen heterocyclischen Ring oder -C(O)(CH₂)_{z}Y, worin z für 0, 1, 2 oder 3 steht und Y unter Wasserstoff, Hydroxy, C₁₋₄-Alkoxy, Amino, Mono-C₁₋₆-alkylamino, Di (C₁₋₆-alkyl)amino oder einem gesättigten oder teilweise gesättigten 3- bis 7-gliedrigen heterocyclischen Ring ausgewählt ist;
und mit der Maßgabe, daß dann, wenn R¹ und/oder R² für eine C₁-Alkanoylgruppe steht, das C₁-Alkanoyl nicht durch Fluor oder Hydroxy substituiert ist;
**R³ und R⁴** unabhängig voneinander unter Wasserstoff, C₁₋₆-Alkyl oder C₁₋₆-Alkoxy ausgewählt sind, worin die C₁₋₆-Alkyl- und die C₁₋₆-Alkoxygruppen gegebenenfalls durch eine oder mehrere unter Fluor, Hydroxy, C₁₋₆-Alkyl, C₁₋₆-Alkoxy, Amino, Mono-C₁₋₆-alkylamino oder Di (C₁₋₆-alkyl) amino, Carbamoyl, Mono-C₁₋₆-alkylcarbamoyl oder Di (C₁₋₆-alkyl)carbamoyl, einem gesättigten oder teilweise gesättigten 3- bis 7-gliedrigen heterocyclischen Ring oder einem 5- oder 6-gliedrigen Heteroarylring ausgewählte Gruppen substituiert sind, worin die heterocyclischen Ringe und Heteroarylringe gegebenenfalls unabhängig voneinander durch eine oder mehrere der folgenden Gruppen substituiert sind: C₁₋₄-Alkyl, C₁₋₄-Alkoxy, Hydroxy, Amino, Mono-C₁₋₆-alkylamino oder Di (C₁₋₆-alkyl)amino oder einen gesättigten oder teilweise gesättigten 3- bis 7-gliedrigen heterocyclischen Ring;
oder eine der Gruppen **R³ und R**⁴ für eine Gruppe **-NR¹R²** gemäß obiger Definition steht;
**A** für eine Arylgruppe oder einen unter Furyl, Pyrrolyl, Thienyl, Oxazolyl, Isoxazolyl, Imidazolyl, Pyrazolyl, Thiazolyl, Isothiazolyl, Oxadiazolyl, Thiadiazolyl, Triazolyl, Tetrazolyl, Pyridyl, Pyridazinyl, Pyrimidinyl, Pyrazinyl oder 1,3,5-Triazinyl ausgewählten 5- oder 6-gliedrigen Heteroarylring steht;
**R⁵** unter Cyclopropyl, Cyano, Halogen, C₁₋₆-Alkoxy oder C₁₋₆-Alkyl ausgewählt ist, worin die C₁₋₆-Alkyl- und die C₁₋₆-Alkoxygruppen gegebenenfalls durch Cyano oder ein oder mehrere Fluor substituiert sind;
**n** für 0, 1, 2 oder 3 steht;
**L** in meta- oder para-Stellung bezüglich des Anbindungspunkts der Ethinylgruppe an den Ring A gebunden ist und für -C(R^{a}R^{b})C(O)N(R⁹)-, -N(R⁸)C(O)CR^{a}R^{b})-, -N(R⁸)C(O)N(R⁹)-, -N(R⁸)C(O)O-oder -OC(O)-N(R⁹)- steht, worin R⁸ und R⁹ unabhängig voneinander für Wasserstoff oder C₁₋₆-Alkyl stehen und worin R^{a} und R^{b} unabhängig voneinander für Wasserstoff oder C₁₋₆-Alkyl stehen oder R^{a} und R^{b} gemeinsam mit dem Kohlenstoffatom, an das sie gebunden sind, für C₃₋₆-Cycloalkyl stehen;
**B** für einen C₃₋₇-Cycloalkylring, einen gesättigten oder teilweise gesättigten 3- bis 7-gliedrigen heterocyclischen Ring, eine Arylgruppe, einen unter Furyl, Pyrrolyl, Thienyl, Oxazolyl, Isoxazolyl, Imidazolyl, Pyrazolyl, Thiazolyl, Isothiazolyl, Oxadiazolyl, Thiadiazolyl, Triazolyl, Tetrazolyl, Pyridyl, Pyridazinyl, Pyrimidinyl, Pyrazinyl oder 1,3,5-Triazinyl ausgewählten 5- oder 6-gliedrigen Heteroarylring oder eine 8-, 9- oder 10-gliedrige bicyclische Gruppe, die gegebenenfalls 1, 2, 3 oder 4 unabhängig voneinander unter N, 0 und S ausgewählte Heteroatome enthält und gesättigt, teilweise gesättigt oder aromatisch ist, steht;
**R⁶** unter Halogen, Cyano, Oxo, einem C₃₋₇-Cycloalkyl-ring, einem gesättigten oder teilweise gesättigten 3- bis 7-gliedrigen heterocyclischen Ring und -N(R^{c})C(O)-C₁₋₆-Alkyl, worin R^{c} für Wasserstoff oder C₁₋₆-Alkyl steht, ausgewählt ist; oder
**R⁶** unter C₁₋₆-Alkyl, -S(O)ₚ-C₁₋₆-Alkyl, worin p für 0, 1 oder 2 steht, oder C₁₋₆-Alkoxy ausgewählt ist, worin die C₁₋₆-Alkyl-, -S(O)ₚ-C₁₋₆-Alkyl- und die C₁₋₆-Alkoxygruppen gegebenenfalls durch eine oder mehrere unabhängig voneinander unter Cyano, Fluor, Hydroxy, C₁₋₆-Alkoxy, Amino, Mono-C₁₋₆-alkylamino, Di (C₁₋₆-alkyl) amino, einem C₃₋₇-Cycloalkyl-ring oder einem gesättigten oder teilweise gesättigten 3- bis 7-gliedrigen heterocyclischen Ring ausgewählte Gruppen substituiert sind; und
worin der C₃₋₇-Cycloalkylring und der gesättigte oder teilweise gesättigte 3- bis 7-gliedrige heterocyclische Ring gegebenenfalls unabhängig voneinander durch eine oder mehrere unter C₁₋₆-Alkyl ausgewählte Gruppen substituiert sind; und
**m** für 0, 1, 2 oder 3 steht;
und dann, wenn B für einen C₃₋₇-Cycloalkylring, einen gesättigten oder teilweise gesättigten 3-bis 7-gliedrigen heterocyclischen Ring oder eine gesättigte oder teilweise gesättigte 8-, 9- oder 10-gliedrige bicyclische Gruppe steht, die Ringe und die bicyclische Gruppe gegebenenfalls 1 oder 2 Oxo- oder Thioxosubstituenten tragen;
und Salze davon.

2. Verbindung der Formel I nach Anspruch 1, worin:
**R⁶** unter Halogen, Cyano, einem C₃₋₇-Cycloalkylring, einem gesättigten oder teilweise gesättigten 3-bis 7-gliedrigen heterocyclischen Ring oder einer Alkanoylaminogruppe -N(R^{c})C(O)-C₁₋₆-Alkyl, worin R^{c} für Wasserstoff oder C₁₋₆-Alkyl steht, ausgewählt ist; oder
**R⁶** unter C₁₋₆-Alkyl oder C₁₋₆-Alkoxy ausgewählt ist, worin die C₁₋₆-Alkyl- und die C₁₋₆-Alkoxygruppen gegebenenfalls durch eine oder mehrere unabhängig voneinander unter Cyano, Fluor, Hydroxy, C₁₋₆-Alkoxy, Amino, Mono-C₁₋₆-alkylamino, Di (C₁₋₆-alkyl)amino, einem C₃₋₇-Cycloalkylring oder einem gesättigten oder teilweise gesättigten 3- bis 7-gliedrigen heterocyclischen Ring ausgewählte Gruppen substituiert sind;
und Salze davon.

3. Verbindung der Formel I nach Anspruch 1, worin:
**R¹ und R²** unabhängig voneinander unter Wasserstoff, C₁₋₆-Alkylsulfonyl, Phenyl (CH₂)ᵤ, worin u für 0, 1, 2, 3, 4, 5 oder 6 steht, C₁₋₆-Alkanoyl, C₁₋₆-Alkyl, C₁₋₆-Alkoxycarbonyl oder C₃₋₆-Cycloalkyl(CH₂)ₓ, worin x für 0, 1, 2, 3, 4, 5 oder 6 steht, ausgewählt sind, oder
**R¹ und R²** gemeinsam mit dem Stickstoffatom, an das sie gebunden sind, für einen gesättigten oder teilweise gesättigten 3- bis 7-gliedrigen heterocyclischen Ring, der gegebenenfalls ein unter N oder 0 ausgewähltes anderes Heteroatom enthält, stehen;
worin die Alkyl- und die Cycloalkylgruppen gegebenenfalls durch eine oder mehrere unabhängig voneinander unter Fluor, Hydroxy, C₁₋₆-Alkyl, C₁₋₆-Alkoxy, Amino, Mono-C₁₋₆-alkylamino oder Di (C₁₋₆-alkyl) amino, einem gesättigten oder teilweise gesättigten 3- bis 7-gliedrigen heterocyclischen Ring oder einem 5- oder 6-gliedrigen Heteroarylring ausgewählte Gruppen substituiert sind, worin die heterocyclischen Ringe und Heteroarylringe gegebenenfalls unabhängig voneinander durch eine oder mehrere der folgenden Gruppen substituiert sind: C₁₋₄-Alkyl, Hydroxy, Amino, Mono-C₁₋₆-alkylamino oder Di (C₁₋₆-alkyl) amino oder einen gesättigten oder teilweise gesättigten 3- bis 7-gliedrigen heterocyclischen Ring;
und worin das Phenyl gegebenenfalls durch eine oder mehrere unter Halogen, C₁₋₆-Alkyl, C₁₋₆-Alkoxy, Amino, Mono-C₁₋₆-alkylamino oder Di (C₁₋₆-alkyl)amino ausgewählte Gruppen substituiert ist, worin die C₁₋₆-Alkyl- oder C₁₋₆-Alkoxygruppen gegebenenfalls durch Hydroxy, Amino, Mono-C₁₋₆-alkylamino oder Di (C₁₋₆-alkyl) amino substituiert sind;
**R³ und R⁴** unabhängig voneinander unter Wasserstoff, C₁₋₆-Alkyl oder C₁₋₆-Alkoxy ausgewählt sind, worin die Alkyl- und die Alkoxygruppen gegebenenfalls durch eine oder mehrere unter Fluor, Hydroxy, C₁₋₆-Alkyl, C₁₋₆-Alkoxy, Amino, Mono-C₁₋₆-alkylamino oder Di(C₁₋₆-alkyl)amino, einem gesättigten oder teilweise gesättigten 3- bis 7-gliedrigen heterocyclischen Ring oder einem 5- oder 6-gliedrigen Heteroarylring ausgewählte Gruppen substituiert sind, worin die heterocyclischen Ringe und Heteroarylringe gegebenenfalls unabhängig voneinander durch eine oder mehrere der folgenden Gruppen substituiert sind: C₁₋₄-Alkyl, Hydroxy, Amino, Mono-C₁₋₆-alkylamino oder Di(C₁₋₆-alkyl)amino oder einen gesättigten oder teilweise gesättigten 3-bis 7-gliedrigen heterocyclischen Ring;
oder eine der Gruppen **R³ und R⁴** für eine Gruppe **-NR¹R²** gemäß obiger Definition steht;
**R⁵** unter Cyano, Halogen, C₁₋₆-Alkoxy oder C₁₋₆-Alkyl, gegebenenfalls substituiert durch Cyano oder ein oder mehrere Fluor, ausgewählt ist;
**B** für einen C₃₋₇-Cycloalkylring, eine Arylgruppe oder einen unter Furyl, Pyrrolyl, Thienyl, Oxazolyl, Isoxazolyl, Imidazolyl, Pyrazolyl, Thiazolyl, Isothiazolyl, Oxadiazolyl, Thiadiazolyl, Triazolyl, Tetrazolyl, Pyridyl, Pyridazinyl, Pyrimidinyl, Pyrazinyl oder 1,3,5-Triazinyl ausgewählten 5- oder 6-gliedrigen Heteroarylring steht;
**R⁶** unter Halogen, Cyano, einem gesättigten oder teilweise gesättigten 3- bis 7-gliedrigen heterocyclischen Ring oder einer Alkanoylaminogruppe -N(R^{c})C(O)-C₁₋₆-Alkyl, worin R^{c} für Wasserstoff oder C₁₋₆-Alkyl steht, ausgewählt ist; oder **R⁶** unter C₁₋₆-Alkyl oder C₁₋₆-Alkoxy ausgewählt ist, worin die Alkyl- und die Alkoxygruppen gegebenenfalls durch eine oder mehrere unter Cyano, Fluor, Hydroxy, C₁₋₆-Alkoxy, Amino, Mono-C₁₋₆-alkylamino, Di (C₁₋₆-alkyl)amino oder einem gesättigten oder teilweise gesättigten 3- bis 7-gliedrigen heterocyclischen Ring ausgewählte Gruppen substituiert sind; und
**m** für 0, 1, 2 oder 3 steht; und dann, wenn m mindestens für 2 steht, zwei Substituenten an benachbarten Kohlenstoffatomen im Ring B gemeinsam für eine Methylendioxygruppe stehen können;
und worin A, L und n die in Anspruch 1 angegebene Bedeutung besitzen;
und Salze davon.

4. Verbindung nach einem der Ansprüche 1, 2 und 3, in der A unter Phenyl, Pyridyl, Thiazolyl, Thiadiazolyl oder Pyrimidinyl ausgewählt ist.

5. Verbindung nach einem der vorhergehenden Ansprüche, in der B unter Phenyl, 2,3-Dihydroindenyl, Piperidinyl, Pyridyl, Pyrazolyl, Isothiazolyl, Thiadiazolyl, Isoxazolyl, Benzodioxinyl, Benzodioxolyl oder Tetrahydropyranyl ausgewählt ist.

6. Verbindung nach einem der vorhergehenden Ansprüche, in der L unter -N(R⁸)C(O)N(R⁹)-, -N(R⁸)C(O)O- oder -N(R⁸)C(O)CH₂-, worin R⁸ und R⁹ unabhängig voneinander für Wasserstoff oder C₁₋₆-Alkyl stehen, ausgewählt ist.

7. Verbindung nach einem der vorhergehenden Ansprüche, in der R¹ und R² beide für Wasserstoff stehen oder R¹ für Wasserstoff oder C₁₋₆-Alkyl steht und R² für C₁₋₆-Alkyl steht,
worin C₁₋₆-Alkyl gegebenenfalls durch Hydroxy, Amino, Mono-C₁₋₆-alkylamino oder Di(C₁₋₆-alkyl)amino, Carbamoyl, C₁₋₆-Alkoxy, C₁₋₆-Alkoxy-C₁₋₆-alkoxy, -N(R^{d})C(O)-C₁₋₆-Alkyl, worin R^{d} für Wasserstoff oder C₁₋₆-Alkyl steht, Aryl (insbesondere Phenyl), einen gesättigten oder teilweise gesättigten 3- bis 7-gliedrigen heterocyclischen Ring oder einen 5- oder 6-gliedrigen Heteroarylring substituiert ist; worin die C₁₋₆-Alkoxy-, Mono-C₁₋₆-alkylamino- und -N(R^{d})C(O)-C₁₋₆-Alkyl-Gruppen gegebenenfalls durch Hydroxy substituiert sind; und
worin ein Arylring, ein gesättigter oder teilweise gesättigter 3- bis 7-gliedriger heterocyclischer Ring oder ein 5- oder 6-gliedriger Heteroarylring gegebenenfalls durch C₁₋₄-Alkyl, C₁₋₄-Alkoxy oder -C(O)CH₂Y, worin Y unter Hydroxy oder Di-C₁₋₆-alkylamino ausgewählt ist, substituiert ist.

8. Verbindung nach einem der vorhergehenden Ansprüche, in der R³ und R⁴ beide für Wasserstoff stehen.

9. Verbindung nach einem der vorhergehenden Ansprüche, in der R⁶ unabhängig voneinander unter Halogen, Cyano, Oxo, C₃₋₇-Cycloalkyl, einem gesättigten 3- bis 7-gliedrigen heterocyclischen Ring (gegebenenfalls substituiert durch C₁₋₄-Alkyl), -N(R^{c})C(O)-C₁₋₆-Alkyl, worin R^{c} für Wasserstoff oder C₁₋₆-Alkyl (insbesondere C₁₋₄-Alkyl) steht, C₁₋₆-Alkyl (gegebenenfalls substituiert durch bis zu drei unabhängig voneinander unter Halogen ausgewählte Gruppen) oder C₁₋₆-Alkoxy ausgewählt ist und m unter 1 und 2 ausgewählt ist.

10. Verbindung nach Anspruch 1, ausgewählt unter:
N-{3-[(2-Aminopyrimidin-5-yl)ethinyl]phenyl}-N'-[2-fluor-5-(trifluormethyl)phenyl]harnstoff;
N-{3-[(2-Aminopyrimidin-5-yl)ethinyl]phenyl}-N'-[2-(trifluormethyl)phenyl]harnstoff;
N-{3-[(2-Aminopyrimidin-5-yl)ethinyl]phenyl}-N'-[4-(trifluormethyl)phenyl]harnstoff;
N-{3-[(2-Aminopyrimidin-5-yl)ethinyl]phenyl}-N'-(2-fluorphenyl)harnstoff;
N-{3-[(2-Aminopyrimidin-5-yl)ethinyl]phenyl}-N'-(3-fluorphenyl)harnstoff;
N-{3-[(2-Aminopyrimidin-5-yl)ethinyl]phenyl}-N'-(4-fluorphenyl)harnstoff;
N-{3-[(2-Aminopyrimidin-5-yl)ethinyl]phenyl}-N'-(3-methoxyphenyl)harnstoff;
N-{3-[(2-Aminopyrimidin-5-yl)ethinyl]phenyl}-N'-(2,5-difluorphenyl)harnstoff;
N-{3-[(2-Aminopyrimidin-5-yl)ethinyl]phenyl}-N'-1,3-benzodioxol-5-ylharnstoff;
N-{3-[(2-Aminopyrimidin-5-yl)ethinyl]phenyl}-N'-[3-(trifluormethyl)phenyl]harnstoff;
N-{3-[(2-Aminopyrimidin-5-yl)ethinyl]phenyl}-N'-(2-methoxyphenyl)harnstoff;
N-{3-[(2-Aminopyrimidin-5-yl)ethinyl]phenyl}-N'-(4-methoxyphenyl)harnstoff;
N-{3-[(2-Aminopyrimidin-5-yl)ethinyl]phenyl}-N'-(3,4-difluorphenyl)harnstoff;
N-{3-[(2-Aminopyrimidin-5-yl)ethinyl]phenyl}-N'-(3-cyanophenyl)harnstoff;
N-{3-[(2-Aminopyrimidin-5-yl)ethinyl]phenyl}-N'-(3-chlorphenyl)harnstoff;
N-{3-[(2-Aminopyrimidin-5-yl)ethinyl]phenyl}-N'-cyclopentylharnstoff;
N-{3-[(2-Aminopyrimidin-5-yl)ethinyl]phenyl}-N'-(3,5-difluorphenyl)harnstoff;
N-{3-[(2-Aminopyrimidin-5-yl)ethinyl]phenyl}-N'-(5-tert-Butyl-1,3,4-thiadiazol-2-yl)harnstoff;
N-{3-[(2-Aminopyrimidin-5-yl)ethinyl]phenyl}-N'-(3-methylisoxazol-5-yl)harnstoff;
N-(3-{[({3-[(2-Aminopyrimidin-5-yl)ethinyl]-phenyl}amino)carbonyl]amino}phenyl)acetamid;
N-{3-[(2-Aminopyrimidin-5-yl)ethinyl]phenyl}-N'-(4-(trifluormethyl)pyridin-2-yl]harnstoff;
N-{3-[(2-Aminopyrimidin-5-yl)ethinyl]phenyl}-N'-(5-tert-butylisoxazol-3-yl)harnstoff;
{3-[(2-Aminopyrimidin-5-yl)ethinyl]phenyl}-carbamidsäurephenylester;
N-{3-[(2-Aminopyrimidin-5-yl)ethinyl]phenyl}-N'-(2-oxopiperidin-3-yl)harnstoff;
N-(5-tert-Butylisoxazol-3-yl)-N'-(3-{[2-(methylamino)pyrimidin-5-yl]ethinyl}phenyl)harnstoff;
N-(5-tert-Butylisoxazol-3-yl)-N'-(3-{[2-(dimethylamino)pyrimidin-5-yl]ethinyl}phenyl)harnstoff;
N-(5-tert-Butylisoxazol-3-yl)-N'-[3-({2-[(2-morpholin-4-ylethyl)amino]pyrimidin-5-yl}-ethinyl)phenyl]harnstoff;
N-(5-tert-Butylisoxazol-3-yl)-N'-[3-({2-[(2-morpholin-4-ylpropyl)amino]pyrimidin-5-yl}-ethinyl)phenyl]harnstoff;
N-(5-tert-Butylisoxazol-3-yl)-N'-[3-({2-[(2-methoxyethyl)amino]pyrimidin-5-yl}ethinyl)phenyl]-harnstoff;
N-(5-tert-Butylisoxazol-3-yl)-N'-{3-[(2-{[3-(1H-imidazol-1-yl)propyl]amino}pyrimidin-5-yl)-ethinyl]phenyl}harnstoff;
N-(5-tert-Butylisoxazol-3-yl)-N'-[3-({2-[(3-methoxypropyl)amino]pyrimidin-5-yl}ethinyl)-phenyl]harnstoff;
N-(5-tert-Butylisoxazol-3-yl)-N'-[3-({2-[(2-hydroxyethyl)amino]pyrimidin-5-yl}ethinyl)phenyl]-harnstoff;
N-(5-tert-Butylisoxazol-3-yl)-N'-[3-({2-[(2-pyrrolidin-1-ylethyl)amino]pyrimidin-5-yl}-ethinyl)phenyl]harnstoff;
N-(5-tert-Butylisoxazol-3-yl)-N'-[3-({2-[(3-pyrrolidin-1-ylpropyl)amino]pyrimidin-5-yl}-ethinyl)phenyl]harnstoff;
N-[3-({2-[(2-Aminoethyl)amino]pyrimidin-5-yl}-ethinyl)phenyl]-N'-(5-tert-butylisoxazol-3-yl)-harnstoff;
N-[3-({2-[(3-Aminopropyl)amino]pyrimidin-5-yl}-ethinyl)phenyl]-N'-(5-tert-butylisoxazol-3-yl)-harnstoff;
N-(5-tert-Butylisoxazol-3-yl)-N'-{3-[(2-(dimethylamino)ethyl]amino}pyrimidin-5-yl)ethinyl]phenyl}-harnstoff;
N-(5-tert-Butylisoxazol-3-yl)-N'-{3-[(2-{[3-(dimethylamino)propyl]amino}pyrimidin-5-yl)-ethinyl]phenyl}harnstoff;
N-2-(5-{[3-({[(5-tert-Butylisoxazol-3-yl)amino]-carbonyl}amino)phenyl]ethinyl}pyrimidin-2-yl)-glycinamid;
N-3-(5-{[3-({[(5-tert-Butylisoxazol-3-yl)amino]-carbonyl}amino)phenyl]ethinyl}pyrimidin-2-yl)-beta-alaninamid;
N-(5-tert-Butylisoxazol-3-yl)-N'-{3-[(2-{[2-(1H-imidazol-4-yl)ethyl]amino}pyrimidin-5-yl)ethinyl]-phenyl}harnstoff;
N-(5-tert-Butylisoxazol-3-yl)-N'-[3-({2-[(2-pyridin-2-ylethyl)amino]pyrimidin-5-yl}ethinyl)-phenyl]harnstoff;
N-(5-tert-Butylisoxazol-3-yl)-N'-{3-[(2-{[3-(isopropylamino)propyl]amino}pyrimidin-5-yl)-ethinyl]phenyl}harnstoff;
N-(5-tert-Butylisoxazol-3-yl)-N'-{3-[(2-{[3-(4-methylpiperazin-1-yl)propyl]amino}pyrimidin-5-yl)-ethinyl]phenyl}harnstoff;
N-(5-tert-Butylisoxazol-3-yl)-N'-[3-({2-[(2-pyridin-4-ylethyl)amino]pyrimidin-5-yl}ethinyl)-phenyl]harnstoff;
N-(5-tert-Butylisoxazol-3-yl)-N'-[3-({2-[(3-piperidin-1-ylpropyl)amino]pyrimidin-5-yl}-ethinyl)phenyl]harnstoff;
N-(5-Methylisoxazol-3-yl)-N'-[3-({2-[(2-pyrrolidin-1-ylethyl)amino]pyrimidin-5-yl}-ethinyl)phenyl]harnstoff;
N-(5-tert-Butyl-1,3,4-thiadiazol-2-yl)-N'-[3-({2-[(2-pyrrolidin-1-ylethyl)amino]pyrimidin-5-yl}-ethinyl)phenyl]harnstoff;
N-(3-Methylisothiazol-5-yl)-N'-[3-({2-[(2-pyrrolidin-1-ylethyl)amino]pyrimidin-5-yl}-ethinyl)phenyl]harnstoff;
N-(3-Fluorphenyl)-N'-[3-({2-[(2-pyrrolidin-1-ylethyl)amino]pyrimidin-5-yl}ethinyl)phenyl]-harnstoff;
N-(4-Methoxyphenyl)-N'-[3-({2-[(2-pyrrolidin-1-ylethyl)amino]pyrimidin-5-yl}ethinyl)phenyl]-harnstoff;
N-(2-Fluorphenyl)-N'-[3-({2-[(2-pyrrolidin-1-ylethyl)amino]pyrimidin-5-yl}ethinyl)phenyl]-harnstoff;
N-(2,5-Difluorphenyl)-N'-[3-({2-[(2-pyrrolidin-1-ylethyl)amino]pyrimidin-5-yl}ethinyl)phenyl]-harnstoff;
N-(3,4-Difluorphenyl)-N'-[3-({2-[(2-pyrrolidin-1-ylethyl)amino]pyrimidin-5-yl}ethinyl)phenyl]-harnstoff;
N-[2-Fluor-5-(trifluormethyl)phenyl]-N'-[3-({2-[(2-pyrrolidin-1-ylethyl)amino]pyrimidin-5-yl}-ethinyl)phenyl]harnstoff;
N-[3-({2-[(2-Pyrrolidin-1-ylethyl)amino]pyrimidin-5-yl}ethinyl)phenyl]-N'-[4-(trifluormethyl)-phenyl]harnstoff;
N-1,3-Benzodioxol-5-yl-N'-[3-({2-[(2-pyrrolidin-1-ylethyl)amino]pyrimidin-5-yl}ethinyl)phenyl]-harnstoff;
N-(4-Fluorphenyl)-N'-[3-({2-[(2-pyrrolidin-1-yl-ethyl)amino]pyrimidin-5-yl}ethinyl)phenyl]-harnstoff;
N-(3-Chlorphenyl)-N'-[3-({2-[(2-pyrrolidin-1-yl-ethyl)amino]pyrimidin-5-yl}ethinyl)phenyl]-harnstoff;
N-(5-Methylisoxazol-3-yl)-N'-[3-({2-[(2-morpholin-4-ylethyl)amino]pyrimidin-5-yl}ethinyl)phenyl]-harnstoff;
N-(5-tert-Butyl-1,3,4-thiadiazol-2-yl)-N'-[3-({2-[(2-morpholin-4-ylethyl)amino]pyrimidin-5-yl}-ethinyl)phenyl]harnstoff;
N-[2-Fluor-5-(trifluormethyl)phenyl]-N'-[3-({2-[(2-morpholin-4-ylethyl)amino]pyrimidin-5-yl}-ethinyl)phenyl]harnstoff;
N-(5-Methylisoxazol-3-yl)-N'-[3-({2-[(3-morpholin-4-ylpropyl)amino]pyrimidin-5-yl}ethinyl)phenyl]-harnstoff;
N-(5-tert-Butyl-1,3,4-thiadiazol-2-yl)-N'-[3-({2-[(3-morpholin-4-ylpropyl)amino]pyrimidin-5-yl}-ethinyl)phenyl]harnstoff;
N-[2-Fluor-5-(trifluormethyl)phenyl]-N'-[3-({2-[(2-morpholin-4-ylpropyl)amino]pyrimidin-5-yl}-ethinyl)phenyl]harnstoff;
N-(5-Methylisoxazol-3-yl)-N'-[4-({2-[(2-pyrrolidin-1-ylethyl)amino]pyrimidin-5-yl}-ethinyl)phenyl]harnstoff;
N-(5-tert-Butylisoxazol-3-yl)-N'-[4-({2-[(2-pyrrolidin-1-ylethyl)amino]pyrimidin-5-yl}-ethinyl)phenyl]harnstoff;
N-(5-tert-Butyl-1,3,4-thiadiazol-2-yl)-N'-[4-({2-[(2-pyrrolidin-1-ylethyl)amino]pyrimidin-5-yl}-ethinyl)phenyl]harnstoff;
N-[2-Fluor-5-(trifluormethyl)phenyl]-N'-[4-({2-[(2-pyrrolidin-1-ylethyl)amino]pyrimidin-5-yl}-ethinyl)phenyl]harnstoff;
N-(5-{[3-({[(5-tert-Butylisoxazol-3-yl)amino]-carbonyl}amino)phenyl]ethinyl}pyrimidin-2-yl)-2-(2-methoxyethoxy)acetamid;
N-{6-[(2-Aminopyrimidin-5-yl)ethinyl]pyridin-2-yl}-N'-(5-tert-butylisoxazol-3-yl)harnstoff;
N-{2-[(2-Aminopyrimidin-5-yl)ethinyl]pyridin-4-yl}-N'-(5-tert-butylisoxazol-3-yl)harnstoff;
N-{5-[(2-Aminopyrimidin-5-yl)ethinyl]-1,3-thiazol-2-yl}-N'-[2-fluor-5-(trifluormethyl)phenyl]-harnstoff;
N-{5-[(2-Aminopyrimidin-5-yl)ethinyl]-1,3,4-thiadiazol-2-yl}-N'-[2-fluor-5-(trifluormethyl)-phenyl]harnstoff;
N-{5-[(2-Aminopyrimidin-5-yl)ethinyl]-1,3-thiazol-2-yl}-N'-(5-tert-butylisoxazol-3-yl)harnstoff;
N-{3-[(2-Aminopyrimidin-5-yl)ethinyl]phenyl}-2-(2-methoxyphenyl)acetamid;
N-{3-[(2-Aminopyrimidin-5-yl)ethinyl]phenyl}-2-phenylacetamid;
N-{3-[(2-Aminopyrimidin-5-yl)ethinyl]phenyl}-2-(3-methoxyphenyl)acetamid;
N-{3-[(2-Aminopyrimidin-5-yl)ethinyl]phenyl}-2-[(3-(trifluormethyl)phenyl]acetamid;
N-{3-[(2-Aminopyrimidin-5-yl)ethinyl]phenyl}-2-[(4-(trifluormethyl)phenyl]acetamid;
N-{3-[(2-Aminopyrimidin-5-yl)ethinyl]phenyl}-2-(3-methylisoxazol-5-yl)acetamid;
N-{4-[(2-Aminopyrimidin-5-yl)ethinyl]phenyl}-2-(2-methoxyphenyl)acetamid;
N-{4-[(2-Aminopyrimidin-5-yl)ethinyl]phenyl}-2-(3-methylisoxazol-5-yl)acetamid;
N-{3-[(2-Aminopyrimidin-5-yl)ethinyl]phenyl}-N'-(2,2-dimethyltetrahydro-2H-pyran-4-yl)harnstoff;
N-{6-[(2-Aminopyrimidin-5-yl)ethinyl]pyrimidin-4-yl}-N'-(5-tert-butylisoxazol-3-yl)harnstoff;
N'-{3-[(2-Aminopyrimidin-5-yl)ethinyl]phenyl}-N-(5-tert-butylisoxazol-3-yl)-N-methylharnstoff;
N-{3-[(2-Aminopyrimidin-5-yl)ethinyl]phenyl}-N'-(1-tert-butyl-3-Cyclopropyl-1H-pyrazol-5-yl)-harnstoff;
N-{3-[(2-Aminopyrimidin-5-yl)ethinyl]phenyl}-N'-(5-methylisoxazol-3-yl)harnstoff;
N-{5-[(2-Aminopyrimidin-5-yl)ethinyl]pyridin-3-yl}-N'-(5-tert-butylisoxazol-3-yl)harnstoff;
N-[3-({2-[(3-Piperidin-1-ylpropyl)amino]pyrimidin-5-yl}ethinyl)phenyl]-N'-[4-(trifluormethyl)-pyridin-2-yl]harnstoff;
N-(5-tert-Butyl-1,3,4-thiadiazol-2-yl)-N'-[3-({2-[(3-piperidin-1-ylpropyl)amino]pyrimidin-5-yl}-ethinyl)phenyl]harnstoff;
N-(3-Methylisoxazol-5-yl)-N'-[3-({2-[(3-piperidin-1-ylpropyl)amino]pyrimidin-5-yl}ethinyl)phenyl]-harnstoff;
N-(2-Methoxyphenyl)-N'-[3-({2-[(3-piperidin-1-ylpropyl)amino]pyrimidin-5-yl}ethinyl)phenyl]-harnstoff;
N-(3-Fluorphenyl)-N'-[3-({2-[(3-piperidin-1-ylpropyl)amino]pyrimidin-5-yl}ethinyl)phenyl]-harnstoff;
N'-{4-[(2-Aminopyrimidin-5-yl)ethinyl]phenyl}-N-(5-tert-butylisoxazol-3-yl)-N-methylharnstoff;
N-{3-[(2-Aminopyrimidin-5-yl)ethinyl]phenyl}-N'-(3-cyclopropyl-1-methyl-1H-pyrazol-5-yl)harnstoff;
N-{3-[(2-Aminopyrimidin-5-yl)ethinyl]phenyl}-N'-phenylharnstoff;
N-[3-({2-[(4-Aminobutyl)amino]pyrimidin-5-yl}-ethinyl)phenyl]-N'-(5-tert-butylisoxazol-3-yl)-harnstoff;
N-(5-tert-Butylisoxazol-3-yl)-N'-[3-({2-[(2-piperidin-1-ylethyl)amino]pyrimidin-5-yl}ethinyl)-phenyl]harnstoff;
N-(5-tert-Butylisoxazol-3-yl)-N'-{3-[(2-{[2-(isopropylamino)ethyl]amino}pyrimidin-5-yl)-ethinyl]phenyl}harnstoff;
N-(5-tert-Butylisoxazol-3-yl)-N'-{3-[(2-{[2-(2-(hydroxyethoxy)ethyl]amino}pyrimidin-5-yl)-ethinyl]phenyl}harnstoff;
N-{3-[(2-Aminopyrimidin-5-yl)ethinyl]phenyl}-N'-(5-cyclopropyl-1,3,4-thiadiazol-2-yl)harnstoff;
N-{3-[(2-Aminopyrimidin-5-yl)ethinyl]phenyl}-N'-[5-(trifluormethyl)-1,3,4-thiadiazol-2-yl)harnstoff;
N-{2-[(5-{[3-({[(5-tert-Butylisoxazol-3-yl)amino]-carbonyl}amino)phenyl]ethinyl}pyrimidin-2-yl)-amino]ethyl}-2-hydroxyacetamid;
N-(5-tert-Butylisoxazol-3-yl)-N'-{3-[(2-{[4-(dimethylamino)butyl]amino}pyrimidin-5-yl)-ethinyl]phenyl}harnstoff;
N'-{3-[(2-Aminopyrimidin-5-yl)ethinyl]phenyl}-N-methyl-N-[5-(trifluormethyl)-1,3,4-thiadiazol-2-yl]harnstoff;
N-Phenyl-N'-[3-({2-[(3-piperidin-1-ylpropyl)-amino]pyrimidin-5-yl}ethinyl)phenyl]harnstoff;
N-(5-Methylisoxazol-3-yl)-N'-[3-({2-[(3-piperidin-1-ylpropyl)amino]pyrimidin-5-yl}ethinyl)phenyl]-harnstoff;
N-{3-[(2-Aminopyrimidin-5-yl)ethinyl]phenyl}-N'-(5-tert-butylisoxazol-3-yl)-N-methylharnstoff;
N-(5-tert-Butylisoxazol-3-yl)-N'-{3-[(2-{[2-(dimethylamino)1-methylethyl]amino}pyrimidin-5-yl)ethinyl]phenyl}harnstoff;
{3-[(2-{[3-(Dimethylamino)propyl]amino}pyrimidin-5-yl)ethinyl]phenyl}carbamidsäurephenylester;
N-{3-[(2-Aminopyrimidin-5-yl)ethinyl]phenyl}-N'-(3-tert-butyl-1-methyl-1H-pyrazol-5-yl)harnstoff;
N-{3-[(2-Aminopyrimidin-5-yl)ethinyl]phenyl}-N'-(4-tert-butyl-1,3-thiazol-2-yl)harnstoff;
N-{3-[(2-Aminopyrimidin-5-yl)ethinyl]phenyl}-N'-(5-methyl-1,3,4-thiadizol-2-yl)harnstoff;
N-{3-[(2-Aminopyrimidin-5-yl)ethinyl]phenyl}-N'-(5-ethyl-1,3,4-thiadizol-2-yl)harnstoff;
N-{3-[(2-Aminopyrimidin-5-yl)ethinyl]phenyl}-N'-(5-isopropyl-1,3,4-thiadizol-2-yl)harnstoff;
N-{3-[(2-{[3-(Dimethylamino)propyl]amino}-pyrimidin-5-yl)ethinyl]phenyl}-N'-(5-methylisoxazol-3-yl)harnstoff;
N-{3-[(2-{[3-(Dimethylamino)propyl]amino}-pyrimidin-5-yl)ethinyl]phenyl}-N'-phenylharnstoff;
N-{5-[(2-Aminopyrimidin-5-yl)ethinyl]pyridin-3-yl}-N'-(5-tert-butylisoxazol-3-yl)harnstoff;
N-(3-tert-Butyl-1-methyl-1H-pyrazol-5-yl)-N'-[3-({2-[(3-morpholin-4-ylpropyl)amino]pyrimidin-5-yl}ethinyl)phenyl]harnstoff;
N-(3-tert-Butyl-1-methyl-1H-pyrazol-5-yl)-N'-[3-({2-[(2-morpholin-4-ylethyl)amino]pyrimidin-5-yl}-ethinyl)phenyl]harnstoff;
N-(3-tert-Butyl-1-methyl-1H-pyrazol-5-yl)-N'-[3-({2-[(3-piperidin-lylpropyl)amino]pyrimidin-5-yl}-ethinyl)phenyl]harnstoff;
N-(5-tert-Butylisoxazol-3-yl)-N'-[3-({2-[(1-methyl-2-morpholin-4-ylethyl)amino]pyrimidin-5-yl}ethinyl)phenyl]harnstoff;
N-(5-tert-Butylisoxazol-3-yl)-N'-{3-[(2-{[(1-glykoloylpyrrolidin-2-yl)methyl]amino}pyrimidin-5-yl)ethinyl]phenyl}harnstoff;
N-(5-tert-Butylisoxazol-3-yl)-N'-(3-{[2-({[1-(N,N-dimethylglycyl)pyrrolidin-2-yl]methyl}amino)-pyrimidin-5-yl]ethinyl}phenyl)harnstoff;
N-(5-tert-Butylisoxazol-3-yl)-N'-[3-({2-[(2-piperazin-1-ylethyl)amino]pyrimidin-5-yl}ethinyl)-phenyl]harnstoff;
N-(5-tert-Butylisoxazol-3-yl)-N'-[3-({2-[(3-piperazin-1-ylpropyl)amino]pyrimidin-5-yl}-ethinyl)phenyl]harnstoff;
N-(5-tert-Butylisoxazol-3-yl)-N-methyl-N'-[3-({2-[(2-morpholin-4-ylethyl)amino]pyrimidin-5-yl}-ethinyl)phenyl]harnstoff;
N-(5-tert-Butylisoxazol-3-yl)-N-methyl-N'-[3-({2-[(3-morpholin-4-ylpropyl)amino]pyrimidin-5-yl}-ethinyl)phenyl]harnstoff;
N-(5-tert-Butylisoxazol-3-yl)-N-methyl-N'-[3-({2-[(3-piperidin-1-ylpropyl)amino]pyrimidin-5-yl}-ethinyl)phenyl]harnstoff;
N-(3-tert-Butyl-1-methyl-1H-pyrazol-5-yl)-N'-[3-({2-[(3-piperidin-1-ylpropyl)amino]pyrimidin-5-yl}ethinyl)phenyl]harnstoff;
N-(3-tert-Butyl-1-methyl-1H-pyrazol-5-yl)-N'-{3-[(2-{[2-(dimethylamino)ethyl]amino}pyrimidin-5-yl)ethinyl]phenyl}harnstoff;
N-(3-tert-Butyl-1-methyl-1H-pyrazol-5-yl)-N'-{3-[(2-{[2-(isopropylamino)ethyl]amino}pyrimidin-5-yl)ethinyl]phenyl}harnstoff;
N-(3-Cyclopropyl-1-methyl-1H-pyrazol-5-yl)-N'-[3-({2-[(3-piperidin-1-ylpropyl)amino]pyrimidin-5-yl}ethinyl)phenyl]harnstoff;
N-(3-Cyclopropyl-1-methyl-1H-pyrazol-5-yl)-N'-[3-({2-[(3-morpholin-4-ylpropyl)amino]pyrimidin-5-yl}ethinyl)phenyl]harnstoff;
N-(3-Cyclopropyl-1-methyl-1H-pyrazol-5-yl)-N'-[3-({2-[(2-morpholin-4-ylethyl)amino]pyrimidin-5-yl}-ethinyl)phenyl]harnstoff;
N-(5-tert-Butylisoxazol-3-yl)-N'-(3-{[2-({2-[(2-hydroxy-1-oxoethyl)amino]ethyl}amino)pyrimidin-5-yl]ethinyl}phenyl)harnstoff;
N-(5-tert-Butylisoxazol-3-yl)-N'-(3-{[2-({3-[(2-hydroxyethyl)amino]propyl}amino)pyrimidin-5-yl]ethinyl}phenyl)harnstoff;
N-(5-tert-Butylisoxazol-3-yl)-N'-{3-[(2-{[3-(dimethylamino)propyl]amino}pyrimidin-5-yl)-ethinyl]phenyl}-N-methylharnstoff;
N-{5-[(2-Aminopyrimidin-5-yl)ethinyl]pyridin-3-yl}-N'-(3-tert-butyl-1-methyl-1H-pyrazol-5-yl)-harnstoff;
N-{5-[(2-Aminopyrimidin-5-yl)ethinyl]pyridin-3-yl}-N'-(3-cyclopropyl-1-methyl-1H-pyrazol-5-yl)-harnstoff;
N-{5-[(2-Aminopyrimidin-5-yl)ethinyl]-1,3-thiazol-2-yl}-N'-phenylharnstoff;
N-{5-[(2-Aminopyrimidin-5-yl)ethinyl]-1,3-thiazol-2-yl}-N'-(2,2-dimethyltetrahydro-2H-pyran-4-yl)-harnstoff;
N-{5-[(2-Aminopyrimidin-5-yl)ethinyl]-1,3-thiazol-2-yl}-N'-(3-cyclopropyl-1-methyl-1H-pyrazol-5-yl)-harnstoff;
N-{5-[(2-Aminopyrimidin-5-yl)ethinyl]-1,3-thiazol-2-yl}-N'-(3-tert-butyl-1-methyl-1H-pyrazol-5-yl)-harnstoff;
N-{5-[(2-Aminopyrimidin-5-yl)ethinyl]-1,3-thiazol-2-yl}-N'-phenylharnstoff;
N-{5-[(2-Aminopyrimidin-5-yl)ethinyl]-1,3-thiazol-2-yl}-N'-(2,2-dimethyltetrahydro-2H-pyran-4-yl)-harnstoff;
N-{3-[(2-Aminopyrimidin-5-yl)ethinyl]phenyl}-N'-(1,3-dimethyl-1H-pyrazol-5-yl)harnstoff;
N-{3-[(2-Aminopyrimidin-5-yl)ethinyl]phenyl}-N'-[5-(ethylthio)-1,3,4-thiadiazol-2-yl]harnstoff;
N-{3-[(2-Aminopyrimidin-5-yl)ethinyl]phenyl}-N'-(1-tert-butyl-1H-pyrazol-4-yl)harnstoff;
N-{3-[(2-Aminopyrimidin-5-yl)ethinyl]phenyl}-N'-(3-isopropyl-1-methyl-1H-pyrazol-5-yl)harnstoff;
N-{3-[(2-Aminopyrimidin-5-yl)ethinyl]phenyl}-N'-(5-isopropyl-1,3,4-thiadiazol-2-yl)harnstoff;
N-{3-[(2-Aminopyrimidin-5-yl)ethinyl]phenyl}-N'-(1-ethyl-1H-pyrazol-3-yl)harnstoff;
N-{3-[(2-Aminopyrimidin-5-yl)ethinyl]phenyl}-N'-(1-isopropyl-1H-pyrazol-3-yl)harnstoff;
N-{3-[(2-Aminopyrimidin-5-yl)ethinyl]phenyl}-N'-[3-fluor-5-(4-methylpiperazin-1-yl)phenyl]harnstoff oder
N-{3-[(2-Aminopyrimidin-5-yl)ethinyl]-4-methylphenyl}-N'-(3-tert-butyl-1-methyl-1H-pyrazol-5-yl)harnstoff.

11. Pharmazeutische Zusammensetzung, die eine Verbindung der Formel I oder ein pharmazeutisch annehmbares Salz davon gemäß einem der Ansprüche 1 bis 10 zusammen mit einem pharmazeutisch annehmbaren Verdünnungsmittel oder Träger enthält.

12. Verbindung der Formel I oder ein pharmazeutisch annehmbares Salz davon nach einem der Ansprüche 1 bis 10 zur Verwendung als Arzneimittel.

13. Verwendung einer Verbindung der Formel I oder eines pharmazeutisch annehmbaren Salzes davon gemäß einem der Ansprüche 1 bis 10 bei der Herstellung eines Arzneimittels zur Verwendung als Tie2-Rezeptor-Tyrosinkinase-Inhibitor bei einem Warmblüter wie dem Menschen.

14. Verwendung einer Verbindung der Formel I oder eines pharmazeutisch annehmbaren Salzes davon gemäß einem der Ansprüche 1 bis 10 bei der Herstellung eines Arzneimittels zur Erzielung einer antiangiogenen Wirkung bei einem Warmblüter wie dem Menschen.

15. Verfahren zur Herstellung einer Verbindung der Formel I gemäß Anspruch 1 oder eines pharmazeutisch annehmbaren Salzes davon (worin R¹, R², R³, R⁴, R⁵, R⁶ , R⁷, R⁸, R⁹, R¹⁰, R¹¹, L, Ring A und Ring B, n und m die in Anspruch 1 angegebene Bedeutung besitzen, sofern nicht anders vermerkt), bei dem man:
(a) für Verbindungen der Formel I, worin L für -N (R⁸) C (O) N (H) - steht, eine Verbindung der Formel II: 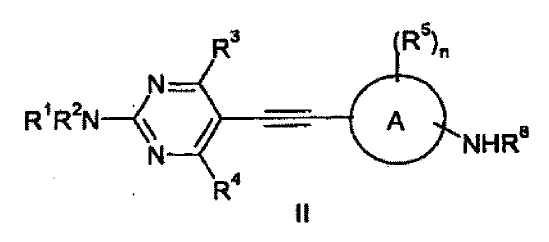 worin R¹, R², R³, R⁴, R⁵, R⁸, n und A eine der oben angegebenen Bedeutungen besitzen, wobei jedoch jede funktionelle Gruppe gegebenenfalls geschützt ist, mit einem Isocyanat der Formel IV: 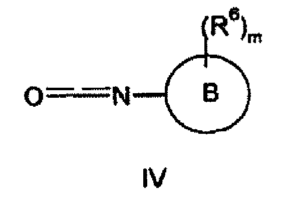 worin R⁶, m und B eine der oben angegebenen Bedeutungen besitzen, wobei jedoch jede funktionelle Gruppe gegebenenfalls geschützt ist, umsetzt; oder
(b) für Verbindungen der Formel I, worin L für -N(R⁸)C(O)N(H)- steht, eine Verbindung der Formel II gemäß obiger Definition mit einem Arylcarbamat der Formel III: 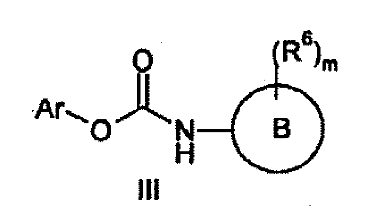 worin Ar für eine geeignete Arylgruppe steht und R⁶, m und B eine der oben angegebenen Bedeutungen besitzen, wobei jedoch jede funktionelle Gruppe gegebenenfalls geschützt ist, umsetzt; oder
(c) für Verbindungen der Formel I, worin L für N (R⁸) C (O) -O- steht, eine Verbindung der Formel II gemäß obiger Definition mit einer Verbindung der Formel XI: 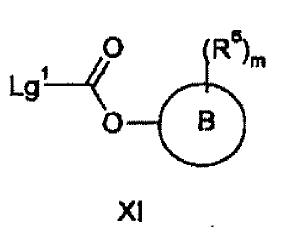 worin Lg¹ für eine geeignete Abgangsgruppe steht und R⁶, m und B eine der oben angegebenen Bedeutungen besitzen, wobei jedoch jede funktionelle Gruppe gegebenenfalls geschützt ist, umsetzt; oder
(d) für Verbindungen der Formel I, worin L für N (R⁸) C (O) C (R^{a}R^{b}) steht, eine Verbindung der Formel II gemäß obiger Definition mit einer Verbindung der Formel IX: 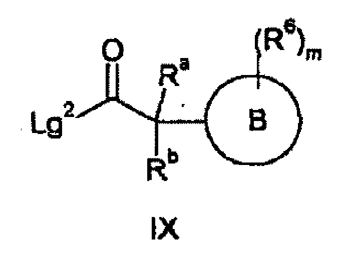 worin Lg² für eine geeignete Abgangsgruppe, R^{x}-C(O)-O- oder R^{x}-O- (worin R^{x} eine geeignete Alkyl- oder Arylgruppe bedeutet) steht und R⁶, R^{a}, R^{b}, m und B eine der oben angegebenen Bedeutungen besitzen, wobei jedoch jede funktionelle Gruppe gegebenenfalls geschützt ist, umsetzt; oder
(e) für Verbindungen der Formel I, worin L für -N (R⁸) C (O) N (H) - steht, eine Verbindung der Formel II gemäß obiger Definition mit einem Trichloracetylamin der Formel XIII: 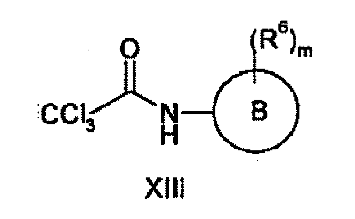 worin R⁶, m und B eine der oben angegebenen Bedeutungen besitzen, wobei jedoch jede funktionelle Gruppe gegebenenfalls geschützt ist, umsetzt; oder
(f) für Verbindungen der Formel I, worin L für -C (R^{a}R^{b}) C (O)N (R⁹) - steht, eine Verbindung der Formel XIV: 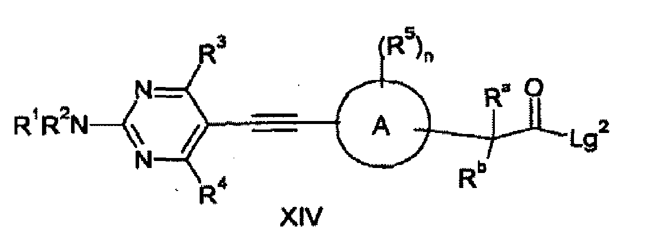 worin Lg² für eine geeignete Abgangsgruppe gemäß obiger Beschreibung steht und R¹, R², R³, R⁴, R⁵, R^{a}, R^{b}, n und A eine der oben angegebenen Bedeutungen besitzen, wobei jedoch jede funktionelle Gruppe gegebenenfalls geschützt ist, mit einem Amin der Formel XV: 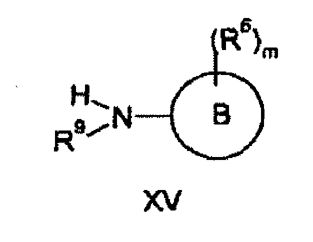 worin R⁶, R⁹, m und B eine der oben angegebenen Bedeutungen besitzen, wobei jedoch jede funktionelle Gruppe gegebenenfalls geschützt ist, umsetzt; oder
(g) eine Verbindung der Formel XVI: 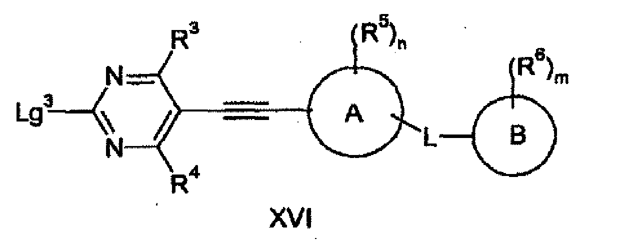 worin Lg³ für eine geeignete Abgangsgruppe, Methylsulfonyl, Methylthio oder Aryloxy steht und R³, R⁴, R⁵, R⁶, n, m, A, B und L eine der oben angegebenen Bedeutungen besitzen, wobei jedoch jede funktionelle Gruppe gegebenenfalls geschützt ist, mit einem Amin der Formel HNR¹R², worin R¹ und R² eine der oben angegebenen Bedeutungen besitzen, wobei jedoch jede funktionelle Gruppe gegebenenfalls geschützt ist, umsetzt; oder
(h) eine Verbindung der Formel XVII: 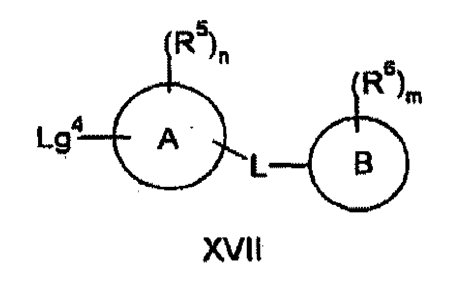 worin Lg⁴ für eine geeignete Abgangsgruppe oder eine Sulfonyloxygruppe steht und R⁵, R⁶, n, m, A, B und L eine der oben angegebenen Bedeutungen besitzen, wobei jedoch jede funktionelle Gruppe gegebenenfalls geschützt ist, mit einem Alkin der Formel XVIII: 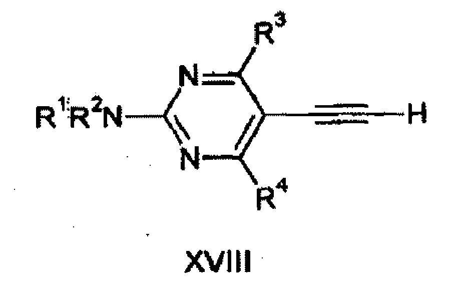 worin R¹, R², R³ und R⁴ eine der oben angegebenen Bedeutungen besitzen, wobei jedoch jede funktionelle Gruppe gegebenenfalls geschützt ist, umsetzt; oder
(i) für Verbindungen der Formel I, worin L für -N (H) C (O) N (R⁹) - steht, ein Isocyanat der Formel XIX: 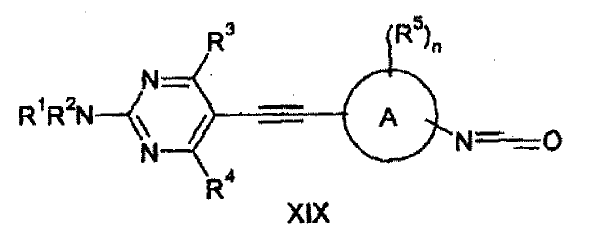 worin R¹, R², R³, R⁴, R⁵, n und A eine der oben angegebenen Bedeutungen besitzen, wobei jedoch jede funktionelle Gruppe gegebenenfalls geschützt ist, mit einem Amin der Formel XV gemäß obiger Definition umsetzt; oder
(j) für Verbindungen der Formel I, worin L für -N (H) C (O) N (R⁹) - steht, eine Verbindung der Formel XX: 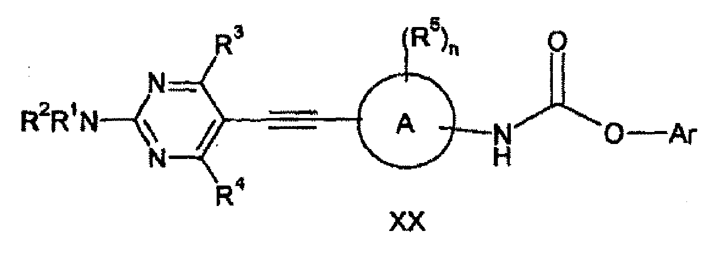 worin Ar für eine geeignete Arylgruppe steht und R¹, R², R³, R⁴, R⁵, n und A eine der oben angegebenen Bedeutungen besitzen, wobei jedoch jede funktionelle Gruppe gegebenenfalls geschützt ist, mit einem Amin der Formel XV gemäß obiger Definition umsetzt;
und danach gegebenenfalls:
i) eine Verbindung der Formel (I) in eine andere Verbindung der Formel (I) umwandelt;
ii) jegliche Schutzgruppen abspaltet;
iii) ein Salz bildet.

16. Verbindung, ausgewählt unter den Formeln II, XIV, XVI, XIX und XX gemäß Anspruch 15, worin A für einen unter Furyl, Pyrrolyl, Thienyl, Oxazolyl, Isoxazolyl, Imidazolyl, Pyrazolyl, Thiazolyl, Isothiazolyl, Oxadiazolyl, Thiadiazolyl, Triazolyl, Tetrazolyl, Pyridyl, Pyridazinyl, Pyrimidinyl, Pyrazinyl oder 1,3,5-Triazinyl ausgewählten 5- oder 6-gliedrigen Heteroarylring steht, oder einer Verbindung der Formel VIc: 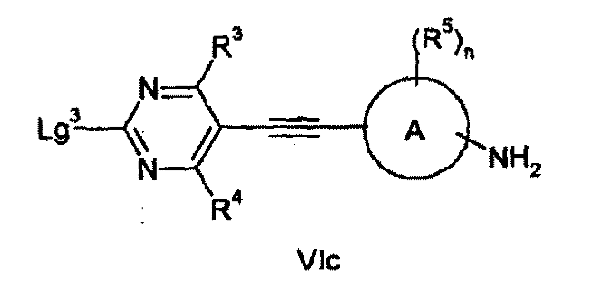 oder ein Salz davon, worin A für einen unter Furyl, Pyrrolyl, Thienyl, Oxazolyl, Isoxazolyl, Imidazolyl, Pyrazolyl, Thiazolyl, Isothiazolyl, Oxadiazolyl, Thiadiazolyl, Triazolyl, Tetrazolyl, Pyridyl, Pyridazinyl, Pyrimidinyl, Pyrazinyl oder 1,3,5-Triazinyl ausgewählten 5- oder 6-gliedrigen Heteroarylring steht und R¹, R², R³, R⁴, R⁵, R⁶, R⁷, R⁸, R⁹, R¹⁰, R¹¹, L, m, Ring B und n die in Anspruch 1 angegebene Bedeutung besitzen und Lg² für Hydroxyl oder Halogen steht und Lg³ für Halogen, Methylsulfonyl, Methylthio oder Aryloxy steht.

## Revendications

1. Composé de formule I : 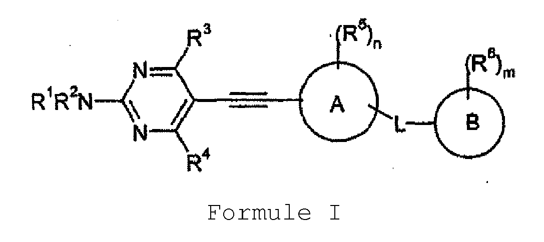 dans laquelle :
**R¹ et R²** sont choisis indépendamment parmi hydrogène, (1-6C)alkylsulfonyle, phényl(CH₂)ᵤ- où u est 0, 1, 2, 3, 4, 5 ou 6, (1-6C)alcanoyle, (1-6C)alkyle, (1-6C)alcoxycarbonyle, (3-6C) cycloalkyl (CH₂) ₓ- dans lequel x est 0, 1, 2, 3, 4, 5 ou 6, ou un cycle hétéroaryle à 5 ou 6 chaînons, ou **R¹ et R²** conjointement avec l'atome d'azote auquel ils sont liés représentent un cycle hétérocyclique saturé ou partiellement saturé à 3 à 7 chaînons contenant éventuellement un autre hétéroatome choisi parmi N ou 0 ;
où les groupements (1-6C)alkyle, (1-6C)alcanoyle et (3-6C)cycloalkyle sont éventuellement substitués par un ou plusieurs groupements choisis indépendamment parmi fluoro, hydroxy, (1-6C)alkyle, (1-6C)alcoxy, (1-6C) alcoxy (1-6C) alcoxy, (1-6C) alcoxy (1-6C)alcoxy(1-6C)alcoxy, amino, mono(1-6C)alkylamino, di-[(1-6C)alkyl]amino, carbamoyle, mono(1-6C)alkylcarbamoyle, di-[(1-6C) alkyl] carbamoyle, -N (R^{d}) C (O) (1-6C) alkyle dans lequel R^{d} est hydrogène ou (1-6C)alkyle, un cycle hétérocyclique saturé ou partiellement saturé à 3 à 7 chaînons, ou un cycle hétéroaryle à 5 ou 6 chaînons,
où les groupements (1-6C)alcoxy, (1-6C) alcoxy (1-6C) alcoxy et (1-6C)alcoxy(1-6C)alcoxy(1-6C)alcoxy et les groupements (1-6C)alkyle des groupements mono(1-6C)alkylamino, di-[(1-6C)alkyl]amino, mono (1-6C)alkylcarbamoyle, di-[(1-6C)alkyl]carbamoyle et/ou -N (R^{d}) C (O) (1-6C)alkyle sont éventuellement substitués par un ou plusieurs groupements hydroxy ;
où le phényle est éventuellement substitué par un ou plusieurs groupements choisis indépendamment parmi halogéno, (1-6C)alkyle, (1-6C)alcoxy, amino, mono(1-6C)alkylamino ou di-[(1-6C)alkyl]amino, où les groupements (1-6C)alkyle et (1-6C)alcoxy sont éventuellement substitués par un ou plusieurs groupements choisis indépendamment parmi hydroxy, amino, mono(1-6C)alkylamino ou di-[(1-6C)alkyl]amino ;
et où tous les cycles hétérocycliques et hétéroaryle dans R¹ et/ou R² sont éventuellement indépendamment substitués par un ou plusieurs de ce qui suit :
(1-4C)alkyle, (1-4C)alcoxy, (1-4C)alcoxy(1-4C)alkyle, hydroxy, amino, mono(1-6C)alkylamino, di-[(1-6C)alkyl]amino, un cycle hétérocyclique saturé ou partiellement saturé à
3 à 7 chaînons ou -C(O) (CH₂)_{z} Y où z est 0, 1, 2 ou 3 et Y est choisi parmi hydrogène, hydroxy, (1-4C)alcoxy, amino, mono(1-6C)alkylamino, di-[(1-6C)alkyl]amino ou un cycle hétérocyclique saturé ou partiellement saturé à 3 à 7 chaînons ;
et à condition que lorsque R¹ et/ou R² est un groupement (1C)alcanoyle, alors le (1C)alcanoyle n'est pas substitué par fluoro ou hydroxy ;
**R³ et R⁴** sont choisis indépendamment parmi hydrogène, (1-6C)alkyle ou (1-6C)alcoxy,
où les groupements (1-6C) alkyle et (1-6C) alcoxy sont éventuellement substitués par un ou plusieurs groupements choisis indépendamment parmi : fluoro, hydroxy, (1-6C)alkyle, (1-6C) alcoxy, amino, mono(1-6C)alkylamino, di-[(1-6C)alkyl]amino, carbamoyle, mono(1-6C)alkylcarbamoyle ou di-[(1-6C)alkyl]carbamoyle, un cycle hétérocyclique saturé ou partiellement saturé à 3 à 7 chaînons ou un cycle hétéroaryle à 5 ou 6 chaînons, où lesdits cycles hétérocyclique et hétéroaryle sont éventuellement indépendamment substitués par un ou plusieurs de ce qui suit :
(1-4C)alkyle, (1-4C)alcoxy, hydroxy, amino, mono(1-6C)alkylamino ou di-[(1-6C)alkyl]amino ou un cycle hétérocyclique saturé ou partiellement saturé à 3 à 7 chaînons ;
ou l'un de **R³** et **R⁴** est tel que défini ci-dessus et l'autre représente un groupement **-NR¹R²** tel que défini ci-dessus ;
**A** représente un groupement aryle ou un cycle hétéroaryle à 5 ou 6 chaînons choisi parmi furyle, pyrrolyle, thiényle, oxazolyle, isoxazolyle, imidazolyle, pyrazolyle, thiazolyle, isothiazolyle, oxadiazolyle, thiadiazolyle, triazolyle, tétrazolyle, pyridyle, pyridazinyle, pyrimidinyle, pyrazinyle ou 1,3,5-triazinyle ;
**R⁵** est choisi parmi cyclopropyle, cyano, halogéno, (1-6C)alcoxy ou (1-6C)alkyle, où les groupements (1-6C)alkyle et (1-6C)alcoxy sont éventuellement substitués par cyano ou par un ou plusieurs fluoro ;
**n** est 0, 1, 2 ou 3 ;
**L** est lié en méta ou para sur le cycle A par rapport au point de liaison du groupement éthynyle et représente -C(R^{a}R^{b})C(O)N(R⁹)-, -N (R⁸) C (O) C (R^{a}R^{b}) -, -N(R⁸)C(O)N(R⁹)-, -N(R⁸)C(O)O- ou -OC(O)-N(R⁹)- où R⁸ et R⁹ représentent indépendamment hydrogène ou (1-6C)alkyle et où R^{a} et R^{b} représentent indépendamment hydrogène ou (1-6C)alkyle ou R^{a} et R^{b} conjointement avec l'atome de carbone auquel ils sont liés représentent (3-6C)cycloalkyle ;
**B** représente un cycle (3-7C)cycloalkyle, un cycle hétérocyclique saturé ou partiellement saturé à 3 à 7 chaînons, un groupement aryle, un cycle hétéroaryle à 5 ou 6 chaînons choisi parmi furyle, pyrrolyle, thiényle, oxazolyle, isoxazolyle, imidazolyle, pyrazolyle, thiazolyle, isothiazolyle, oxadiazolyle, thiadiazolyle, triazolyle, tétrazolyle, pyridyle, pyridazinyle, pyrimidinyle, pyrazinyle ou 1,3,5-triazinyle, ou un groupement bicyclique à 8, 9 ou 10 chaînons qui contient éventuellement 1, 2, 3 ou 4 hétéroatomes choisis indépendamment parmi N, 0 et S et qui est saturé, partiellement saturé ou aromatique ;
**R⁶** est choisi parmi halogéno, cyano, oxo, un cycle (3-7C)cycloalkyle, un cycle hétérocyclique saturé ou partiellement saturé à 3 à 7 chaînons et - N(R^{c})C(O)(1-6C) alkyle dans lequel R^{c} est hydrogène ou (1-6C)alkyle ; ou
**R⁶** est choisi parmi (1-6C)alkyle, -S(O)ₚ-(1-6C)alkyle où p est 0, 1 ou 2, ou (1-6C)alcoxy, où les groupements (1-6C)alkyle, -S(O)ₚ-(1-6C)alkyle et (1-6C)alcoxy sont éventuellement substitués par un ou plusieurs groupements choisis indépendamment parmi cyano, fluoro, hydroxy, (1-6C)alcoxy, amino, mono(1-6C)alkylamino, di-[(1-6C)alkyl]amino, un cycle (3-7C)cycloalkyle ou un cycle hétérocyclique saturé ou partiellement saturé à 3 à 7 chaînons ; et
où le cycle (3-7C)cycloalkyle et le cycle hétérocyclique saturé ou partiellement saturé à 3 à 7 chaînons sont éventuellement indépendamment substitués par un ou plusieurs groupements choisis parmi (1-6C)alkyle ; et
**m** est 0, 1, 2 ou 3 ;
et lorsque B est un cycle (3-7C)cycloalkyle, un cycle hétérocyclique saturé ou partiellement saturé à 3 à 7 chaînons ou un cycle bicyclique saturé ou partiellement saturé à 8, 9 ou 10 chaînons, les cycles et le groupement bicyclique portent éventuellement 1 ou 2 substituants oxo ou thioxo ;
et les sels de celui-ci.

2. Composé de formule I selon la revendication 1, **caractérisé en ce que** :
**R⁶** est choisi parmi halogéno, cyano, un cycle (3-7C)cycloalkyle, un cycle hétérocyclique saturé ou partiellement saturé à 3 à 7 chaînons ou un groupement alcanoylamino -N(R^{c})C(O)(1-6C)alkyle dans lequel R^{c} est hydrogène ou (1-6C)alkyle ; ou
**R⁶** est choisi parmi (1-6C)alkyle ou (1-6C)alcoxy, où les groupements (1-6C) alkyle et (1-6C)alcoxy sont éventuellement substitués par un ou plusieurs groupements choisis indépendamment parmi cyano, fluoro, hydroxy, (1-6C)alcoxy, amino, mono(1-6C)alkylamino, di-[(1-6C)alkyl]amino, un cycle (3-7C)cycloalkyle ou un cycle hétérocyclique saturé ou partiellement saturé à 3 à 7 chaînons ;
et les sels de celui-ci.

3. Composé de formule I selon la revendication 1, **caractérisé en ce que** :
**R¹ et R²** sont choisis indépendamment parmi hydrogène, (1-6C)alkylsulfonyle, phényl(CH₂)ᵤ₋ où u est 0, 1, 2, 3, 4, 5 ou 6, (1-6C)alcanoyle, (1-6C)alkyle, (1-6C)alcoxycarbonyle ou (3-6C)cycloalkyl(CH₂)ₓ- dans lequel x est 0, 1, 2, 3, 4, 5 ou 6 ou **R¹ et R²** conjointement avec l'atome d'azote auquel ils sont liés représentent un cycle hétérocyclique saturé ou partiellement saturé à 3 à 7 chaînons contenant éventuellement un autre hétéroatome choisi parmi N ou 0 ;
où les groupements alkyle et cycloalkyle sont éventuellement substitués par un ou plusieurs groupements choisis parmi fluoro, hydroxy, (1-6C)alkyle, (1-6C)alcoxy, amino, mono(1-6C)alkylamino ou di-[(1-6C)alkyl]amino, un cycle hétérocyclique saturé ou partiellement saturé à 3 à 7 chaînons ou un cycle hétéroaryle à 5 ou 6 chaînons, où lesdits cycles hétérocyclique et hétéroaryle sont éventuellement indépendamment substitués par un ou plusieurs de ce qui suit : (1-4C)alkyle, hydroxy, amino, mono(1-6C)alkylamino ou di-[(1-6C)alkyl]amino ou un cycle hétérocyclique saturé ou partiellement saturé à 3 à 7 chaînons ;
et où le phényle est éventuellement substitué par un ou plusieurs groupements choisis parmi halogéno, (1-6C)alkyle, (1-6C)alcoxy, amino, mono(1-6C)alkylamino ou di-[(1-6C)alkyl]amino, où le (1-6C)alkyle ou le (1-6C)alcoxy sont éventuellement substitués par hydroxy, amino, mono(1-6C)alkylamino ou di-[(1-6C)alkyl]amino ;
**R³ et R⁴** sont choisis indépendamment parmi hydrogène, (1-6C)alkyle ou (1-6C)alcoxy où les groupements alkyle et alcoxy sont éventuellement substitués par un ou plusieurs groupements choisis parmi fluoro, hydroxy, (1-6C)alkyle, (1-6C)alcoxy, amino, mono(1-6C)alkylamino ou di-[(1-6C)alkyl]amino, un cycle hétérocyclique saturé ou partiellement saturé à 3 à 7 chaînons ou un cycle hétéroaryle à 5 ou 6 chaînons, où lesdits cycles hétérocyclique et hétéroaryle sont éventuellement indépendamment substitués par un ou plusieurs de ce qui suit : (1-4C)alkyle, hydroxy, amino, mono(1-6C)alkylamino ou di-[(1-6C)alkyl]amino ou un cycle hétérocyclique saturé ou partiellement saturé à 3 à 7 chaînons ;
ou l'un de **R³** et **R⁴** est tel que défini ci-dessus et l'autre représente un groupement **-NR¹R²** tel que défini ci-dessus ;
**R⁵** est choisi parmi cyano, halogéno, (1-6C)alcoxy ou (1-6C)alkyle éventuellement substitués par cyano ou par un ou plusieurs fluoro ;
**B** représente un cycle (3-7C)cycloalkyle, un aryle ou un cycle hétéroaryle à 5 ou 6 chaînons choisi parmi furyle, pyrrolyle, thiényle, oxazolyle, isoxazolyle, imidazolyle, pyrazolyle, thiazolyle, isothiazolyle, oxadiazolyle, thiadiazolyle, triazolyle, tétrazolyle, pyridyle, pyridazinyle, pyrimidinyle, pyrazinyle ou 1,3,5-triazinyle ;
**R⁶** est choisi parmi halogéno, cyano, un cycle hétérocyclique saturé ou partiellement saturé à 3 à 7 chaînons ou un groupement alcanoylamino -N(R^{c})C(O)(1-6C)alkyle dans lequel R^{c} est hydrogène ou (1-6C)alkyle ; ou **R⁶** est choisi parmi (1-6C)alkyle ou (1-6C) alcoxy, où les groupements alkyle et alcoxy sont éventuellement substitués par un ou plusieurs groupements choisis parmi cyano, fluoro, hydroxy, (1-6C)alcoxy, amino, mono(1-6C)alkylamino, di-[(1-6C)alkyl]amino ou un cycle hétérocyclique saturé ou partiellement saturé à 3 à 7 chaînons ; et
**m est 0, 1, 2 ou 3 ;** et lorsque m est au moins 2, alors deux substituants sur des atomes de carbone adjacents dans le cycle B peuvent représenter ensemble un groupement méthylènedioxy ;
et où A, L et n sont tels que définis dans la revendication 1,
et les sels de celui-ci.

4. Composé selon l'une quelconque des revendications 1, 2 et 3, **caractérisé en ce que** A est choisi parmi phényle, pyridyle, thiazolyle, thiadiazolyle ou pyrimidinyle.

5. Composé selon l'une quelconque des revendications précédentes, **caractérisé en ce que** B est choisi parmi phényle, 2,3-di-hydro-indényle, pipéridinyle, pyridyle, pyrazolyle, isothiazolyle, thiadiazolyle, isoxazolyle, benzodioxinyle, benzodioxolyle ou tétrahydropyranyle.

6. Composé selon l'une quelconque des revendications précédentes, **caractérisé en ce que** L est choisi parmi -N(R⁸)C(O)N(R⁹)-, -N(R⁸)C(O)O- ou - N(R⁸)C(O)CH₂- où R⁸ et R⁹ représentent indépendamment hydrogène ou (1-6C)alkyle.

7. Composé selon l'une quelconque des revendications précédentes, **caractérisé en ce que** R¹ et R² sont tous deux hydrogène ou R¹ est hydrogène ou (1-6C)alkyle et R² est (1-6C)alkyle
où (1-6C)alkyle est éventuellement substitué par hydroxy, amino, mono(1-6C)alkylamino ou di(1-6C)alkylamino, carbamoyle, (1-6C)alcoxy, (1-6C) alcoxy (1-6C) alcoxy, -N(R^{d})C(O)(1-6C) alkyle dans lequel R^{d} est hydrogène ou (1-6C)alkyle, aryle (particulièrement phényle), un cycle hétérocyclique saturé ou partiellement saturé à 3 à 7 chaînons ou un cycle hétéroaryle à 5 ou 6 chaînons ;
où les groupements (1-6C) alcoxy, mono(1-6C)alkylamino et -N(R^{d})C(O)(1-6C) alkyle sont éventuellement substitués par hydroxy ;
où un cycle aryle, un cycle hétérocyclique saturé ou partiellement saturé à 3 à 7 chaînons ou un cycle hétéroaryle à 5 ou 6 chaînons est éventuellement substitué par (1-4C)alkyle, (1-4C)alcoxy ou -C(O)CH₂Y où Y est choisi parmi hydroxy ou di- (1-6C) alkylamino.

8. Composé selon l'une quelconque des revendications précédentes, **caractérisé en ce que** R³ et R⁴ sont tous deux hydrogène.

9. Composé selon l'une quelconque des revendications précédentes, **caractérisé en ce que** R⁶ est choisi indépendamment parmi halogéno, cyano, oxo, (3-7C)cycloalkyle, un cycle hétérocyclique saturé à 3 à 7 chaînons (éventuellement substitué par (1-4C)alkyle), -N(R^{c})C(O) (1-6C) alkyle où R^{c} est hydrogène ou (1-6C)alkyle (particulièrement (1-4C)alkyle), (1-6C)alkyle (éventuellement substitué par jusqu'à trois groupements choisis indépendamment parmi halogéno) ou (1-6C)alcoxy et m est choisi parmi 1 ou 2.

10. Composé selon la revendication 1, choisi parmi :
la N-{3-[(2-aminopyrimidin-5-yl)éthynyl]phényl}-N'-[2-fluoro-5-(trifluorométhyl)phényl]urée
la N-{3-[(2-aminopyrimidin-5-yl)éthynyl]phényl}-N'-[2-(trifluorométhyl)phényl]urée
la N-{3-[(2-aminopyrimidin-5-yl)éthynyl]phényl}-N'-[4-(trifluorométhyl)phényl]urée
la N-{3-[(2-aminopyrimidin-5-yl)éthynyl]phényl}-N'-(2-fluorophényl)urée
la N-{3-[(2-aminopyrimidin-5-yl)éthynyl]phényl}-N'-(3-fluorophényl)urée
la N-{3-[(2-aminopyrimidin-5-yl)éthynyl]phényl}-N'-(4-fluorophényl)urée
la N-{3-[(2-aminopyrimidin-5-yl)éthynyl]phényl}-N'-(3-méthoxyphényl)urée
la N-{3-[(2-aminopyrimidin-5-yl)éthynyl]phényl}-N'-(2,5-difluorophényl)urée
la N-{3-[(2-aminopyrimidin-5-yl)éthynyl]phényl}-N'-1,3-benzodioxol-5-ylurée
la N-{3-[(2-aminopyrimidin-5-yl)éthynyl]phényl}-N'-[3-(trifluorométhyl)phényl]urée
la N-{3-[(2-aminopyrimidin-5-yl)éthynyl]phényl}-N'-(2-méthoxyphényl)urée
la N-{3-[(2-aminopyrimidin-5-yl)éthynyl]phényl}-N'-(4-méthoxyphényl)urée
la N-{3-[(2-aminopyrimidin-5-yl)éthynyl]phényl}-N'-(3,4-difluorophényl)urée
la N-{3-[(2-aminopyrimidin-5-yl)éthynyl]phényl}-N'-(3-cyanophényl)urée
la N-{3-[(2-aminopyrimidin-5-yl)éthynyl]phényl}-N'-(3-chlorophényl)urée
la N-{3-[(2-aminopyrimidin-5-yl)éthynyl]phényl}-N'-cyclopentylurée
la N-{3-[(2-aminopyrimidin-5-yl)éthynyl]phényl}-N'-(3,5-difluorophényl)urée
la N-{3-[(2-aminopyrimidin-5-yl)éthynyl]phényl}-N'-(5-tert-butyl-1,3,4-thiadiazol-2-yl)urée
la N-{3-[(2-aminopyrimidin-5-yl)éthynyl]phényl}-N'-(3-méthylisoxazol-5-yl)urée
le N-(3-{[({3-[(2-aminopyrimidin-5-yl)éthynyl]phényl}amino)carbonyl]amino}phényl)acétamide
la N-{3-[(2-aminopyrimidin-5-yl)éthynyl]phényl}-N'-[4-(trifluorométhyl)pyridin-2-yl]urée
la N-{3-[(2-aminopyrimidin-5-yl)éthynyl]phényl}-N'-(5-tert-butylisoxazol-3-yl)urée
le {3-[(2-aminopyrimidin-5-yl)éthynyl]phényl}carbamate de phényle
la N-{3-[(2-aminopyrimidin-5-yl)éthynyl]phényl}-N'-(2-oxopipéridin-3-yl)urée
la N-(5-tert-butylisoxazol-3-yl)-N'-(3-{[2-(méthylamino)pyrimidin-5-yl]éthynyl}phényl)urée
la N-(5-tert-butylisoxazol-3-yl)-N'-(3-{[2-(diméthylamino)pyrimidin-5-yl]éthynyl}phényl)urée
la N-(5-tert-butylisoxazol-3-yl)-N'-[3-({2-[(2-morpholin-4-yléthyl)amino]pyrimidin-5-yl}éthynyl)phényl]urée
la N-(5-tert-butylisoxazol-3-yl)-N'-[3-({2-[(3-morpholin-4-ylpropyl)amino]pyrimidin-5-yl}éthynyl)phényl]urée
la N-(5-tert-butylisoxazol-3-yl)-N'-[3-({2-[(2-méthoxyéthyl)amino]pyrimidin-5-yl}éthynyl)phényl]urée
la N-(5-tert-butylisoxazol-3-yl)-N'-{3-[(2-{[3-(1H-imidazol-1-yl)propyl]amino}pyrimidin-5-yl)éthynyl]phényl}urée
la N-(5-tert-butylisoxazol-3-yl)-N'-[3-({2-[(3-méthoxypropyl)amino]pyrimidin-5-yl}éthynyl)phényl]urée
la N-(5-tert-butylisoxazol-3-yl)-N'-[3-({2-[(2-hydroxyéthyl)amino]pyrimidin-5-yl}éthynyl)phényl]urée
la N-(5-tert-butylisoxazol-3-yl)-N'-[3-({2-[(2-pyrrolidin-1-yléthyl)amino]pyrimidin-5-yl}éthynyl)phényl]urée
la N-(5-tert-butylisoxazol-3-yl)-N'-[3-({2-[(3-pyrrolidin-1-ylpropyl)amino]pyrimidin-5-yl}éthynyl)phényl]urée
la N-[3-({2-[(2-aminoéthyl)amino]pyrimidin-5-yl}éthynyl)phényl]-N'-(5-tert-butylisoxazol-3-yl)urée
la N-[3-({2-[(3-aminopropyl)amino]pyrimidin-5-yl}éthynyl)phényl]-N'-(5-tert-butylisoxazol-3-yl)urée
la N-(5-tert-butylisoxazol-3-yl)-N'-{3-[(2-{[2-(diméthylamino)éthyl]amino}pyrimidin-5-yl)éthynyl]phényl}urée
la N-(5-tert-butylisoxazol-3-yl)-N'-{3-[(2-{[3-(diméthylamino)propyl]amino}pyrimidin-5-yl)éthynyl]phényl}urée
le N-2-(5-{[3-({[(5-tert-butylisoxazol-3-yl)amino]carbonyl}amino)phényl]éthynyl}pyrimidin-2-yl)glycinamide
le N-3-(5-{[3-({[(5-tert-butylisoxazol-3-yl)amino]carbonyl}amino)phényl]éthynyl}pyrimidin-2-yl)-bêta-alaninamide
la N-(5-tert-butylisoxazol-3-yl)-N'-{3-[(2-{[2-(1H-imidazol-4-yl)éthyl]amino}pyrimidin-5-yl)éthynyl]phényl}urée
la N-(5-tert-butylisoxazol-3-yl)-N'-[3-({2-[(2-pyridin-2-yléthyl)amino]pyrimidin-5-yl}éthynyl)phényl]urée
la N-(5-tert-butylisoxazol-3-yl)-N'-{3-[(2-{[3-(isopropylamino)propyl]amino}pyrimidin-5-yl)éthynyl]phényl}urée
la N-(5-tert-butylisoxazol-3-yl)-N'-{3-[(2-{[3-(4-méthylpipérazin-1-yl)propyl]amino}pyrimidin-5-yl)éthynyl]phényl}urée
la N-(5-tert-butylisoxazol-3-yl)-N'-[3-({2-[(2-pyridin-4-yléthyl)amino]pyrimidin-5-yl}éthynyl)phényl]urée
la N-(5-tert-butylisoxazol-3-yl)-N'-[3-({2-[(3-pipéridin-1-ylpropyl)amino]pyrimidin-5-yl}éthynyl)phényl]urée
la N-(5-méthylisoxazol-3-yl)-N'-[3-({2-[(2-pyrrolidin-1-yléthyl)amino]pyrimidin-5-yl}éthynyl)phényl]urée
la N-(5-tert-butyl-1,3,4-thiadiazol-2-yl)-N'-[3-({2-[(2-pyrrolidin-1-yléthyl)amino]pyrimidin-5-yl}éthynyl)phényl]urée
la N-(3-méthylisothiazol-5-yl)-N'-[3-({2-[(2-pyrrolidin-1-yléthyl)amino]pyrimidin-5-yl}éthynyl)phényl]urée
la N-(3-fluorophényl)-N'-[3-({2-[(2-pyrrolidin-1-yléthyl)amino]pyrimidin-5-yl}éthynyl)phényl]urée
la N-(4-méthoxyphényl)-N'-[3-({2-[(2-pyrrolidin-1-yléthyl)amino]pyrimidin-5-yl}éthynyl)phényl]urée
la N-(2-fluorophényl)-N'-[3-({2-[(2-pyrrolidin-1-yléthyl)amino]pyrimidin-5-yl}éthynyl)phényl]urée
la N-(2,5-difluorophényl)-N'-[3-({2-[(2-pyrrolidin-1-yléthyl)amino]pyrimidin-5-yl}éthynyl)phényl]urée
la N-(3,4-difluorophényl)-N'-[3-({2-[(2-pyrrolidin-1-yléthyl)amino]pyrimidin-5-yl}éthynyl)phényl]urée
la N-[2-fluoro-5-(trifluorométhyl)phényl]-N'-[3-({2-[(2-pyrrolidin-1-yléthyl)amino]pyrimidin-5-yl}éthynyl)phényl]urée
la N-[3-({2-[(2-pyrrolidin-1-yléthyl)amino]pyrimidin-5-yl}éthynyl)phényl]-N'-[4-(trifluorométhyl)phényl]urée
la N-1,3-benzodioxol-5-yl-N'-[3-({2-[(2-pyrrolidin-1-yléthyl)amino]pyrimidin-5-yl}éthynyl)phényl]urée
la N-(4-fluorophényl)-N'-[3-({2-[(2-pyrrolidin-1-yléthyl)amino]pyrimidin-5-yl}éthynyl)phényl]urée
la N-(3-chlorophényl)-N'-[3-({2-[(2-pyrrolidin-1-yléthyl)amino]pyrimidin-5-yl}éthynyl)phényl]urée
la N-(5-méthylisoxazol-3-yl)-N'-[3-({2-[(2-morpholin-4-yléthyl)amino]pyrimidin-5-yl}éthynyl)phényl]urée
la N-(5-tert-butyl-1,3,4-thiadiazol-2-yl)-N'-[3-({2-[(2-morpholin-4-yléthyl)amino]pyrimidin-5-yl}éthynyl)phényl]urée
la N-[2-fluoro-5-(trifluorométhyl)phényl]-N'-[3-({2-[(2-morpholin-4-yléthyl)amino]pyrimidin-5-yl}éthynyl)phényl]urée
la N-(5-méthylisoxazol-3-yl)-N'-[3-({2-[(3-morpholin-4-ylpropyl)amino]pyrimidin-5-yl}éthynyl)phényl]urée
la N-(5-tert-butyl-1,3,4-thiadiazol-2-yl)-N'-[3-({2-[(3-morpholin-4-ylpropyl)amino]pyrimidin-5-yl}éthynyl)phényl]urée
la N-[2-fluoro-5-(trifluorométhyl)phényl]-N'-[3-({2-[(3-morpholin-4-ylpropyl)amino]pyrimidin-5-yl}éthynyl)phényl]urée
la N-(5-méthylisoxazol-3-yl)-N'-[4-({2-[(2-pyrrolidin-1-yléthyl)amino]pyrimidin-5-yl}éthynyl)phényl]urée
la N-(5-tert-butylisoxazol-3-yl)-N'-[4-({2-[(2-pyrrolidin-1-yléthyl)amino]pyrimidin-5-yl}éthynyl)phényl]urée
la N-(5-tert-butyl-1,3,4-thiadiazol-2-yl)-N'-[4-({2-[(2-pyrrolidin-1-yléthyl)amino]pyrimidin-5-yl}éthynyl)phényl]urée
la N-[2-fluoro-5-(trifluorométhyl)phényl]-N'-[4-({2-[(2-pyrrolidin-1-yléthyl)amino]pyrimidin-5-yl}éthynyl)phényl]urée
le N-(5-{[3-({[(5-tert-butylisoxazol-3-yl)amino]carbonyl}amino)phényl]éthynyl}pyrimidin-2-yl)-2-(2-méthoxyéthoxy)acétamide
la N-{6-[(2-aminopyrimidin-5-yl)éthynyl]pyridin-2-yl}-N'-(5-tert-butylisoxazol-3-yl)urée
la N-{2-[(2-aminopyrimidin-5-yl)éthynyl]pyridin-4-yl}-N'-(5-tert-butylisoxazol-3-yl)urée
la N-{5-[(2-aminopyrimidin-5-yl)éthynyl]-1,3-thiazol-2-yl}-N'-[2-fluoro-5-(trifluorométhyl)phényl]urée
la N-{5-[(2-aminopyrimidin-5-yl)éthynyl]-1,3,4-thiadiazol-2-yl}-N'-[2-fluoro-5-(trifluorométhyl)phényl]urée
la N-{5-[(2-aminopyrimidin-5-yl)éthynyl]-1,3-thiazol-2-yl}-N'-(5-tert-butylisoxazol-3-yl)urée
le N-{3-[(2-aminopyrimidin-5-yl)éthynyl]phényl}-2-(2-méthoxyphényl)acétamide
le N-{3-[(2-aminopyrimidin-5-yl)éthynyl]phényl}-2-phénylacétamide
le N-{3-[(2-aminopyrimidin-5-yl)éthynyl]phényl}-2-(3-méthoxyphényl)acétamide
le N-{3-[(2-aminopyrimidin-5-yl)éthynyl]phényl}-2-[3-(trifluorométhyl)phényl]acétamide
le N-{3-[(2-aminopyrimidin-5-yl)éthynyl]phényl}-2-[4-(trifluorométhyl)phényl]acétamide
le N-{3-[(2-aminopyrimidin-5-yl)éthynyl]phényl}-2-(3-méthylisoxazol-5-yl)acétamide
le N-{4-[(2-aminopyrimidin-5-yl)éthynyl]phényl}-2-(2-méthoxyphényl)acétamide
le N-{4-[(2-aminopyrimidin-5-yl)éthynyl]phényl}-2-(3-méthylisoxazol-5-yl)acétamide
la N-{3-[(2-aminopyrimidin-5-yl)éthynyl]phényl}-N'-(2,2-diméthyltétrahydro-2H-pyran-4-yl)urée
la N-{6-[(2-aminopyrimidin-5-yl)éthynyl]pyrimidin-4-yl}-N'-(5-tert-butylisoxazol-3-yl)urée
la N'-{3-[(2-aminopyrimidin-5-yl)éthynyl]phényl}-N-(5-tert-butylisoxazol-3-yl)-N-méthylurée
la N-{3-[(2-aminopyrimidin-5-yl)éthynyl]phényl}-N'-(1-tert-butyl-3-cyclopropyl-1H-pyrazol-5-yl)urée
la N-{3-[(2-aminopyrimidin-5-yl)éthynyl]phényl}-N'-(5-méthylisoxazol-3-yl)urée
la N-{5-[(2-aminopyrimidin-5-yl)éthynyl]pyridin-3-yl}-N'-(5-tert-butylisoxazol-3-yl)urée
la N-[3-({2-[(3-pipéridin-1-ylpropyl)amino]pyrimidin-5-yl}éthynyl)phényl]-N'-[4-(trifluorométhyl)pyridin-2-yl]urée
la N-(5-tert-butyl-1,3,4-thiadiazol-2-yl)-N'-[3-({2-[(3-pipéridin-1-ylpropyl)amino]pyrimidin-5-yl}éthynyl)phényl]urée
la N-(3-méthylisoxazol-5-yl)-N'-[3-({2-[(3-pipéridin-1-ylpropyl)amino]pyrimidin-5-yl}éthynyl)phényl]urée
la N-(2-méthoxyphényl)-N'-[3-({2-[(3-pipéridin-1-ylpropyl)amino]pyrimidin-5-yl}éthynyl)phényl]urée
la N-(3-fluorophényl)-N'-[3-({2-[(3-pipéridin-1-ylpropyl)amino]pyrimidin-5-yl}éthynyl)phényl]urée
la N'-{4-[(2-aminopyrimidin-5-yl)éthynyl]phényl}-N-(5-tert-butylisoxazol-3-yl)-N-méthylurée
la N-{3-[(2-aminopyrimidin-5-yl)éthynyl]phényl}-N'-(3-cyclopropyl-l-méthyl-1H-pyrazol-5-yl)urée
la N-{3-[(2-aminopyrimidin-5-yl)éthynyl]phényl}-N'-phénylurée
la N-[3-({2-[(4-aminobutyl)amino]pyrimidin-5-yl}éthynyl)phényl]-N'-(5-tert-butylisoxazol-3-yl)urée
la N-(5-tert-butylisoxazol-3-yl)-N'-[3-({2-{(2-pipéridin-1-yléthyl)amino]pyrimidin-5-yl}éthynyl)phényl]urée
la N-(5-tert-butylisoxazol-3-yl)-N'-{3-[(2-{[2-(isopropylamino)éthyl]amino}pyrimidin-5-yl)éthynyl]phényl}urée
la N-(5-tert-butylisoxazol-3-yl)-N'-{3-[(2-{[2-(2-hydroxyéthoxy)éthyl]amino}pyrimidin-5-yl)éthynyl]phényl}urée
la N-{3-[(2-aminopyrimidin-5-yl)éthynyl]phényl}-N'-(5-cyclopropyl-1,3,4-thiadiazol-2-yl)urée
la N-{3-[(2-aminopyrimidin-5-yl)éthynyl]phényl}-N'-[5-(trifluorométhyl)-1,3,4-thiadiazol-2-yl]urée
le N-{2-[(5-{[3-({[(5-tert-butylisoxazol-3-yl)aminocarbonyl}amino)]phényl]éthynyl}pyrimidin-2-yl)amino]éthyl}-2-hydroxyacétamide
la N-(5-tert-butylisoxazol-3-yl)-N'-{3-[(2-{[4-(diméthylamino)butyl]amino}pyrimidin-5-yl)éthynyl]phényl}urée
la N'-{3-[(2-aminopyrimidin-5-yl)éthynyl]phényl}-N-méthyl-N-[5-(trifluorométhyl)-1,3,4-thiadiazol-2-yl]urée
la N-phényl-N'-[3-({2-[(3-pipéridin-1-ylpropyl)amino]pyrimidin-5-yl}éthynyl)phényl]urée
la N-(5-méthylisoxazol-3-yl)-N'-[3-({2-[(3-pipéridin-1-ylpropyl)amino]pyrimidin-5-yl}éthynyl)phényl]urée
la N-{3-[(2-aminopyrimidin-5-yl)éthynyl]phényl}-N'-(5-tert-butylisoxazol-3-yl)-N-méthylurée
la N-(5-tert-butylisoxazol-3-yl)-N'-{3-[(2-{[2-(diméthylamino)-1-méthyléthyl]amino}pyrimidin-5-yl)éthynyl]phényl}urée
le {3-[(2-{[3-(diméthylamino)propyl]amino}pyrimidin-5-yl)éthynyl]phényl}carbamate de phényle
la N-{3-[(2-aminopyrimidin-5-yl)éthynyl]phényl}-N'-(3-tert-butyl-1-méthyl-1H-pyrazol-5-yl)urée
la N-{3-[(2-aminopyrimidin-5-yl)éthynyl]phényl}-N'-(4-tert-butyl-1,3-thiazol-2-yl)urée
la N-{3-[(2-aminopyrimidin-5-yl)éthynyl]phényl}-N'-(5-méthyl-1,3,4-thiadiazol-2-yl)urée
la N-{3-[(2-aminopyrimidin-5-yl)éthynyl]phényl}-N'-(5-éthyl-1,3,4-thiadiazol-2-yl)urée
la N-{3-[(2-aminopyrimidin-5-yl)éthynyl]phényl}-N'-(5-isopropyl-1,3,4-thiadiazol-2-yl)urée
la N-{3-[(2-{[3-(diméthylamino)propyl]amino}pyrimidin-5-yl)éthynyl]phényl}-N'-(5-méthylisoxazol-3-yl)urée
la N-{3-[(2-{[3-(diméthylamino)propyl]amino}pyrimidin-5-yl)éthynyl]phényl}-N'-phénylurée
la N-{5-[(2-aminopyrimidin-5-yl)éthynyl]pyridin-3-yl}-N'-(5-tert-butylisoxazol-3-yl)urée ;
la N-(3-tert-butyl-1-méthyl-1H-pyrazol-5-yl)-N'-[3-({2-[(3-morpholin-4-ylpropyl)amino]pyrimidin-5-yl}éthynyl)phényl]urée ;
la N-(3-tert-butyl-1-méthyl-1H-pyrazol-5-yl)-N'-[3-({2-[(2-morpholin-4-yléthyl)amino]pyrimidin-5-yl}éthynyl)phényl]urée ;
la N-(3-tert-butyl-1-méthyl-1H-pyrazol-5-yl)-N'-[3-({2-[(3-pipéridin-1-ylpropyl)amino]pyrimidin-5-yl}éthynyl)phényl]urée ;
la N-(5-tert-butylisoxazol-3-yl)-N'-[3-({2-[(1-méthyl-2-morpholin-4-yléthyl)amino]pyrimidin-5-yl}éthynyl)phényl]urée ;
la N-(5-tert-butylisoxazol-3-yl)-N'-{3-[(2-{[(1-glycoloylpyrrolidin-2-yl)méthyl]amino}pyrimidin-5-yl)éthynyl]phényl}urée ;
la N-(5-tert-butylisoxazol-3-yl)-N'-(3-{[2-({[1-(N,N-diméthylglycyl)pyrrolidin-2-yl]méthyl}amino)pyrimidin-5-yl]éthynyl}phényl)urée ;
la N-(5-tert-butylisoxazol-3-yl)-N'-[3-({2-[(2-pipérazin-1-yléthyl)amino]pyrimidin-5-yl}éthynyl)phényl]urée ;
la N-(5-tert-butylisoxazol-3-yl)-N'-[3-({2-[(3-pipérazin-1-ylpropyl)amino]pyrimidin-5-yl}éthynyl)phényl]urée ;
la N-(5-tert-butylisoxazol-3-yl)-N-méthyl-N'-[3-({2-[(2-morpholin-4-yléthyl)amino]pyrimidin-5-yl}éthynyl)phényl]urée ;
la N-(5-tert-butylisoxazol-3-yl)-N-méthyl-N'-[3-({2-[(3-morpholin-4-ylpropyl)amino]pyrimidin-5-yl}éthynyl)phényl]urée ;
la N-(5-tert-butylisoxazol-3-yl)-N-méthyl-N'-[3-({2-[(3-pipéridin-1-ylpropyl)amino]pyrimidin-5-yl}éthynyl)phényl]urée ;
la N-(3-tert-butyl-1-méthyl-1H-pyrazol-5-yl)-N'-[3-({2-[(3-pipéridin-1-ylpropyl)amino]pyrimidin-5-yl}éthynyl)phényl]urée ;
la N-(3-tert-butyl-1-méthyl-1H-pyrazol-5-yl)-N'-{3-[(2-{[2-(diméthylamino)éthyl]amino}pyrimidin-5-yl)éthynyl]phényl}urée ;
la N-(3-tert-butyl-1-méthyl-1H-pyrazol-5-yl)-N'-{3-[(2-{[2-(isopropylamino)éthyl]amino}pyrimidin-5-yl)éthynyl]phényl}urée ;
la N-(3-cyclopropyl-l-méthyl-1H-pyrazol-5-yl)-N'-[3-({2-[(3-pipéridin-1-ylpropyl)amino]pyrimidin-5-yl}éthynyl)phényl]urée ;
la N-(3-cyclopropyl-l-méthyl-1H-pyrazol-5-yl)-N'-[3-({2-[(3-morpholin-4-ylpropyl)amino]pyrimidin-5-yl}éthynyl)phényl]urée ;
la N-(3-cyclopropyl-1-méthyl-1H-pyrazol-5-yl)-N'-[3-({2-[(2-morpholin-4-yléthyl)amino]pyrimidin-5-yl}éthynyl)phényl]urée ;
la N-(5-tert-butylisoxazol-3-yl)-N'-(3-{[2-({2-[(2-hydroxy-1-oxo-éthyl)amino]éthyl}amino)-pyrimidin-5-yl]éthynyl}phényl)urée ;
la N-(5-tert-butylisoxazol-3-yl)-N'-(3-{[2-({3-[(2-hydroxyéthyl)amino]propyl}amino)pyrimidin-5-yl]éthynyl}phényl)urée ;
la N-(5-tert-butylisoxazol-3-yl)-N'-{3-[(2-{[3-(diméthylamino)propyl]amino}pyrimidin-5-yl)éthynyl]phényl}-N-méthylurée ;
la N-{5-[(2-aminopyrimidin-5-yl)éthynyl]pyridin-3-yl}-N'-(3-tert-butyl-1-méthyl-1H-pyrazol-5-yl)urée ;
la N-{5-[(2-aminopyrimidin-5-yl)éthynyl]pyridin-3-yl}-N'-(3-cyclopropyl-1-méthyl-1H-pyrazol-5-yl)urée ;
la N-{5-[(2-aminopyrimidin-5-yl)éthynyl]-1,3-thiazol-2-yl}-N'-phénylurée ;
la N-{5-[(2-aminopyrimidin-5-yl)éthynyl]-1,3-thiazol-2-yl}-N'-(2,2-diméthyltétrahydro-2H-pyran-4-yl)urée ;
la N-{5-[(2-aminopyrimidin-5-yl)éthynyl]-1,3-thiazol-2-yl}-N'-(3-cyclopropyl-1-méthyl-1H-pyrazol-5-yl)urée ;
la N-{5-[(2-aminopyrimidin-5-yl)éthynyl]-1,3-thiazol-2-yl}-N'-(3-tert-butyl-1-méthyl-1H-pyrazol-5-yl)urée ;
la N-{5-[(2-aminopyrimidin-5-yl)éthynyl]-1,3,4-thiadiazol-2-yl}-N'-phénylurée ;
la N-{5-[(2-aminopyrimidin-5-yl)éthynyl]-1,3,4-thiadiazol-2-yl}-N'-(2,2-diméthyltétrahydro-2H-pyran-4-yl)urée ;
la N-{3-[(2-aminopyrimidin-5-yl)éthynyl]phényl}-N'-(1,3-diméthyl-1H-pyrazol-5-yl)urée ;
la N-{3-[(2-aminopyrimidin-5-yl)éthynyl]phényl}-N'-[5-(éthylthio)-1,3,4-thiadiazol-2-yl]urée ;
la N-{3-[(2-aminopyrimidin-5-yl)éthynyl]phényl}-N'-(1-tert-butyl-1H-pyrazol-4-yl)urée ;
la N-{3-[(2-aminopyrimidin-5-yl)éthynyl]phényl}-N'-(3-isopropyl-1-méthyl-1H-pyrazol-5-yl)urée ;
la N-{3-[(2-aminopyrimidin-5-yl)éthynyl]phényl}-N'-(5-isopropyl-1,3,4-oxadiazol-2-yl)urée ;
la N-{3-[(2-aminopyrimidin-5-yl)éthynyl]phényl}-N'-(1-éthyl-1H-pyrazol-3-yl)urée ;
la N-{3-[(2-aminopyrimidin-5-yl)éthynyl]phényl}-N'-(1-isopropyl-1H-pyrazol-3-yl)urée ;
la N-{3-[(2-aminopyrimidin-5-yl)éthynyl]phényl}-N'-[3-fluoro-5-(4-méthylpipérazin-1-yl)phényl]urée ou
la *N*-{3-[(2-aminopyrimidin-5-yl)éthynyl]-4-méthylphényl}-*N'*-(3-*tert*-butyl-1-méthyl-1*H-*pyrazol-5-yl)urée.

11. Composition pharmaceutique, **caractérisée en ce qu'**elle comprend un composé de formule I, ou un sel pharmaceutiquement acceptable de celui-ci, tel que défini dans les revendications 1 à 10 en association avec un diluant ou un support pharmaceutiquement acceptable.

12. Composé de formule I, ou un sel pharmaceutiquement acceptable de celui-ci, tel que défini dans les revendications 1 à 10, destiné à être utilisé comme médicament.

13. Utilisation d'un composé de formule I, ou d'un sel pharmaceutiquement acceptable de celui-ci, tel que défini dans les revendications 1 à 10, dans la fabrication d'un médicament destiné à être utilisé comme inhibiteur du récepteur tyrosine-kinase Tie2 chez un animal à sang chaud tel que l'homme.

14. Utilisation d'un composé de formule I ou d'un sel pharmaceutiquement acceptable de celui-ci, tel que défini dans les revendications 1 à 10, dans la fabrication d'un médicament destiné à être utilisé dans la production d'un effet anti-angiogénique chez un animal à sang chaud tel que l'homme.

15. Procédé de préparation d'un composé de formule I, ou d'un sel de celui-ci, tel que défini dans la revendication 1, ou d'un sel pharmaceutiquement acceptable de celui-ci (où R¹, R², R³, R⁴, R⁵, R⁶, R⁷, R⁸, R⁹, R¹⁰, R¹¹, L, le cycle A et le cycle B, n et m sont, sauf précision contraire, tels que définis dans la revendication 1) comprenant :
(a) pour les composés de formule I dans laquelle L est -N(R⁸)C(O)N(H)-, la réaction d'un composé de formule II : 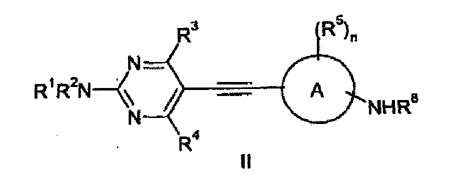 dans laquelle R¹, R², R³, R⁴, R⁵, R⁸, n et A ont l'une quelconque des significations définies précédemment sauf qu'une fonction quelconque est protégée le cas échéant, avec un isocyanate de formule IV _{:} 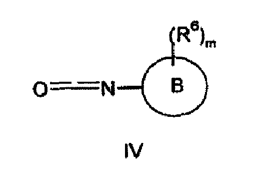 dans laquelle R⁶ m et B ont l'une quelconque des significations définies précédemment sauf qu'une fonction quelconque est protégée le cas échéant ; ou
(b) pour les composés de formule I dans laquelle L est -N(R⁸)C(O)N(H)-, la réaction d'un composé de formule II tel que défini ci-dessus avec un carbamate d'aryle de formule III _{:} 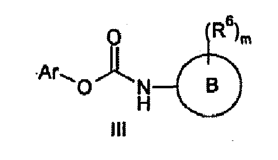 dans laquelle Ar est un groupement aryle convenable et R⁶, m et B ont l'une quelconque des significations définies précédemment sauf qu'une fonction quelconque est protégée le cas échéant ; ou
(c) pour les composés de formule I dans laquelle L est N(R⁸)C(O)-O-, la réaction d'un composé de formule II telle que définie ci-dessus avec un composé de formule XI : 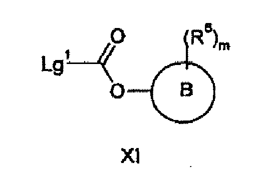 dans laquelle Lg¹ est un groupement déplaçable convenable et R⁶ m et B ont l'une quelconque des significations définies précédemment sauf qu'une fonction quelconque est protégée le cas échéant ; ou
(d) pour les composés de formule I dans laquelle L est N(RB)C(O)C (R^{a}R^{b}), la réaction d'un composé de formule II telle que définie ci-dessus avec un composé de formule IX _{:} 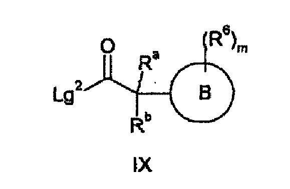 dans laquelle Lg² est un groupement déplaçable convenable, R^{x}-C(O)-O- ou R^{x}-O- (où R^{x} est un groupement alkyle ou aryle convenable) et R⁶, R^{a}, R^{b} m et B ont l'une quelconque des significations définies précédemment sauf qu'une fonction quelconque est protégée le cas échéant ; ou
(e) pour les composés de formule I dans laquelle L est -N(R⁸)C(O)N(H)-, la réaction d'un composé de formule II telle que définie ci-dessus avec une trichloroacétylamine de formule XIII: 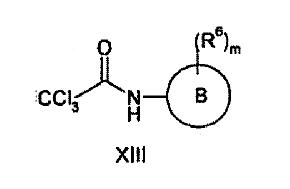 dans laquelle R⁶, m et B ont l'une quelconque des significations définies précédemment sauf qu'une fonction quelconque est protégée le cas échéant ; ou
(f) pour les composés de formule I dans laquelle L est -C(R^{a}R^{b})C(O)N(R⁹)-, la réaction d'un composé de formule XIV: 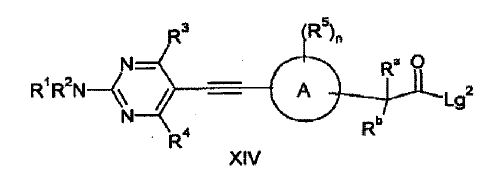 dans laquelle Lg² est un groupement déplaçable convenable tel que décrit ci-dessus et R¹, R², R³, R⁴, R⁵, R^{a}, R^{b}, n et A ont l'une quelconque des significations définies précédemment sauf qu'une fonction quelconque est protégée le cas échéant, avec une amine de formule XV: 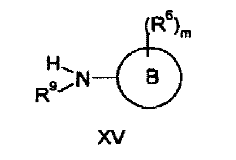 dans laquelle R⁶, R⁹, m et B ont l'une quelconque des significations définies précédemment sauf qu'une fonction quelconque est protégée le cas échéant ; ou
(g) la réaction d'un composé de formule XVI: 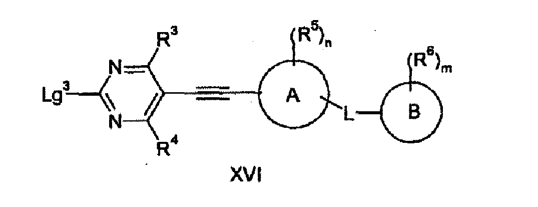 dans laquelle Lg³ est un groupement déplaçable convenable, méthylsulfonyle, méthylthio ou aryloxy et R³, R⁴ , R⁵ , R⁶, n, m, A, B et L ont l'une quelconque des significations définies précédemment sauf qu'une fonction quelconque est protégée le cas échéant, avec une amine de formule HNR¹R², dans laquelle R¹ et R² ont l'une quelconque des significations définies précédemment sauf qu'une fonction quelconque est protégée le cas échéant ; ou
(h) la réaction d'un composé de formule XVII : 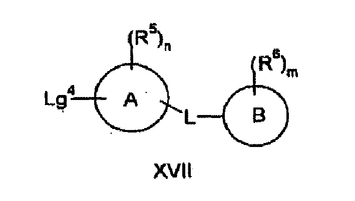 dans laquelle Lg⁴ est un groupement déplaçable convenable ou un groupement sulfonyloxy et R⁵, R⁶ n, m, A, B et L ont l'une quelconque des significations définies précédemment sauf qu'une fonction quelconque est protégée le cas échéant, avec un alcyne de formule XVIII _{:} 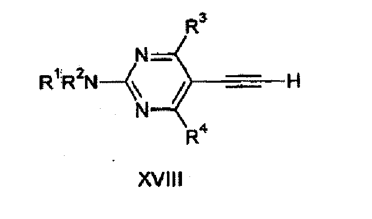 dans laquelle R¹, R², R³, et R⁴ ont l'une quelconque des significations définies précédemment sauf qu'une fonction quelconque est protégée le cas échéant ; ou
(i) pour les composés de formule I dans laquelle L est -N(H)C(O)N(R⁹)-, la réaction d'un isocyanate de formule XIX: 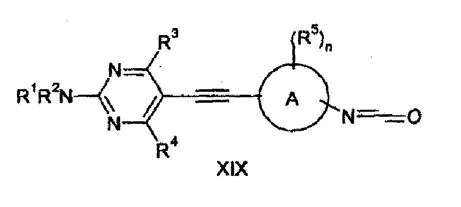 dans laquelle R¹, R², R³, R⁴, R⁵, n et A ont l'une quelconque des significations définies précédemment sauf qu'une fonction quelconque est protégée le cas échéant, avec une amine de formule XV telle que définie ci-dessus ; ou
(j) pour les composés de formule I dans laquelle L est -N(H)C(O)N(R⁹)-, la réaction d'un composé de formule XX : 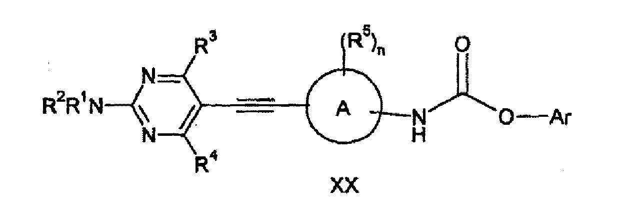 dans laquelle Ar est un groupement aryle convenable et R¹, R², R³, R⁴, R⁵, n et A ont l'une quelconque des significations définies précédemment sauf qu'une fonction quelconque est protégée le cas échéant, avec une amine de formule XV telle que définie ci-dessus,
et ensuite le cas échéant :
i) la transformation d'un composé de formule (I) en un autre composé de formule (I) ;
ii) l'élimination de tous les groupements protecteurs ;
iii) la formation d'un sel.

16. Composé choisi parmi les formules II, XIV, XVI, XIX et XX telles que définies dans la revendication 15, dans lesquelles A est un cycle hétéroaryle à 5 ou 6 chaînons choisi parmi furyle, pyrrolyle, thiényle, oxazolyle, isoxazolyle, imidazolyle, pyrazolyle, thiazolyle, isothiazolyle, oxadiazolyle, thiadiazolyle, tirzolyle, tétrazolyle, pyridyle, pyridazinyle, pyrimidinyle, pyrazinyle ou 1,3,5-triazinyle
ou un composé de formule VIc: 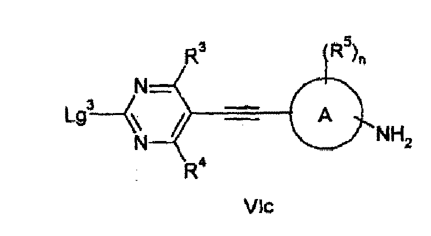 ou un sel de celui-ci, dans laquelle A est un cycle hétéroaryle à 5 ou 6 chaînons choisi parmi furyle, pyrrolyle, thiényle, oxazolyle, isoxazolyle, imidazolyle, pyrazolyle, thiazolyle, isothiazolyle, oxadiazolyle, thiadiazolyle, triazolyle, tétrazolyle, pyridyle, pyridazinyle, pyrimidinyle, pyrazinyle ou 1,3,5-triazinyle et R¹, R², R³, R⁴, R⁵, R⁶, R⁷, R⁸, R⁹, R¹⁰, R¹¹, L, m le cycle B et n sont tels que définis dans la revendication 1, et Lg² est hydroxy ou halogéno et Lg³ est halogéno, méthylsulfonyle, méthylthio ou aryloxy.
